# EUROPEAN PATENT SPECIFICATION

(11) **EP 4 568 960 B1**
(45) Date of publication and mention of the grant of the patent: **29.04.2026**
(21) Application number: 23755359.9
(22) Date of filing: 10.08.2023
(51) Int. Cl.: C07D 417/04, C07D 417/14, A61P 35/00, A61K 31/554

(54) **BICYCLIC TETRAHYDROAZEPINE DERIVATIVES**
BICYCLISCHE TETRAHYDROAZEPINDERIVATE
DÉRIVÉS BICYCLIQUES DE TÉTRAHYDROAZÉPINE

(30) Priority: 11.08.2022 EP 22189830
(43) Date of publication of application: 18.06.2025
(73) Proprietor: F. Hoffmann-La Roche AG, 4070 Basel (CH)
(72) Inventor: BRANDSTAETTER, Marco, 4070 Basel (CH); KUEHNE, Holger, 4070 Basel (CH); LUEBBERS, Thomas, 4070 Basel (CH); MARTIN, Laetitia Janine, 4070 Basel (CH)
(74) Representative: Belkacemi, Doria
(86) International application number: PCT/EP2023/072129
(87) International publication number: WO 2024/033458

(56) References cited:
- WO-A1-2014/023708
- WO-A1-2016/139181

## Description

### Field of the invention

The present invention relates to bicyclic tetrahydroazepine compounds which inhibit Diacylglycerol kinases (DGK) α and ζ and are useful as T-Cell activators, their manufacture and pharmaceutical compositions comprising said compounds.

The present compounds may be useful as immunotherapeutic agents for the treatment of human diseases. More specifically, the present compounds can be used alone or in combination with other immunotherapeutic agents in order to boost anti-cancer immunity.

### Background of the invention

Cancer immunity is a multistep process that is regulated by a series of negative immune checkpoint and positive co-stimulatory receptors and related intracellular signaling cascades that when effectively triggered can achieve antitumor response (Mellman, I., et al. (2011) Cancer Immunotherapy Comes of Age, Nature 480(7378), 480-489). Indeed, PD1/PDL1 targeting and other immune-checkpoint inhibitors have revolutionized cancer immunotherapy, but still more than 70% of patients do not benefit from immune-checkpoint inhibition. Similarly, for T-cell bispecific antibodies, even in the most promising indication (Non-Hodgkin lymphoma), these T-cell binders (TCBs) achieve complete remissions in less than 50% of patients. T-cell exhaustion seems to play an important role in many of these examples of primary or secondary resistance to cancer immunotherapy. A possible reason for this lack of efficacy is that T-cell activation occurs via targeting and crosslinking of CD3 (signal 1), but co-stimulation e.g. via CD28 or 4-1BB (signal 2) is missing. This hypothesis was verified clinically for CAR T-cell therapy where it was shown that only after the incorporation of costimulatory domains, clinically relevant efficacy was observed.

Diacylglycerol kinases (DGKs) are lipid kinases that catalyze the conversion of Diacylglycerol (DAG) to phosphatidic acid (PA), thus limiting DAG-regulated and promoting PA-dependent functions (Merida, I., Avila-Flores, A., and Merino, E. 2008: Diacylglycerol kinases: at the hub of cell signalling. Biochem. J. 409 (1), 1-18). The DGK family consist of ten isoforms that can be grouped into five subtypes based on the presence of different regulatory domains within their structure. Beyond that, the lack of structural data as of now still hinders a more thorough understanding of the DGKs mode of action. Also information on certain prokaryotic DGK and other lipid kinases like sphingosine kinase and phosphatidylinositol-3-kinase (PI3K) has provided only limited insight into the DGK catalytic mechanisms which seems to be distinct from classical kinases (Arranz-Nicolás, J. and Mérida, I., 2020. Biological regulation of diacylglycerol kinases in normal and neoplastic tissues: New opportunities for cancer immunotherapy, Advances in Biological Regulation, Volume 75; Ma, Q., Gabelli, S.B., Raben, D.M., 2019: Diacylglycerol kinases: relationship to other lipid kinases. Adv Biol Regul 71, 104-110).

Although several isoforms within the DGK family have been described to play a role in cancer, the α and ζ isoforms are the ones that have been most deeply studied in this regard. As PA producers, both enzymes have been implicated in various processes promoting tumor growth and metastasis. On the other hand, as DAG consumers, DGKα and ζ have been extensively characterized as negative regulators of T cell responses (Riese, M.J., Moon, E.K., Johnson, B.D., Albelda, S.M., 2016. Diacylglycerol kinases (DGKs): novel targets for improving T cell activity in cancer. Front Cell Dev Biol 4,108; Noessner, E., 2017. DGK-alpha: a checkpoint in cancer-mediated immuno-inhibition and target for immunotherapy. Front Cell Dev Biol 5, 16; Sakane, F., Mizuno, S., Komenoi, S., 2016. Diacylglycerol kinases as emerging potential drug targets for a variety of diseases: an update. Front Cell Dev Biol 4, 82; Arranz-Nicolás, J. and Mérida, I., 2020. Biological regulation of diacylglycerol kinases in normal and neoplastic tissues: New opportunities for cancer immunotherapy, Advances in Biological Regulation, Volume 75).

These two isozymes DGKα and DGKζ are active downstream of CD28 and other costimulatory receptors as well as the T cell receptor (TCR), and their function is to limit the amount of DAG generated - and ultimately T-cell activation (Merida, I., Andrada, E., Gharbi, S.I., Avila-Flores, A., 2015. Redundant and specialized roles for diacylglycerol kinases alpha and zeta in the control of T cell functions. Sci. Signal. 8 (374); Shulga, Y.V., Topham, M.K., Epand, R.M., 2011. Regulation and functions of diacylglycerol kinases. Chem. Rev. 111 (10), 6186-6208.) A summary of representative DGK-regulated signaling pathways is shown in Figure 1 (Sim, J.A.; Kim, J.; Yang, D. Beyond Lipid Signaling: Pleiotropic Effects of Diacylglycerol Kinases in Cellular Signaling. Int. J. Mol. Sci. 2020, 21, 6861): Activated PLC1 cleaves PIP2 in the plasma membrane to generate two secondary messengers, DAG and IP3. DAG activates PKC, Ras/MEK/ERK/AP-1 and NF-kB, while IP3 is involved in the activation of intracellular Ca2+ flux. The upregulated Ca2+ signaling in turn activates the transcription factor NFAT. In short, DAG production and levels determine the duration and intensity of the Ras/MEK/ERK and PKC-dependent signaling pathways, and they are central to T-cell activation. Thus, DGKs serve as intracellular checkpoints and inhibition of DGKs is expected to enhance T cell signaling pathways and T cell activation.

Experimental evidence suggests that enhanced DGK function and / or expression in tumor infiltrating T-cells (TILs) limits tumor destruction. Experiments with CAR T cells directed against human mesothelioma engrafted into nude mice demonstrated that tumorinfiltrating CAR T cells express elevated concentrations of surface inhibitory receptors, as well as the inhibitory enzymes SHIP-1, DGKα and DGKζ (Moon et al., 2014). Further, high DGKα expression was also observed in TIL isolated from human renal tumors (Prinz et al., 2012). In mouse mesoCAR T cells, dual deletion of DGKα and DGKζ results in enhanced cytokine expression and cytotoxicity against tumor cells (Riese et al., 2013). Similar results have been reported for human CAR T cells in which both DGKα and DGKζ expression were silenced using CRISPR/Cas9 (Jung et al., 2018). All these studies support a rationale for targeting DGKα/ζ in the development of anti-cancer therapies (Arranz-Nicolás, J. and Mérida, I., 2020. Biological regulation of diacylglycerol kinases in normal and neoplastic tissues: New opportunities for cancer immunotherapy, Advances in Biological Regulation, Volume 75; Riese, M.J., Moon, E.K., Johnson, B.D., Albelda, S.M., 2016. Diacylglycerol kinases (DGKs): novel targets for improving T cell activity in cancer. Front Cell Dev Biol 4, 108.). Knock out mouse models provide further evidence: Mice lacking either DGKα or DGKζ showed a hyperresponsive T cell phenotype and improved anti-tumor immune activity (Riese, M.J., Grewal, J., Das, J., Zou, T., Patil, V., Chakraborty, A.K., Koretzky, G.A., 2011. Decreased diacylglycerol metabolism enhances ERK activation and augments CD8+ T cell functional responses. J. Biol. Chem. 286 (7), 5254-5265; Zha, Y., Marks, R., Ho, A.W., Peterson, A.C., Janardhan, S., Brown, I., Praveen, K., Stang, S., Stone, J.C., Gajewski, T.F., 2006. T cell anergy is reversed by active Ras and is regulated by diacylglycerol kinase-alpha. Nat. Immunol. 7 (11), 1166-1173; Olenchock, B.A., Guo, R., Carpenter, J.H., Jordan, M., Topham, M.K., Koretzky, G.A., Zhong, X.P., 2006a. Disruption of diacylglycerol metabolism impairs the induction of T cell anergy. Nat. Immunol. 7 (11), 1174-1181.)

Taken together, there is substantial evidence that DGKα and DGKζ are high value targets for cancer immunotherapy. At the same time, there is a lack of compounds with the ability to potently inhibit both DGKα and DGKζ with good selectivity over other diacylglycerol kinases, protein kinases, and/or other lipid kinases.

This invention describes such dual DGK α/ζ inhibitors with excellent selectivity over other protein kinases, across safety / off-target panels and vs. other lipid kinases. These compounds potently activate suboptimally stimulated T-cells and thereby act as intracellular enhancers of co-stimulatory signaling cascades. These DGK α/ζ inhibitors have the potential to increase proliferation, cytotoxicity and the life span of targeted T-cells which may result in improved anticancer activity of CPIs, CD3 engaging T-cell bispecifics and CAR T-cells. Further, by engaging a signaling node central to both TCR and co-stimulatory receptors, it is plausible that these molecules enhance both signals 1 and 2 and thus single agent activity can be achieved, e.g. in inflamed tumors.

There is an ongoing need for new compounds capable of activating and proliferating T-cells, thus enabling the treatment, prevention and/or delay of progression of cancer.

It is, therefore, an object of this invention to provide compounds useful as DGKα/ζ inhibitors for the treatment or prevention or amelioration of such diseases with improved therapeutic properties, in particular improved pharmacokinetic properties.

### Summary of the invention

A first object of the present invention is a compound of formula (I) 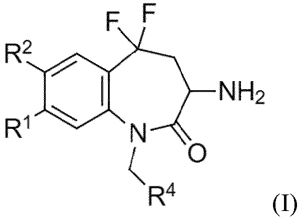 or a pharmaceutically acceptable salt thereof, wherein:
R¹ is 5 or 6-membered heteroaryl, wherein R¹ is optionally substituted with one or more R¹⁰ which can be the same or different;
R² is selected from hydrogen and halogen;
R⁴ is selected from phenyl and pyridinyl, wherein R⁴ is optionally substituted with one or more R¹¹ which can be the same or different;
R¹⁰ is selected from:
   i) C₁₋₆-alkyl, optionally substituted with one or more halogen, amino, C₁₋₆-alkoxy, - S(O)₂(C₁₋₆-alkyl), cyano;
   ii) C₃₋₁₀-cycloalkyl, optionally substituted with one or more halogen, cyano, amino;
   iii) 3-10 membered heterocyclyl, optionally substituted with one or more halogen, C₁₋₁₀-alkyl, amino, halo-C₁₋₆-alkyl, hydroxy, cyano, -C(O)O-(R^{10q}), C₃₋₁₀-cycloalkyl, wherein C₁₋₁₀-alkyl is optionally substituted with one or more hydroxy, C₁₋₆-alkoxy;
   iv) -N(R^{10e}R^{10f});
   v) heteroaryl, optionally substituted with one or more C₁₋₁₀-alkyl, halogen;
R^{10e} and R^{10f} are each independently selected from:
   i) hydrogen;
   ii) C₁₋₆-alkyl, optionally substituted with one or more, cyano, halogen, hydroxy;
   iii) C₃₋₁₀-cycloalkyl, optionally substituted with one or more halogen, C₁₋₁₀-alkyl;
R^{10q} is C₁₋₅-alkyl, wherein C₁₋₅-alkyl is optionally substituted with one or more hydroxy;
R¹¹ is selected from:
   i) halogen;
   ii) C₁₋₆-alkoxy, optionally substituted with one or more C₁₋₆-alkyl, C₅₋₆-aryl, C₃₋₁₀-cycloalkyl, halo-C₁₋₆-alkyl, C₃₋₁₀-heterocyclyl, wherein 3-10 membered heterocyclyl is optionally substituted with C₁₋₆-alkyl;
   iii) 5-6 membered heteroaryl, optionally substituted with one or more halogen, halo-C₁₋₆-alkyl, C₁₋₆-alkoxy, C₃₋₁₀-cycloalkyl, C₁₋₆-alkyl, halo-C₁₋₆-alkoxy;
   iv) phenyl, optionally substituted with one or more C₁₋₆-alkoxy, halo-C₁₋₆-alkyl;
   v) -O(R^{11a});
R^{11a} is selected from C₁₋₆-alkyl, C₃₋₆-cycloalkyl, phenyl and halo-C₁₋₆-alkyl.

A second object of the present invention is a process for the preparation of a compound of formula (I) as described herein, or a pharmaceutically acceptable salt thereof, comprising reacting a compound of formula (XVIII) 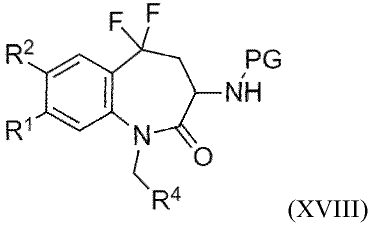 wherein R¹, R² and R⁴ are as defined herein and PG is an amino protecting group, with a suitable deprotection agent to form said compound of formula (I).

A third object of the present invention is a pharmaceutical composition comprising a compound of formula (I) as described herein, or a pharmaceutically acceptable salt thereof, and a pharmaceutically acceptable excipient.

A forth object of the present invention is a compound of formula (I) as described herein, or a pharmaceutically acceptable salt thereof, for use in the treatment, prevention and/or delay of progression of cancer.

Unless otherwise defined, all technical and scientific terms used herein have the same meaning as commonly understood by one of ordinary skill in the art to which this invention belongs.

The nomenclature used in this application is based on IUPAC systematic nomenclature, unless indicated otherwise.

### Detailed description of the invention

### Definitions

"Alkoxy" refers to an alkyl group, as previously defined, attached to the parent molecular moiety via an oxygen atom. Unless otherwise specified, the alkoxy group contains 1 to 12 carbon atoms ("C₁₋₁₂-alkoxy"), preferably 1 to 10 carbon atoms ("C₁₋₁₀-alkoxy"), more preferably 1 to 6 carbon atoms ("C₁₋₆-alkoxy"). In some preferred embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy.

"Alkoxyalkyl" refers toan alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably one hydrogen atom of the alkyl group have been replaced by an alkoxy group. Particularly preferred, yet non-limiting examples of alkoxyalkyl is methoxymethyl and 2-methoxyethyl.

"Alkyl" refers to a saturated linear (i.e. unbranched) or branched univalent hydrocarbon chain or combination thereof, having the number of carbon atoms designated (*i.e.,* C₁-₁₀ means one to ten carbon atoms). Particular alkyl groups are those having 1 to 20 carbon atoms (a "C₁-₂₀ alkyl"), having 1 to 12 carbon atoms (a "C₁-₁₂ alkyl"), having 1 to 10 carbon atoms (a "C₁-₁₀ alkyl"), having 1 to 8 carbon atoms (a "C₁-₈ alkyl"), having 1 to 6 carbon atoms (a "C₁-₆ alkyl"), having 2 to 6 carbon atoms (a "C₂-₆ alkyl"), or having 1 to 4 carbon atoms (a "C₁₋₄ alkyl"). Examples of alkyl group include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like.

"Alkynyl" refers to an unsaturated linear (i.e. unbranched) or branched univalent hydrocarbon chain or combination thereof, having at least one site of acetylenic unsaturation (*i.e.,* having at least one moiety of the formula C≡C) having the number of carbon atoms designated (*i.e.* C₂₋₁₀ means two to ten carbon atoms). Particular alkynyl groups are those having 2 to 20 carbon atoms (a "C₂₋₂₀ alkynyl"), having 2 to 8 carbon atoms (a "C₂₋₈ alkynyl"), having 2 to 6 carbon atoms (a "C₂₋₆ alkynyl"), having 2 to 4 carbon atoms (a "C₂₋₄ alkynyl"). Examples of alkynyl group include, but are not limited to, groups such as ethynyl (or acetylenyl), prop-1-ynyl, prop-2-ynyl (or propargyl), but-1-ynyl, but-2-ynyl, but-3-ynyl, homologs and isomers thereof, and the like.

"Amino", alone or in combination with other groups, refers to NH₂.

"Aminoalkyl" refers to an alkyl group wherein one or more of the hydrogen atoms of the alkyl group have been replaced by an amino moiety.

"Aromatic" denotes the conventional idea of aromaticity as defined in the literature, in particular in IUPAC - Compendium of Chemical Terminology, 2nd Edition, A. D. McNaught & A. Wilkinson (Eds). Blackwell Scientific Publications, Oxford (1997).

"Aryl" refers to a cyclic aromatic hydrocarbon moiety having a mono-, bi- or tricyclic aromatic ring of 5 to 14 carbon ring atoms ("C₅₋₁₄-aryl"). Bicyclic aryl ring systems include fused bicyclics having two fused five-membered aryl rings (denoted as 5-5), having a five-membered aryl ring and a fused six-membered aryl ring (denoted as 5-6 and as 6-5), and having two fused six-membered aryl rings (denoted as 6-6). The aryl group can be optionally substituted as defined herein. Examples of aryl moieties include, but are not limited to, phenyl, naphthyl, phenanthryl, fluorenyl, indenyl, pentalenyl, azulenyl, and the like. The term "aryl" also includes partially hydrogenated derivatives of the cyclic aromatic hydrocarbon moiety provided that at least one ring of the cyclic aromatic hydrocarbon moiety is aromatic, each being optionally substituted.

"Cancer" refers to a disease characterized by the presence of a neoplasm or tumor resulting from abnormal uncontrolled growth of cells (such cells being "cancer cells"). As used herein, the term cancer explicitly includes, but is not limited to, hepatocellular cancer, malignancies and hyperproliferative disorders of the colon (colon cancer), lung cancer, breast cancer, prostate cancer, melanoma, and ovarian cancer.

"Cyano", alone or in combination with other groups, refers to CN (i.e. nitrile).

"Cyanoalkyl" refers to an alkyl group wherein one or more of the hydrogen atoms of the alkyl group have been replaced by a cyano moiety.

"Cycloalkyl" refers to a saturated or partially unsaturated carbocyclic moiety having mono-, bi- (including bridged bicyclic and cycloalkyl spiro moieties) or tricyclic rings and 3 to 10 carbon atoms i.e., (C₃-C₁₀)cycloalkyl) in the ring. The cycloalkyl moiety can optionally be substituted with one or more substituents. In particular aspects cycloalkyl contains from 3 to 8 carbon atoms (i.e., (C₃-C₈)cycloalkyl). In other particular aspects cycloalkyl contains from 3 to 6 carbon atoms (i.e., (C₃-C₆)cycloalkyl). Examples of cycloalkyl moieties include, but are not limited to, cyclopropyl, cyclobutyl, cyclopentyl, cyclohexyl, cycloheptyl, and partially unsaturated (cycloalkenyl) derivatives thereof (e.g. cyclopentenyl, cyclohexenyl, and cycloheptenyl), bicyclo[3.1.0]hexanyl, bicyclo[3.1.0]hexenyl, bicyclo[3.1.1]heptanyl, bicyclo[3.1.1]heptenyl and bicyclo[1.1.1]pentane. The cycloalkyl moiety can be attached in a "spiro-cycloalkyl" or "cycloalkyl spiro" fashion such as "spirocyclopropyl"

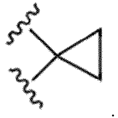

"ECₓ" refers to the effective concentration e.g. in medium or in the plasma of a particular compound required for obtaining x% of the maximum of a particular effect in vitro or in vivo. Examples of "ECₓ" are EC₂₀, EC₅₀ and EC₁₀₀ denoting the concentration of a particular compound in medium or plasma required for obtaining 20%, 50% and 100%, respectively, of the maximum of a particular effect in vitro or in vivo. "Halo" or "Halogen" refers to fluoro, chloro, bromo and/or iodo. Where a residue is substituted with more than one halogen, it can be referred to by using a prefix corresponding to the number of halogen moieties attached, e.g., dihaloaryl, dihaloalkyl, trihaloaryl etc. refer to aryl and alkyl substituted with two ("di") or three ("tri") Halo groups, which can be but are not necessarily the same Halo; thus 4-chloro-3-fluorophenyl is within the scope of dihaloaryl. An alkyl group in which one or more hydrogen is replaced with a Halo group is referred to as a "haloalkyl", for example, "C₁₋₆ haloalkyl." A preferred haloalkyl group is trifluoroalkyl (-CF₃).

"Haloalkoxy" refers to an alkoxy group in which at least one halogen takes the place of each H in the hydrocarbon making up the alkyl moiety of the alkoxy group. An example of a haloalkoxy group is difluoromethoxy (-OCHF₂), trifluoromethoxy (-OCF₃).

"Haloaryl" refers to refers to an aryl wherein at least one hydrogen has been substituted with an halogen.

"Heteroaryl" refers to an aromatic heterocyclic mono-, bi- or tricyclic ring system of 5 to 14 ring atoms, preferably from 5 to 10 ring atoms, more preferably from 5 to 6 ring atoms, comprising 1, 2, 3 or 4 heteroatoms selected from N, O and S, the remaining ring atoms being carbon. In some aspects, monocyclic heteroaryl rings may be 5-6 membered. Bicyclic heteroaryl ring systems include fused bicyclics having two fused five-membered heteroaryl rings (denoted as 5-5), having a five-membered heteroaryl ring and a fused six-membered heteroaryl ring (denoted as 5-6 and 6-5), and having two fused six-membered heteroaryl rings (denoted as 6-6). The heteroaryl group can be optionally substituted as defined herein. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, isoxazolyl, benzofuranyl, isothiazolyl, benzothienyl, benzothiophenyl, indolyl, aza-indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, pyrrolopyridinyl, furopyridinyl, thienopyridinyl, pyrrolopyridazinyl, pyrrolopyrimidinyl, pyrrolopyrazinyl, thienopyridazinyl, thienopyrimidinyl, thienopyrazinyl, furopyridazinyl, furopyrimidinyl, and furopyrazinyl. Most preferably, "5-membered heteroaryl" refers to the following groups:

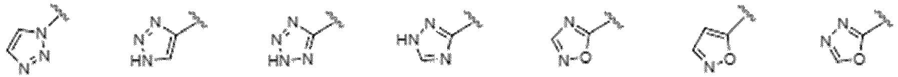

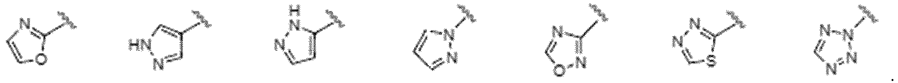

"Heterocycle" or "heterocyclyl" refers to a 3, 4, 5, 6, 7, 8, 9, 10-membered monocyclic, 7, 8, 9 and 10-membered bicyclic (including bridged bicyclic and cycloalkyl spiro moieties) or 10, 11, 12, 13, 14 and 15-membered bicyclic heterocyclic moiety that is saturated or partially unsaturated, and has one or more (e.g., 1, 2, 3 or 4 heteroatoms selected from oxygen, nitrogen and sulfur in the ring with the remaining ring atoms being carbon. In some aspects, the heterocycle is a heterocycloalkyl. In particular aspects heterocycle or heterocyclyl refers to a 4, 5, 6 or 7-membered heterocycle. When used in reference to a ring atom of a heterocycle, a nitrogen or sulfur may also be in an oxidized form, and a nitrogen may be substituted with one or more (C₁-C₆)alkyl or groups. The heterocycle can be attached to its pendant group at any heteroatom or carbon atom that results in a stable structure. Any of the heterocycle ring atoms can be optionally substituted with one or more substituents described herein. Examples of such saturated or partially unsaturated heterocycles include, without limitation, tetrahydrofuranyl, tetrahydrothienyl, pyrrolidinyl, pyrrolidonyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, pyrrolidine 1-oxide, N-hydroxypiperidine, 1-methylpyrrolidine N-oxide, diazirinyl and quinuclidinyl. The term heterocycle also includes groups in which a heterocycle is fused to one or more aryl, heteroaryl, or cycloalkyl rings, such as indolinyl, 3H-indolyl, chromanyl, azabicyclo[2.2.1]heptanyl, azabicyclo[3.1.0]hexanyl, azabicyclo[3.1.1]heptanyl, octahydroindolyl, or tetrahydroquinolinyl.

"Hydroxy", alone or in combination with other groups, refers to OH.

"Hydroxyalkyl" refers to an alkyl group wherein one or more of the hydrogen atoms of the alkyl group have been replaced by a hydroxy moiety. Examples include alcohols and diols.

"Moiety" and "substituent" refer to an atom or group of chemically bonded atoms that is attached to another atom or molecule by one or more chemical bonds thereby forming part of a molecule.

When indicating the number of substituents, the term "one or more" refers to the range from one substituent to the highest possible number of substitution, i.e. replacement of one hydrogen up to replacement of all hydrogens by substituents, in particular wherein "one or more" refers to one, two or three, most particularly "one or more" refers to one or two.

"Optional" or "optionally" means that the subsequently described event or circumstance may but need not occur, and that the description includes instances where the event or circumstance occurs and instances in which it does not. For example, "aryl group optionally substituted with an alkyl group" means that the alkyl may but need not be present, and the description includes situations where the aryl group is substituted with an alkyl group and situations where the aryl group is not substituted with the alkyl group.

"Optionally substituted" refers to means unsubstituted or substituted. Generally these substituents can be the same or different.

"Oxo", alone or in combination with other groups, refers to =O.

"Pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein.

Particularly preferred pharmaceutically acceptable salts of compounds of formula (I) are the salts of hydrochloric acid, hydrobromic acid, sulfuric acid, phosphoric acid and methanesulfonic acid.

The term "protecting group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxyprotective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

"Prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

"Substituted" refers to the replacement of at least one of hydrogen atoms of a compound or moiety with another substituent or moiety. Examples of such substituents include, without limitation, halogen, -OH, -CN, oxo, alkoxy, alkyl, alkylene, aryl, heteroaryl, haloalkyl, haloalkoxy, cycloalkyl and heterocycle. For example, the term "haloalkyl" refers to the fact that one or more hydrogen atoms of an alkyl (as defined below) is replaced by one or more halogen atoms (e.g., trifluoromethyl, difluoromethyl, fluoromethyl, chloromethyl, etc.). In one aspect, substituted as used herein can refer to replacement of at least one hydrogen atom of a compound or moiety described herein with halogen or alkyl.

"Therapeutically effective amount" refers to an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

"Therapeutically inert carrier" refers to any ingredient having no therapeutic activity and being non-toxic such as disintegrators, binders, fillers, solvents, buffers, tonicity agents, stabilizers, antioxidants, surfactants or lubricants used in formulating pharmaceutical products.

In particular, the chemical groups whose definitions are given above are those specifically exemplified in the examples.

The following abbreviations are used in the present text:
BOP = benzotriazol-1-yloxytris(dimethylamino)phosphonium hexafluorophosphate, Brine = saturated aqueous NaCl solution, CAS = chemical abstracts registration number, CDI = 1,1'-Carbonyldiimidazole, DAD = diode-array detection, DBU = 1,8-diazabicyclo[5,4,0]undec-7-ene, DCM = dichloromethane, DDQ = 2,3-dichloro-5,6-dicyano-1,4-benzoquinone, DMF = N,N-dimethylformamide, DIPEA = N,N-diisopropylethylamine, EDC = 1-ethyl-3-(3-dimethylaminopropyl)carbodiimide, ESI = electrospray ionization, EtOAc = ethyl acetate, EtOH = ethanol, h = hour(s), HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluoro-phosphate, HFIP = hexafluoroisopropanol, HOBt = hydroxybenzotriazole, HPLC = high performance liquid chromatography, IPA = isopropyl alcohol, m-CPBA = meta-chloroperoxybenzoic acid, MeCN = acetonitrile, MeI = methyliodide, MeOH = methanol, min = minute(s), MS = mass spectrum, NBS = N-bromosuccinimide, PDA = photodiode-array detection, PE = petroleum ether, PyBroP = bromo-tris-pyrrolidino-phosphonium hexafluorophosphate, RT = room temperature, TBAF = tetrabutylammonium fluoride, TBAOH = tetrabutylammonium hydroxide, TBDMS = tert-butyldimethylsilyl, TEA = triethylamine, TFA = trifluoroacetic acid, THF = tetrahydrofuran, TMSOTF = trifluoromethanesulfonic acid trimethylsilylester, TLC = thin layer chromatography, T3P = 2,4,6-tripropyl-1,3,5,2,4,6-trioxatriphosphinane 2,4,6-trioxide.

In the description herein, if there is a discrepancy between a depicted structure and a name given to that structure, then the depicted structure controls. Additionally, if the stereochemistry of a structure or a portion of a structure is not indicated with, for example, bold wedged, or dashed lines, the structure or portion of the structure is to be interpreted as encompassing all stereoisomers of it. In some cases, however, where more than one chiral center exists, the structures and names may be represented as single enantiomers to help describe the relative stereochemistry.

Unless otherwise indicated, the terms "a compound of the formula" or "a compound of formula" or "compounds of the formula" or "compounds of formula" refer to any compound selected from the genus of compounds as defined by the formula (including any pharmaceutically acceptable salt of any such compound if not otherwise noted).

Certain compounds may exhibit tautomerism. Tautomeric compounds can exist as two or more interconvertable species. Prototropic tautomers result from the migration of a covalently bonded hydrogen atom between two atoms. Tautomers generally exist in equilibrium and attempts to isolate an individual tautomers usually produce a mixture whose chemical and physical properties are consistent with a mixture of compounds. The position of the equilibrium is dependent on chemical features within the molecule. For example, in many aliphatic aldehydes and ketones, such as acetaldehyde, the keto form predominates while; in phenols, the enol form predominates. Common prototropic tautomers include keto/enol (-C(=O)-CH- ↔ -C(-OH)=CH-), amide/imidic acid (-C(=O)-NH- ↔ -C(-OH)=N-) and amidine (-C(=NR)-NH- ↔ -C(-NHR)=N-) tautomers. The latter two are particularly common in heteroaryl and heterocyclic rings and the present invention encompasses all tautomeric forms of the compounds.

Furthermore, the invention includes all optical isomers, i.e. diastereoisomers, diastereomeric mixtures, racemic mixtures, all their corresponding enantiomers and/or tautomers as well as their solvates of the compounds of formula (I).

The compounds of formula (I) may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90% of the desired isomer by weight, particularly > 95% of the desired isomer by weight, or more particularly > 99% of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Compounds of the invention

In one embodiment, there is provided a compound of formula (I) as described herein, 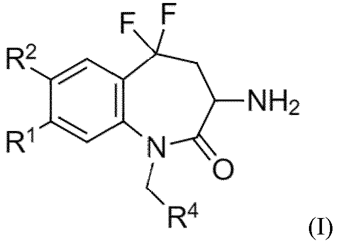 or a pharmaceutically acceptable salt thereof, wherein:
R¹ is 5 or 6-membered heteroaryl, wherein R¹ is optionally substituted with one or more R¹⁰ which can be the same or different;
R² is selected from hydrogen and halogen;
R⁴ is selected from phenyl and pyridinyl, wherein R⁴ is optionally substituted with one or more R¹¹ which can be the same or different;
R¹⁰ is selected from:
   i) C₁₋₆-alkyl, optionally substituted with one or more halogen, amino, C₁₋₆-alkoxy, - S(O)₂(C₁₋₆-alkyl), cyano;
   ii) C₃₋₁₀-cycloalkyl, optionally substituted with one or more halogen, cyano, amino;
   iii) 3-10 membered heterocyclyl, optionally substituted with one or more halogen, C₁₋₁₀-alkyl, amino, halo-C₁₋₆-alkyl, hydroxy, cyano, -C(O)O-(R^{10q}), C₃₋₁₀-cycloalkyl, wherein C₁₋₁₀-alkyl is optionally substituted with one or more hydroxy, C₁₋₆-alkoxy;
   iv) -N(R^{10e}R^{10f});
   v) heteroaryl, optionally substituted with one or more C₁₋₁₀-alkyl, halogen;
R^{10e} and R^{10f} are each independently selected from:
   i) hydrogen;
   ii) C₁₋₆-alkyl, optionally substituted with one or more, cyano, halogen, hydroxy;
   iii) C₃₋₁₀-cycloalkyl, optionally substituted with one or more halogen, C₁₋₁₀-alkyl;
R^{10q} is C₁₋₅-alkyl, wherein C₁₋₅-alkyl is optionally substituted with one or more hydroxy;
R¹¹ is selected from:
   i) halogen;
   ii) C₁₋₆-alkoxy, optionally substituted with one or more C₁₋₆-alkyl, C₅₋₆-aryl, C₃₋₁₀-cycloalkyl, halo-C₁₋₆-alkyl, C₃₋₁₀-heterocyclyl, wherein 3-10 membered heterocyclyl is optionally substituted with C₁₋₆-alkyl;
   iii) 5-6 membered heteroaryl, optionally substituted with one or more halogen, halo-C₁₋₆-alkyl, C₁₋₆-alkoxy, C₃₋₁₀-cycloalkyl, C₁₋₆-alkyl, halo-C₁₋₆-alkoxy;
   iv) phenyl, optionally substituted with one or more C₁₋₆-alkoxy, halo-C₁₋₆-alkyl;
   v) -O(R^{11a});
R^{11a} is selected from C₁₋₆-alkyl, C₃₋₆-cycloalkyl, phenyl and halo-C₁₋₆-alkyl.

In another embodiment, there is provided a compound of formula (I) as described herein, or a pharmaceutically acceptable salt thereof, wherein wherein R¹ is 5-membered heteroaryl, wherein R¹ is optionally substituted with one or more R¹⁰ which can be the same or different.

In a preferred embodiment, there is provided a compound of formula (I) as described herein, or a pharmaceutically acceptable salt thereof, wherein R¹ is oxadiazole, optionally substituted with one or more R¹⁰ which can be the same or different.

In a more preferred embodiment, there is provided a compound of formula (I) as described herein, or a pharmaceutically acceptable salt thereof, wherein R¹ is oxadiazole, substituted with one R¹⁰.

In a even more preferred embodiment, there is provided a compound of formula (I) as described herein, or a pharmaceutically acceptable salt thereof, wherein R¹ is 1,3,4-oxadiazole, substituted with one R¹⁰ .

In another embodiment, there is provided a compound of formula (I) as described herein, or a pharmaceutically acceptable salt thereof, wherein R² is fluorine.

In another embodiment, there is provided a compound of formula (I) as described herein, or a pharmaceutically acceptable salt thereof, wherein R⁴ is phenyl.

In another embodiment, there is provided a compound of formula (I) as described herein, or a pharmaceutically acceptable salt thereof, wherein R¹⁰ is selected from methyl-methylsulfonyl-ethyl, pyrrolidin, 4-oxa-7-azaspiro[2.5]octan-7-yl, methyl-propanenitrile, 1,2,2,2-tetrafluoro-methoxy-ethyl, tertbutyl, 1-ethyl-5,5-difluoro-3-piperidyl, 4,4-difluoro-1-piperidyl and azabicyclo[3.1.1]heptane-3-carboxylate.

In another embodiment, there is provided a compound of formula (I) as described herein, or a pharmaceutically acceptable salt thereof, wherein R¹⁰ is selected from methyl-methylsulfonyl-ethyl, pyrrolidin, oxa-azaspiro[2.5]octanyl, methyl-propanenitrile, tetrafluoro-methoxy-ethyl.

In another embodiment, there is provided a compound of formula (I) as described herein, or a pharmaceutically acceptable salt thereof, wherein R¹¹ is selected from 5-(trifluoromethyl)-2-pyridyl, phenoxy, trifluoromethoxy, methoxy-pyridyl, cyclopentoxy, 4-methoxyphenyl, (trifluoromethyl)-1,2,4-oxadiazol-3-yl, 5-(trifluoromethoxy)-2-pyridyl, 4-(trifluoromethyl)pyrazol-1-yl, chloro, 5-(difluoromethoxy)-2-pyridyl, 4-[4-(trifluoromethoxy)pyrazol-1-yl, 4-[5-(trifluoromethyl)tetrazol-2-yl.

In another embodiment, there is provided a compound of formula (I) as described herein, or a pharmaceutically acceptable salt thereof, wherein R¹¹ is selected from (trifluoromethyl)-pyridyl, phenoxy, trifluoromethoxy, (trifluoromethyl)-oxadiazolyl, methoxy-pyridyl.

In another embodiment, there is provided a compound of formula (I) as described herein, or a pharmaceutically acceptable salt thereof, wherein:
R¹ is 5-membered heteroaryl, wherein R¹ is optionally substituted with one or more R¹⁰ which can be the same or different;
R² is halogen;
R⁴ is phenyl or pyridinyl;
R¹⁰ is selected from:
   iv) C₁₋₆-alkyl, optionally substituted with one or more halogen, C₁₋₆-alkoxy, -S(O)₂(C₁₋₆-alkyl), cyano;
   v) 3-10 membered heterocyclyl, optionally substituted with one or more halogen, C₁₋₁₀-alkyl, -C(O)O-(R^{10q}), C₃₋₁₀-cycloalkyl, wherein C₁₋₁₀-alkyl is optionally substituted with one or more C₁₋₆-alkoxy;
   vi) heteroaryl, optionally substituted with one or more C₁₋₁₀-alkyl;
R¹¹ is selected from:
   i) halogen;
   ii) 5-6 membered heteroaryl, optionally substituted with one or more halogen, halo-C₁₋₆-alkyl, C₁₋₆-alkoxy, halo-C₁₋₆-alkoxy;
   iii) phenyl, optionally substituted with one or more C₁₋₆-alkoxy, halo-C₁₋₆-alkyl;
   iv) -O(R^{11a});
R^{11a} is selected from C₁₋₆-alkyl, C₃₋₆-cycloalkyl, phenyl and halo-C₁₋₆-alkyl.

In another embodiment, there is provided a compound of formula (I) as described herein, or a pharmaceutically acceptable salt thereof, wherein:
R¹ is 5-membered heteroaryl, wherein R¹ is optionally substituted with one or more R¹⁰ which can be the same or different;
R² is halogen;
R⁴ is phenyl;
R¹⁰ is selected from:
   i) C₁₋₆-alkyl, optionally substituted with one or more halogen, amino, C₁₋₆-alkoxy, - S(O)₂(C₁₋₆-alkyl), cyano;
   ii) C₃₋₁₀-cycloalkyl, optionally substituted with one or more halogen, cyano, amino;
   iii) 3-10 membered heterocyclyl, optionally substituted with one or more -C(O)O-(R1^{0ql});
R¹¹ is selected from
   i) halogen;
   ii) 5-6 membered heteroaryl, optionally substituted with one or more halogen, halo-C₁₋₆-alkyl, C₁₋₆-alkoxy;
   iii) phenyl, optionally substituted with one or more C₁₋₆-alkoxy, halo-C₁₋₆-alkyl;
   iv) -O(R^{11a});
R^{11a} is selected from C₁₋₆-alkyl, C₃₋₆-cycloalkyl, phenyl and halo-C₁₋₆-alkyl.

In another embodiment, there is provided a compound of formula (I) as described herein, or a pharmaceutically acceptable salt thereof, wherein:
R¹ is 1,3,4-oxadiazole, substituted with one R¹⁰ ;
R² is fluorine;
R⁴ is phenyl;
R¹⁰ is selected from methyl-methylsulfonyl-ethyl, pyrrolidin, 4-oxa-7-azaspiro[2.5]octan-7-yl, methyl-propanenitrile, 1,2,2,2-tetrafluoro-methoxy-ethyl, tertbutyl, 1-ethyl-5,5-difluoro-3-piperidyl, 4,4-difluoro-1-piperidyl and azabicyclo[3.1.1]heptane-3-carboxylate;
R¹¹ is selected from 5-(trifluoromethyl)-2-pyridyl, phenoxy, trifluoromethoxy, methoxy-pyridyl, cyclopentoxy, 4-methoxyphenyl, (trifluoromethyl)-1,2,4-oxadiazol-3-yl, 5-(trifluoromethoxy)-2-pyridyl, 4-(trifluoromethyl)pyrazol-1-yl, chloro, 5-(difluoromethoxy)-2-pyridyl, 4-[4-(trifluoromethoxy)pyrazol-1-yl and 4-[5-(trifluoromethyl)tetrazol-2-yl.

In another embodiment, there is provided a compound of formula (I) as described herein, or a pharmaceutically acceptable salt thereof, wherein:
R¹ is 5-membered heteroaryl, wherein R¹ is optionally substituted with one or more R¹⁰ which can be the same or different;
R² is fluorine;
R⁴ is phenyl;
R¹⁰ is selected from difluoro-(methoxyethyl)piperidyl, azabicyclo[3.1.1]heptane-carboxylate, amino-trifluoro-methyl-ethyl, methyl-methylsulfonyl-ethyl, pyrrolidin, oxaazaspiro[2.5]octanyl, methyl-propanenitrile, tert-butyl, tetrafluoro-methoxy-ethyl, morpholino;
R¹¹ is selected from methoxyphenyl, (trifluoromethyl)-pyridyl, cyclopentoxy, phenoxy, trifluoromethoxy, (trifluoromethyl)-oxadiazolyl, chloro, methoxy-pyridyl.

In another embodiment, there is provided a compound of formula (I) as described herein, or a pharmaceutically acceptable salt thereof, wherein:
R¹ is 1,3,4-oxadiazole, substituted with one R¹⁰ ;
R² is fluorine;
R⁴ is phenyl;
R¹⁰ is selected from methyl-methylsulfonyl-ethyl, pyrrolidin, oxa-azaspiro[2.5]octanyl, methyl-propanenitrile, tetrafluoro-methoxy-ethyl;
R¹¹ is selected from (trifluoromethyl)-pyridyl, phenoxy, trifluoromethoxy, (trifluoromethyl)-oxadiazolyl, methoxy-pyridyl.

In another embodiment, there is provided a compound of formula (I) as described herein, wherein the compound is selected from:
3-amino-8-(5-tert-butyl-1,3,4-oxadiazol-2-yl)-5,5,7-trifluoro-1-[[4-(trifluoromethoxy)phenyl]methyl]-3,4-dihydro-1-benzazepin-2-one;
2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
(3S)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one;
(3R)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one;
(3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3S)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3R)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3 S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3 S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
(3 S)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one;
(3R)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one;
2-methyl-2-[5-[(3 S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
(3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-8-(5-tert-butyl-3-pyridyl)-1-[(4-chlorophenyl)methyl]-5,5,7-trifluoro-3,4-dihydro-1-benzazepin-2-one;
(3R)-3-amino-8-(5-tert-butyl-3-pyridyl)-1-[(4-chlorophenyl)methyl]-5,5,7-trifluoro-3,4-dihydro-1-benzazepin-2-one;
2-[5-[(3S)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methyl-propanenitrile;
2-[5-[(3R)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methyl-propanenitrile;
2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
(3*S*)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one;
(3*S*)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one;
(3*R*)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one;
2-methyl-2-[5-[(3S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
(3S)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one;
2-[5-[(3S)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile;
2-[5-[(3S)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile;
(3S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one;
(3R)-3-amino-5,5,7-trifluoro-8-[ 5-(1-methyl-1-methylsulfonyl-ethyl)-1,3,4-oxadiazol-2-yl]-1-[[4-[4-(trifluoromethyl)pyrazol-1-yl phenyl Jmethyl]-3,4-dihydro-1-benzazepin-2-one;
2-[5-[(3 S)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile;
(3S)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one;
2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
(3S)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one;
(3R)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one;
methyl 1-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5, 5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-methyl-2-[5-[(3S)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile;
(3S)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one;
(3R)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one;
2-methyl-2-[5-[(3S)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3R)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
methyl 1-[5-[(3S)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate;
2-methyl-2-[5-[(3*S*)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile;
2-methyl-2-[5-[(3*R*)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*R*)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile;
2-methyl-2-[5-[(3*R*)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile;
2-methyl-2-[5-[(3*R*)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*R*)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile; and
2-methyl-2-[5-[(3*R*)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile;
or a pharmaceutically acceptable salt thereof.

In a preferred embodiment, there is provided a compound of formula (I) as described herein, wherein the compound is selected from:
2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
(3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
(3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
(3S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl phenyl Jmethyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl phenyl Jmethyl]-3,4-dihydro-1-benzazepin-2-one;
2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
methyl 1-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5, 5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile; and
2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
or a pharmaceutically acceptable salt thereof.

In a more preferred embodiment, there is provided a compound of formula (I) as described herein, wherein the compound is selected from:
2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
(3 S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3 S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
(3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
(3S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl phenyl Jmethyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl phenyl Jmethyl]-3,4-dihydro-1-benzazepin-2-one;
2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
methyl 1-[5-[(3 S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5, 5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile; and
2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile.

### Processes of manufacturing

Processes for the manufacture of compounds of formula (I), or pharmaceutically acceptable salts thereof, as described herein are also an object of the invention.

The present invention provides a process for the preparation of a compound of formula (I) as described herein, or a pharmaceutically acceptable salt thereof, comprising reacting a compound of formula (XVIII) 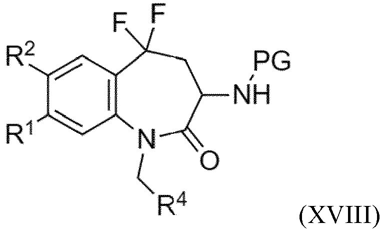 wherein R¹, R² and R⁴ are as defined herein and PG is an amino protecting group, with a suitable deprotection agent to form said compound of formula (I).

The preparation of compounds of formula (I) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (I) contain one or more functional groups which are not stable or are reactive under the reaction conditions of one or more reaction steps, appropriate protective groups (as described e.g., in "Protective Groups in Organic Chemistry" by T. W. Greene and P. G. M. Wutts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

Processes for the manufacture of compounds of formula (I) as described herein are also an object of the invention.

The preparation of compounds of formula (I) of the present invention may be carried out following sequential or convergent synthetic routes. Syntheses of the invention are shown in the following general schemes. The substituents and indices used in the following description of the processes have the significance given herein before unless indicated to the contrary.

In more detail, the compounds of formula (I) can be manufactured by the methods given below, by the methods given in the examples or by analogous methods. Appropriate reaction conditions for the individual reaction steps are known to a person skilled in the art. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The present compounds of formula (I) and their pharmaceutically acceptable salts may be prepared by a process described below (Scheme 1)

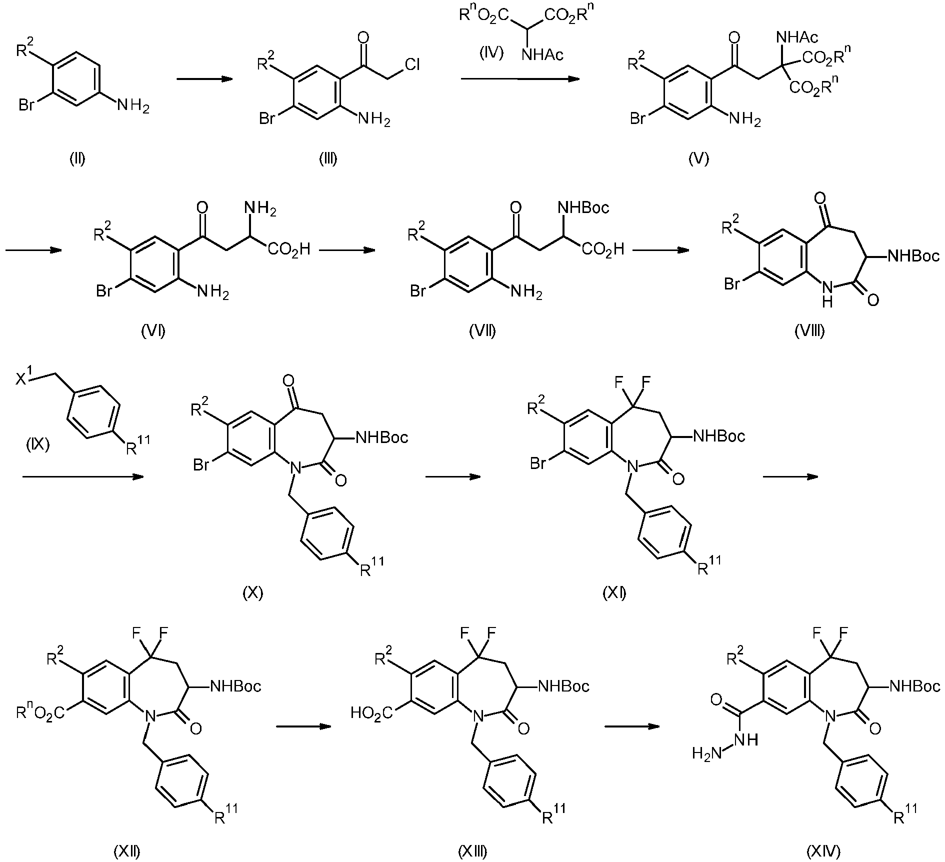

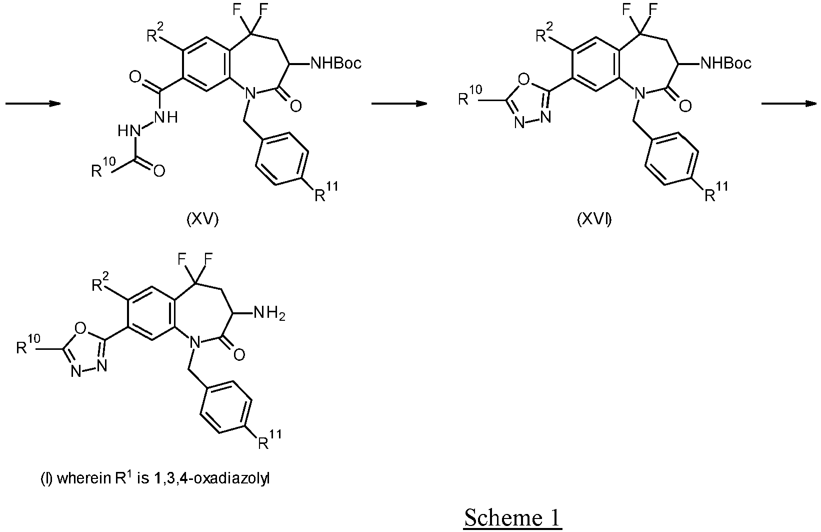

Suitable starting materials for the preparation of compounds of formula (I) are aniline derivatives of formula (II). Compounds of formula (II) can be converted into compounds of formula (III) by reaction with chloroacetonitrile in the presence of AlCl₃ and BCl₃ in a solvent such as toluene at elevated temperatures. Derivatives (III) can be reacted with aminomalonate derivatives (IV) (wherein Rⁿ is alkyl) in the presence of sodium iodide and a base such as sodium alkoxide in an alcoholic solvent to obtain compounds of formula (V). Reaction of formula (V) compounds with aqueous HCl at elevated temperatures provides amino acid derivatives of formula (VI) that can subsequently be N-protected by reaction with di-t-butyldicarbonate in a solvent mixture such as dioxane and water in the presence of a base such as TEA to obtain compounds of formula (VII). Derivatives (VII) can be cyclized to compounds of formula (VIII) emploing T3P in presence of a base such as DIPEA or TEA in a solvent such as DCM or THF. Introduction of a benzyl substituent to obtain compounds of formula (X) can be accomplished by reaction with formula (IX) halides or sulfonates (X¹ is Cl, Br, I or OSO₂R) in presence of a base such as K₂CO₃ in a solvent such as DMF. Optionally, potassium iodide can be added. Reaction of compounds of formula (X) with DAST provides derivatives of formula (XI) which can be converted into formula (XII) compounds by carbonylation reaction employing catalysts such as Pd(OAc)₂/dppp or Pd(dppp)Cl₂ in the presence of a base such as TEA or DIPEA in a solvent mixture of DMF and an alcohol at elevated temperatures. Reaction of compounds of formula (XII) with alkali hydroxides such as LiOH in an aqueous solvent mixture such as THF and water provides compounds of formula (XIII). Alternatively, acid derivatives (XIII) can be obtained from compounds of formula (XI) by carbonylation reaction employing catalysts such as Pd(OAc)₂/dppp or Pd(dppp)Cl₂ in the presence of a base such as TEA or DIPEA in a solvent mixture of DMF and water at elevated temperatures. Conversion of compounds of formula (XIII) into compounds of formula (XIV) can be accomplished by reaction with hydrazine after activation of derivatives (XIII) with reagents such as T3P in the presence of DIPEA or oxalyl chloride in the presence of DIPEA and catalytic amounts of DMF in a solvent such as DCM. Activation of compounds of formula (XIV) with reagents such as HATU or T3P in the presence of a base such as DIPEA and subsequent reation with an acid derivative R¹⁰CO₂H provides compounds of formula (XV). Alternatively, compounds of formula (XV) can be obtained from compounds of formula (XIII) by reaction with hydrazide derivatives R¹⁰CONHNH₂ and a coupling reagent such as HATU in the presence of a base such as DIPEA in a solvent such as THF. Formula (XVI) compounds can be obtained from compounds of formula (XV) by reaction with dehydrating agents such as Burgess reagent in a solvent such as toluene or THF. Compounds of formula (I, wherein R¹ is 1,3,4-oxadiazolyl) can be obtained from compounds of formula (XVI) by reaction with HCl in EtOAc, TFA in DCM or HFIP.

Compounds of formula (I) wherein R¹ is a 6-membered heteroaryl group may be prepared as illustrated in scheme 2.

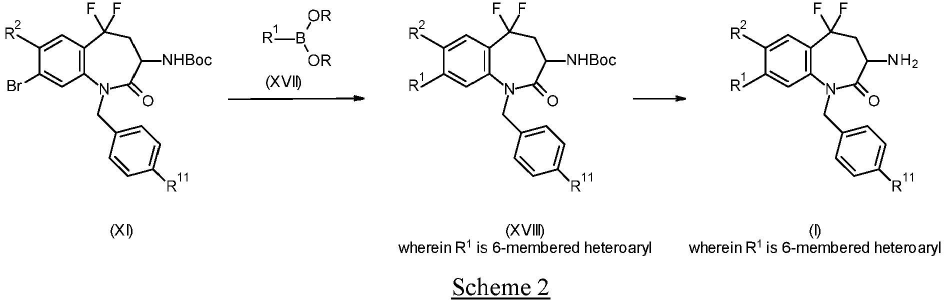

Reaction of compounds of formula (XI) with a boronic acid (R is H) or a boronic acid ester derivative (XVII) in the presence of a palladium catalyst such as Pd(dppf)Cl₂-DCM and a base such as K₂CO₃ in an aqueous solvent mixture such as dioxane and water provides compounds of formula (XVIII). Compounds of formula (XVIII) can be converted into compounds of formula (I) wherein R¹ is a 6-membered heteroaryl group as described for the conversion of compounds of formula (XVI) to compounds of formula (I) in scheme 1.

### Pharmaceutical compositions and administration

Another object of the present invention is a pharmaceutical composition comprising a compound of formula (I) as described herein, or a pharmaceutically acceptable salt thereof, and a pharmaceutically acceptable excipient.

The compounds of formula (I) and their pharmaceutically acceptable salts can be used as medicaments, in the form of pharmaceutical preparations. The pharmaceutical preparations can be administered internally, such as orally (e.g. in the form of tablets, coated tablets, dragées, hard and soft gelatine capsules, solutions, emulsions or suspensions), nasally (e.g. in the form of nasal sprays) or rectally (e.g. in the form of suppositories). However, the administration can also be effected parenterally, such as intramuscularly or intravenously (e.g. in the form of injection solutions). The administration can also be effected topically, e.g. transdermal administration, or in form of eye drops or ear drops.

The compounds of formula (I) and their pharmaceutically acceptable salts can be processed with pharmaceutically inert, inorganic or organic carriers for the production of pharmaceutical preparations, such as tablets, coated tablets, dragées, hard gelatin capsules, injection solutions or topical formulations. Lactose, corn starch or derivatives thereof, talc, stearic acids or salts thereof, and the like can be used, for example, as such carriers for tablets, coated tablets, dragées and hard gelatin capsules.

Suitable carriers for soft gelatin capsules are, for example, vegetable oils, waxes, fats, semi-solid substances and liquid polyols and the like. Depending on the nature of the active substance no carriers are, however, usually required in the case of soft gelatin capsules.

Suitable carriers for the production of solutions and syrups are, for example, water, alcohols, polyols, saccharose, glucose, invert sugar, vegetable oil, etc.

Suitable carriers for injection solutions are, for example, water, alcohols, polyols, glycerol, vegetable oils, etc.

Suitable carriers for suppositories are, for example, natural or hardened oils, waxes, fats, semi-liquid or liquid polyols, etc.

Suitable carriers for topical ocular formulations are, for example, cyclodextrins, mannitol or many other carriers and excipients known in the art.

Moreover, the pharmaceutical preparations can contain preservatives, solubilizers, viscosity increasing substances, stabilizers, wetting agents, emulsifiers, sweeteners, colorants, flavorants, salts for varying the osmotic pressure, buffers, masking agents or antioxidants. They can also contain other therapeutically valuable substances.

Medicaments containing a compound of formula (I) or a pharmaceutically acceptable salt thereof and a pharmaceutically acceptable excipient are also an object of the present invention, as is a process for their production, which comprises bringing one or more compounds of formula (I) and/or pharmaceutically acceptable salts thereof and, if desired, one or more other therapeutically valuable substances into a galenical administration form together with one or more pharmaceutically acceptable excipients.

The dosage can vary within wide limits and will, of course, have to be adjusted to the individual requirements in each particular case. In general, in the case of oral administration a daily dosage of about 0.1 mg to 20 mg per kg body weight, preferably 0.5 mg to 4 mg per kg body weight (e.g. about 300 mg per person), divided into preferably 1-3 individual doses, which can consist, for example, of the same amounts, should be appropriate. **In** the case of topical administration, the formulation can contain 0.001% to 15% by weight of medicament and the required dose, which can be between 0.1 and 25 mg, and can be administered either by single dose per day or per week, or by multiple doses (2 to 4) per day, or by multiple doses per week. It will, however, be clear that the upper or lower limit given herein can be exceeded when this is shown to be indicated.

The pharmaceutical composition according to the invention may be prepared as follows.

### Preparation of pharmaceutical compositions comprising compounds of the invention

**Tablet Formulation (Wet Granulation)**

| **Ingredient** | **mg/tablet** | | | |
|---|---|---|---|---|
| | 5 | 25 | 100 | 500 |
| 1) Compound of formula (I) | 5 | 25 | 100 | 500 |
| 2) Lactose Anhydrous DTG | 125 | 105 | 30 | 150 |
| 3) Sta-Rx 1500 | 6 | 6 | 6 | 30 |
| 4) Microcrystalline Cellulose | 30 | 30 | 30 | 150 |
| 5) Magnesium Stearate | 1 | 1 | 1 | 1 |
| Total | 167 | 167 | 167 | 831 |

### Manufacturing Procedure:

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Capsule Formulation

| **Ingredient** | **mg/capsule** | | | |
|---|---|---|---|---|
| | 5 | 25 | 100 | 500 |
| 1) Compound of formula (I) | 5 | 25 | 100 | 500 |
| 2) Hydrous Lactose | 159 | 123 | 148 | - |
| 3) Corn Starch | 25 | 35 | 40 | 70 |
| 4) Talc | 10 | 15 | 10 | 25 |
| 5) Magnesium Stearate | 1 | 2 | 2 | 5 |
| Total | 200 | 200 | 300 | 600 |

### Manufacturing Procedure:

1. Mix ingredients 1, 2 and 3 in a suitable mixer for 30 minutes.
2. Add ingredients 4 and 5 and mix for 3 minutes.
3. Fill into a suitable capsule.

### Injection Solutions

| **Ingredient** | **mg/injection solution** |
|---|---|
| Compound of formula I | 3 |
| Polyethylene Glycol 400 | 150 |
| acetic acid | q.s. ad pH 5.0 |
| water for injection solutions | ad 1.0 ml |

### Manufacturing Procedure:

A compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Indications

The compounds of formula (I) can be used in an effective amount to treat a subject, in particular a human, affected by cancer.

In one aspect, the present invention provides a compound of formula (I) described herein, or a pharmaceutically acceptable thereof, for use as a therapeutically active substance.

In a further aspect, the present invention provides a compound of formula (I) as described herein, or a pharmaceutically acceptable thereof, for use in the treatment, prevention and/or delay of progression of cancer.

In a further aspect, the present invention provides the use of a compound of formula (I) as described herein, or a pharmaceutically acceptable salt thereof, for the preparation of a medicament for the treatment, prevention and/or delay of progression of cancer.

By the term "treatment" or "treating" and grammatical variations thereof as used herein, is meant therapeutic therapy. In reference to a particular condition, treating means: (1) to ameliorate the condition or one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy using the methods and/or compositions of the invention is also contemplated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As immunotherapeutic agents acting on immune cells rather than directly acting on the cancer cells, the present disclosure could also be foreseen for the use as anti-cancer vaccines. This also comprises approaches in which immune cells are cultured and manipulated *ex vivo* and the herein disclosed molecules are used as a way of conferring co-stimulation of the *ex vivo* manipulated cells.

In one embodiment, the cancer is a hematologic cancer such as lymphoma, a leukemia or a myeloma. A hematologic cancer contemplated herein includes, but is not limited to, one or more leukemias such as B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML) and chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, mucosa-associated lymphoid tissue (MALT) lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia," which are a diverse collection of hematological conditions united by ineffective production (or dysplasia) of myeloid blood cells.

In a further embodiment, the cancer is a non-hematologic cancer such as a sarcoma, a carcinoma, or a melanoma. A non-hematologic cancer contemplated herein includes, but is not limited to, a neuroblastoma, renal cell carcinoma, colon cancer, colorectal cancer, breast cancer, epithelial squamous cell cancer, melanoma, stomach cancer, brain cancer, lung cancer (e.g. nonsmall cell lung cancer - NSCLC), pancreatic cancer, cervical cancer, ovarian cancer, liver cancer, bladder cancer, prostate cancer, testicular cancer, thyroid cancer, uterine cancer, adrenal cancer and head and neck cancer.

### Co-administration of compounds of formula (I) and other agents

The compounds of formula (I) or salts thereof or a compound disclosed herein or a pharmaceutically acceptable salt thereof may be employed alone or in combination with other agents for treatment. For example, the second agent of the pharmaceutical combination formulation or dosing regimen may have complementary activities to the compound of formula (I) such that they do not adversely affect each other. The compounds may be administered together in a unitary pharmaceutical composition or separately. In one embodiment a compound or a pharmaceutically acceptable salt can be co-administered with a cytotoxic agent to treat proliferative diseases and cancer.

The term "co-administering" refers to either simultaneous administration, or any manner of separate sequential administration, of a compound of formula (I) or a salt thereof or a compound disclosed herein or a pharmaceutically acceptable salt thereof and a further active pharmaceutical ingredient or ingredients, including cytotoxic agents and radiation treatment. If the administration is not simultaneous, the compounds are administered in a close time proximity to each other. Furthermore, it does not matter if the compounds are administered in the same dosage form, e.g. one compound may be administered topically and another compound may be administered orally.

Typically, any agent that has anti-cancer activity may be co-administered. Examples of such agents can be found in Cancer Principles and Practice of Oncology by V.T. Devita and S. Heilman (editors), 6th edition (February 15, 2001), Lippincott Williams & Wilkins Publishers. A person of ordinary skill in the art would be able to discern which combinations of agents would be useful based on the particular characteristics of the drugs and the disease involved.

In one aspect, the present invention provides a pharmaceutical composition described herein, further comprising an additional therapeutic agent.

In one embodiment, said additional therapeutic agent is a chemotherapeutic agent.

In one embodiment, said additional therapeutic agent is a cytotoxic agent.

In one embodiment, said additional therapeutic agent is an immuno-oncology agent.

The term "cytotoxic agent" as used herein refers to a substance that inhibits or prevents a cellular function and/or causes cell death or destruction. Cytotoxic agents include, but are not limited to, radioactive isotopes (At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents; growth inhibitory agents; enzymes and fragments thereof such as nucleolytic enzymes; and toxins such as small molecule toxins or enzymatically active toxins of bacterial, fungal, plant or animal origin, including fragments and/or variants thereof.

Exemplary cytotoxic agents can be selected from anti-microtubule agents, platinum coordination complexes, alkylating agents, antibiotic agents, topoisomerase II inhibitors, antimetabolites, topoisomerase I inhibitors, hormones and hormonal analogues, signal transduction pathway inhibitors, non-receptor tyrosine kinase angiogenesis inhibitors, immunotherapeutic agents, proapoptotic agents, inhibitors of LDH-A; inhibitors of fatty acid biosynthesis; cell cycle signaling inhibitors; HDAC inhibitors, proteasome inhibitors; and inhibitors of cancer metabolism.

"Chemotherapeutic agent" includes chemical compounds useful in the treatment of cancer. Examples of chemotherapeutic agents include erlotinib (TARCEVA^{®}, Genentech/OSI Pharm.), bortezomib (VELCADE^{®}, Millennium Pharm.), disulfiram , epigallocatechin gallate , salinosporamide A, carfilzomib, 17-AAG(geldanamycin), radicicol, lactate dehydrogenase A (LDH-A), fulvestrant (FASLODEX^{®}, AstraZeneca), sunitib (SUTENT^{®}, Pfizer/Sugen), letrozole (FEMARA^{®}, Novartis), imatinib mesylate (GLEEVEC^{®}., Novartis), finasunate (VATALANIB^{®}, Novartis), oxaliplatin (ELOXATIN^{®}, Sanofi), 5-FU (5-fluorouracil), leucovorin, Rapamycin (Sirolimus, RAPAMUNE^{®}, Wyeth), Lapatinib (TYKERB^{®}, GSK572016, Glaxo Smith Kline), Lonafamib (SCH 66336), sorafenib (NEXAVAR^{®}, Bayer Labs), gefitinib (IRESSA^{®}, AstraZeneca), AG1478, alkylating agents such as thiotepa and CYTOXAN^{®} cyclosphosphamide; alkyl sulfonates such as busulfan, improsulfan and piposulfan; Eiziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including topotecan and irinotecan); bryostatin; callystatin; CC-1065 (including its adozelesin, carzelesin and bizelesin synthetic analogs); cryptophycins (particularly cryptophycin I and cryptophycin 8); adrenocorticosteroids (including prednisone and prednisolone); cyproterone acetate; 5a-reductases including finasteride and dutasteride); vorinostat, romidepsin, panobinostat, valproic acid, mocetinostat dolastatin; aldesleukin, talc duocarmycin (including the synthetic analogs, KW-2189 and CBI-TM I); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin γḯI and calicheamicin coll (Angew Chem. Inti. Ed. Engl. 1994 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} (doxorubicin), morpholino-doxorubicin, cyanomorpholino-doxorubicin, 2-pyrrolinodoxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidamnol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL (paclitaxel; Bristol-Myers Squibb Oncology, Princeton, N.J.), ABRAXANE^{®} (Cremophor-free), albumin-engineered nanoparticle formulations of paclitaxel (American Pharmaceutical Partners, Schaumberg, 111.), and TAXOTERE^{®} (docetaxel, doxetaxel; Sanofi-Aventis); chloranmbucil; GEMZAR^{®} (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE^{®} (vinorelbine); novantrone; teniposide; edatrexate; daunomycin; aminopterin; capecitabine (XELODA^{®}); ibandronate; CPT-I I; topoisomerase inhibitor RFS 2000; difluoromethylomithine (DMFO); retinoids such as retinoic acid; and pharmaceutically acceptable salts, acids and derivatives of any of the above.

Chemotherapeutic agent also includes (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, iodoxyfene , 4-hydroxytamoxifen, trioxifene, keoxifene,LYl 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RIVISOR^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide and goserelin; buserelin, tripterelin, medroxyprogesterone acetate, diethylstilbestrol, premarin, fluoxymesterone, all transretionic acid, fenretinide, as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAXID^{®}; PROLEUKIN^{®}, rIL-2; a topoisomerase I inhibitor such as LURTOTECAN^{®}; ABARELIX^{®} rmRH; and (ix) pharmaceutically acceptable salts, acids and derivatives of any of the above.

Chemotherapeutic agent also includes antibodies such as alemtuzumab (Campath), bevacizumab (AVASTIN^{®}, Genentech); cetuximab (ERBITUX^{®}, Imclone); panitumumab (VECTIBIX^{®}, Amgen), rituximab (RITUXAN^{®}, Genentech/Biogen Idee), pertuzumab (OMNITARG^{®}, 2C4, Genentech), trastuzumab (HERCEPTIN^{®}, Genentech), tositumomab (Bexxar, Corixia), and the antibody drug conjugate, gemtuzumab ozogamicin (MYLOTARG^{®}, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, fulllength IgGi λ antibody genetically modified to recognize interleukin-12 p40 protein.

Chemotherapeutic agent also includes "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind toEGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX^{®}) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.ll, E6. 3 and E7.6. 3 and described in US 6,235,883; MDX-447 (Medarex Inc); and mAb 806 or humanized mAb 806 (Johns et al, J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105,5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFRantagonists include OSI-774 (CP-358774, erlotinib, TARCEVA^{®} Genentech/OSI Pharmaceuticals); PD 183805 (Cl 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSA^{®}) 4-(3'-Chloro-4'-fluoroanilino)-7-methoxy-6-(3-morpholinopropoxy)quinazoline, AstraZeneca); ZM 105180 ((6-amino-4-(3-methylphenylamino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4- [4- [(I -phenylethyl)amino] -1 H-pyrrolo[2,3 -d]pyrimidin-6-yl] -phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl] -2-butynamide); EKB-569 (N- [4- [(3 -chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB^{®}, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule FIER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Raf-I inhibitors such as antisense agent ISIS-5132 available from ISIS Pharmaceuticals which inhibit Raf-I signaling; non-HER targeted TK inhibitors such as imatinib mesylate (GLEEVEC^{®}, available from Glaxo SmithKline); multi-targeted tyrosine kinase inhibitors such as sunitinib (SUTENT^{®}, available from Pfizer); VEGF receptor tyrosine kinase inhibitors such as vatalanib (PTK787/ZK222584, available from Novartis/Schering AG); MAPK extracellular regulated kinase I inhibitor Cl-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Wamer-Lamber); antisense molecules (e.g. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI- 1033 (Pfizer); Affinitac (ISIS 3521; Isis/Lilly); imatinib mesylate (GLEEVEC^{®}); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-ICl I (Imclone), rapamycin (sirolimus, RAPAMUNE^{®}); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include dexamethasone, interferons, colchicine, metoprine, cyclosporine, amphotericin, metronidazole, alemtuzumab, alitretinoin, allopurinol, amifostine, arsenic trioxide, asparaginase, BCG live, bevacuzimab, bexarotene, cladribine, clofarabine, darbepoetin alfa, denileukin, dexrazoxane, epoetin alfa, elotinib, filgrastim, histrelin acetate, ibritumomab, interferon alfa-2a, interferon alfa-2b, lenalidomide, levamisole, mesna, methoxsalen, nandrolone, nelarabine, nofetumomab, oprelvekin, palifermin, pamidronate, pegademase, pegaspargase, pegfilgrastim, pemetrexed disodium, plicamycin, porfimer sodium, quinacrine, rasburicase, sargramostim, temozolomide, VM-26, 6-TG, toremifene, tretinoin, ATRA, valrubicin, zoledronate, and zoledronic acid, and pharmaceutically acceptable salts thereof.

Chemotherapeutic agents also include hydrocortisone, hydrocortisone acetate, cortisone acetate, tixocortol pivalate, triamcinolone acetonide, triamcinolone alcohol, mometasone, amcinonide, budesonide, desonide, fluocinonide, fluocinolone acetonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (PEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomideminocycline, sulfasalazine, tumor necrosis factor alpha (TNFa) blockers such as etanercept (Enbrel), infliximab (Remicade), adalimumab (Humira), certolizumab pegol (Cimzia), golimumab (Simponi), Interleukin I (IL-I) blockers such as anakinra (Kineret), T cell costimulation blockers such as abatacept (Orencia), Interleukin 6 (IL-6) blockers such as tocilizumab (ACTEMERA^{®}); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-Ml prime; Secreted homotrimeric LTa3 and membrane bound heterotrimer LTa I/β2 blockers such as Anti-lymphotoxin alpha (LTa); radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); miscellaneous investigational agents such as thioplatin, PS-341, phenylbutyrate, ET-18- OCH3, or famesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; delta-9-tetrahydrocannabinol (dronabinol, MARINOL^{®}); beta-lapachone; lapachol; colchicines; betulinic acid; acetylcamptothecin, scopolectin, and 9-aminocamptothecin); podophyllotoxin; tegafur (UFTORAL^{®}); bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTQNEL^{®}); and epidermal growth factor receptor (EGF-R); vaccines such as THERATOPE^{®} vaccine; perifosine, COX-2 inhibitor (e.g. celecoxib or etoricoxib), proteosome inhibitor (e.g. PS341); CCI-779; tipifamib (R11577); orafenib, ABT510; Bcl-2 inhibitor such as oblimersen sodium (GENASENSE^{®}); pixantrone; famesyltransferase inhibitors such as lonafamib (SCH 6636, SARASAR^{™}); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN^{™}) combined with 5-FU and leucovorin.

In another embodiment, compounds of formula (I) can be co-formulated with an immuno-oncology agent. Immuno-oncology agents include, for example, a small molecule drug, antibody, or other biologic or small molecule. Examples of biologic immuno-oncology agents include, but are not limited to, cancer vaccines, antibodies, and cytokines. In one aspect, the antibody is a monoclonal antibody. In another aspect, the monoclonal antibody is humanized or human. In another aspect, the antibody is a bispecific antibody.

In one aspect, the immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fnl4, TWEAK, BAFFR, EDAR, XEDAR, TACI, APRIL, BCMA, LTfiR, LIGHT, DcR3, HVEM, VEGI/TL1A, TRAMP/DR3, EDAR, EDA1, XEDAR, EDA2,TNFRl, Lymphotoxin α/TNPβ, TNFR2, TNF a, LT R, Lymphotoxin a 1β2, FAS, FASL, RELT, DR6, TROY, NGFR.

In one aspect, T cell responses can be stimulated by a combination of a compound of formula (I) and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of formula (I) for the treatment of cancer include antagonists of inhibitory receptors on NK cells or agonists of activating receptors on NK cells. For example, compounds of formula (I) can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 or FPA-008.

In another aspect, compounds of formula (I) can be used with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-Ll/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

In some embodiments, the immuno-oncology agent is a CTLA-4 antagonist, such as an antagonistic CTLA-4 antibody. Suitable CTLA-4 antibodies include, for example, YERVOY (ipilimumab) or tremelimumab. In another aspect, the immuno-oncology agent is a PD-1 antagonist, such as an antagonistic PD-1 antibody. Suitable PD-1 antibodies include, for example, OPDIVO (nivolumab), KEYTRUDA (pembrolizumab), or MEDI-0680 (AMP-514; WO2012/145493). The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgGl, called AMP-224

In another aspect, the immuno-oncology agent is a PD-L1 antagonist, such as an antagonistic PD-L1 antibody. Suitable PD-L1 antibodies include, for example, TECENTRIQ (atezolizumab) (RG7446; WO2010/077634), durvalumab (MEDI4736), BMS-936559 (WO2007/005874), and MSB0010718C (WO2013/79174).

In another aspect, the immuno-oncology agent is a LAG-3 antagonist, such as an antagonistic LAG-3 antibody. Suitable LAG3 antibodies include, for example, BMS-986016 (WO2010/19570, WO2014/08218), or IMP-731 or IMP-321 (WO2008/132601, WO2009/44273).

In another aspect, the immuno-oncology agent is a CD137 (4-1BB) agonist, such as an agonistic CD137 antibody. Suitable CD137 antibodies include, for example, urelumab and PF-05082566 (WO2012/32433).

In another aspect, the immuno-oncology agent is a GITR agonist, such as an agonistic GITR antibody. Suitable GITR antibodies include, for example, BMS-986153, BMS-986156, TRX-518 (WO2006/105021, WO2009/009116) and MK-4166 (WO2011/028683).

In another aspect, the immuno-oncology agent is an IDO antagonist. Suitable IDO antagonists include, for example, INCB-024360 (WO2006/122150, WO2007/75598, WO2008/36653, WO2008/36642), indoximod, or NLG-919 (WO2009/73620, WO2009/1156652, WO2011/56652, WO2012/142237).

In another aspect, the immuno-oncology agent is an OX40 agonist, such as an agonistic OX40 antibody. Suitable OX40 antibodies include, for example, MEDI-6383 or MEDI-6469. In another aspect, the immuno-oncology agent is an OX40L antagonist, such as an antagonistic OX40 antibody. Suitable OX40L antagonists include, for example, RG-7888 (WO06/029879).

In another aspect, the immuno-oncology agent is a CD40 agonist, such as an agonistic CD40 antibody. In yet another embodiment, the immuno-oncology agent is a CD40 antagonist, such as an antagonistic CD40 antibody. Suitable CD40 antibodies include, for example, lucatumumab or dacetuzumab.

In another aspect, the immuno-oncology agent is a CD27 agonist, such as an agonistic CD27 antibody. Suitable CD27 antibodies include, for example, varlilumab.

In another aspect, the immuno-oncology agent is MGA271 (to B7H3) (WO2011/109400).

### Examples

The invention will be more fully understood by reference to the following examples. The claims should not, however, be construed as limited to the scope of the examples.

### 1) Preparative examples

### 1.1) Intermediates

### Intermediate 1

### 2-ethoxy-2,3,3,3-tetrafluoro-propanehydrazide

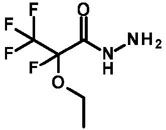

To a solution of 2-ethoxy-2,3,3,3-tetrafluoropropanoic acid [CAS 10186-67-1] (200 mg, 0.92 mmol, 1 eq) in EtOH (1.5 ml) was added hydrazine monohydrate (54.25 mg, 1.1 mmol, 1.2 eq) and the mixture was heated to 80°C for 8 h. The resulting colorless solution was concentrated under reduced pressure and dried under high vacuum to afford the title compound (119 mg, 60%) as white solid. MS (ESI): 205.1 [M+H]⁺.

### Intermediate 2

### 1-ethyl-5,5-difluoro-piperidine-3-carboxylic acid

### Step a) methyl 1-ethyl-5,5-difluoro-piperidine-3-carboxylate

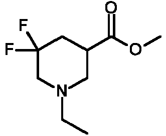

To a solution of methyl 5,5-difluoropiperidine-3-carboxylate; hydrochloride [CAS 1359656-87-3] (370.0 mg, 1.72 mmol, 1.0 eq) and TEA (0.24 mL, 1.72 mmol, 1.0 eq) in MeOH (5 mL) was added acetaldehyde (0.14 mL, 2.57 mmol, 1.5 eq) at 25°C and the mixture was stirred for 1 h. Sodium cyanoborohydride (323.48 mg, 5.15 mmol, 3.0 eq) was added at 25°C and then the mixture was stirred at 25°C for 2 h. The reaction mixture was concentrated in vacuum and the remaining residue was purified by column chromatography on silica gel (PE/EtOAc = 1:0 to 5:1) to give the title compound (300.0 mg, 1.45 mmol, 84% yield) as yellow oil. ¹H NMR (400 MHz, CDCl₃) δ = 3.72 (s, 3H), 3.19 - 3.15 (m, 1H), 3.13 - 3.05 (m, 1H), 2.95 - 2.84 (m, 1H), 2.65 - 2.48 (m, 2H), 2.47 - 2.37 (m, 1H), 2.31 - 2.18 (m, 1H), 2.14 (t, J = 11.2 Hz, 1H), 1.97 - 1.80 (m, 1H), 1.11 (t, J = 7.2 Hz, 3H).

### Step b) 1-ethyl-5,5-difluoro-piperidine-3-carboxylic acid

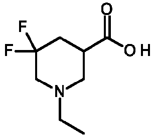

To a solution of methyl 1-ethyl-5,5-difluoro-piperidine-3-carboxylate (340.0 mg, 1.64 mmol, 1.0 eq) in THF (10 mL) was added a solution of LiOH monohydrate (131.26 mg, 3.28 mmol, 2.0 eq) in water (2 mL) dropwise and the reaction mixture was stirred at 20°C for 2 h. The mixture was poured into water (10 mL) and the pH was adjusted to 5 by addition of HCl (1 M). The miture was then lyophilized to give a white solid (300 mg) containing the title compound. ¹H NMR (400 MHz, D₂O) δ = 4.04 - 3.58 (m, 2H), 3.57 - 2.96 (m, 5H), 2.87 - 2.08 (m, 2H), 1.35 (t, J = 7.2 Hz, 3H).

### Intermediate 3

### 2-[4-(chloromethyl)phenyl]-3-fluoro-5-(trifluoromethyl)pyridine

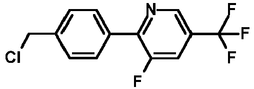

### Step a) [4-[3-fluoro-5-(trifluoromethyl)-2-pyridyl]phenyl]methanol

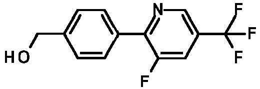

To a mixture of 4-(hydroxymethyl)phenylboronic acid [CAS# 59016-93-2] (1000.0 mg, 6.58 mmol, 1.0 eq), Pd(dppf)Cl₂ (70.03 mg, 0.66 mmol, 0.1 eq) and potassium carbonate (2728.47 mg, 19.74 mmol, 3.0 eq) in a solvent mixture of water (3 mL) and 1,4-dioxane (24 mL) was added 2-bromo-3-fluoro-5-(trifluoromethyl)pyridine [CAS# 89402-29-9] (1926.66 mg, 7.9 mmol, 1.2 eq) under an atmosphere of nitrogen at 25°C. The reaction mixture was stirred at 90°C for 12 h. Then water (50 mL) was added and the mixture was extracted with EtOAc (100 mL x 3). The combined extracts were washed with brine (200 mL x 2), dried (Na₂SO₄) and concentrated and the remaining residue was purified by column chromatography on silica gel (PE/EtOAc = 1:0 to 3:1) to give the title compound (1650.0 mg, 6.08 mmol, 92% yield) as yellow solid. MS (ESI): 272.1 [M+H]⁺.

### Step b) 2-[4-(chloromethyl)phenyl]-3-fluoro-5-(trifluoromethyl)pyridine

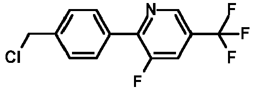

To a solution of [4-[3-fluoro-5-(trifluoromethyl)-2-pyridyl]phenyl]methanol (1600.0 mg, 5.9 mmol, 1.0 eq) in DCM (10 mL) was added thionyl chloride (1.39 mL, 19.11 mmol, 3.0 eq) at 25°C and the mixture was stirred at 25°C for 1 h. Water (30 mL) was added and the mixture was extracted with EtOAc (50 mL x 3). The combined extracts were washed with brine (100 mL x 2), dried (Na₂SO₄) and concentrated to afford the title compound (1600.0 mg) as yellow solid. ¹H NMR (400 MHz, DMSO-d₆) δ = 8.96 (s, 1H), 8.43 (d, J = 10.5 Hz, 1H), 8.00 - 7.96 (m, 2H), 7.63 (d, J = 8.3 Hz, 2H), 4.85 (s, 2H).

### Intermediate 4

### 2-[4-(bromomethyl)phenyl]-5-(trifluoromethyl)tetrazole

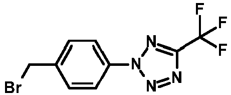

A solution of 2-(p-tolyl)-5-(trifluoromethyl)tetrazole [CAS#1817810-19-7] (300.0 mg, 1.31 mmol, 1.0 eq), N-bromosuccinimide (304.22 mg, 1.71 mmol, 1.3 eq) and AIBN (107.95 mg, 0.66 mmol, 0.5 eq) in chlorobenzene (10 mL) was stirred at 100°C for 12 h. The mixture was concentrated and the remaining residue was purified by column chromatography on silica gel (PE/EtOAc = 1:0 to 50:1) to give the title compound (200.0 mg, 0.65 mmol, 50% yield) as a yellow oil. ¹H NMR (400 MHz, CDCl₃) δ = 8.17 (d, J = 8.8 Hz, 2H), 7.64 (d, J = 8.8 Hz, 2H), 4.56 (s, 2H).

### Intermediate 5

### 1-[4-(bromomethyl)phenyl]-3-(trifluoromethyl)-1,2,4-triazole

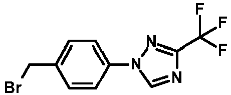

The title compound was prepared in analogy to Intermediate 4 from 1-(p-tolyl)-3-(trifluoromethyl)-1,2,4-triazole [CAS# 2850321-26-3] and was obtained as white solid. MS (ESI): 308.0 [M+H]⁺.

### Intermediate 6

### 2-[4-(chloromethyl)phenyl]-4-(trifluoromethyl)pyridine; hydrochloride

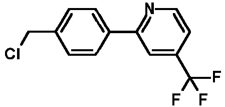

The title compound was prepared in analogy to Intermediate 3, step b from [4-[4-(trifluoromethyl)-2-pyridyl]phenyl]methanol [CAS# 1185444-74-9] and was obtained as white solid. MS (ESI): 272.0 [M+H]⁺.

### Intermediate 7

### 2-[4-(chloromethyl)phenyl]-5-(1,1-difluoroethyl)pyridine

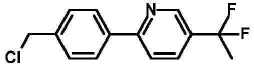

### Step a) [4-[5-(1,1-difluoroethyl)-2-pyridyl]phenyl]methanol

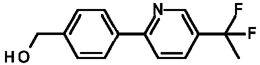

The title compound was prepared in analogy to Intermediate 3, step a from 2-bromo-5-(1,1-difluoroethyl)pyridine [CAS# 1211521-60-6] and 4-(hydroxymethyl)phenylboronic acid [CAS# 59016-93-2] and was obtained as light yellow solid. MS (ESI): 250.3 [M+H]⁺.

### Step b) 2-[4-(chloromethyl)phenyl]-5-(1,1-difluoroethyl)pyridine

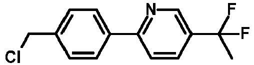

The title compound was prepared in analogy to Intermediate 3, step b from [4-[5-(1,1-difluoroethyl)-2-pyridyl]phenyl]methanol and was obtained as light yellow solid. MS (ESI): 268.1 [M+H]⁺.

### Intermediate 8

### 2-[4-(chloromethyl)phenyl]-5-(difluoromethyl)pyridine

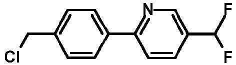

### Step a) [4-[5-(difluoromethyl)-2-pyridyl]phenyl]methanol

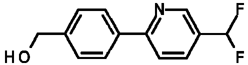

The title compound was prepared in analogy to Intermediate 3, step a from 2-bromo-5-(difluoromethyl)pyridine [CAS# 1221272-81-6] and 4-(hydroxymethyl)phenylboronic acid [CAS# 59016-93-2] and was obtained as white solid. MS (ESI): 236.1 [M+H]⁺.

### Step b) 2-[4-(chloromethyl)phenyl]-5-(difluoromethyl)pyridine

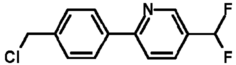

The title compound was prepared in analogy to Intermediate 3, step b from [4-[5-(difluoromethyl)-2-pyridyl]phenyl]methanol and was obtained as light yellow solid. MS (ESI): 254.1 [M+H]⁺.

### Intermediate 9

### 2-[4-(chloromethyl)phenyl]-5-(difluoromethoxy)pyridine

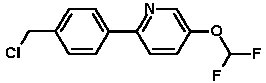

### Step a) [4-[5-(difluoromethoxy)-2-pyridyl]phenyl]methanol

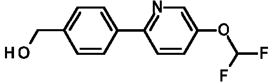

The title compound was prepared in analogy to Intermediate 3, step a from 2-bromo-5-(difluoromethoxy)pyridine [CAS# 845827-14-7] and 4-(hydroxymethyl)phenylboronic acid [CAS# 59016-93-2] and was obtained as white solid. MS (ESI): 252.1 [M+H]⁺.

### Step b) 2-[4-(chloromethyl)phenyl]-5-(difluoromethoxy)pyridine

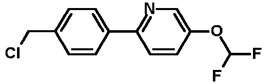

The title compound was prepared in analogy to Intermediate 3, step b from [4-[5-(difluoromethoxy)-2-pyridyl]phenyl]methanol and was obtained as light yellow solid. MS (ESI): 270.0 [M+H]⁺.

### Intermediate 10

### 1-[4-(bromomethyl)phenyl]-4-(trifluoromethoxy)pyrazole

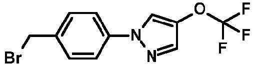

### Step a) O-[1-(p-tolyl)pyrazol-4-yl] methylsulfanylmethanethioate

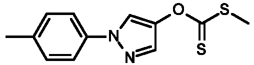

To a solution of 1-(p-tolyl)pyrazol-4-ol (2300.0 mg, 13.2 mmol, 1.0 eq) [CAS# 77458-34-5] and methyl 3-methylimidazol-3-ium-1-carbodithioate (3431.78 mg, 19.8 mmol, 1.5 eq) in MeCN (40 mL) was added TEA (3.68 mL, 26.41 mmol, 2.0 eq) at 25°C and the mixture was stirred at 25°C for 1 h. The reaction mixture was poured into water (40 mL) and the resulting mixture was extracted with EtOAc (30 mL x 3). The combined organic extracts were washed with brine (100 mL), dried (Na₂SO₄) and concentrated. The remaining residue was purified by column chromatography on silica gel (PE/EtOAc = 1:0 to 10:1) to give the title compound (2.7 g, 10.21 mmol, 76% yield) as a yellow solid. ¹H NMR (400 MHz, CDCl₃) δ = 8.04 (s, 1H), 7.69 (s, 1H), 7.57 (d, J = 8.4 Hz, 2H), 7.29 (s, 2H), 2.71 (s, 3H), 2.42 (s, 3H).

### Step b) 3,5-dibromo-1-(p-tolyl)-4-(trifluoromethoxy)pyrazole and 5-bromo-1-(p-tolyl)-4-(trifluoromethoxy)pyrazole

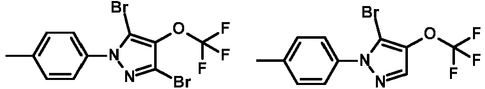

A solution of 1,3-dibromo-5,5-dimethylimidazolidine-2,4-dione (8652.12 mg, 30.26 mmol, 4.0 eq) and HF-pyridine (1165.27 mg, 7.57 mmol, 1.0 eq) in DCM (15 mL) was stirred at -70°C for 0.5 h. Then O-[1-(p-tolyl)pyrazol-4-yl] methylsulfanylmethanethioate (2.0 g, 7.57 mmol, 1.0 eq) in DCM (15 mL) was added dropwise at -70°C and the mixture was allowed to warm to 0°C and was stirred at 0°C for 0.5 h. The mixture was poured into a saturated aqueous solution of NaHCO₃ (80 mL) and the resulting mixture was extracted with EtOAC (3 x 60 mL). The combined organic extracts were washed with brine (200 mL), dried over anhydrous sodium sulfate, filtered, and concentrated. The remaining residue was purified by column chromatography on silica gel (PE/EtOAc = 1:0 to 10:1) to give a the mixture of 3,5-dibromo-1-(p-tolyl)-4-(trifluoromethoxy)pyrazole (1550.0 mg, 3.88 mmol, 51% yield) and 5-bromo-1-(p-tolyl)-4-(trifluoromethoxy)pyrazole (600.0 mg, 1.87 mmol, 25% yield) as a yellow solid. MS (ESI): 400.9 [M+H]⁺ and 323.0 [M+H]⁺.

### Step c) 1-(p-tolyl)-4-(trifluoromethoxy)pyrazole

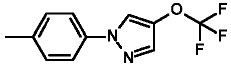

A solution of a mixture of 3,5-dibromo-1-(p-tolyl)-4-(trifluoromethoxy)pyrazole (1550.0 mg, 3.88 mmol, 1.0 eq) and 5-bromo-1-(p-tolyl)-4-(trifluoromethoxy)pyrazole (600.0 mg, 1.87 mmol, 0.48 eq) in MeOH (5 mL) was added to a suspension of Pd/C (500.0 mg, 18.69 mmol, 4.82 eq) in MeOH (5 mL) under an atmosphere of nitrogen. The reaction mixture was degassed an charged with hydrogen for three times and then the mixture was stirred at 25°C for 1 h under an atmosphere of hydrogen. The mixture was filtered with celite and concentrated under reduced pressure to afford the title compound (1360.0 mg, 5.62 mmol, 98% yield) as a yellow solid. MS (ESI): 243.3 [M+H]⁺.

### Step d) 1-[4-(bromomethyl)phenyl]-4-(trifluoromethoxy)pyrazole

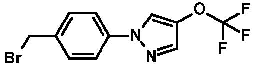

The title compound was prepared in analogy to Intermediate 4 from 1-(p-tolyl)-4-(trifluoromethoxy)pyrazole and was obtained as yellow solid. MS (ESI): 323.0 [M+H]⁺.

### Intermediate 11

### 1-[4-(chloromethyl)-3-fluoro-phenyl]-4-(trifluoromethyl)pyrazole

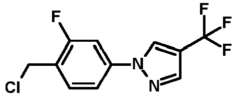

### Step a) methyl 2-fluoro-4-[4-(trifluoromethyl)pyrazol-1-yl]benzoate

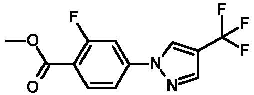

To a solution of methyl 4-bromo-2-fluorobenzoate [CAS# 179232-29-2] (5.14 g, 22.05 mmol, 1.0 eq), 4-(trifluoromethyl)-1H-pyrazole [CAS# 52222-73-8] (3.0 g, 22.05 mmol, 1.0 eq) and trans-N,N-dimethylcyclohexane-1,2-diamine (6.27 g, 44.09 mmol, 2.0 eq) in DMSO (50 mL) was added CuI (4.2 g, 22.05 mmol, 1.0 eq) at 25°C under an atmosphere of nitogen and the mixture was stirred at 100°C for 3 h. Then the mixture was filtered with celite and water (200 mL) was added to the filtrate. The mixture was extracted with EtOAc (150 mL × 3) and the organic extracts were washed with brine (300 mL), dried (Na₂SO₄) and concentrated. The remaining residue was purified by column chromatography on silica gel (PE/EtOAc = 10:1) to give the title compound (4.4 g, 15.27 mmol, 69% yield) as a yellow solid. MS (ESI): 289.1 [M+H]⁺.

### Step b) [2-fluoro-4-[4-(trifluoromethyl)pyrazol-1-yl]phenyl]methanol

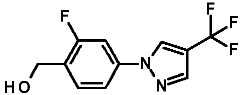

To a solution of methyl 2-fluoro-4-[4-(trifluoromethyl)pyrazol-1-yl]benzoate (3400.0 mg, 11.8 mmol, 1.0 eq) in THF (40 mL) was added diisobutylaluminium hydride (50.0 mL, 50.0 mmol, 4.24 eq) at 0°C and then the mixture was warmed up to 25°C and stirred for 12 h. The reaction mixture was poured into water (100 mL) and the resulting mixture was extracted with EtOAc (300 mL). The organic phase was washed with brine (100 mL), dried (Na₂SO₄) and concentrated to give the title compound (2500.0 mg, 9.61 mmol, 79% yield) as a white solid, which was used for the next step without further purification. MS (ESI): 261.1 [M+H]⁺.

### Step c) 1-[4-(chloromethyl)-3-fluoro-phenyl]-4-(trifluoromethyl)pyrazole

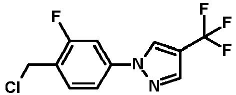

The title compound was prepared in analogy to Intermediate 3, step b from [2-fluoro-4-[4-(trifluoromethyl)pyrazol-1-yl]phenyl]methanol and was obtained as brown oil. ¹H NMR (400 MHz, CDCl₃) δ = 8.00 (s, 1H), 7.72 (s, 1H), 7.38 - 7.27 (m, 3H), 4.47 (s, 2H).

### Intermediate 12

### 3-(chloromethyl)-2-fluoro-6-[4-(trifluoromethyl)pyrazol-1-yl]pyridine

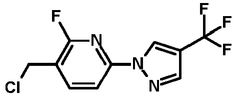

### Step a) methyl 2-fluoro-6-[4-(trifluoromethyl)pyrazol-1-yl]pyridine-3-carboxylate

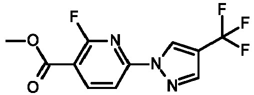

A mixture of 4-(trifluoromethyl)-1H-pyrazole (3.0 g, 22.05 mmol, 1.0 eq), potassium carbonate (9140.61 mg, 66.14 mmol, 3.0 eq) and methyl 2,6-difluoropyridine-3-carboxylate (4579.89 mg, 26.46 mmol, 1.2 eq) in DMSO (60 mL) was stirred at 20°C for 8 h. The mixture was poured into water (300 mL) and the resulting mixtuire was extracted with EtOAc (500 mL). The organic extract was washed with brine (300 mL), dried (Na₂SO₄) and concentrated. The remaining residue was purified by column chromatography on silica gel (PE/EtOAc = 20:1 to 5:1) to give a light yellow solid which was further purified by reversed-phase chromatography (water (containing formic acid)/MeCN = 5:1 to 1:1) to give the title compound (2.1 g, 7.26 mmol, 32% yield) as a light yellow solid. MS (ESI): 290.0 [M+H]⁺.

### Step b) [2-fluoro-6-[4-(trifluoromethyl)pyrazol-1-yl]-3-pyridyl]methanol

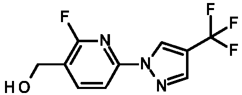

The title compound was prepared in analogy to Intermediate 11, step b from methyl 2-fluoro-6-[4-(trifluoromethyl)pyrazol-1-yl]pyridine-3-carboxylate and was obtained as light yellow solid. MS (ESI): 262.1 [M+H]⁺.

### Step c) 3-(chloromethyl)-2-fluoro-6-[4-(trifluoromethyl)pyrazol-1-yl]pyridine

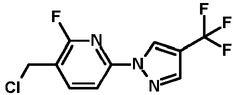

The title compound was prepared in analogy to Intermediate 3, step b from [2-fluoro-6-[4-(trifluoromethyl)pyrazol-1-yl]-3-pyridyl]methanol and was obtained as light yellow solid. MS (ESI): 280.1 [M+H]⁺.

### Intermediate 13

### 5-(chloromethyl)-2-[4-(trifluoromethyl)pyrazol-1-yl]pyridine

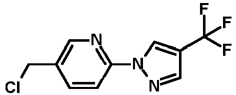

### Step a) methyl 6-[4-(trifluoromethyl)pyrazol-1-yl]pyridine-3-carboxylate

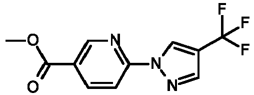

To a mixture of 4-(trifluoromethyl)-1H-pyrazole (2.0 g, 14.7 mmol, 1.0 eq) and potassium carbonate (6.1 g, 44.1 mmol, 3.0 eq) in DMF (48 mL) was added a solution of methyl 6-chloronicotinate (2.27 g, 13.2 mmol, 0.9 eq) in DMF (24 mL) dropwise at 25°C and then the mixture was stirred at 100°C for 12 h. Water (50 mL) was added and the mixture was extracted with EtOAc (30 mL x 3). The organic extracts were washed with brine (30 mL x 5), dried (Na₂SO₄) and concentrated. The remaining residue was purified by column chromatography on silica gel (PE/EtOAc = 1:0 to 10:1) to give the title compound (1650.0 mg, 6.08 mmol, 41% yield) as a white solid. MS (ESI): 272.2 [M+H]⁺.

### Step b) 6-[4-(trifluoromethyl)pyrazol-1-yl]-3-pyridyl]methanol

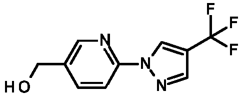

The title compound was prepared in analogy to Intermediate 11, step b from methyl 6-[4-(trifluoromethyl)pyrazol-1-yl]pyridine-3-carboxylate and was obtained as yellow solid. MS (ESI): 244.3 [M+H]⁺.

### Step c) 5-(chloromethyl)-2-[4-(trifluoromethyl)pyrazol-1-yl]pyridine

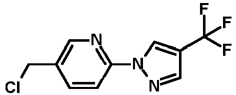

The title compound was prepared in analogy to Intermediate 3, step b from 6-[4-(trifluoromethyl)pyrazol-1-yl]-3-pyridyl]methanol and was obtained as yellow oil. MS (ESI): 262.2 [M+H]⁺.

### Intermediate 14

### 1-[4-(chloromethyl)-2-fluoro-phenyl]-4-(trifluoromethyl)pyrazole

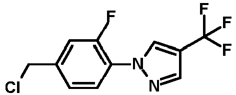

### Step a) methyl 3-fluoro-4-[4-(trifluoromethyl)pyrazol-1-yl]benzoate

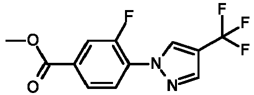

A mixture of 4-(trifluoromethyl)-1H-pyrazole (200.0 mg, 1.47 mmol, 1.0 eq), potassium carbonate (609.37 mg, 4.41 mmol, 3.0 eq) and methyl 3,4-difluorobenzoate (379.48 mg, 2.2 mmol, 1.5 eq) in DMF (10 mL) was stirred at 100°C for 8 h. The reaction mixture was poured into water (30 mL) and the resulting mixture was extracted with EtOAc (50 mL). The organic phase was washed with brine (50 mL), dried (Na₂SO₄) and concentrated. The remaining residue was purified by column chromatography on silica gel (PE/EtOAc = 1:0 to 5:1) to give the title compound (400.0 mg, 1.39 mmol, 80% yield) as a light yellow solid. MS (ESI): 289.0 [M+H]⁺.

### Step b) [3-fluoro-4-[4-(trifluoromethyl)pyrazol-1-yl]phenyl]methanol

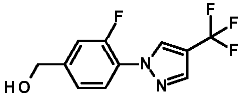

The title compound was prepared in analogy to Intermediate 11, step b from methyl 3-fluoro-4-[4-(trifluoromethyl)pyrazol-1-yl]benzoate and was obtained as white solid. MS (ESI): 261.0 [M+H]⁺.

### Step c) 1-[4-(chloromethyl)-2-fluoro-phenyl]-4-(trifluoromethyl)pyrazole

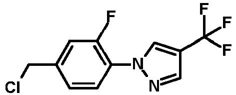

The title compound was prepared in analogy to Intermediate 3, step b from [3-fluoro-4-[4-(trifluoromethyl)pyrazol-1-yl]phenyl]methanol and was obtained as light yellow solid. MS (ESI): 279.0 [M+H]⁺.

### Intermediate 15

### 4-[4-(chloromethyl)phenyl]-1-(trifluoromethyl)pyrazole

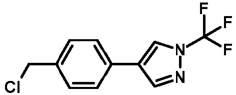

### Step a) [4-[1-(trifluoromethyl)pyrazol-4-yl]phenyl]methanol

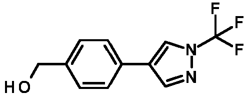

A mixture of potassium carbonate (2571.19 mg, 18.6 mmol, 2.5 eq), 4-bromobenzyl alcohol [CAS# 873-75-6] (1391.8 mg, 7.44 mmol, 1.0 eq), 4-(4,4,5,5-tetramethyl-1,3,2-dioxaborolan-2-yl)-1-(trifluoromethyl)pyrazole [CAS# 1046831-98-4] (1950.0 mg, 7.44 mmol, 1.0 eq), 1,1'-bis(diphenylphosphino)ferrocene-palladium(II)dichloride dichloromethane complex (607.24 mg, 0.74 mmol, 0.1 eq) in a mixture of water (1 mL) and 1,4-dioxane (8 mL) was stirred at 90°C for 3 h under an atmosphere of nitrogen. The reaction mixture was concentrated in vacuu and the remaining residue was purified by column chromatography on silica gel to give the title compound (1620.0 mg, 6.69 mmol, 90% yield) as yellow oil. MS (ESI): 243.1 [M+H]⁺.

### Step b) 4-[4-(chloromethyl)phenyl]-1-(trifluoromethyl)pyrazole

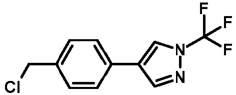

To a solution of [4-[1-(trifluoromethyl)pyrazol-4-yl]phenyl]methanol (1600.0 mg, 6.61 mmol, 1.0 eq) in DCM (10 mL) was added thionyl chloride (1.4 mL, 19.82 mmol, 3.0 eq) slowly at 20°C and the solution was stirred at 20°C for 1 h . The reaction mixture was concentrated in vacuum to give the crude title compound (1710.0 mg, 6.56 mmol, 99% yield) as yellow solid which was used without further purification. MS (ESI): 261.1 [M+H]⁺.

### Intermediate 16

### 5,5-difluoro-1-(2-methoxyethyl)piperidine-3-carboxylic acid

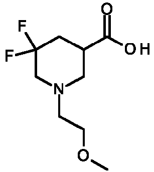

### Step a) methyl 5,5-difluoro-1-(2-methoxyethyl)piperidine-3-carboxylate

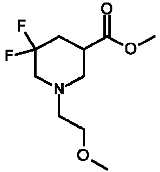

To a mixture of methyl 5,5-difluoropiperidine-3-carboxylate;hydrochloride (1100.0 mg, 5.1 mmol, 1.0 eq), 2-bromoethyl methyl ether (1.44 mL, 15.3 mmol, 3.0 eq), potassium carbonate (2115.1 mg, 15.3 mmol, 3.0 eq) in MeCN (20 mL) was added potassium iodide (0.14 mL, 2.55 mmol, 0.5 eq) at 20°C and the mixture was stirred at 80°C for 12 h. The reaction mixture was poured into water (50 mL) and the resulting mixture was extracted with ethyl acetate (50 mL×3). The combined organic extracts were washed with brine (100 mL), dried (Na₂SO₄) and concentrated in vacuum. The remaining residue was purified by column chromatography on silica gel (PE/EtOAc = 100:1 to 5:1) to give the title compound (1200.0 mg, 5.06 mmol, 99% yield) as yellow oil. ¹H NMR (400 MHz, CDCl₃) δ = 3.71 (s, 3H), 3.55 - 3.49 (m, 2H), 3.35 (s, 3H), 3.22 - 3.09 (m, 2H), 2.95 - 2.86 (m, 1H), 2.82 - 2.65 (m, 2H), 2.48 - 2.35 (m, 2H), 2.34 - 2.27 (m, 1H), 1.97 - 1.80 (m, 1H).

### Step b) 5,5-difluoro-1-(2-methoxyethyl)piperidine-3-carboxylic acid

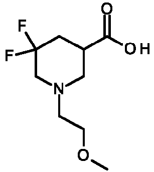

To a solution of methyl 5,5-difluoro-1-(2-methoxyethyl)piperidine-3-carboxylate (500.0 mg, 2.11 mmol, 1.0 eq) in THF (10 mL) was added a solution of LiOH-H₂O (168.61 mg, 4.22 mmol, 2.0 eq) in water (2 mL) drop wise and the reaction mixture was stirred at 20°C for 2 h. The reaction mixture was poured into water (10 mL) and the pH of the mixture was adjusted to 5 by addition of HCl (1M). The miture was lyophilized to give the title compound (450.0 mg) as white solid (containing LiCl). ¹H NMR (400 MHz, DMSO-d₆) δ = 3.87 - 3.71 (m, 4H), 3.55 - 3.47 (m, 1H), 3.38 - 3.34 (m, 2H), 3.25 (s, 3H), 3.17 - 3.15 (m, 1H), 2.43 - 2.41 (m, 2H), 2.29 - 2.08 (m, 1H).

### 1.2) Syntheses of examples

### Example 1

### 3-amino-8-(5-tert-butyl-1,3,4-oxadiazol-2-yl)-5,5,7-trifluoro-1-[[4-(trifluoromethoxy)phenyl]methyl]-3,4-dihydro-1-benzazepin-2-one

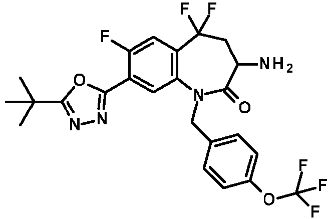

### Step a) 1-(2-amino-4-bromo-5-fluoro-phenyl)-2-chloro-ethanone

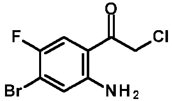

Chloroacetonitrile (4.6 mL, 72.63 mmol, 1.38 eq) was added dropwise to a mixture of AlCl₃ (8.84 g, 66.31 mmol, 1.26 eq) and BCl₃ (1M in DCM, 61.05 mL, 61.05 mmol, 1.16 eq) in toluene (200 mL) and the mixture was stirred at 0°C for 0.5 h under an atmosphere of nitrogen. 3-Bromo-4-fluoroaniline (6.33 mL, 52.63 mmol, 1 eq) was added and the reaction mixture was stirred at 20°C for 0.5 h and then heated to 70°C for 16 h. Then HCl (100 mL) was added at 50°C over a period of 30 min. The mixture was cooled to RT and extracted with DCM (2 x 30 mL). The combined organic extracts were dried over Na₂SO₄ and the solvent was removed under reduced pressure to afford a residue that was purified by prep-HPLC. The title compound was obtained as brown solid (3.3 g, 12.38 mmol, 23% yield).
¹H NMR (400 MHz, DMSO-d6) δ = 7.76 - 7.68 (m, 1H), 7.34 - 7.20 (m, 2H), 7.18 - 7.14 (m, 1H), 5.07 - 4.99 (m, 2H)

### Step b) diethyl 2-acetamido-2-[2-(2-amino-4-bromo-5-fluoro-phenyl)-2-oxo-ethyl]propanedioate

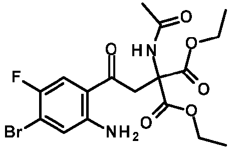

Sodium ethylate (21 wt. % in ethanol, 1.09 mL, 13.9 mmol, 1.61 eq) was added to a solution of diethyl 2-acetamidopropanedioate (1.87 g, 8.63 mmol, 1 eq) in ethanol (20 mL) at 20°C and the mixture was stirred for 0.5 h. Sodium iodide (0.26 g, 1.72 mmol, 0.200 eq) and a solution of 1-(2-amino-4-bromo-5-fluoro-phenyl)-2-chloro-ethanone (2.3 g, 8.63 mmol, 1 eq) in THF (10 mL) were added and the resulting mixture was stirred at 50°C for 16 h. The reaction mixture was added to ice/water (15 mL) and the resulting mixture was extracted with ethyl acetate (3 x 20 mL). The combined organic layers were washed with brine (2 x 20 mL), dried over anhydrous sodium sulfate and concentrated under vacuum to afford the crude product which was purified by column chromatography on silica gel (5% to 40% EtOAc in PE) to afford the title compound (2.5 g, 5.59 mmol, 60% yield) as a brown oil. MS (ESI): 447.0 [M +H]⁺.

### Step c) 2-amino-4-(2-amino-4-bromo-5-fluoro-phenyl)-4-oxo-butanoic acid

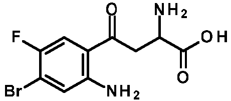

A solution of diethyl 2-acetamido-2-[2-(2-amino-4-bromo-5-fluoro-phenyl)-2-oxoethyl]propanedioate (1.95 g, 4.36 mmol, 1 eq) in hydrochloric acid (0.218 M, 20.0 mL, 4.36 mmol, 1 eq) was stirred at 110 °C for 16 h. The mixture was concentrated under vacuum to obtain a brown solid (1.33 g) which was dissolved in water. The pH of the aqueous solution was then adjusted to 7 with NaOH (2 M) and the aqueous solution was used as such in the next reaction step.

### Step d) 4-(2-amino-4-bromo-5-fluoro-phenyl)-2-(tert-butoxycarbonylamino)-4-oxo-butanoic acid

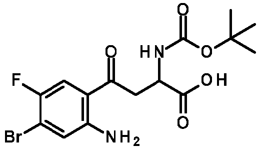

To a solution of 2-amino-4-(2-amino-4-bromo-5-fluoro-phenyl)-4-oxo-butanoic acid (1.33 g, 4.36 mmol, 1 eq) and di-t-butyldicarbonate (1.14 g, 5.23 mmol, 1.2 eq) in a solvent mixture of dioxane (15 mL) and water (10 mL) was added TEA (1.21 mL, 8.72 mmol, 2 eq) and the mixture was stirred at 25°C for 16 h. Ice water (10 mL) and EtOAc (20 mL) were added and the pH of the solution was adjusted to ~4 with 2N HCl. The organic phase was separated, washed with brine, dried over Na₂SO₄ and evaporated in vacuo. The remaining residue was purified by column chromatography on silica gel (20% to 40% EtOAc (containing 0.1% AcOH) in PE) to afford the title compound (1.2 g, 2.96 mmol, 56% yield) as brown oil. MS (ESI): 351.0 [M-isobutene+H]⁺.

### Step e) tert-butyl N-(8-bromo-7-fluoro-2,5-dioxo-3,4-dihydro-1H-1-benzazepin-3-yl)carbamate

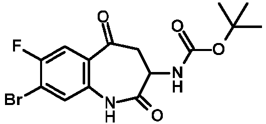

To a solution of 4-(2-amino-4-bromo-5-fluoro-phenyl)-2-(tert-butoxycarbonylamino)-4-oxo-butanoic acid (1.0 g, 2.47 mmol, 1 eq) and DIPEA (1.29 mL, 7.4 mmol, 3 eq) in DCM (20 mL) was added T3P (2.36 g, 3.7 mmol, 1.5 eq) and the reaction mixture was stirred at 20°C for 16 h. Water ( 30 mL ) was added and the mixture was extracted with EtOAc (10 mL x 2). The organic extracts were washed with brine (10 mL x 3), dried over Na₂SO₄ and concentrated under vacuum to provide the title compound (870 mg, 2.25 mmol, 91% yield) as yellow solid. MS (ESI): 330.7 [M-isobutene+H]⁺.

### Step f) tert-butyl N-[8-bromo-7-fluoro-2,5-dioxo-1-[[4-(trifluoromethoxy)phenyl]methyl]-3,4-dihydro-1-benzazepin-3-yl]carbamate

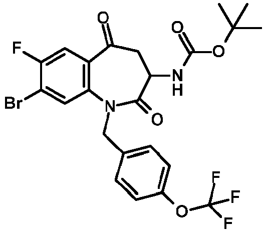

To a solution of tert-butyl N-(8-bromo-7-fluoro-2,5-dioxo-3,4-dihydro-1H-1-benzazepin-3-yl)carbamate (900.0 mg, 2.32 mmol, 1 eq) in DMF (30 mL) were added K₂CO₃ (706.76 mg, 5.11 mmol, 2.2 eq) and 4-(trifluoromethoxy)benzyl bromide (0.37 mL, 2.32 mmol, 1 eq) at 0°C and the reaction mixture was then stirred for 16 h at 25°C. Water (10 mL) was added and the mixture was extracted with EtOAc (10 mL x 3). The combined extracts were dried over Na₂SO₄ and concentrated to obtain a liquid which was purified by column chromatography on silica gel (4% to 80% EtOAc in PE) to affort the title compound (1.07 g, 1.91 mmol, 63% yield) as yellow oil. MS (ESI): 504.9 [M-isobutene+H]⁺.

### Step g) tert-butyl N-[8-bromo-5,5,7-trifluoro-2-oxo-]-[[4-(trifluoromethoxy)phenyl]methyl]-3,4-dihydro-1-benzazepin-3-yl]carbamate

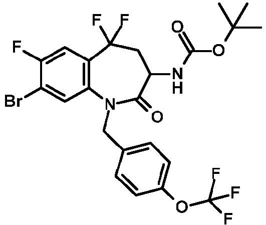

To tert-butyl N-[8-bromo-7-fluoro-2,5-dioxo-1-[[4-(trifluoromethoxy)phenyl]methyl]-3,4-dihydro-1-benzazepin-3-yl]carbamate (2.0 g, 2.85 mmol, 1 eq) was added dropwise DAST (20.0 mL) at 0-10°C under an atmosphere of nitrogen and the mixture was stirred at 25°C for 12 h. Then MeOH (60 mL) was added dropwise and the mixture was concentrated. The crude material was dissloved in EtOAc (30 mL) and water (20 mL) was added. The pH of the mixture was adjusted to 8 by addition of NaHCO₃. The phases were seperated and the organic phase was washed with brine (100 mL), dried (Na₂SO₄) and concentrated in vacuum. The remaining residue was purified by column chromatography on silica gel (MeCN/water (containing formic acid) = 1:5 to 4: 1) to obtain the title compound (0.8 g, 1.37 mmol, 48% yield) as a light yellow solid. MS (ESI): 528.9 [M-isobutene+H]⁺.

### Step h) ethyl 3-(tert-butoxycarbonylamino)-5,5,7-trifluoro-2-oxo-1-[[4-(trifluoromethoxy)phenyl]methyl]-3,4-dihydro-1-benzazepine-8-carboxylate

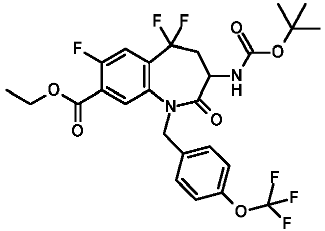

To a mixture of tert-butyl N-[8-bromo-5,5,7-trifluoro-2-oxo-1-[[4-(trifluoromethoxy)phenyl]methyl]-3,4-dihydro-1-benzazepin-3-yl]carbamate (480.0 mg, 0.820 mmol, 1 eq), 1,3-bis-(diphenylphosphino)propane (67.88 mg, 0.160 mmol, 0.200 eq) and palladium diacetate (18.47 mg, 0.080 mmol, 0.100 eq) in a solvent mixture of ethanol (6 mL) and DMF (12 mL) was added TEA (0.34 mL, 2.47 mmol, 3 eq). Then the mixture was degassed with nitrogen for three times and then charged with CO (230.41 mg, 8.23 mmol, 10 eq) for three times and then stirred at 80°C for 16 h under CO (45 psi). Water (10 mL) was added and the mixture was extracted with EtOAc (10 mL x 3). The combined extracts were dried over Na₂SO₄, filtered and concentrated under vacuum. The remaining residue was purified by column chromatography on silica gel (4% to 80% EtOAc in PE) to affort the title compound (437 mg, 0.760 mmol, 87% yield) as yellow oil. MS (ESI): 521.0 [M-isobutene+H]⁺.

### Step i) 3-(tert-butoxycarbonylamino)-5,5, 7-trifluoro-2-oxo-1-[[4-(trifluoromethoxy)phenyl]methyl]-3,4-dihydro-1-benzazepine-8-carboxylic acid

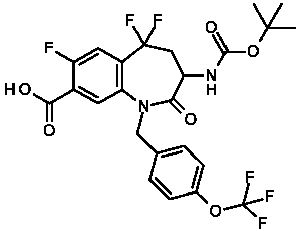

To a solution of ethyl 3-(tert-butoxycarbonylamino)-5,5,7-trifluoro-2-oxo-1-[[4-(trifluoromethoxy)phenyl]methyl]-3,4-dihydro-1-benzazepine-8-carboxylate (890.0 mg, 1.54 mmol, 1 eq) in a solvent mixture of THF (8 mL) and water (8 mL) was added LiOH (147.9 mg, 6.18 mmol, 4 eq) at 0°C and the reaction mixture was then stirred at 20°C for 4 h. The pH of the mixture was then adjusted to 3 to 4 by addition of 1N HCl. The mixture was extracted with EtOAc (10 mL x 3) and the extracts were dried over Na₂SO₄ and concentrated under vacuum to afford the title compound (790 mg, 1.44 mmol, 84% yield) as yellow foam. MS (ESI): 493.0 [M-isobutene+H]⁺.

### Step j) tert-butyl N-[5,5, 7-trifluoro-8-(hydrazinecarbonyl)-2-oxo-1-[[4-(trifluoromethoxy)phenyl]methyl]-3,4-dihydro-1-benzazepin-3-yl]carbamate

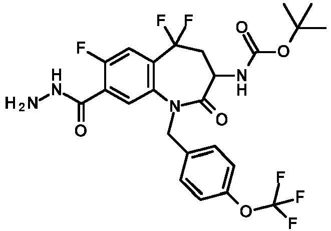

To a solution of 3-(tert-butoxycarbonylamino)-5,5,7-trifluoro-2-oxo-1-[[4-(trifluoromethoxy)phenyl]methyl]-3,4-dihydro-1-benzazepine-8-carboxylic acid (430.0 mg, 0.780 mmol, 1 eq) and T3P (748.41 mg, 1.18 mmol, 1.5 eq) in DCM (10 mL) were added DIPEA (0.41 mL, 2.35 mmol, 3 eq) and hydrazine monohydrate (0.16 mL, 3.14 mmol, 4 eq) and the reaction mixture was stirred at 20°C for 16 h. The reaction mixture was diluted with water (5 mL ) and extracted with DCM (5 mL x 3). The combined extracts were washed with brine (5 mL x 3), dried over Na₂SO₄, filtered and concentrated under vacuum and the remaining residue was purified by column chromatography on silica gel (4% to 40% EtOAc in PE) to afford the title compound (220 mg, 0.390 mmol, 49% yield) as yellow oil. MS (ESI): 507.0 [M-isobutene+H]⁺.

### Step k) tert-butyl N-[8-[(2,2-dimethylpropanoylamino)carbamoyl]-5,5, 7-trifluoro-2-oxo-1-[[4-(trifluoromethoxy)phenyl]methyl]-3,4-dihydro-1-benzazepin-3-yl]carbamate

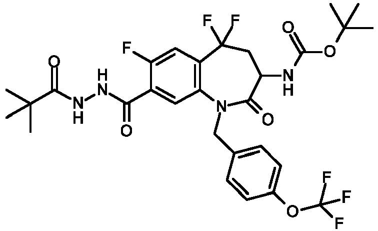

To a solution of tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate (70.0 mg, 0.120 mmol, 1 eq) in THF (60 mL) were added DIPEA (0.07 mL, 0.370 mmol, 3 eq), HATU (56.79 mg, 0.150 mmol, 1.2 eq) and pivalic acid (13.98 mg, 0.140 mmol, 1.1 eq) at 25°C and the mixture was stirred at 25°C for 4 h. The reaction mixture was concentrateed under vacuum to remove THF. Then EtOAc (20 mL) was added and the mixture was washed with water (10 mL) and brine (10 mL x 3), dried over anhydrous Na₂SO₄ and concentrated under vacuum to obtain the crude product. The crude product was purified by preperative TLC (PE/EA = 1: 2) to obtain the title compound (45 mg, 0.070 mmol, 39% yield) as yellow oil. MS (ESI): 591.0 [M-isobutene+H]⁺.

### Step 1) tert-butyl N-[8-(5-tert-butyl-1,3,4-oxadiazol-2-yl)-5,5,7-trifluoro-2-oxo-1-[[4-(trifluoromethoxy)phenyl]methyl]-3,4-dihydro-1-benzazepin-3-yl]carbamate

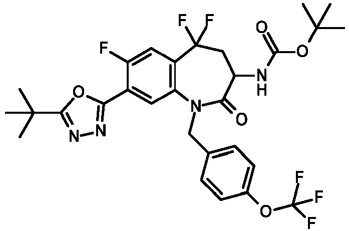

To a solution of tert-butyl N-[8-[(2,2-dimethylpropanoylamino)carbamoyl]-5,5,7-trifluoro-2-oxo-1-[[4-(trifluoromethoxy)phenyl]methyl]-3,4-dihydro-1-benzazepin-3-yl]carbamate (30.0 mg, 0.050 mmol, 1 eq) in toluene (40 mL) was added Burgess Reagent (44.23 mg, 0.190 mmol, 4 eq) at 25°C and the reaction mixture was heated to 100°C for 16 h. Then water (10 mL) was added and the mixture was extracted with EtOAc (10 mL x 3). The combined extracts were washed with brine (10 mL x 3), dried over Na₂SO₄ and concentrated under vacuum to afford the title compound (30 mg, 0.050 mmol, 84% yield) as yellow oil. MS (ESI): 573.0 [M-isobutene+H]⁺.

### Step m) 3-amino-8-(5-tert-butyl-1,3,4-oxadiazol-2-yl)-5,5,7-trifluoro-1-[[4-(trifluoromethoxy)phenyl]methyl]-3,4-dihydro-1-benzazepin-2-one

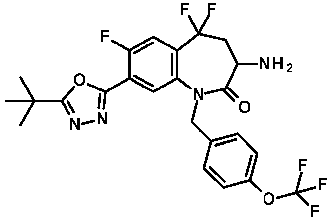

A solution of tert-butyl N-[8-(5-tert-butyl-1,3,4-oxadiazol-2-yl)-5,5,7-trifluoro-2-oxo-1-[[4-(trifluoromethoxy)phenyl]methyl]-3,4-dihydro-1-benzazepin-3-yl]carbamate (30.0 mg, 0.05 mmol, 1.0 eq) in HCl/EtOAc (4 M, 2.0 mL, 8.0 mmol, 167.62 eq) was stirred at 20°C for 2 h. The mixture was concentrated in vacuum and the remaining residue was purified by prep-HPLC to obtain the title compound (12.4 mg, 0.020 mmol, 47% yield) as yellow oil. MS (ESI): 529.5 [M+H]⁺.

### Example 2

### 2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-(trifluoromethoxy)phenyl] methyl]-3,4-dihydro-1-benzazepin-8-yl]-1,3,4-oxadiazol-2-yl]propanenitrile

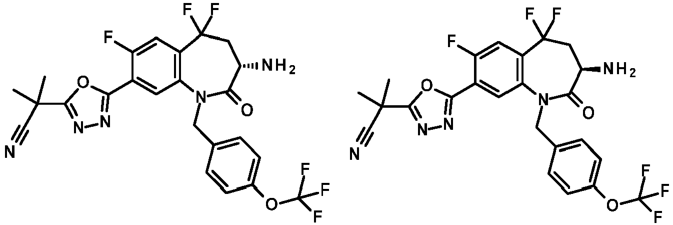

### Step a) tert-butyl N-[8-[[(2-cyano-2-methyl-propanoyl)amino]carbamoyl]-5,5, 7-trifluoro-2-oxo-1-[[4-(trifluoromethoxy)phenyl]methyl]-3,4-dihydro-1-benzazepin-3-yl]carbamate

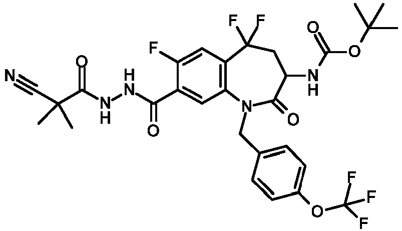

To a solution of 2-cyano-2,2-dimethylacetic acid (0.05 mL, 0.51 mmol, 1.7 eq) in THF (1 mL) were added DIPEA (0.13 mL, 0.91 mmol, 3.0 eq) and T3P (345.46 mg, 0.45 mmol, 1.5 eq) and the mixture was stirred at 15°C for 15 min. Then tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate (Example 1, step j) (170.0 mg, 0.3 mmol, 1.0 eq) was added at 15°C and the reaction mixture was stirred at 20°C for 16 h. The reaction mixture was concentrateed under vacuum to remove THF, then it was diluted with EtOAc (20 mL), washed with H₂O (10 mL), brine (10 mL x 3) and dried over anhydrous Na₂SO₄. The organic layer was concentrated under vacuum to obtain a yellow oil (310.0 mg) containing the title compound which was used without further purification in the next reaction step. MS (ESI): 602.1 [M-isobutene+H]⁺.

### Step b) tert-butyl N-[8-[5-(1-cyano-1-methyl-ethyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamte

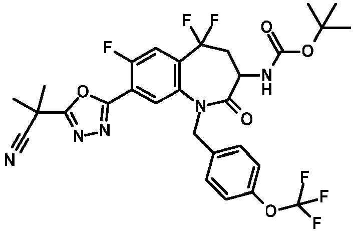

The title compound was prepared in analogy to Example 1, step 1 from tert-butyl N-[8-[[(2-cyano-2-methyl-propanoyl)amino]carbamoyl]-5,5,7-trifluoro-2-oxo-1-[[4-(trifluoromethoxy)phenyl]methyl]-3,4-dihydro-1-benzazepin-3-yl]carbamate (310.0 mg, 0.47 mmol) and was obtained as yellow oil (120.0 mg, 0.19 mmol, 36% yield). MS (ESI): 584.1 [M-isobutene+H]⁺.

### Step c) 2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile and 2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile

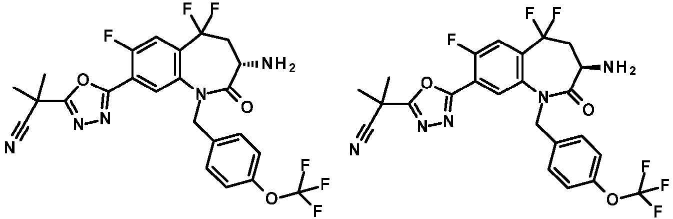

A solution of tert-butyl N-[8-[5-(1-cyano-1-methyl-ethyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate (60.0 mg, 0.09 mmol, 1.0 eq) in HCl/EtOAc (4 M, 4.0 mL, 16.0 mmol, 170.55 eq) was stirred at 20°C for 0.5 h. The mixture was concentrated under vacuum and the remaining residue was purified by preparative-HPLC (neutral) to obtain pure 2-[5-[3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]-2-methylpropanenitrile. This was subjected to chiral SFC to obtain 2-methyl-2-[5-[3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile [isomer B] (8.1 mg, 0.02 mmol, 16% yield) (MS (ESI): 540.1 [M+H]⁺, retention time 1.40 minutes (see conditions below)) and 2-methyl-2-[5-[3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile [isomer A] (8.5 mg, 0.02 mmol, 17% yield) as a white powder. MS (ESI): 540.1 [M+H]⁺; retention time 1.05 minutes (column: Chiralcel OD-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: PDA; column temperatur: 35°C; back pressure: 100 bar).

### Example 3

### (3S)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one and

### (3R)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one

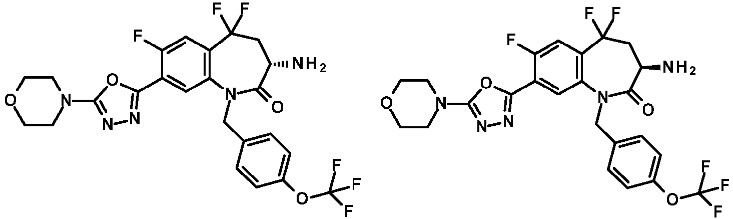

### Step a) tert-butyl N-[5,5,7-trifluoro-8-[(morpholine-4-carbonylamino)carbamoyl]-2-oxo-1-[[4-(trifluoromethoxy)phenyl]methyl]-3,4-dihydro-1-benzazepin-3-yl]carbamate

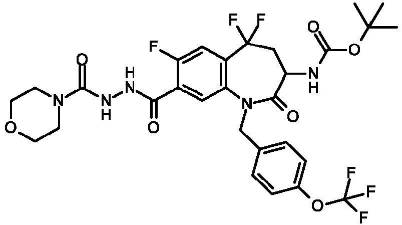

To a solution tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate (Example 1, step j) (100.0 mg, 0.18 mmol, 1.0 eq) in DCM (1 mL) were added pyridine (0.5 mL, 6.18 mmol, 34.77 eq) and morpholine-4-carbonyl chloride (0.06 mL, 0.51 mmol, 2.89 eq) at 25°C and the mixture was stirred at 30°C for 16 h. The mixture was diluted with EtOAc (10 mL), washed with 1 N HCl (3 x 8 mL) and then concentrated under vacuum to obtain the title compound (130.0 mg, 0.19 mmol, 94% yield) as yellow oil which was used in the next reaction step without further purification. MS (ESI): 620.1 [M-isobutene+H]⁺.

### Step b) tert-butyl N-[5,5,7-trifluoro-8-(5-morpholino-1,3,4-oxadiazol-2-yl)-2-oxo-1-[[4-(trifluoromethoxy)phenyl]methyl]-3,4-dihydro-1-benzazepin-3-yl]carbamate

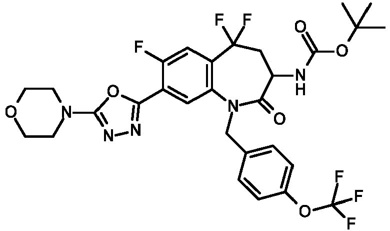

The title compound was prepared in analogy to Example 1, step 1 from tert-butyl N-[5,5,7-trifluoro-8-[(morpholine-4-carbonylamino)carbamoyl]-2-oxo-1-[[4-(trifluoromethoxy)phenyl]methyl]-3,4-dihydro-1-benzazepin-3-yl]carbamate (280.0 mg, 0.41 mmol) and was obtained as yellow oil (120.0 mg, 0.18 mmol, 44% yield). MS (ESI): 658.1 [M+H] ⁺.

### Step c) (3S)-3-amino-5,5,7-trifluoro-8-(5-morpholino-1, 3, 4-oxadiazol-2-yl)-1-[[4-(trifluoromethoxy)phenyl]methyl]-3,4-dihydro-1-benzazepin-2-one and (3R)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one

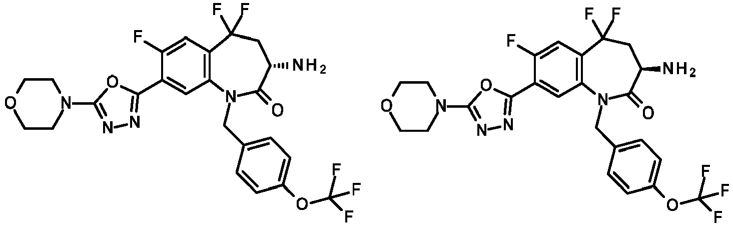

A solution of tert-butyl N-[5,5,7-trifluoro-8-(5-morpholino-1,3,4-oxadiazol-2-yl)-2-oxo-1-[[4-(trifluoromethoxy)phenyl]methyl]-3,4-dihydro-1-benzazepin-3-yl]carbamate (120.0 mg, 0.18 mmol, 1.0 eq) in HCl/EtOAc (4 M, 6.0 mL, 24.0 mmol, 131.51 eq) was stirred at 20°C for 1 h. The mixture was concentrated under vacuum and the remaining residue was purified by preparative-HPLC (neutral) to obtain pure 3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one. This was subjected to chiral SFC to obtain (3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one [isomer B] (16.7 mg, 0.03 mmol, 16% yield) (MS (ESI): 558.1 [M+H]⁺, retention time 1.53 minutes (see conditions below)) and (3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one [isomer A] (19.5 mg, 0.03 mmol, 19% yield) as a white powder. MS (ESI): 540.1 [M+H]⁺; retention time 1.86 minutes (column: Chiralpak AD-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: PDA; column temperatur: 35°C; back pressure: 100 bar).

### Example 4

### (3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one and

### (3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one

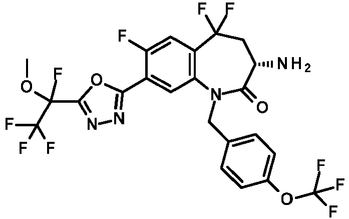

### Step a) (3S)-3-(tert-butoxycarbonylamino)-5,5, 7-trifluoro-2-oxo-1-[[4-(trifluoromethoxy)phenyl]methyl]-3,4-dihydro-1-benzazepine-8-carboxylic acid and (3R)-3-(tert-butoxycarbonylamino)-5,5,7-trifluoro-2-oxo-1-[[4-(trifluoromethoxy)phenyl]methyl]-3,4-dihydro-1-benzazepine-8-carboxylic acid

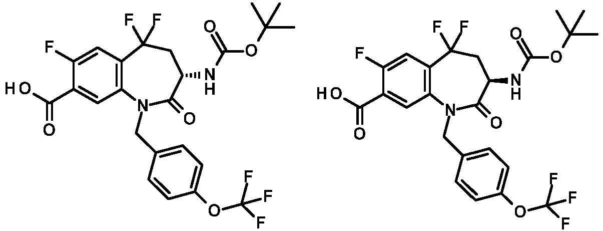

To a mixture of tert-butyl N-[8-bromo-5,5,7-trifluoro-2-oxo-1-[[4-(trifluoromethoxy)phenyl]methyl]-3,4-dihydro-1-benzazepin-3-yl]carbamate (Example 1, step 1) (490.0 mg, 0.84 mmol, 1.0 eq) 1,3-bis-(diphenylphosphino)propane (69.29 mg, 0.17 mmol, 0.2 eq), palladium diacetate (18.86 mg, 0.08 mmol, 0.1 eq) in a mixture of DMF (10 mL) and water (2 mL) was added TEA (0.35 mL, 2.52 mmol, 3.0 eq) and then the mixture was degassed with nitrogen for three times, then charged with CO for three times and then the mixture was stirred at 80°C for 16 h under CO (45 psi). The mixture was diluted with 1 N HCl (10 mL) and extracted with EtOAc (3 x 15 mL). The orgnanic extracts were dried over Na₂SO₄ and concentrated under vacuum and the remaining residue was purified by prep-HPLC (HCl) to obtain pure 3-(tert-butoxycarbonylamino)-5,5,7-trifluoro-2-oxo-1-[[4-(trifluoromethoxy)phenyl]methyl]-3,4-dihydro-1-benzazepine-8-carboxylic acid. This was subjected to chiral SFC to obtain (3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer B] (67.1 mg, 0.12 mmol, 15% yield) (MS (ESI): 492.9 [M-isobutene+H]⁺, retention time 1.47 minutes (see conditions below)) as yellow powder and (3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer A] (63.7 mg, 0.12 mmol, 14% yield) as white powder. MS (ESI): 492.9 [M-isobutene+H]⁺, retention time 1.19 minutes (column: Chiralpak IG-3 50×4.6 mm I.D., 3 µm; mobile phase: phase A for CO₂, and phase B for IPA (0.05%DEA); gradient elution: IPA (0.05% DEA) in CO₂ from 5% to 40%; flow rate: 3 mL/min; detector: DAD; column temperatur: 35°C; back pressure: 100 bar).

### Step b) tert-butyl N-[(3S)-5,5,7-trifluoro-2-oxo-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-3-yl]carbamate and tert-butyl N-[(3R)-5,5,7-trifluoro-2-oxo-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-3-yl]carbamate

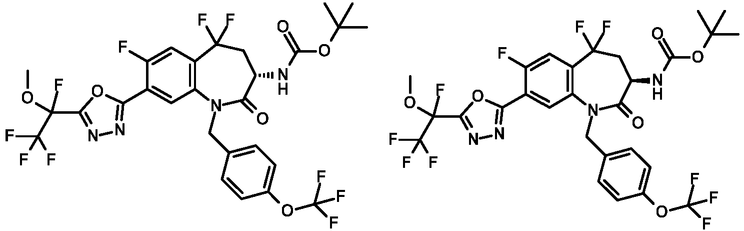

A mixture of (3-(tert-butoxycarbonylamino)-5,5,7-trifluoro-2-keto-1-[4-(trifluoromethoxy)benzyl]-3,4-dihydro-1-benzazepine-8-carboxylic acid [isomer A] (63 mg, 0.114 mmol, 1 eq), 2,3,3,3-tetrafluoro-2-methoxy-propionohydrazide (Intermediate 1) (29.03 mg, 0.148 mmol, 1.3 eq) and DIPEA (29.45 mg, 39.01 uL, 0.228 mmol, 2 eq) in THF (0.940 mL) was stirred at RT. HATU (64.99 mg, 0.171 mmol, 1.5 eq) was added and stirring was continued for 40 min. The solution was concentrated to give a light yellow foam which was dissolved in THF (0.940 mL) and Burgess Reagent (135.78 mg, 0.570 mmol, 5 eq) was added in one portion and the mixture was stirred for 1 h. The mixture was diluted with EtOAc and water and extracted with EtOAc. The combined organic phases were dried over Na₂SO₄, filtered and concentrated and the remaining residue was purified by column chromatography on silica gel (0% to 30% EtOAc in heptane) to give tert-butyl N-[5,5,7-trifluoro-2-oxo-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-3-yl]carbamate [isomer A] (54 mg, 68%) as colorless solid. MS (ESI): 647.3 [M-isobutene+H]⁺. tert-butyl N-[5,5,7-trifluoro-2-oxo-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-3-yl]carbamate [isomer B] was obtained in analogy from (3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer B]. MS (ESI): 647.1 [M-isobutne+H]⁺.

### Step c) (3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one and (3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one

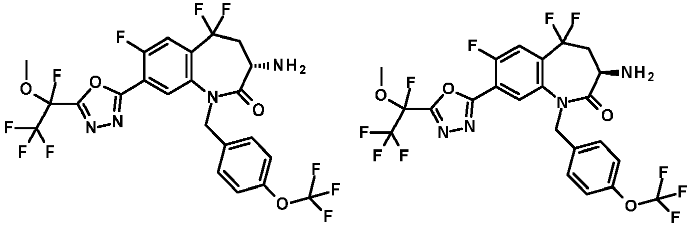

N-[5,5,7-trifluoro-2-keto-8-[5-(1,2,2,2-tetrafluoro-1-methoxy-ethyl)-1,3,4-oxadiazol-2-yl]-1-[4-(trifluoromethoxy)benzyl]-3,4-dihydro-1-benzazepin-3-yl]carbamic acid tert-butyl ester [isomer A] (50 mg, 0.071 mmol, 1 eq) was stirred with HCl (2M in Et₂O) (53.38 uL, 0.107 mmol, 1.5 eq) in 1,1,1,3,3,3-hexafluoro-2-propanol (3.56 mL) at RT for 2 h. The reaction mixture was concentrated in vacuo. The solid was suspended in DCM and again concentrated. This process was repeated three times to afford the title compound as hydrochloride salt (45 mg, 97%) as off-white solid after drying in high vacuum. MS (ESI): 603.2 [M+H]⁺. 3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one [isomer B] was obtained in analogy from 3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer B] as an off-white solid. MS (ESI): 603.2 [M+H]⁺.

### Example 5

### (3S)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one

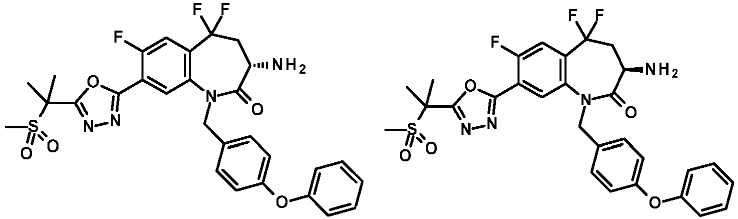

### Step a) tert-butyl N-[8-bromo-7-fluoro-2,5-dioxo-1-[(4-phenoxyphenyl)methyl]-3,4-dihydro-1-benzazepin-3-yl]carbamate

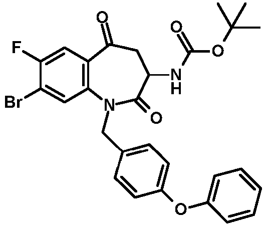

The title compound was prepared in analogy to Example 1, step f from tert-butyl N-(8-bromo-7-fluoro-2,5-dioxo-3,4-dihydro-1H-1-benzazepin-3-yl)carbamate (1000.0 mg, 2.58 mmol) and 1-(chloromethyl)-4-phenoxy-benzene (700.0 mg, 3.2 mmol, 1.24 eq) and was obtained as light yellow oil (1.6 g) containing the title compound.. MS (ESI): 513.0 [M-isobutene+H]⁺.

### Step b) tert-butyl N-[8-bromo-5,5,7-trifluoro-2-oxo-1-[(4-phenoxyphenyl)methyl]-3,4-dihydro-1-benzazepin-3-yl]carbamate

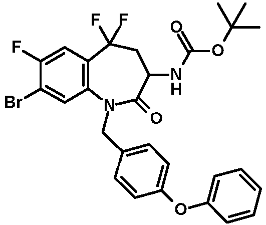

To tert-butyl N-[8-bromo-7-fluoro-2,5-dioxo-1-[(4-phenoxyphenyl)methyl]-3,4-dihydro-1-benzazepin-3-yl]carbamate (100.0 mg, 0.18 mmol, 1.0 eq) was added dropwise DAST (5.0 mL) at 0-10°C under an atmosphere of nitrogen and the mixture was stirred at 25°C for 12 h. The reaction was quenched by addition of water (20 mL) and was extracted with EtOAc (2 x 20 mL). The combined extracts were dried over Na₂SO₄ and concentrated. The remeining residue was purified by preparative TLC (PE/EtOAc = 5:1) to obtain the title compound (50.0 mg, 0.08 mmol, 54% yield) as a light yellow solid. MS (ESI): 593.1 [M+H]⁺.

### Step c) 3-(tert-butoxycarbonylamino)-5,5,7-trifluoro-2-oxo-1-[(4-phenoxyphenyl)methyl]-3,4-dihydro-1-benzazepine-8-carboxylic acid

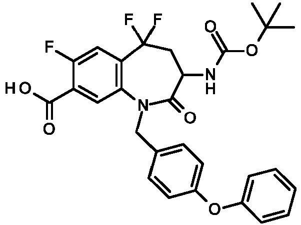

To a mixture of tert-butyl N-[8-bromo-5,5,7-trifluoro-2-oxo-1-[(4-phenoxyphenyl)methyl]-3,4-dihydro-1-benzazepin-3-yl]carbamate (250.0 mg, 0.42 mmol, 1.0 eq), DPPP (35.0 mg, 0.08 mmol, 0.2 eq) and TEA (130.0 mg, 1.28 mmol, 3.04 eq) in a mixture of DMF (10 mL) and water (2 mL) was added Pd(OAc)₂ (10.0 mg, 0.04 mmol, 0.11 eq) at 25°C. The mixture was degassed with nitrogen for three times, charged with CO for three times, and then stirred at 80°C for 12 h under CO (45 psi). The mixture was poured into water (50 mL) and the pH was adjusted to 6 by addition of HCl (0.5 M). The mixture was extracted with EtOAc (100 mL) and the organic phase was washed with brine (100 mL), dried (Na₂SO₄) and concentrated under vacuum to give the title compound (160.0 mg, 0.29 mmol, 55% yield) as a light yellow solid and was used for next reaction step without further purification. MS (ESI): 557.1 [M+H]⁺.

### Step d) tert-butyl N-[5,5,7-trifluoro-8-(hydrazinecarbonyl)-2-oxo-1-[(4-phenoxyphenyl)methyl]-3,4-dihydro-1-benzazepin-3-yl]carbamate

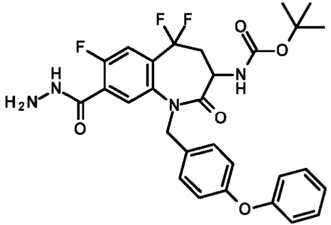

The title compound was prepared in analogy to Example 1, step j from 3-(tert-butoxycarbonylamino)-5,5,7-trifluoro-2-oxo-1-[(4-phenoxyphenyl)methyl]-3,4-dihydro-1-benzazepine-8-carboxylic acid (150.0 mg, 0.27 mmol, 1.0 eq) and was obtained as light yellow solid (120 mg, 0.180 mmol, 69% yield). MS (ESI): 515.2 [M-isobutene+H]⁺.

### Step e) tert-butyl N-[5,5,7-trifluoro-8-[[(2-methyl-2-methylsulfonyl-propanoyl)amino]carbamoyl]-2-oxo-1-[(4-phenoxyphenyl)methyl]-3,4-dihydro-1-benzazepin-3-yl]carbamate

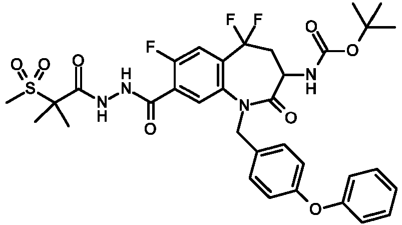

The title compound was prepared in analogy to Example 1, step k from tert-butyl N-[5,5,7-trifluoro-8-(hydrazinecarbonyl)-2-oxo-1-[(4-phenoxyphenyl)methyl]-3,4-dihydro-1-benzazepin-3-yl]carbamate (110.0 mg, 0.19 mmol, 1.0 eq) and was obtained as light yellow solid (120.0 mg, 0.17 mmol, 80% yield) which was used in the next reaction step without further purification. MS (ESI): 719.3 [M+H]⁺.

### Step f) tert-butyl N-[(3S)-5,5, 7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate

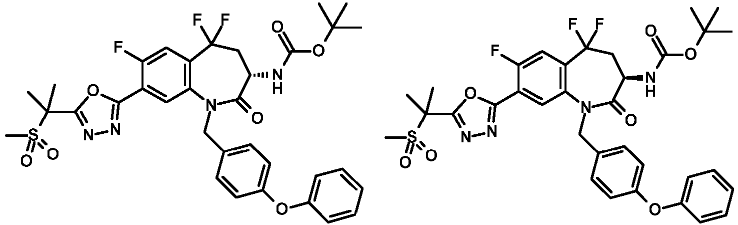

A solution of Burgess reagent (220.0 mg, 0.92 mmol, 6.03 eq) and tert-butyl N-[5,5,7-trifluoro-8-[[(2-methyl-2-methylsulfonyl-propanoyl)amino]carbamoyl]-2-oxo-1-[(4-phenoxyphenyl)methyl]-3,4-dihydro-1-benzazepin-3-yl]carbamate (110.0 mg, 0.15 mmol, 1.0 eq) in toluene (10 mL) was stirred at 110°C for 16 h. The reaction mixture was poured into water (20 mL) and extracted with EtOAc (40 mL). The organic phase was washed with brine (40 mL), dried (Na₂SO₄) and concentrated under vacuum. The remaining residue was purified by preparative-TLC (PE/EtOAc = 2: 1) to give pure tert-butyl N-[5,5,7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate (80.0 mg, 0.11 mmol, 72% yield) as a light yellow solid (MS (ESI): 701.4 [M+H] ⁺). This material was subjected to chiral SFC to obtain tert-butyl N-[5,5,7-trifluoro-8-[5-(1-methyl-1-methylsulfonylethyl)-1,3,4-oxadiazol-2-yl]-2-oxo-1-[(4-phenoxyphenyl)methyl]-3,4-dihydro-1-benzazepin-3-yl]carbamate [isomer B] (29.0 mg, 0.04 mmol, 34% yield) (MS (ESI): 701.4 [M+H] ⁺, retention time 1.33 minutes (see conditions below)) as light yellow solid and tert-butyl N-[5,5,7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer A] (28.0 mg, 0.04 mmol, 34% yield) as light yellow solid. MS (ESI): 701.4 [M+H]⁺, retention time 1.03 minutes (column: Chiralpak AS-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: PDA; column temperatur: 35°C; back pressure: 100 bar).

### Step g) (3S)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one and (3R)-3-amino-5,5, 7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one

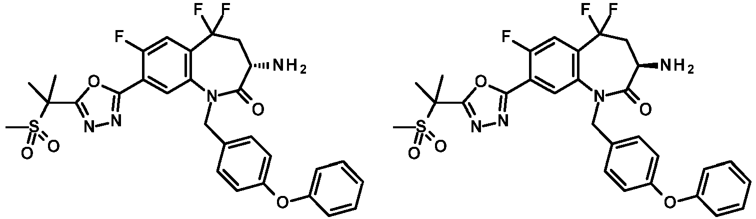

A solution of tert-butyl N-[,5,7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer A] (25.0 mg, 0.04 mmol, 1.0 eq) in HCl/EtOAc (4 M, 5.0 mL, 20.0 mmol, 560.58 eq) was stirred at 20°C for 0.5 h. The reaction mixture was concentrated and the remaining solid was dissloved in H₂O to obtain 3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one [isomer A] after lyophilization as hydrochloride salt (20.7 mg, 0.03 mmol, 91 % yield) as a white solid. MS (ESI): 601.4 [M+H]⁺. Chiral SFC: retention time 2.0 minutes. tert-Butyl N-[5,5,7-trifluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer B] was obtained in analogy. MS (ESI): 601.4 [M+H]⁺. Chiral SFC: retention time 2.32 minutes (column: Chiralcel OD-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: DAD; column temperatur: 35°C; back pressure: 100 bar).

### Example 6

### (3S)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one and

### (3R)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one

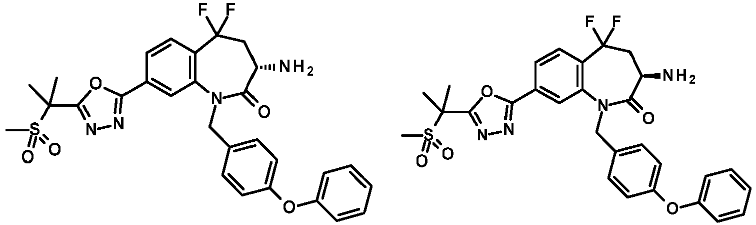

### Step a) 1-(2-amino-4-bromo-phenyl)-2-chloro-ethanone

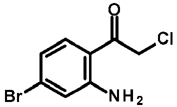

To a solution of BCl₃ (1 M in DCM, 63.95 mL, 63.95 mmol, 1.1 eq) in toluene (100 mL) was added 3-bromo aniline (6.33 mL, 58.13 mmol, 1.0 eq) dropwise at 0-5°C under an atmosphere of nitrogen. Then AlCl₃ (9.3 g, 69.76 mmol, 1.2 eq) and chloroacetonitrile (4.78 mL, 75.57 mmol, 1.3 eq) were added and the mixture was stirred at 0-5°C for 0.5 h and then at 70°C for 12 h. The reaction mixture was quenched by slow addition of 100 mL HCl (1 M ) at 50°C and then stirred at 50°C for 0.5 h. The mixture was poured into water (100 mL) and extracted with EtOAc (2 x 200 mL). The combined extracts were washed with brine (100 mL), dried over Na₂SO₄, filtered, and concentrated under vacuum. The remaining residue was purified first by column chromatography on silica gel (PE/EtOAc = 1:0 to 10:1) and then by preparative HPLC (XB-CN 10µm, hexane/EtOH) to give the title compound (4.73 g) as yellow solid. MS (ESI): 250.0 [M+H]⁺.

### Step b) diethyl 2-acetamido-2-[2-(2-amino-4-bromo-phenyl)-2-oxo-ethyl]propanedioate

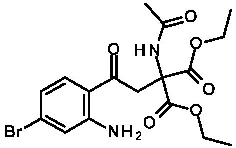

The title compound was prepared in analogy to Example 1, step b from 1-(2-amino-4-bromophenyl)-2-chloro-ethanone (9.28 g) and was obtained as green oil (8.7 g). MS (ESI): 431.0 [M+H]⁺.

### Step c) 2-amino-4-(2-amino-4-bromo-phenyl)-4-oxo-butanoic acid

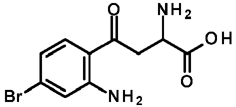

A solution of diethyl 2-acetamido-2-[2-(2-amino-4-bromo-phenyl)-2-oxo-ethyl]propanedioate (5.5 g, 12.81 mmol, 1.0 eq) in HCl (12 M, 60.0 mL, 720.0 mmol, 56.19 eq) was stirred at 100°C for 12 h. The reaction mixture was concentrated under vacuum to give the title compound as hydrochloride salt (3.7 g) as green oil which was used in the next reaction step without further purification. MS (ESI): 289.0 [M+H]⁺.

### Step d) 4-(2-amino-4-bromo-phenyl)-2-(tert-butoxycarbonylamino)-4-oxo-butanoic acid

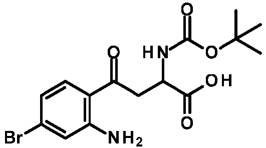

The title compound was prepared in analogy to Example 1, step d from 2-amino-4-(2-amino-4-bromo-phenyl)-4-oxo-butanoic acid (2.0 g, 6.97 mmol, 1.0 eq) and was obtained as light yellow oil (2.51 g, 6.48 mmol, 93% yield). MS (ESI): 333.1 [M-isobutene+H]⁺.

### Step e) tert-butyl N-(8-bromo-2,5-dioxo-3,4-dihydro-1H-1-benzazepin-3-yl)carbamate

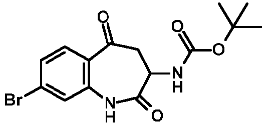

The title compound was prepared in analogy to Example 1, step e from 4-(2-amino-4-bromophenyl)-2-(tert-butoxycarbonylamino)-4-oxo-butanoic acid (2.6 g, 6.71 mmol, 1.0 eq) and was obtained as light yellow solid (800.0 mg, 2.17 mmol, 29% yield). MS (ESI): 271.0 [M-isobutene-CO₂+H]⁺.

### Step f) tert-butyl N-[8-bromo-2, 5-dioxo-1-[(4-phenoxyphenyl)methyl]-3, 4-dihydro-1-benzazepin-3-yl]carbamate

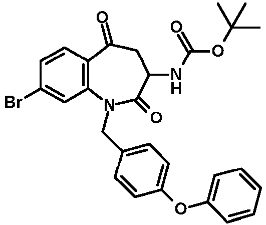

The title compound was prepared in analogy to Example 1, step f from tert-butyl N-(8-bromo-2,5-dioxo-3,4-dihydro-1H-1-benzazepin-3-yl)carbamate (650 mg, 1.76 mmol, 1.0 eq) and 1-(chloromethyl)-4-phenoxy-benzene (385 mg, 1.76 mmol, 1.0 eq) and was obtained as light yellow solid (350.0 mg, 0.635 mmol, 36% yield). MS (ESI): 497.0 [M-isobutene+H]⁺.

### Step g) tert-butyl N-[8-bromo-5,5-difluoro-2-oxo-1-[(4-phenoxyphenyl)methyl]-3,4-dihydro-1-benzazepin-3-yl]carbamate

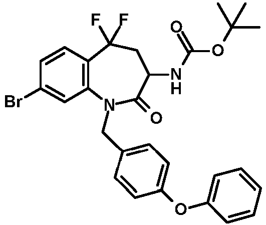

The title compound was prepared in analogy to Example 1, step g from tert-butyl N-[8-bromo-2,5-dioxo-1-[(4-phenoxyphenyl)methyl]-3,4-dihydro-1-benzazepin-3-yl]carbamate (350.0 mg, 0.63 mmol, 1.0 eq). A light yellow oil (400 mg) containing the title compound was obtained which was used in the next reaction step without further purification. MS (ESI): 475.0 [M-isobutene-CO₂+H]⁺.

### Step h) 3-(tert-butoxycarbonylamino)-5,5-difluoro-2-oxo-1-[(4-phenoxyphenyl)methyl]-3,4-dihydro-1-benzazepine-8-carboxylic acid

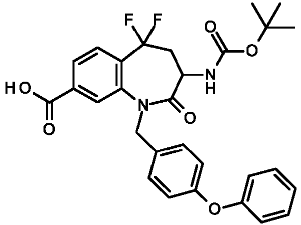

To a solution of tert-butyl N-[8-bromo-5,5-difluoro-2-oxo-1-[(4-phenoxyphenyl)methyl]-3,4-dihydro-1-benzazepin-3-yl]carbamate (200.0 mg, 0.35 mmol, 1.0 eq) and DIPEA (90.16 mg, 0.70 mmol, 2.0 eq) in a mixture of THF (8 mL) and water (2 mL) was added Pd(dppp)Cl₂ (61.71 mg, 0.105 mmol, 0.3 eq) at 25°C. The mixture was degassed with nitrogen for three times, then charged with CO for three times and then the mixture was stirred at 80°C for 12 h under carbon monoxide (50 psi). The reaction mixture was filtered and the filtrated was poured into water (20 mL). The pH was carefully adjusted to 3-4 by addition of 0.5 N HCl and the mixture extracted with EtOAc (3 x 30 mL). The combined extracts were washed with brine (10 mL), dried over Na₂SO₄, filtered and concentrated under vacuum. The remaining brown oil (200 mg) containg the title compound was used in the next reaction step without further purification. MS (ESI): 483.2 [M-isobutene+H]⁺.

### Step i) tert-butyl N-[5,5-difluoro-8-(hydrazinecarbonyl)-2-oxo-1-[(4-phenoxyphenyl)methyl]-3,4-dihydro-1-benzazepin-3-yl]carbamate

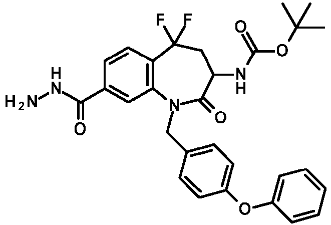

The title compound was prepared in analogy to Example 1, step j from 3-(tert-butoxycarbonylamino)-5,5-difluoro-2-oxo-1-[(4-phenoxyphenyl)methyl]-3,4-dihydro-1-benzazepine-8-carboxylic acid (270.0 mg, 0.5 mmol, 1.0 eq). A light yellow solid (260.0 mg) was obtained which was used in the next reaction step without further purification. MS (ESI): 497.2 [M-isobutene+H]⁺.

### Step j) tert-butyl N-[5,5-difluoro-8-[[(2-methyl-2-methylsulfonyl-propanoyl)amino]carbamoyl]-2-oxo-1-[(4-phenoxyphenyl)methyl]-3,4-dihydro-1-benzazepin-3-yl]carbamate

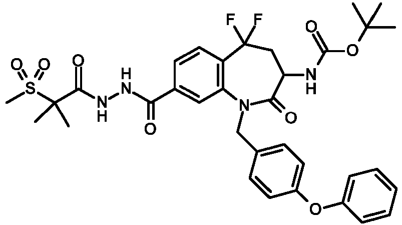

The title compound was prepared in analogy to Example 1, step k from tert-butyl N-[5,5-difluoro-8-(hydrazinecarbonyl)-2-oxo-1-[(4-phenoxyphenyl)methyl]-3,4-dihydro-1-benzazepin-3-yl]carbamate (260.0 mg, 0.47 mmol, 1.0 eq) and 2-methyl-2-methylsulfonyl-propanoic acid (93.84 mg, 0.56 mmol, 1.2 eq). A yellow oil (270 mg) containing the title compound was obtained which was used in the next reaction step without further purification. MS (ESI): 701.2 [M+H]⁺.

### Step k) tert-butyl N-[5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate

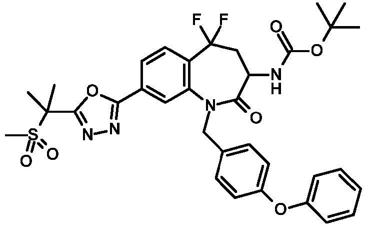

The title compound was prepared in analogy to Example 1, step 1 from tert-butyl N-[5,5-difluoro-8-[[(2-methyl-2-methylsulfonyl-propanoyl)amino]carbamoyl]-2-oxo-1-[(4-phenoxyphenyl)methyl]-3,4-dihydro-1-benzazepin-3-yl]carbamate (260.0 mg, 0.37 mmol, 1.0 eq) and was obtained as light yellow solid (170.0 mg, 0.25 mmol, 67% yield). MS (ESI): 627.3 [M-isobutene+H]⁺.

### Step 1) tert-butyl N-[(3S)-5,5-difluoro-8-[5-(1-methyl-1-methylsulfonyl-ethyl)-1,3,4-oxadiazol-2-yl]-2-oxo-1-[(4-phenoxyphenyl)methyl]-3, 4-dihydro-1-benzazepin-3-yl]carbamate and tert-butyl N-[(3R)-5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate

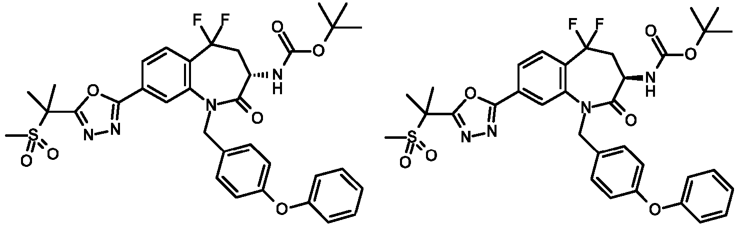

tert-butyl N-[5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate (100 mg) was subjected to preparative SFC (Daicel Chiralpak AS (250 mm x 30 mm,10 µm), 0.1% NH₃-H₂O in MeOH) to obtain tert-butyl N-[5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer B] (45 mg, 0.07 mmol) (MS (ESI): 627.2 [M-isobutene+H]⁺, retention time 1.35 minutes (see conditions below) as light yellow oil and obtain tert-butyl N-[5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer A] (45 mg, 0.07 mmol) as light yellow oil. MS (ESI): 627.2 [M-isobutene+H]⁺, retention time 1.09 minutes (column: Chiralpak AS-3 50×4.6 mm I.D., 3 µm; mobile phase: phase A for CO₂, and phase B for MeOH (0.05%DEA); gradient elution: MeOH (0.05% DEA) in CO₂ from 5% to 40%; flow rate: 3 mL/min; detector: PDA; column temperatur: 35°C; back pressure: 100 bar).

### Step m) (3S)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one and (3R)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one

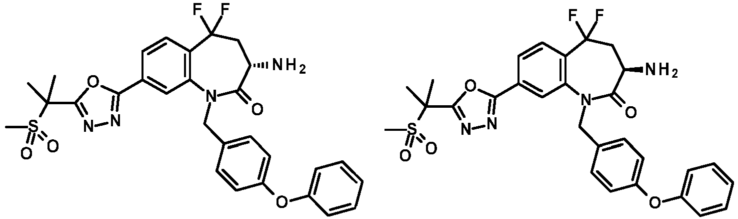

A solution of tert-butyl N-[5,5-difluoro-8-[5-(1-methyl-1-methylsulfonyl-ethyl)-1,3,4-oxadiazol-2-yl]-2-oxo-1-[(4-phenoxyphenyl)methyl]-3,4-dihydro-1-benzazepin-3-yl]carbamate [isomer A] (40.0 mg, 0.06 mmol, 1.0 eq) in HCl/EtOAc (4 M, 4.0 mL, 20.0 mmol, 560.58 eq) was stirred at 20°C for 0.5 h. The reaction mixture was concentrated and the remaining solid was dissloved in H₂O and lyophilized. The solid that was obtained was purified by preparative HPLC (HCl as additive in the eluent) to obtain 3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one [isomer A] after lyophilization as hydrochloride salt (8.9 mg, 0.01 mmol, 26% yield) as a white solid. MS (ESI): 583.3 [M+H]⁺. Chiral SFC: retention time 2.09 minutes. 3-amino-5,5-difluoro-8-[5-(1-methyl-1-methylsulfonylethyl)-1,3,4-oxadiazol-2-yl]-1-[(4-phenoxyphenyl)methyl]-3,4-dihydro-1-benzazepin-2-one [isomer B] was obtained in analogy. MS (ESI): 583.2 [M+H]⁺. Chiral SFC: retention time 2.43 minutes (column: Chiralcel OD-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: PDA; column temperatur: 35°C; back pressure: 100 bar).

### Example 7

### (3S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one and

### (3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one

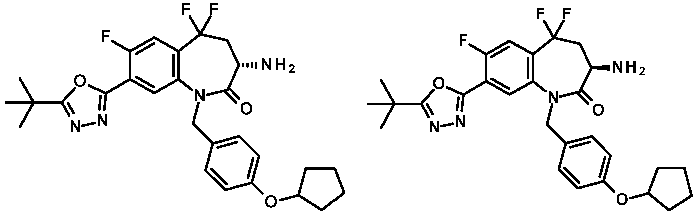

### Step a) tert-butyl N-[8-(5-tert-butyl-1,3,4-oxadiazol-2-yl)-1-[[4-(cyclopentoxy)phenyl]methyl]-5,5,7-trifluoro-2-oxo-3, 4-dihydro-1-benzazepin-3-yl]carbamate

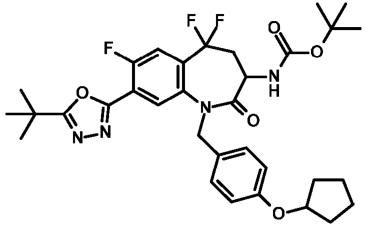

The title compound was prepared in analogy to Example 1, steps a to 1 (using 1-(bromomethyl)-4-(cyclopentoxy)benzene [CAS 1094272-95-3] instead of 4-(trifluoromethoxy)benzyl bromide in step f) and was obtained as white solid. MS (ESI): 629.2 [M+H]⁺.

### Step b) tert-butyl N-[(3S)-8-(5-tert-butyl-1,3,4-oxadiazol-2-yl)-1-[[4-(cyclopentoxy)phenyl]methyl]-5,5, 7-trifluoro-2-oxo-3,4-dihydro-1-benzazepin-3-yl]carbamate and tert-butyl N-[(3R)-8-(5-tert-butyl-1,3,4-oxadiazol-2-yl)-1-[[4-(cyclopentoxy)phenyl]methyl]-5,5,7-trifluoro-2-oxo-3,4-dihydro-1-benzazepin-3-yl]carbamate

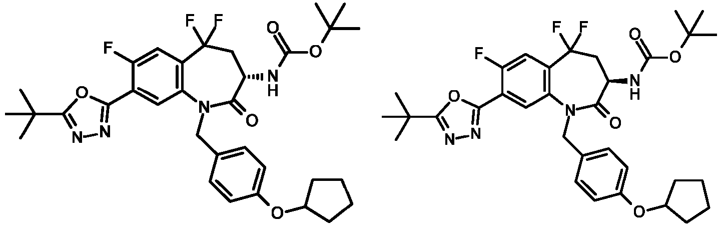

N-[8-(5-tert-butyl-1,3,4-oxadiazol-2-yl)-1-[[4-(cyclopentoxy)phenyl]methyl]-5,5,7-trifluoro-2-oxo-3,4-dihydro-1-benzazepin-3-yl]carbamate (35 mg) was subjected to preparative SFC (Daicel Chiralpak IC (250 mm x 30 mm,10 µm), 0.1% NH₃-H₂O in MeOH) to obtain tert-butyl N-[8-(5-tert-butyl-1,3,4-oxadiazol-2-yl)-1-[[4-(cyclopentoxy)phenyl]methyl]-5,5,7-trifluoro-2-oxo-3,4-dihydro-1-benzazepin-3-yl]carbamate [isomer B] (12 mg) (MS (ESI): 629.4 [M+H]⁺, retention time 1.19 minutes (see conditions below) as white solid and tert-butyl N-[8-(5-tert-butyl-1,3,4-oxadiazol-2-yl)-1-[[4-(cyclopentoxy)phenyl]methyl]-5,5,7-trifluoro-2-oxo-3,4-dihydro-1-benzazepin-3-yl]carbamate [isomer A] compound (14 mg) as white solid. MS (ESI): 629.4 [M+H]⁺, retention time 0.84 minutes (column: Chiralpak IC-3 50×4.6 mm I.D., 3 µm; mobile phase: phase A for CO₂, and phase B for MeOH (0.05% DEA); gradient elution: MeOH (0.05% DEA) in CO₂ from 5% to 40%; flow rate: 3 mL/min; detector: PDA; column temperatur: 35°C; back pressure: 100 bar).

### Step c) (3S)-3-amino-8-(5-tert-butyl-1,3,4-oxadiazol-2-yl)-1-[[4-(cyclopentoxy)phenyl]methyl]-5,5, 7-trifluoro-3, 4-dihydro-1-benzazepin-2-one and (3R)-3-amino-8-(5-tert-butyl-1, 3, 4-oxadiazol-2-yl)-1-[[4-(cyclopentoxy)phenyl]methyl]-5,5,7-trifluoro-3,4-dihydro-1-benzazepin-2-one

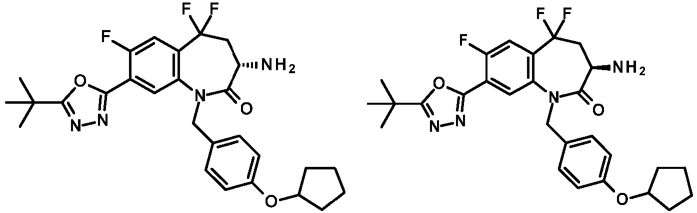

A solution of tert-butyl N-[8-(5-tert-butyl-1,3,4-oxadiazol-2-yl)-1-[[4-(cyclopentoxy)phenyl]methyl]-5,5,7-trifluoro-2-oxo-3,4-dihydro-1-benzazepin-3-yl]carbamate [isomer A] (13.0 mg, 0.02 mmol, 1.0 eq) in HCl/EtOH (4 M, 5.0 mL) was stirred at 20°C for 0.5 h. All volatiles were removed and the remaining solid was dissloved in water to obtain 3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one [isomer A] (11.2 mg, 0.02 mmol, 94% yield) as a light yellow solid as hydrochloride salt after lyophilization. MS (ESI): 529.3 [M+H]⁺, retention time 1.43 minutes. 3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one [isomer B] was obtained in analogy. MS (ESI): 529.3 [M+H]⁺, retention time 1.85 minutes (column: Chiralcel OD-3 50×4.6 mm I.D., 3 µm; mobile phase: phase A for CO₂, and phase B for MeOH (0.05% DEA); gradient elution: B in A from 5% to 40%; flow rate: 3 mL/min; detector: DAD; column temperatur: 35°C; back pressure: 100 bar).

### Example 8

### (3S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one

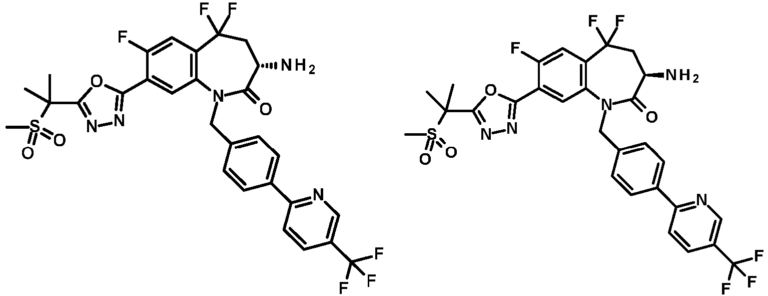

### Step a) tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate

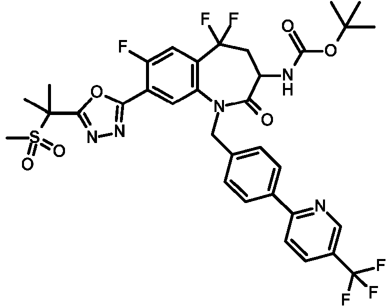

The title compound was prepared in analogy to Example 1, steps a to 1 (using 2-[4-(bromomethyl)phenyl]-5-(trifluoromethyl)pyridine [CAS 1056641-21-4]; hydrochloride instead of 4-(trifluoromethoxy)benzyl bromide in step f and 2-methyl-2-methylsulfonyl-propanoic acid instead of pivalic acid in step k) and was obtained as white solid. MS (ESI): 754.4 [M+H]⁺.

### Step b) tert-butyl N-[(3S)-5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate

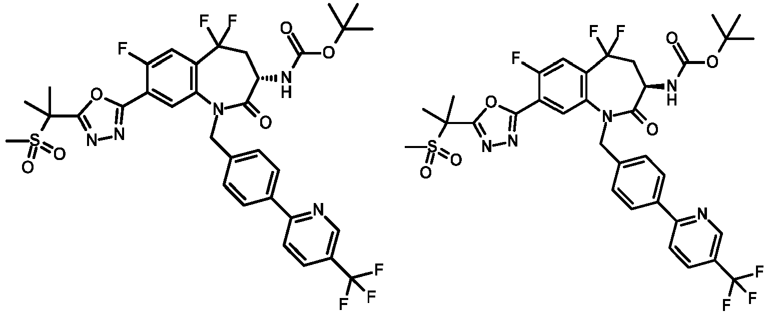

tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate (95 mg) was subjected to preparative SFC (Daicel Chiralpak IC (250 mm x 30 mm,10 µm), 0.1% NH₃-H₂O in IPA) to obtain tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate [isomer B] (28 mg) (MS (ESI): 754.3 [M+H]⁺, retention time 2.43 minutes (see conditions below) as white solid and tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate [isomer A] (30 mg) as white solid. MS (ESI): 754.3 [M+H]⁺, retention time 1.88 minutes (column: Chiralpak IC-3 50×4.6 mm I.D., 3 µm; mobile phase: phase A for CO₂, and phase B for IPA (0.05% DEA); gradient elution: IPA (0.05% DEA) in CO₂ from 5% to 40%; flow rate: 3 mL/min; detector: PDA; column temperatur: 35°C; back pressure: 100 bar).

### Step c) (3S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one and (3R)-3-amino-3,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one

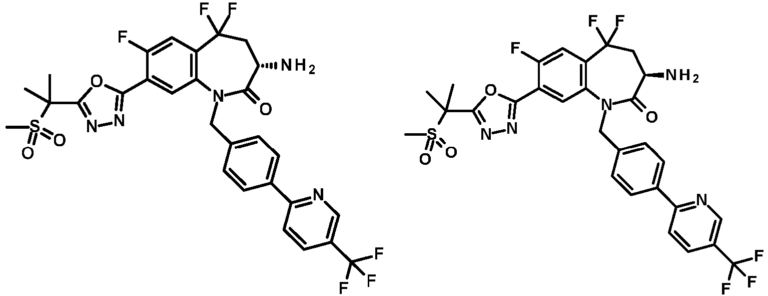

A solution of tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-2-pyridyl]phenyl]methyl]-3,4-dihydro-1-benzazepin-3-yl]carbamate [isomer A] (25.0 mg, 0.03 mmol, 1.0 eq) in HCl/EtOH (4 M, 6.25 mL) was stirred at 20°C for 0.3 h. All volatiles were removed and the remaining solid was dissloved in water to obtain 3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one [isomer A] (18.7 mg, 0.03 mmol, 75% yield) as a white solid as dihydrochloride salt after lyophilization. MS (ESI): 654.2 [M+H]⁺, retention time 1.82 minutes. 3-amino-5,5,7-trifluoro-8-[5-(1-methyl-1-methylsulfonylethyl)-1,3,4-oxadiazol-2-yl]-1-[[4-[5-(trifluoromethyl)-2-pyridyl]phenyl]methyl]-3,4-dihydro-1-benzazepin-2-one [isomer B] was obtained in analogy. MS (ESI): 654.2 [M+H]⁺, retention time 2.10 minutes (column: Chiralcel OD-3 50×4.6 mm I.D., 3 µm; mobile phase: phase A for CO₂, and phase B for MeOH (0.05% DEA); gradient elution: B in A from 5% to 40%; flow rate: 3 mL/min; detector: PDA; column temperatur: 35°C; back pressure: 100 bar).

### Example 9

### 2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile and

### 2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile

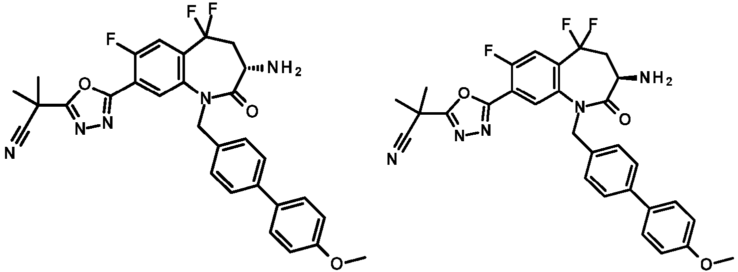

### Step a) tert-butyl N-[8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate

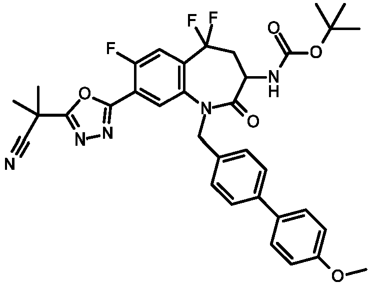

The title compound was prepared in analogy to Example 1, steps a to l (using 1-(chloromethyl)-4-(4-methoxyphenyl)benzene [CAS 93258-73-2] instead of 4-(trifluoromethoxy)benzyl bromide in step f and 2-cyano-2-methyl-propanoic acid instead of pivalic acid in step k) and was obtained as off-white solid. MS (ESI): 606.2 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate

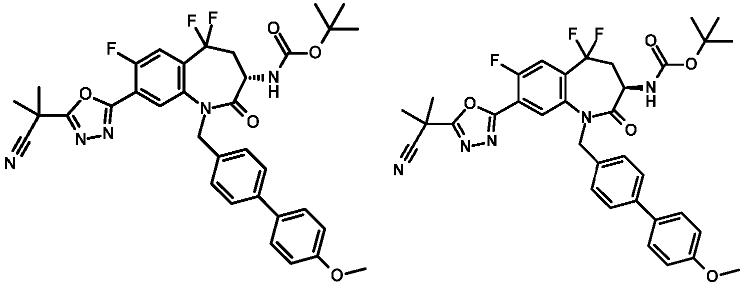

tert-butyl N-[8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate (80 mg) was subjected to preparative SFC (Daicel Chiralpak AD (250 mm x 30 mm, 10 µm), 0.1% NH₃-H₂O in IPA) to obtain tert-butyl N-[8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate [isomer B] (38 mg) (MS (ESI): 606.2 [M-isobutene+H]⁺, retention time 2.22 minutes (see conditions below) as yellow solid and tert-butyl N-[8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate [isomer A] (38 mg) as yellow solid. MS (ESI): 606.2 [M-isobutene+H]⁺, retention time 1.87 minutes (column: Chiralpak IG-3 50×4.6 mm I.D., 3 µm; mobile phase: phase A for CO₂, and phase B for EtOH (0.05%DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrileand2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile

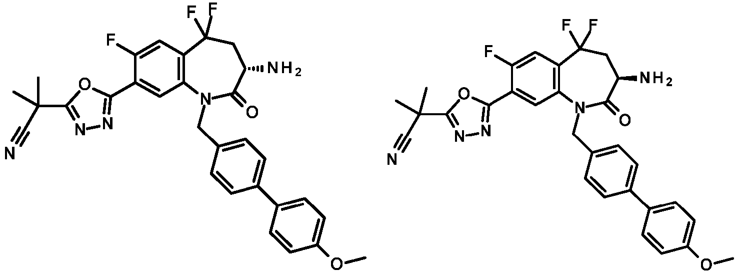

To a solution of tert-butyl N-[8-[5-(1-cyano-1-methyl-ethyl)-1,3,4-oxadiazol-2-yl]-5,5,7-trifluoro-1-[[4-(4-methoxyphenyl)phenyl]methyl]-2-oxo-3,4-dihydro-1-benzazepin-3-yl]carbamate [isomer A] (36.0 mg, 0.05 mmol, 1.0 eq) in EtOAc (1 mL) was added HCl/EtOAc (4 M, 8.0 mL, 32.0 mmol, 588.15 eq) at 20°C and the mixture was stirred at 20°C for 1 h. The reaction mixture was concentrated in vacuum and water was added to the remaining residue. 2-methyl-2-[5-[3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile [isomer A] (29.2 mg, 0.05 mmol, 84% yield) was obtained after lyophilization as off-white solid. MS (ESI): 562.1 [M+H]⁺, retention time 0.62 minutes. 2-methyl-2-[5-[3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile [isomer B] was obtained in analogy. MS (ESI): 562.1 [M+H]⁺, retention time 1.22 minutes (column: Chiralpak AD-3 50×4.6 mm I.D., 3 µm; mobile phase: phase A for CO₂, and phase B for MeOH/MeCN (0.05%DEA); gradient elution: 30% MeOH/MeCN (0.05% DEA) in CO₂; flow rate: 3 mL/min; detector: PDA; column temperatur: 35°C; back pressure: 100 bar).

### Example 10

### (3S)-3-amino-5,5,7-trifluoro-1-[[4-(4-methoxyphenyl)phenyl] methyl]-8-[5-(1-methyl-1-methylsulfonyl-ethyl)-1,3,4-oxadiazol-2-yl]-3,4-dihydro-1-benzazepin-2-one and

### (3R)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one

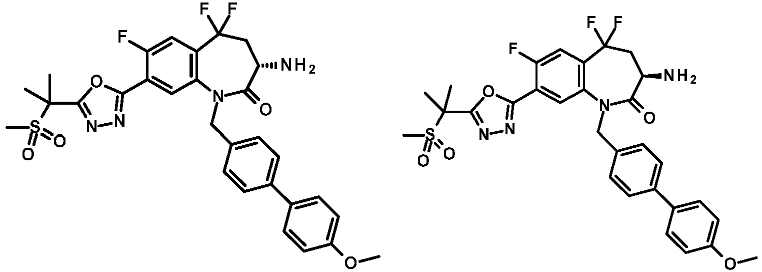

### Step a) tert-butyl N-[5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate

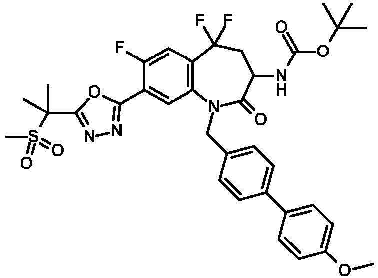

The title compound was prepared in analogy to Example 1, steps a to l (using 1-(chloromethyl)-4-(4-methoxyphenyl)benzene instead of 4-(trifluoromethoxy)benzyl bromide in step f and 2-methyl-2-methylsulfonyl-propanoic acid instead of pivalic acid in step k) and was obtained as white solid. MS (ESI): 659.2 [M-isobutene+H]⁺.

### Step b) tert-butyl N-[(3S)-5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamateand tert-butylN-[(3R)-5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate

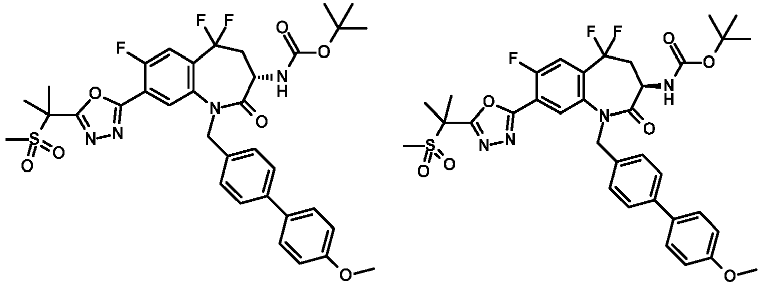

tert-butyl N-[5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate (60 mg) was subjected to preparative SFC (Daicel Chiralpak IG (250 mm x 30 mm, 10 µm), 0.1% NH₃-H₂O in MeCN/EtOH) to obtain tert-butyl N-[5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer B] (22 mg) (MS (ESI): 659.1 [M-isobutene+H]⁺, retention time 2.11 minutes (see conditions below) as yellow solid and tert-butyl N-[(5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer A] (22 mg) as yellow solid. MS (ESI): 659.1 [M-isobutene+H]⁺, retention time 0.95 minutes (column: Chiralpak IG-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) (3S)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one and (3R)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one

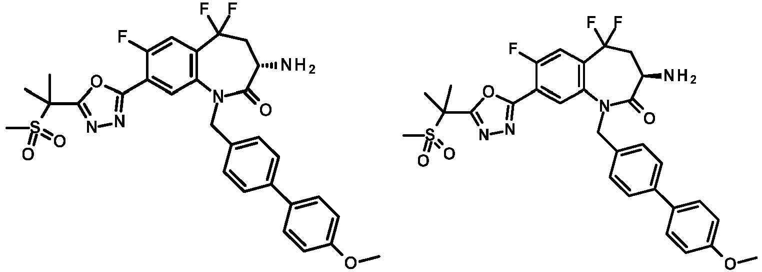

To a solution of tert-butyl N-[5,5,7-trifluoro-1-[[4-(4-methoxyphenyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer A] (20.0 mg, 0.03 mmol, 1.0 eq) in EtOAc (1 mL) was added HCl/EtOAc (4 M, 8.0 mL, 32.0 mmol, 1143.6 eq) dropwised at 20°C and the mixture was stirred at 20°C for 1 h. The reaction mixture was concentrated in vacuum and water was added to the remaining residue. 3-amino-5,5,7-trifluoro-1-[[4-(4-methoxyphenyl)phenyl]methyl]-8-[5-(1-methyl-1-methylsulfonylethyl)-1,3,4-oxadiazol-2-yl]-3,4-dihydro-1-benzazepin-2-one [isomer A] (14.6 mg, 0.02 mmol, 77% yield) was obtained after lyophilization as off-white solid. MS (ESI): 615.1 [M+H]⁺, retention time 0.97 minutes. 3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one [isomer B] was obtained in analogy. MS (ESI): 615.1 [M+H]⁺, retention time 1.45 minutes (column: Chiralpak AD-3 50×4.6 mm I.D., 3 µm; mobile phase: phase A for CO₂, and phase B for MeOH/MeCN (0.05% DEA); gradient elution: 30% MeOH/MeCN (0.05% DEA) in CO₂; flow rate: 3 mL/min; detector: PDA; column temperatur: 35°C; back pressure: 100 bar).

### Example 11

### 2-methyl-2-[5-[(3S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile

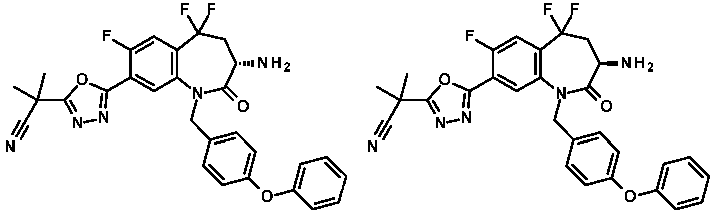

### Step a) tert-butyl N-[8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate

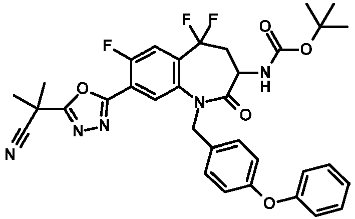

The title compound was prepared in analogy to Example 1, steps a to l (using 1-(chloromethyl)-4-phenoxy-benzene instead of 4-(trifluoromethoxy)benzyl bromide in step f and 2-cyano-2-methyl-propanoic acid instead of pivalic acid in step k) and was obtained as light yellow solid. MS (ESI): 592.3 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate

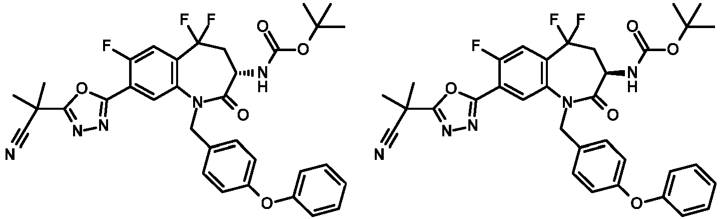

tert-butyl N-[8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate (140 mg) was subjected to preparative SFC (Daicel Chiralpak IC (250 mm x 30 mm, 10 µm), 0.1% NH₃-H₂O in MeOH) to obtain tert-butyl N-[8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate [isomer B] (70 mg) (MS (ESI): 592.3 [M-isobutene+H]⁺, retention time 2.39 minutes (see conditions below) as light yellow solid and tert-butyl N-[8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate [isomer A] (60 mg) as light yellow solid. MS (ESI): 592.3 [M-isobutene+H]⁻, retention time 1.75 minutes (column: Chiralpak IC-3 50×4.6 mm I.D., 3 µm; mobile phase: phase A for CO₂, and phase B for MeOH (0.05%DEA); gradient elution: from 5% to 40% MeOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-]-[(4-phenoxyphenyl)methyl]-3,4-dihydro-1-benzazepin-8-yl]-1,3,4-oxadiazol-2yl]propanenitrile and 2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile

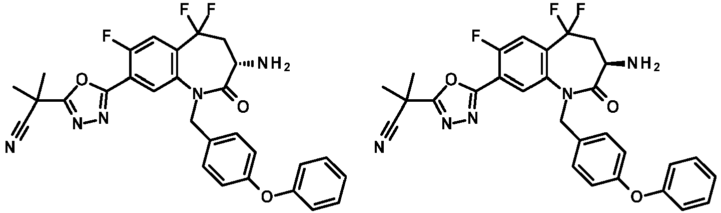

A solution of tert-butyl N-[8-[5-(1-cyano-1-methyl-ethyl)-1,3,4-oxadiazol-2-yl]-5,5,7-trifluoro-2-oxo-1-[(4-phenoxyphenyl)methyl]-3,4-dihydro-1-benzazepin-3-yl]carbamate [isomer A] (60.0 mg, 0.09 mmol, 1.0 eq) in HCl/EtOAc (4 M, 0.12 mL, 0.46 mmol, 5.0 eq) was stirred at 20°C for 0.5 h and then all volatiles were removed. The remaining solid was dissloved in water and 2-methyl-2-[5-[3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile [isomer A] (33.6 mg, 0.06 mmol, 60% yield) was obtained as a white solid by lyophilization as hydrochloride salt. MS (ESI): 548.3 [M+H]⁺, retention time 1.66 minutes. 2-methyl-2-[5-[3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile [isomer B] was obtained in analogy. MS (ESI): 548.3 [M+H]⁺, retention time 2.02 minutes (column: Chiralcel OD-3 50×4.6 mm I.D., 3 µm; mobile phase: phase A for CO₂, and phase B for MeOH (0.05%DEA); gradient elution: 5% to 40% MeOH (0.05% DEA) in CO₂; flow rate: 3 mL/min; detector: DAD; column temperatur: 35°C; back pressure: 100 bar).

### Example 12

### 2-methyl-2-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile

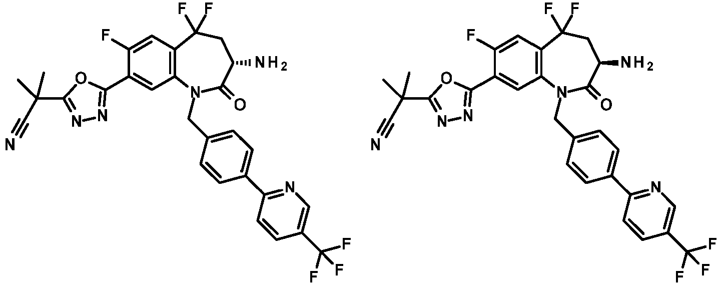

### Step a) tert-butyl N-[8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate

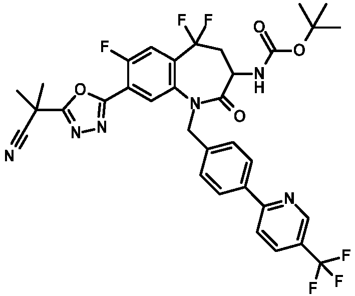

The title compound was prepared in analogy to Example 1, steps a to l (using 2-[4-(bromomethyl)phenyl]-5-(trifluoromethyl)pyridine; hydrochloride instead of 4-(trifluoromethoxy)benzyl bromide in step f and 2-cyano-2-methyl-propanoic acid instead of pivalic acid in step k) and was obtained as light yellow solid. MS (ESI): 701.3 [M+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate

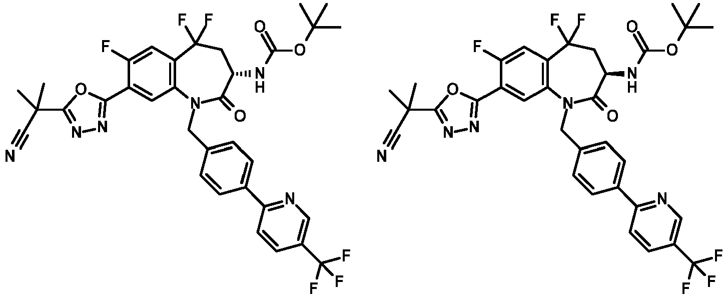

tert-butyl N-[8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate (95 mg) was subjected to preparative SFC (Daicel Chiralpak IC (250 mm x 30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer B] (33 mg) (MS (ESI): 701.2 [M+H]⁺, retention time 2.01 minutes (see conditions below) as white solid and tert-butyl N-[8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer A] (33 mg) as white solid. MS (ESI): 701.1 [M+H]⁺, retention time 1.44 minutes (column: Chiralpak IC-3 50×4.6 mm I.D., 3 µm; mobile phase: phase A for CO₂, and phase B for EtOH (0.05%DEA); gradient elution: from 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile and 2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile

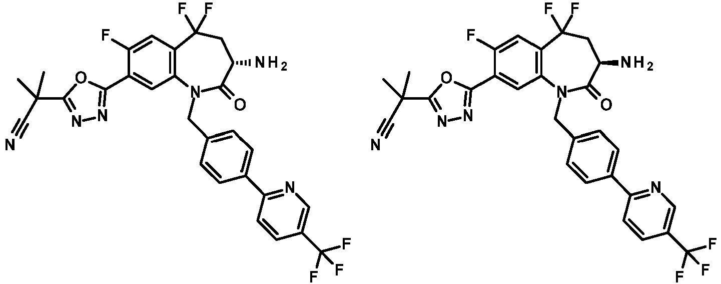

A solution of tert-butyl N-[8-[5-(1-cyano-1-methyl-ethyl)-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer A] (30.0 mg, 0.04 mmol, 1.0 eq) in HCl/EtOAc (4 M, 4.0 mL, 16.0 mmol, 373.67 eq) was stirred at 20°C for 0.5 h and then all volatiles were removed. The remaining solid was dissloved in water and 2-methyl-2-[5-[3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile [isomer A] (28.0 mg, 0.04 mmol, 93% yield) was obtained as a light yellow solid by lyophilization as dihydrochloride salt. MS (ESI): 601.3 [M+H]⁺, retention time 1.51 minutes. 2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile [isomer B] was obtained in analogy. MS (ESI): 601.2 [M+H]⁺, retention time 1.84 minutes (column: Chiralcel OD-3 50×4.6 mm I.D., 3 µm; mobile phase: phase A for CO₂, and phase B for MeOH (0.05%DEA); gradient elution: 5% to 40% MeOH (0.05% DEA) in CO₂; flow rate: 3 mL/min; detector: DAD; column temperatur: 35°C; back pressure: 100 bar).

### Example 13

### (3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one and

### (3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one

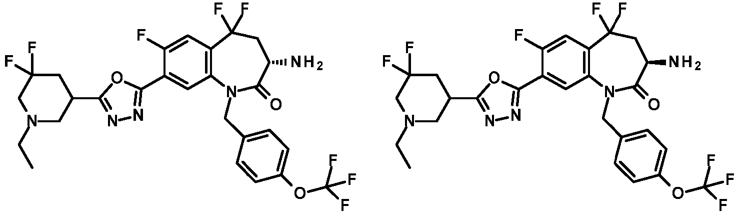

### Step a) tert-butyl N-[(3S)-5,5,7-trifluoro-8-(hydrazinecarbonyl)-2-oxo-1-[[4-(trifluoromethoxy)phenyl]methyl]-3,4-dihydro-1-benzazepin-3-yl]carbamate and tert-butyl N-[(3R)-5,5,7-trifluoro-8-(hydrazinecarbonyl)-2-oxo-1-[[4-(trifluoromethoxy)phenyl]methyl]-3,4-dihydro-1-benzazepin-3-yl]carbamate

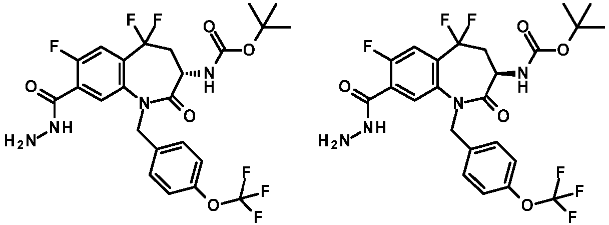

To a mixture of 3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer A] (Example 4, step a) (280.0 mg, 0.51 mmol, 1.0 eq) and DMF (0.1 mL) in DCM (10 mL) was added oxalyl chloride (77.76 mg, 0.61 mmol, 1.2 eq) at 20°C and the mixture was stirred for 0.5 h. Then hydrazine monohydrochloride (127.0 mg, 1.53 mmol, 3.0 eq) and DIPEA (0.27 mL, 1.53 mmol, 3.0 eq) were added at 20°C and the mixture was stirred at 20°C for 0.5 h and then poured into water. The mixture was extracted with DCM (3 x 20 mL) and the combined extracts were washed with brine (50 mL), dried with anhydrous Na₂SO₄, filtered and concentrated in vacuum. The crude tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate [isomer A] (218.0 mg, 0.39 mmol, 76% yield) was obtained as yellow solid and was used in the next step without further purification. MS (ESI): 507.1 [M-isobutene+H]⁺. tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate [isomer B] was obtained in analogy from 3-(tert-butoxycarbonylamino)-5,5,7-trifluoro-2-oxo-1-[[4-(trifluoromethoxy)phenyl]methyl]-3,4-dihydro-1-benzazepine-8-carboxylic acid [isomer B] (Example 4, step a). MS (ESI): 507.2 [M-isobutene+H]⁺.

### Step b) tert-butylN-[(3S)-8-[[(1-ethyl-5,5-piperidine-3-carbonyl)amino]carbamoyl]-5,5,7-trifluoro-2-oxo-1-[[4-(trifluoromethoxy)phenyl]methyl]-3,4-dihydro-1-benzazepin-3-yl]carbamate and tert-butyl N-[(3R)-8-[[(1-ethyl-5,5-difluoro-piperidine-3-carbonyl)amino]carbamoyl]-5,5,7-trifluoro-2-oxo-1-[[4-(trifluoromethoxy)phenyl]methyl]-3,4-dihydro-1-benzazepin-3-yl)carbamate

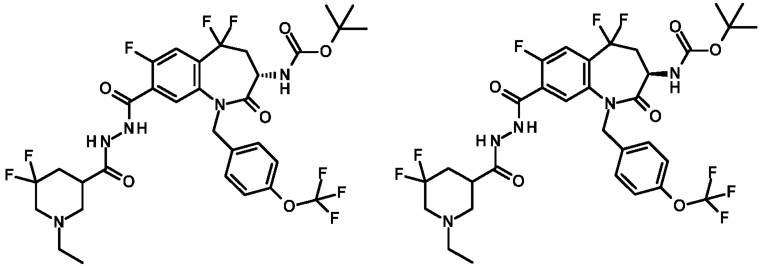

To a mixture of 1-ethyl-5,5-difluoro-piperidine-3-carboxylic acid (Intermediate 2) (159.72 mg, 0.83 mmol, 1.5 eq) and T3P (50% in EtOAc, 526.1 mg, 0.83 mmol, 1.5 eq) in DCM (5 mL) was added DIPEA (0.29 mL, 1.65 mmol, 3.0 eq) at 25°C and the mixture was stirred for 0.5 h. Then tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate [isomer A] (310.0 mg, 0.55 mmol, 1.0 eq) was added at 25°C and the mixture was stirred at 25°C for 0.5 h and then poured into water. The mixture was extracted with DCM (3 x 15 mL) and the combined extracts were washed with brine (40 mL), dried with anhydrous Na₂SO₄, filtered and concentrated in vacuum to give N-[8-[[(1-ethyl-5,5-difluoropiperidine-3-carbonyl)amino]carbamoyl]-5,5,7-trifluoro-2-oxo-1-[[4-(trifluoromethoxy)phenyl]methyl]-3,4-dihydro-1-benzazepin-3-yl]carbamate [isomer A] (155.0 mg, 0.21 mmol, 38% yield) as yellow solid which was used in next step without further purification. MS (ESI): 738.2 [M+H]⁺. N-[8-[[(1-ethyl-5,5-difluoro-piperidine-3-carbonyl)amino]carbamoyl]-5,5,7-trifluoro-2-oxo-1-[[4-(trifluoromethoxy)phenyl]methyl]-3,4-dihydro-1-benzazepin-3-yl]carbamate [isomer B] was obtained in analogy from tert-butyl N-[5,5,7-trifluoro-8-(hydrazinecarbonyl)-2-oxo-1-[[4-(trifluoromethoxy)phenyl]methyl]-3,4-dihydro-1-benzazepin-3-yl]carbamate [isomer B].

### Step c) tert-butyl N-[(3S)-8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate and tert-butyl N-[(3R)-8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate

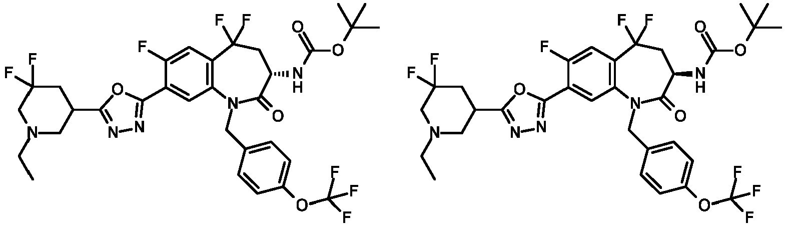

A mixture of tert-butyl N-[8-[[(1-ethyl-5,5-difluoro-piperidine-3-carbonyl)amino]carbamoyl]-5,5,7-trifluoro-2-oxo-1-[[4-(trifluoromethoxy)phenyl]methyl]-3,4-dihydro-1-benzazepin-3-yl]carbamate [isomer A] (390.0 mg, 0.34 mmol, 1.0 eq) and Burgess reagent (245.69 mg, 1.03 mmol, 3.0 eq) in toluene (15 mL) was stirred at 100°C for 12 h and then poured into water (50 mL). The mixture was extracted with EtOAc (3 x 40 mL) and the combined extracts were washed with brine (100 mL), dried with anhydrous Na₂SO₄, filtered and concentrated in vacuum. The remaining residue was purified by prep-HPLC (Column: Phenomenex Synergi C18 150x25mm, 10µm, Condition: water (containing formic acid)/ACN) to give tert-butyl N-[8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate [isomer A] (59.0 mg, 0.08 mmol, 23% yield) as white solid. MS (ESI): 720.2 [M+H]⁺. tert-butyl N-[8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate [isomer B] was obtained in analogy from tert-butyl N-[8-[[(1-ethyl-5,5-difluoro-piperidine-3-carbonyl)amino]carbamoyl]-5,5,7-trifluoro-2-oxo-1-[[4-(trifluoromethoxy)phenyl]methyl]-3,4-dihydro-1-benzazepin-3-yl]carbamate [isomer B]. MS (ESI): 720.2 [M+H]⁺.

### Step d) (3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one and (3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one

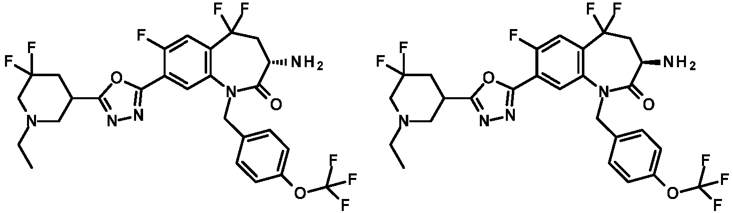

To a solution of tert-butyl N-[8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate [isomer A] (55.0 mg, 0.08 mmol, 1.0 eq) in EtOAc (1 mL) was added HCl/EtOAc (4 M, 5.87 mL, 23.47 mmol, 307.04 eq) at 25°C and the mixture was stirred at 25°C for 1 h. The reaction mixture was concentrated in vacuum and the remaining residue was purified by prep-HPLC (Column: Phenomenex Synergi C18 150x25mm, 5 µm, Condition: water (containing NH₄HCO₃)/ACN) to give 3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one [isomer A] (12.6 mg, 0.02 mmol, 26% yield) as off-white solid. MS (ESI): 620.2 [M+H]⁺, retention time 1.25 minutes. 3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one [isomer B] was obtained in analogy from tert-butyl N-[8-[5-(1-ethyl-5,5-difluoro-3-piperidyl)-1,3,4-oxadiazol-2-yl]-5,5,7-trifluoro-2-oxo-1-[[4-(trifluoromethoxy)phenyl]methyl]-3,4-dihydro-1-benzazepin-3-yl]carbamate [isomer B]. MS (ESI): 620.2 [M+H]⁺, retention times 1.74 and 1.94 minutes (column: Chiralcel OD-3 50×4.6 mm I.D., 3 µm; mobile phase: phase A for CO₂, and phase B for EtOH (0.05%DEA); gradient elution: 5% to 40% EtOH (0.05% DEA) in CO₂; flow rate: 3 mL/min; detector: PDA; column temperatur: 35°C; back pressure: 100 bar).

### Example 14

### (3S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one and

### (3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one

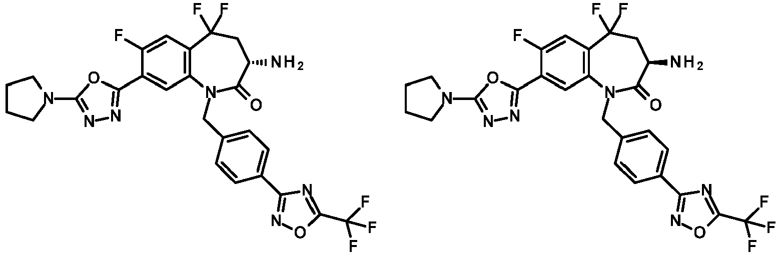

### Step a) tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol3yl]phenyl]methyl]-3,4-dihydro-1-benzazepin-3yl]carbamate

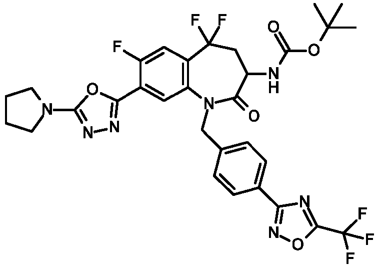

The title compound was prepared in analogy to Example 15, steps d and e from tert-butyl N-[1-[(4-cyanophenyl)methyl]-5,5,7-trifluoro-2-oxo-8-(5-pyrrolidin-1-yl-1,3,4-oxadiazol-2-yl)-3,4-dihydro-1-benzazepin-3-yl]carbamate (Example 15, step c) and was obtained as light yellow solid. MS (ESI): 694.1 [M+H]⁺.

### Step b) tert-butyl N-[(3S)-5,5,7-trifluoro-2-oxo-8-(5-pyrrolidin-1-yl-1,3,4-oxadiazol-2-yl)-1-[[4-[5-(trifluoromethyl)-1,2,4-oxadiazol3yl]phenyl]methyl]-3,4-dihydro-1-benzazepin-3yl]carbamate and tert-butyl N-[(3R)-5,5,7-trifluoro-2-oxo-8-(5-pyrrolidin-1-yl-1,3, 4-oxadiazol-2-yl)-1-[[4-[5-(trifluoromethyl)-1,2,4-oxadiazol3yl]phenyl]methyl]-3,4-dihydro-1-benzazepin-3yl]carbamate

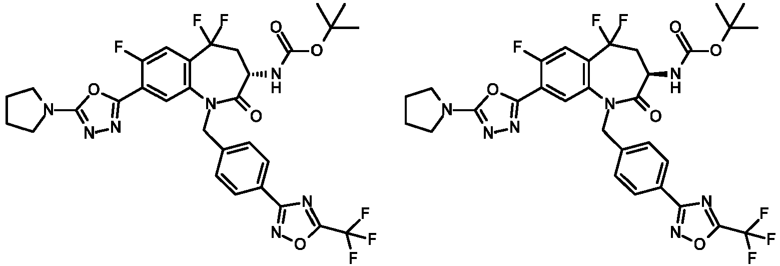

tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-3-yl]carbamate (60 mg) was subjected to preparative SFC (Daicel Chiralpak AD (250 mm x 30 mm, 10 µm), 0.1% NH₃-H₂O in iPrOH) to obtain tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-3-yl]carbamate [isomer B] (25 mg) (MS (ESI): 694.1 [M+H]⁺, retention time 1.76 minutes (see conditions below) as light yellow solid and tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-3-yl]carbamate [isomer A] (23 mg) as light yellow solid. MS (ESI): 694.1 [M+H]⁺, retention time 1.27 minutes (column: Chiralcel OD-3 50×4.6 mm I.D., 3 µm; mobile phase: phase A for CO₂, and phase B for MeOH (0.05% DEA); gradient elution: from 5% to 40% MeOH (0.05% DEA) in CO₂; flow rate: 3 mL/min; detector: PDA; column temperatur: 35°C; back pressure: 100 bar).

### Step c) (3S)-3-amino-5,5,7-trifluoro-8-(5-pyrrolidin-1-yl-1,3,4-oxadiazol-2-yl)-]-[[4-[5-(trifluoromethyl)-1,2,4-oxadiazol-3-yl]phenyl]methyl]-3,4-dihydro-1-benzazepin-2-one and (3R)-3-amino-3,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one

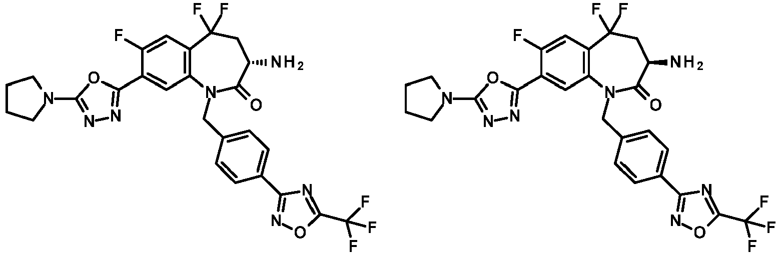

A solution of tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-3-yl]carbamate [isomer A] (23.0 mg, 0.03 mmol, 1.0 eq) in HCl/EtOAc (4 M, 5.0 mL, 20.0 mmol, 603.13 eq) was stirred at 20°C for 0.5 h. All volatiles were removed in vacuum and the remaining residue was dissolved in water to obtain 3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one [isomer A] (13.5 mg, 0.02 mmol, 64% yield) as a white solid by lyophilization as a hydrochloride salt. MS (ESI): 594.2 [M+H]⁺, retention time 1.59 minutes. 3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one [isomer B] was obtained in analogy from tert-butyl N-[5,5,7-trifluoro-2-oxo-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-3-yl]carbamate [isomer B]. MS (ESI): 594.2 [M+H]⁺, retention time 2.28 (column: Chiralcel OD-3 50×4.6 mm I.D., 3 µm; mobile phase: phase A for CO₂, and phase B for MeOH (0.05%DEA); gradient elution: 5% to 40% MeOH (0.05% DEA) in CO₂; flow rate: 3 mL/min; detector: PDA; column temperatur: 35°C; back pressure: 100 bar).

### Example 15

### (3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one and

### (3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one

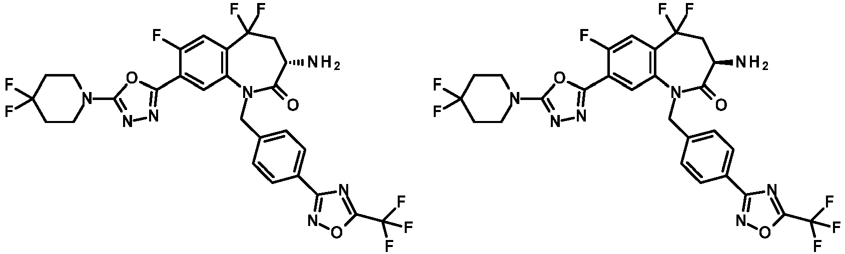

### Step a) tert-butyl N-[1-[(4-cyanophenyl)methyl]-5,5,7-trifluoro-8-(hydrazinecarbonyl)-2-oxo-3,4-dihydro-1-benzazepin-3-yl]carbamate

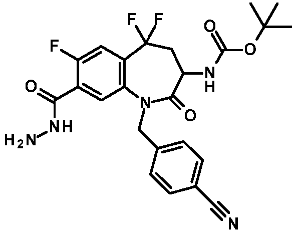

The title compound was prepared in analogy to Example 1, steps a to j (using 4-(bromomethyl)benzonitrile instead of 4-(trifluoromethoxy)benzyl bromide in step f) and was obtained as light yellow solid. MS (ESI): 448.1 [M-isobutene+H]⁺.

### Step b) tert-butyl N-[1-[(4-cyanophenyl)methyl]-5,5,7-trifluoro-2-oxo-8-(2-oxo-3H-1,3,4-oxadiazol-5-yl)-3,4-dihydro-1-benzazepin-3-yl]carbamate

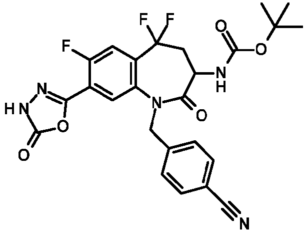

A mixture of tert-butyl N-[1-[(4-cyanophenyl)methyl]-5,5,7-trifluoro-8-(hydrazinecarbonyl)-2-oxo-3,4-dihydro-1-benzazepin-3-yl]carbamate (1.5 g, 2.98 mmol, 1.0 eq), TEA (0.93 mL, 6.67 mmol, 2.24 eq) and CDI (0.72 g, 4.47 mmol, 1.5 eq) in THF (20 mL) was stirred at 20°C for 3 h and then the reactin mixture was poured into water (50 mL). The mixture was extracted with EtOAc (100 mL) and the organic phase was washed with brine (50 mL), dried (Na₂SO₄) and concentrated under vacuum to give the title compound (1.5 g, 2.83 mmol, 87% yield) as a light yellow solid which was used for next step without further purification. MS (ESI): 474.2 [M-isobutene+H]⁺.

### Step c) tert-butyl N-[1-[(4-cyanophenyl)methyl]-8-[5-(4,4-difluoro-1-piperidyl)-1piperidyl)-1,3,4-oxadiazol-2-yl]-5,5,7-trifluoro-2-oxo-3,4-dihydro-1-benzazepin-3-yl]carbamate and tert-butyl N-[1-[(4-cyanophenyl)methyl]-5,5,7-trifluoro-2-oxo-8-(5-pyrrolidin-1-yl-1,3,4-oxadiazol-2-yl)-3,4-dihydro-1-benzazepin-3-yl]carbamate

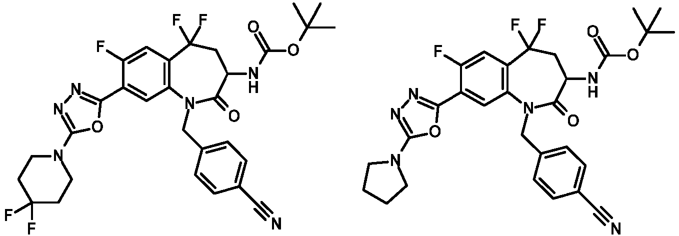

To a mixture of tert-butyl N-[1-[(4-cyanophenyl)methyl]-5,5,7-trifluoro-2-oxo-8-(2-oxo-3H-1,3,4-oxadiazol-5-yl)-3,4-dihydro-1-benzazepin-3-yl]carbamate (450.0 mg, 0.85 mmol, 1.0 eq), 4,4-difluoropiperidine hydrochloride (202.5 mg, 1.28 mmol, 1.51 eq) and DIPEA (0.45 mL, 2.55 mmol, 3.0 eq) in dry 1,4-dioxane (10 mL) was added PyBroP (475.47 mg, 1.02 mmol, 1.2 eq) at 25°C and the mixture was stirred for 3 h at 20°C under an atmosphere of nitrogen. The mixture was poured into water (100 mL). The aqueous phase was extracted with EtOAc (200 mL) and the organic extracts were washed with brine (100 mL), dried over anhydrous sodium sulfate, filtered and concentrated in vacuum. The remaining residue was purified by column chromatography on silica gel (PE/EtOAc 10:1 to 1:1) to obtain tert-butyl N-[1-[(4-cyanophenyl)methyl]-8-[5-(4,4-difluoro-1-piperidyl)-1,3,4-oxadiazol-2-yl]-5,5,7-trifluoro-2-oxo-3,4-dihydro-1-benzazepin-3-yl]carbamate (400.0 mg, 0.63 mmol, 74% yield) as a light yellow solid (MS (ESI): 633.4 [M+H]⁺) and a second fraction which was further purified by prep-HPLC (Column: Phenomenex luna C18 150x25mm, 10µm; condition: water (containing formic acid)/ACN from 1:1 to 1:4) to obtain tert-butyl N-[1-[(4-cyanophenyl)methyl]-5,5,7-trifluoro-2-oxo-8-(5-pyrrolidin-1-yl-1,3,4-oxadiazol-2-yl)-3,4-dihydro-1-benzazepin-3-yl]carbamate (45.0 mg, 0.08 mmol, 9% yield) as a white solid. MS (ESI): 583.5 [M+H]⁺.

### Step d) tert-butyl N-[8-[5-(4,4-difluoro-1-piperidyl)-1,3,4-oxadiazol-2-yl]-5,5,7-trifluoro-1-[[4-[(Z)-N'-hydroxycarbamimidoyl]phenyl]methyl]-2-oxo-3,4-dihydro-1-benzazepin-3-yl]carbamate

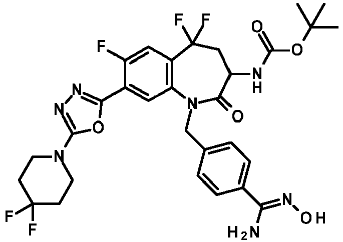

A mixture of tert-butyl N-[1-[(4-cyanophenyl)methyl]-8-[5-(4,4-difluoro-1-piperidyl)-1,3,4-oxadiazol-2-yl]-5,5,7-trifluoro-2-oxo-3,4-dihydro-1-benzazepin-3-yl]carbamate (35.0 mg, 0.06 mmol, 1.0 eq), sodium acetate (13.62 mg, 0.17 mmol, 3.0 eq) and hydroxylamine hydrochloride (5.77 mg, 0.08 mmol, 1.5 eq) in ethanol (5 mL) was stirred at 50°C for 12 h and then poured into water (20 mL). The mixture was extracted with EtOAc (50 mL) and the organic extrat was washed with brine (30 mL), dried (Na₂SO₄) and concentrated in vacuum to give tert-butyl N-[8-[5-(4,4-difluoro-1-piperidyl)-1,3,4-oxadiazol-2-yl]-5,5,7-trifluoro-1-[[4-[(Z)-N'-hydroxycarbamimidoyl]phenyl]methyl]-2-oxo-3,4-dihydro-1-benzazepin-3-yl]carbamate (33.0 mg, 0.05 mmol, 71% yield) as a white solid which was used in the next step without further purification. MS (ESI): 666.2 [M+H]⁺.

### Step e) tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-benzazepin-3-yl]carbamate

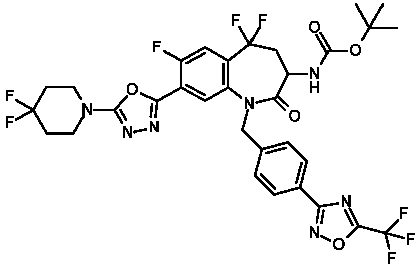

A solution of tert-butyl N-[8-[5-(4,4-difluoro-1-piperidyl)-1,3,4-oxadiazol-2-yl]-5,5,7-trifluoro-1-[[4-[(Z)-N'-hydroxycarbamimidoyl]phenyl]methyl]-2-oxo-3,4-dihydro-1-benzazepin-3-yl]carbamate (190.0 mg, 0.29 mmol, 1.0 eq) and trifluoroacetic anhydride (0.06 mL, 0.46 mmol, 1.6 eq) in THF (10 mL) was stirred at 20°C for 2 h. The solution was poured into water (20 mL) and the pH of the solution was adjusted to 7-8 by addition of NaHCO₃. Then the mixture was extracted with EtOAc (40 mL) and the organic extract was washed with brine (30 mL), dried (Na₂SO₄) and concentrated in vacuum. The remaining residue was purified by prep-HPLC (Column: Phenomenex luna C18 150x25mm, 10µm; condition: water (containing formic acid)/ACN from 1:1 to 1:4) to obtain tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-benzazepin-3-yl]carbamate (120.0 mg, 0.16 mmol, 55% yield) as a white solid. MS (ESI): 744.4 [M+H]⁺.

### Step f) tert-butyl N-[(3S)-8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate and tert-butyl N-[(3R)-8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate

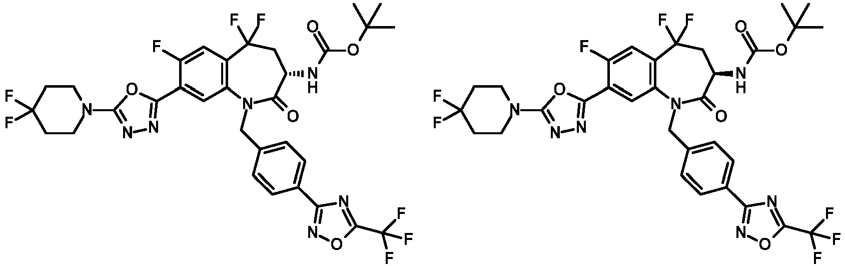

tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-benzazepin-3-yl]carbamate (120 mg) was subjected to preparative SFC (Daicel Chiralcel OD (250 mm x 30 mm, 10 µm), 0.1% NH₃-H₂O in EtOH) to obtain tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate [isomer B] (45 mg) (MS (ESI): 744.4 [M+H]⁺, retention time 1.52 minutes (see conditions below) as white solid and tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate [isomer A] (50 mg) as white solid. MS (ESI): 744.4 [M+H]⁺, retention time 1.10 minutes (column: Chiralcel OD-3 50×4.6 mm I.D., 3 µm; mobile phase: phase A for CO₂, and phase B for EtOH (0.05% DEA); gradient elution: from 5% to 40% EtOH (0.05% DEA) in CO₂; flow rate: 3 mL/min; detector: PDA; column temperatur: 35°C; back pressure: 100 bar).

### Step g) (3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one and (3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one

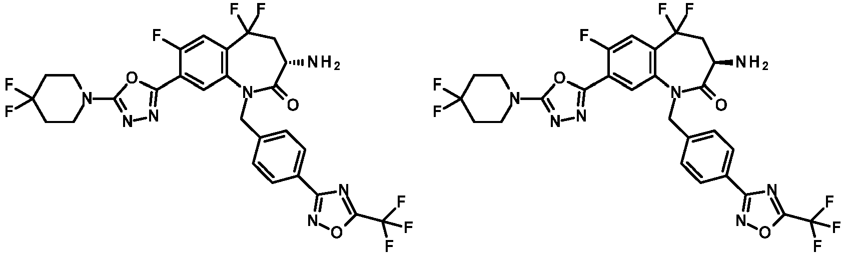

A solution of tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate [isomer A] (40.0 mg, 0.05 mmol, 1.0 eq) in HCl in EtOAc (4 M, 10.0 mL, 40.0 mmol, 743.6 eq) was stirred at 20 °C for 0.5 h. Then all volatiles were removed in vacuum and the remaining residue was dissolved in water. 3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one [isomer A] was obtained as white solid (28.4 mg, 0.04 mmol, 77% yield) after lyophilization as hydrochloride salt. MS (ESI): 644.1 [M+H]⁺, retention time 1.47 minutes. 3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one [isomer B] was obtained in analogy from tert-butyl N-[8-[5-(4,4-difluoro-1-piperidyl)-1,3,4-oxadiazol-2-yl]-5,5,7-trifluoro-2-oxo-1-[[4-[5-(trifluoromethyl)-1,2,4-oxadiazol-3-yl]phenyl]methyl]-3,4-dihydro-1-b enzazepin-3-yl]carbamate [isomer B]. MS (ESI): 644.1 [M+H]⁺, retention time 2.23 minutes (column: Chiralcel OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Example 16

### (3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one and

### (3R)- 3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one

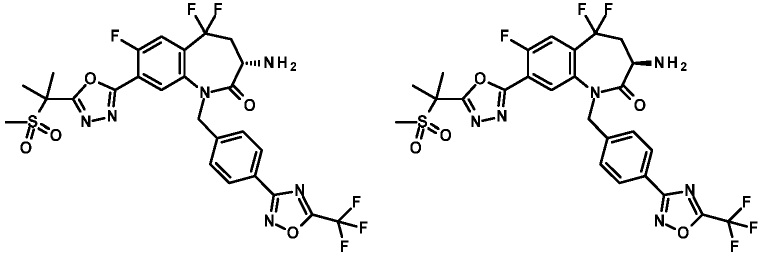

### Step a) tert-butyl N-[1-[(4-cyanophenyl)methyl]-5,5,7-trifluoro-8-[5-(1-methyl-1-methylsulfonylethyl)-1,3,4-oxadiazol-2yl]-2-oxo-3,4-dihydro-1-benzazepin-3yl]carbamate

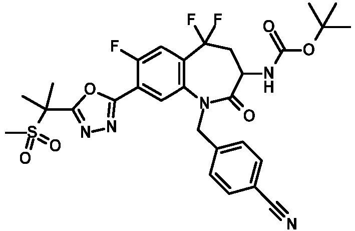

The title compound was prepared in analogy to Example 1, steps a to l (using 4-(bromomethyl)benzonitrile instead of 4-(trifluoromethoxy)benzyl bromide in step f and 2-methyl-2-methylsulfonyl-propanoic acid instead of pivalic acid in step k) and was obtained as light yellow solid. MS (ESI): 634.4 [M+H]⁺.

### Step b) tert-butyl N-[5,5,7-trifluoro-1-[[4-[(Z)-N'-hydroxycarbamimidoyl]phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate

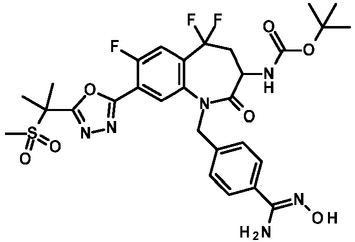

A mixture of tert-butyl N-[1-[(4-cyanophenyl)methyl]-5,5,7-trifluoro-8-[5-(1-methyl-1-methylsulfonyl-ethyl)-1,3,4-oxadiazol-2-yl]-2-oxo-3,4-dihydro-1-benzazepin-3-yl]carbamate (190.0 mg, 0.3 mmol, 1.0 eq), potassium acetate (0.06 mL, 0.9 mmol, 3.0 eq) and hydroxylamine hydrochloride (27.0 mg, 0.39 mmol, 1.3 eq) in EtOH (10 mL) was stirred at 50°C for 12 h and then the reaction mixture was poured into water (50 mL). The mixture was extracted with EtOAc (100 mL), the organic extract was washed with brine (50 mL), dried (Na₂SO₄) and concentrated in vacuum. The remaining residue was purified by prep-TLC (PE/EtOAc = 1:2) to give the title compound (150.0 mg, 0.22 mmol, 74% yield) as a light yellow solid. MS (ESI): 667.4 [M+H]⁺.

### Step c) tert-butyl N-[(3S)-5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate

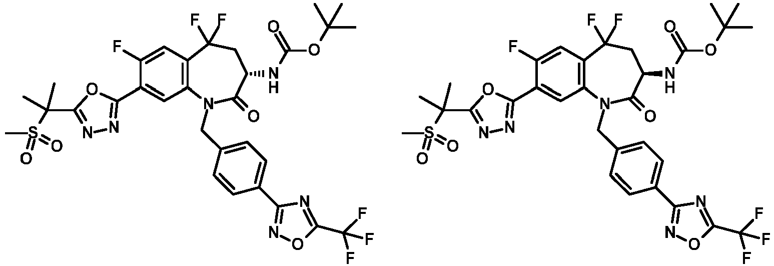

A solution of tert-butyl N-[5,5,7-trifluoro-1-[[4-[(Z)-N'-hydroxycarbamimidoyl]phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate (140.0 mg, 0.21 mmol, 1.0 eq) and trifluoroacetic anhydride (0.04 mL, 0.31 mmol, 1.5 eq) in THF (10 mL) was stirred at 20°C for 3 h. The reaction mixture was poured into water (30 mL) and the pH of the mixture was adjusted to 7-8 by addition of NaHCO₃. The mixture was extracted with EtOAc (50 mL), the organic extract was washed with brine (30 mL), dried (Na₂SO₄) and concentrated in vacuum and the remaining residue was purified by prep-TLC (PE/EtOAc = 3:1) to obtain tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate. This was subjected to preparative SFC (Daicel Chiralpak AD (250 mm x 30 mm, 10 µm), 0.1% NH₃-H₂O in iPrOH) to obtain tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer B] (30 mg) (MS (ESI): 745.4 [M+H]⁺, retention time 1.64 minutes (see conditions below) as white solid and tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer A] (40 mg) as white solid. MS (ESI): 745.4 [M+H]⁺, retention time 1.24 minutes (column: Chiralpak IC-3 50×4.6 mm I.D., 3 µm; mobile phase: phase A for CO₂, and phase B for EtOH (0.05%DEA); gradient elution: from 5% to 40% EtOH (0.05% DEA) in CO₂; flow rate: 3 mL/min; detector: PDA; column temperatur: 35°C; back pressure: 100 bar).

### Step d) (3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one and (3R)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one

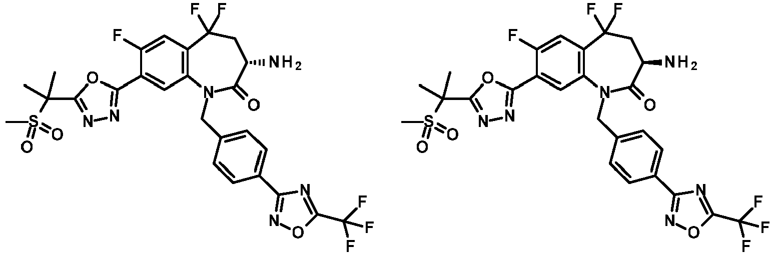

A solution of tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer A] (35.0 mg, 0.05 mmol, 1.0 eq) in HCl/EtOAc (4 M, 10.0 mL, 40.0 mmol, 851.04 eq) was stirred at 20°C for 0.5 h. All volatiles were removed in vacuum and the remaining residue was dissolved in water to obtain 3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one [isomer A] (24.9 mg, 0.04 mmol, 81% yield) as a white soild by lyophilization as hydrochloride salt. MS (ESI): 645.1 [M+H]⁺, retention time 1.86 minutes. 3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one [isomer B] was obtained in analogy from tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer B]. MS (ESI): 645.1 [M+H]⁺, retention time 2.38 minutes (column: Chiralpak IG-3 50×4.6 mm I.D., 3 µm; mobile phase: phase A for CO₂, and phase B for EtOH (0.05%DEA); gradient elution: 5% to 40% EtOH (0.05% DEA) in CO₂; flow rate: 3 mL/min; detector: PDA; column temperatur: 35°C; back pressure: 100 bar).

### Example 17

### (3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one and

### (3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one

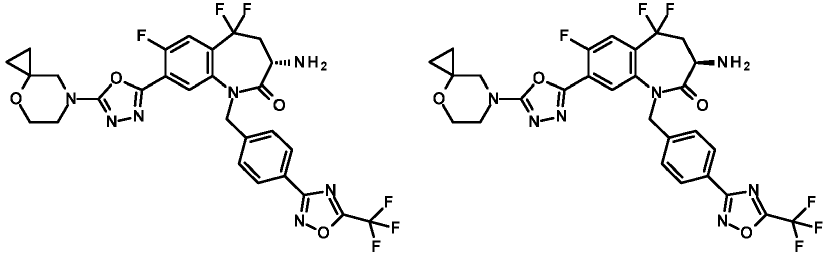

### Step a) tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate

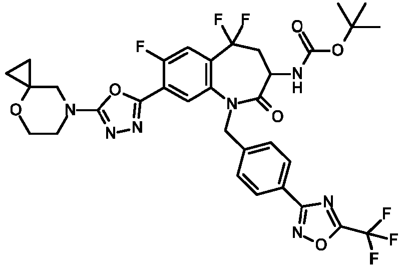

The title compound was prepared in analogy to Example 15, steps a to e (using 4-oxa-7-azaspiro[2.5]octane hydrochloride instead of 4,4-difluoropiperidine hydrochloride in step c) and was obtained as light yellow solid. MS (ESI): 625.2 [M+H]⁺.

### Step b) tert-butyl N-[(3S)-5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate and tert-butyl N-[(3R)-5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate

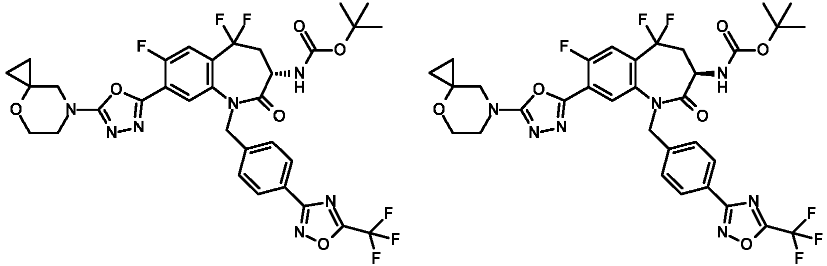

tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate (70 mg) was subjected to preparative SFC (Daicel Chiralpak AD (250 mm x 30 mm, 10 µm), 0.1% NH₃-H₂O in iPrOH) to obtain tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer B] (31 mg) (MS (ESI): 736.3 [M+H]⁺, retention time 1.46 minutes (see conditions below) as light yellow solid and tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer A] (30 mg) as light yellow solid. MS (ESI): 736.3 [M+H]⁺, retention time 1.09 minutes (column: Chiralcel OD-3 50×4.6 mm I.D., 3 µm; mobile phase: phase A for CO₂, and phase B for MeOH (0.05% DEA); gradient elution: from 5% to 40% MeOH (0.05% DEA) in CO₂; flow rate: 3 mL/min; detector: PDA; column temperatur: 35°C; back pressure: 100 bar).

### Step c) (3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one and (3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one

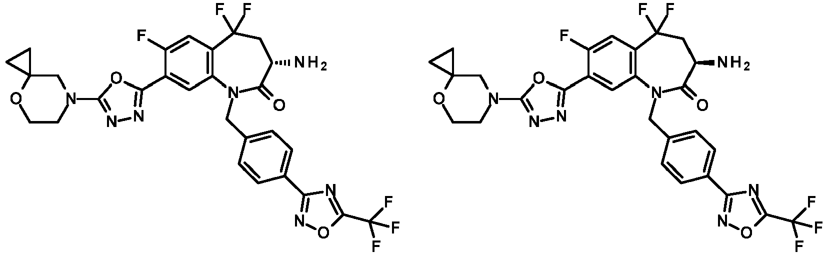

A solution of tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer A] (30.0 mg, 0.04 mmol, 1.0 eq) in HCl/EtOAc (4 M, 5.0 mL, 20.0 mmol, 490.42 eq) was stirred at 20°C for 0.5 h. All volatiles were removed in vacuum and the remaining residue was dissolved in water. 3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one [isomer A] (21.4 mg, 0.03 mmol, 82% yield) was obtained as a light yellow solid as hydrochloride salt by lyophilization. MS (ESI): 636.3 [M+H]⁺, retention time 1.53 minutes. 3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one [isomer B] was obtained in analogy from tert-butyl N-[5,5,7-trifluoro-8-[5-(4-oxa-7-azaspiro[2.5]octan-7-yl)-1,3,4-oxadiazol-2-yl]-2-oxo-1-[[4-[5-(trifluoromethyl)-1,2,4-oxadiazol-3-yl]phenyl]methyl]-3,4-dihydro-1-benzazepin-3-yl]carbamate [isomer B]. MS (ESI): 636.3 [M+H]⁺, retention time 2.30 minutes (column: Chiralcel OD-3 50×4.6 mm I.D., 3 µm; mobile phase: phase A for CO₂, and phase B for MeOH (0.05%DEA); gradient elution: from 5% to 40% MeOH (0.05% DEA) in CO₂; flow rate: 3 mL/min; detector: PDA; column temperatur: 35°C; back pressure: 100 bar).

### Example 18

### (3S)-3-amino-8-(5-tert-butyl-3-pyridyl)-1-[(4-chlorophenyl)methyl]-5,5,7-trifluoro-3,4-dihydro-1-benzazepin-2-one and

### (3R)-3-amino-8-(5-tert-butyl-3-pyridyl)-1-[(4-chlorophenyl)methyl]-5,5,7-trifluoro-3,4-dihydro-1-benzazepin-2-one

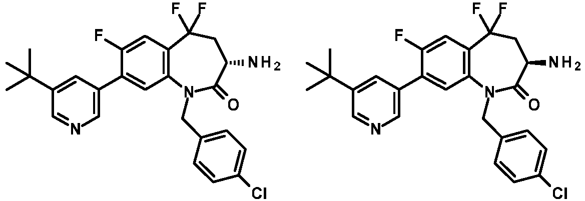

### Step a) tert-butyl N-[8-bromo-1-[(4-chlorophenyl)methyl]-5,5,7-trifluoro-2-oxo-3,4-dihydro-1-benzazepin-3-yl]carbamate

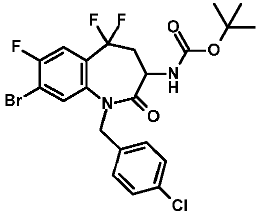

The title compound was prepared in analogy to Example 1, steps a to g (using 1-(bromomethyl)-4-chloro-benzene instead 4-(trifluoromethoxy)benzyl bromide in step f) and was obtained as yellow solid. MS (ESI): 478.9 [M-isobutene+H]⁺.

### Step b) tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate

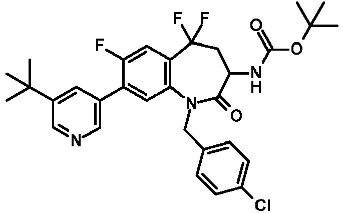

Nitrogen was bubbled through a mixture of tert-butyl N-[8-bromo-1-[(4-chlorophenyl)methyl]-5,5,7-trifluoro-2-oxo-3,4-dihydro-1-benzazepin-3-yl]carbamate (100.0 mg, 0.19 mmol, 1.0 eq), 3-tert-butyl-5-(4,4,5,5-tetramethyl-1,3,2-dioxaborolan-2-yl)pyridine [CAS 1627723-04-9] (53.82 mg, 0.21 mmol, 1.1 eq), K₂CO₃ (77.68 mg, 0.56 mmol, 3.0 eq) and Pd(dppf)Cl₂-DCM (15.3 mg, 0.02 mmol, 0.1 eq) in a mixture of 1,4-dioxane (5 mL) and water (0.7 mL) for 2 minutes. The reaction mixture was then heated to 80 °C and stirred for 3 h. All volatiles were remove in vacuum and the remaining residue was purified by prep-TLC (PE/EtOAc = 5:1) to give the title compound (95.0 mg, 0.16 mmol, 72% yield) as yellow oil. MS (ESI): 588.2 [M+H]⁺.

### Step c) tert-butyl N-[(3S)-8-(5-tert-butyl-3-pyridyl)-1-[(4-chlorophenyl)methyl]-5,5,7-trifluoro-2-oxo-3,4-dihydro-1-benzazepin-3-yl]carbamate and tert-butyl N-[(3R)-8-(5-tert-butyl-3-pyridyl)-1-[(4-chlorophenyl)methyl]-5,5,7-trifluoro-2-oxo-3,4-dihydro-1-benzazepin-3-yl]carbamate

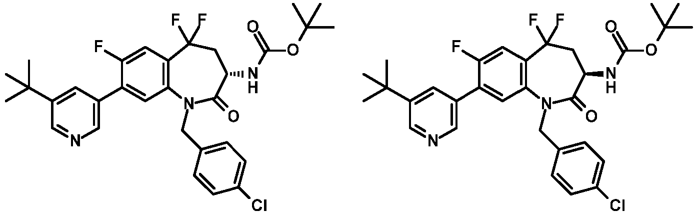

tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate (95 mg) was subjected to preparative SFC (Daicel Chiralpak AD (250 mm x 30 mm, 10 µm), 0.1% DEA in MeOH) to obtain tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate [isomer B] (45 mg) (MS (ESI): 588.2 [M+H]⁺, retention time 1.45 minutes (see conditions below) as yellow oil and tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate [isomer A] (45 mg) as yellow oil. MS (ESI): 588.2 [M+H]⁺, retention time 0.89 minutes (column: Chiralpak AD-3 50×4.6 mm I.D., 3 µm; mobile phase: phase A for CO₂, and phase B for MeOH (0.05% DEA); gradient elution: from 5% to 40% MeOH (0.05% DEA) in CO₂; flow rate: 3 mL/min; detector: PDA; column temperatur: 35°C; back pressure: 100 bar).

### Step d) (3S)-3-amino-8-(5-tert-butyl-3-pyridyl)-1-[(4-chlorophenyl)methyl]-5,5,7-trifluoro-3,4-dihydro-1-benzazepin-2-one and (3R)-3-amino-8-(5-tert-butyl-3-pyridyl)-1-[(4-chlorophenyl)methyl]-5,5,7-trifluoro-3,4-dihydro-1-benzazepin-2-one

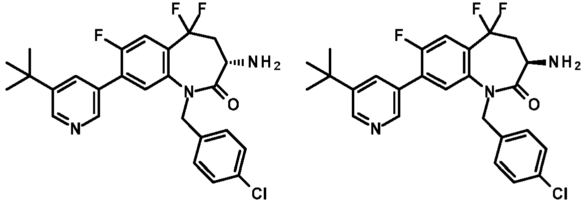

To a solution of tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate [isomer A] (40.0 mg, 0.07 mmol, 1.0 eq) in EtOAc (1 mL) was added HCl/EtOAc (4 M, 8.0 mL, 32.0 mmol, 470.45 eq) at 25 °C and the mixture was stirred at 25 °C for 1 h. The reaction mixture was concentrated in vacuum and the remaining residue was dissolved in water. 3-amino-8-(5-tert-butyl-3-pyridyl)-1-[(4-chlorophenyl)methyl]-5,5,7-trifluoro-3,4-dihydro-1-benzazepin-2-one [isomer A] (25.8 mg, 0.05 mmol, 65% yield) was obtained as dihydrochloride salt as yellow solid by lyophilization. MS (ESI): 488.2 [M+H]⁺, retention time 2.13 minutes. 3-amino-8-(5-tert-butyl-3-pyridyl)-1-[(4-chlorophenyl)methyl]-5,5,7-trifluoro-3,4-dihydro-1-benzazepin-2-one [isomer B] was obtained in analogy from tert-butyl N-[8-(5-tert-butyl-3-pyridyl)-1-[(4-chlorophenyl)methyl]-5,5,7-trifluoro-2-oxo-3,4-dihydro-1-benzazepin-3-yl]carbamate [isomer B]. MS (ESI): 488.2 [M+H]⁺, retention time 1.67 minutes (column: Chiralpak AD-3 50×4.6 mm I.D., 3 µm; mobile phase: phase A for CO₂, and phase B for EtOH (0.05% DEA); gradient elution: from 5% to 40% EtOH (0.05% DEA) in CO₂; flow rate: 3 mL/min; detector: DAD; column temperature: 35°C; back pressure: 100 bar).

### Example 19

### 2-[5-[(3S)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methylpropanenitrile and

### 2-[5-[(3R)-3-amino-5,5,7-trifluoro-1-[[4-[3-fluoro-S-(trifluoromethyl)-2-pyridyl]phenyl]methyl]-2-oxo-3,4-dihydro-1-benzazepin-8-yl]-1,3,4-oxadiazol-2-yl]-2-methylpropanenitrile

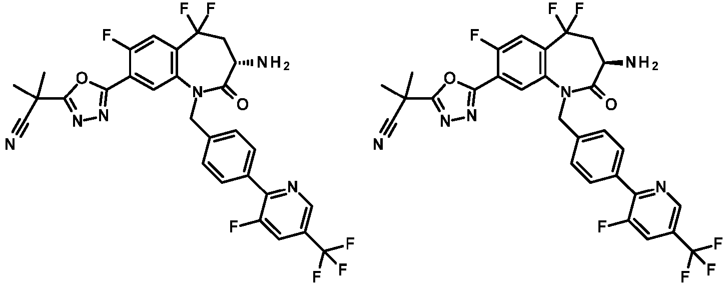

### Step a) tert-butyl N-[8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate

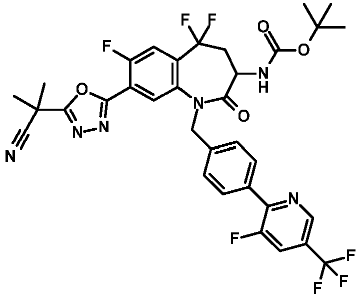

The title compound was prepared in analogy to Example 1, steps a to l (using 2-[4-(chloromethyl)phenyl]-3-fluoro-5-(trifluoromethyl)pyridine (Intermediate 3) instead of 4-(trifluoromethoxy)benzyl bromide in step f and 2-cyano-2-methyl-propanoic acid instead of pivalic acid in step k) and was obtained as white solid. MS (ESI): 719.4 [M+H]⁺.

### Step b) tert-butyl N-[(3S)-8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate

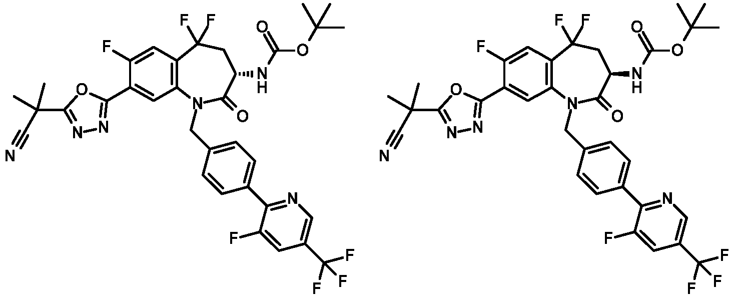

tert-butyl N-[8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate (95 mg) was subjected to preparative SFC (Daicel Chiralcel OD-H (250 mm x 30 mm, 10 µm), 0.1% NH₃-H₂O in MeOH) to obtain tert-butyl *tert-*butyl *N*-[8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate [isomer A] (40 mg) (MS (ESI): 719.4 [M+H]⁺, retention time 1.01 minutes, see conditions below) as white solid and tert-butyl N-[8-[5-(1-cyano-1-methylethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate [isomer B] (41 mg) as white solid. MS (ESI): 719.6 [M+H]⁺, retention time 1.17 minutes (column: Chiralcel OD 50×4.6 mm I.D., 3 µm; mobile phase: phase A for CO₂, and phase B for MeOH (0.05% DEA); gradient elution: from 5% to 40% MeOH (0.05% DEA) in CO₂; flow rate: 3 mL/min; detector: PDA; column temperatur: 35°C; back pressure: 100 bar).

### Step c) 2-[5-[(3S)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methylpropanenitrile and 2-[5-[(3R)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methylpropanenitrile

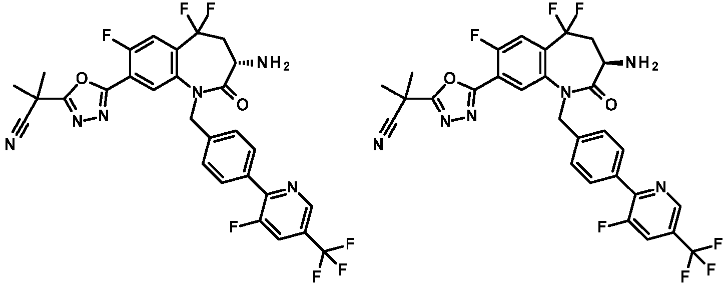

A solution of *tert-*butyl *N*-[8-[5-(1-cyano-1-methyl-ethyl)-1,3,4-oxadiazol-2-yl]-5,5,7-trifluoro-1-[[4-[3-fluoro-5-(trifluoromethyl)-2-pyridyl]phenyl]methyl]-2-oxo-3,4-dihydro-1-benzazepin-3-yl]carbamate [isomer A] (40.0 mg, 0.06 mmol, 1.0 eq) in a mixture of DCM/TFA (3:1, 4.0 mL) was stirred at 25°C for 1 h. The mixture was then diluted with DCM (3 mL) and the pH was adjusted to 8 by addition of saturated NaHCO₃ solution. The resulting mixture was extracted with DCM (10 mL x 3) and the combined organic layers were dried (Na₂SO₄) and concentrated. The residue was dissolved in EtOAc (30 mL) and HCl/EtOAc (4M, 0.14 mL) was added. The mixture was concentrated to obtain 2-[5-[3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methylpropanenitrile [isomer A] (33.0 mg, 0.04 mmol, 83% yield) as a white solid as hydrochloride salt. MS (ESI): 619.3 [M+H]⁺, retention time 1.34 minutes (see conditions below). 2-[5-[3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methyl-propanenitrile [isomer B] was obtained in analogy. MS (ESI): 619.1 [M+H]⁺, retention time 1.70 minutes (column: Chiralcel OD-3 50×4.6 mm I.D., 3 µm; mobile phase: phase A for CO₂, and phase B for MeOH (0.05%DEA); gradient elution: 5% to 40% MeOH (0.05% DEA) in CO₂; flow rate: 3 mL/min; detector: DAD; column temperatur: 35°C; back pressure: 100 bar).

### Example 20

### 2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile

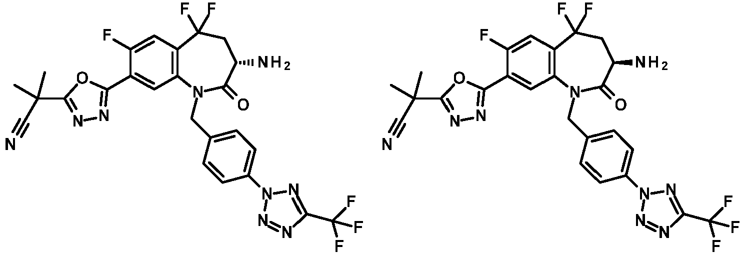

### Step a) tert-butyl N-[8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate

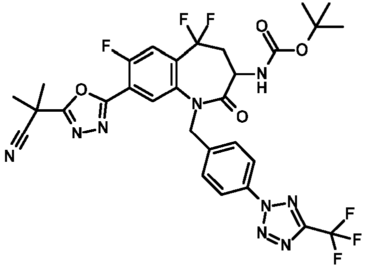

The title compound was prepared in analogy to Example 1, steps a to l (using 2-[4-(bromomethyl)phenyl]-5-(trifluoromethyl)tetrazole (Intermediate 4) instead of 4-(trifluoromethoxy)benzyl bromide in step f and 2-cyano-2-methyl-propanoic acid instead of pivalic acid in step k) and was obtained as light yellow solid. MS (ESI): 636.1 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate

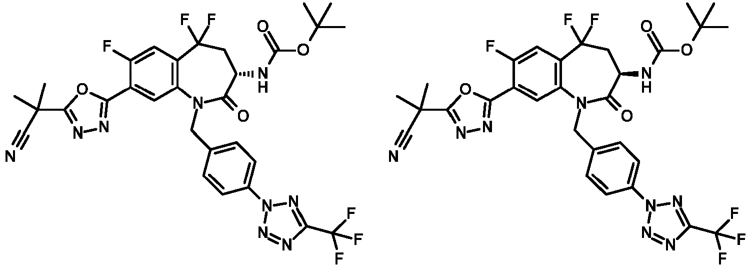

tert-butyl N-[8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate (340 mg) was subjected to preparative SFC (Daicel Chiralpak AD (250 mm x 30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate [isomer A] (105 mg) (MS (ESI): 636.1 [M-isobutene+H]⁺, retention time 0.86 minutes (see conditions below) as light yellow solid and tert-butyl N-[8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate [isomer B] (110 mg) as light yellow solid. MS (ESI): 636.1 [M-isobutene+H]⁺, retention time 1.13 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile and 2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile

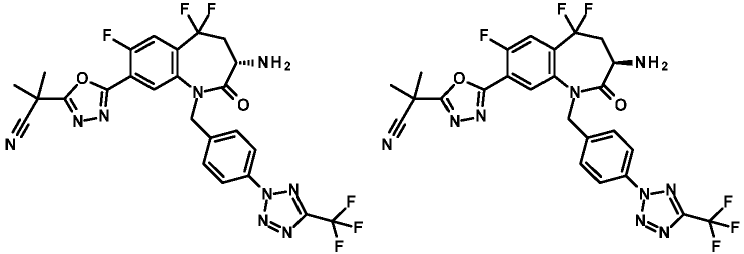

A solution of tert-butyl N-[8-[5-(1-cyano-1-methyl-ethyl)-1,3,4-oxadiazol-2-yl]-5,5,7-trifluoro-2-oxo-1-[[4-[5-(trifluoromethyl)tetrazol-2-yl]phenyl]methyl]-3,4-dihydro-1-benzazepin-3-yl]carbamate [isomer A] (103.0 mg, 0.15 mmol, 1.0 eq) in a mixture of DCM/TFA (3:1, 4.0 mL) was stirred at 20°C for 0.5 h. The pH of the reaction mixture was adjusted to 8 by careful addition of saturated NaHCO₃ solution and the resulting mixture was extracted with DCM (10 mL x 3). The combined organic layers were washed with brine (5 mL), dried (Na₂SO₄) and concentrated. The remaining residue was dissolved in EtOAc (20 mL) and HCl/EtOAc (4M, 0.2 mL) was added. The mixture was concentrated and water (20 mL) was added to the remaining solid. 2-methyl-2-[5-[(3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile [isomer A] was obtained by lyophilization as light yellow solid as a hydrochloride salt (90 mg, 0.14 mmol, 92%). MS (ESI): 592.2 [M+H]⁺, retention time 1.40 minutes (see conditions below). 2-methyl-2-[5-[(3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile [isomer B] was obtained in analogy. MS (ESI): 592.3 [M+H]⁺, retention time 1.83 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 21

### (3S)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one and

### (3R)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one

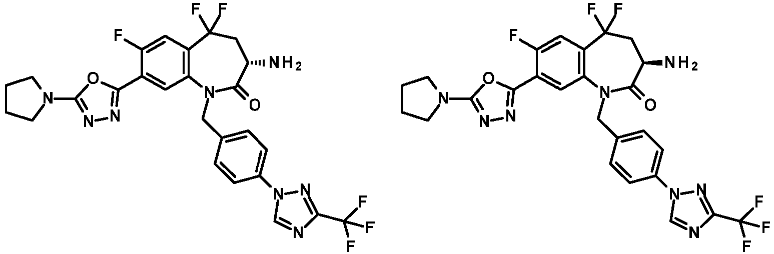

### Step a) tert-butyl N-[(3S)-5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate and tert-butyl N-[(3R)-5,5,7-trifluoro-2-oxo-8-(5-pyrrolidin-1-yl-1,3,4-oxadiazol-2 -yl)-1-[[4-[3-(trifluoromethyl)-1,2,4-triazol-1-yl]phenyl]methyl]-3,4-dihydro-1-benzazepin-3-yl]carbamate

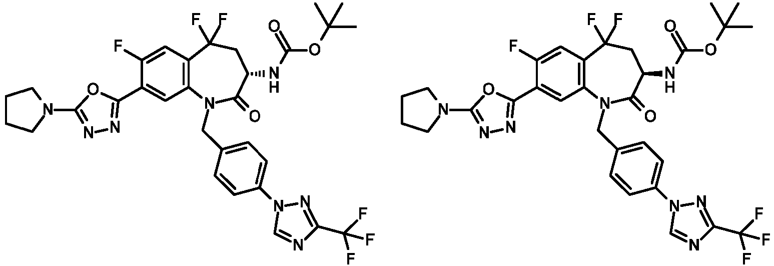

tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate (Example 22, step c) (30 mg) was subjected to preparative SFC (Daicel Chiralpak OD (250 mm x 30 mm, 10 µm), 0.1% NH₃-H₂O in EtOH) to obtain tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate [isomer A] (10 mg) (MS (ESI): 693.2 [M+H]⁺, retention time 1.61 minutes (see conditions below) as white solid and tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate [isomer B] (10 mg) as white solid. MS (ESI): 693.2 [M+H]⁺, retention time 2.07 minutes (column: Chiralpak OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step b) (3S)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one and (3R)-3-amino-5,5,7-trifluoro-8-(5-pyrrolidin-1-yl-1,3,4-oxadiazol-2 -yl)-1-[[4-[3-(trifluoromethyl)-1,2,4-triazol-1-yl]phenyl]methyl]-3,4-dihydro-1-benzazepin-2-one

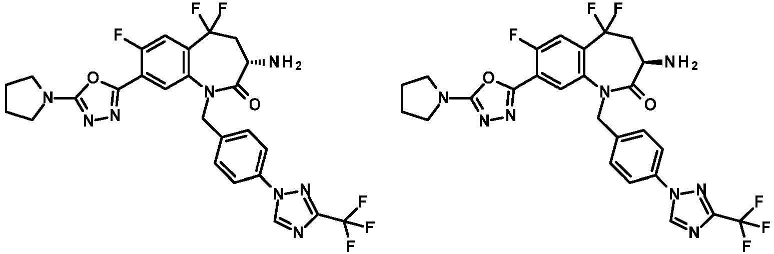

A solution of tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate [isomer A] (10.0 mg, 0.01 mmol, 1.0 eq) in HCl/EtOAc (4 M, 2.1 mL, 8.42 mmol, 582.9 eq) was stirred at 20°C for 0.5 h. The mixture was concentrated and the remaining residue was dissolved in MeCN/water to obtain 3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one [isomer A] (2.0 mg, 20% yield) by lyophilization as a white solid as hydrochloride salt. MS (ESI): 593.2 [M+H]⁺, retention time 0.57 minutes (see conditions below). 3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one [isomer B] was obtained in analogy. MS (ESI): 593.1 [M+H]⁺, retention time 1.69 minutes (column: Chiralcel OD-3 50×4.6 mm I.D., 3 µm; mobile phase: phase A for CO₂, and phase B for EtOH (0.05% DEA); elution: 50% EtOH (0.05% DEA) in CO₂; flow rate: 3 mL/min; detector: PDA; column temperatur: 35°C; back pressure: 100 bar).

### Example 22

### (3S)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one and

### (3R)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one

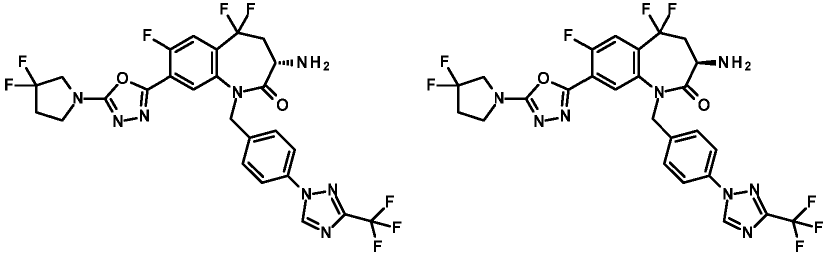

### Step a) tert-butyl N-[5,5,7-trifluoro-8-(hydrazinecarbonyl)-2-oxo-1-[[4-[3-(trifluoromethyl)-1,2,4-triazol-1-yl]phenyl]methyl]-3,4-dihydro-1-benzazepin-3-yl]carbamate

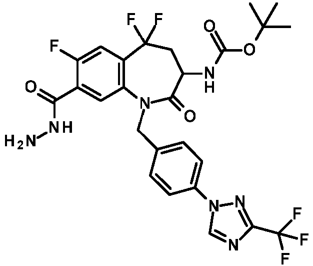

The title compound was prepared in analogy to Example 1, steps a to j (using 1-[4-(bromomethyl)phenyl]-3-(trifluoromethyl)-1,2,4-triazole (Intermediate 5) instead of 4-(trifluoromethoxy)benzyl bromide in step f) and was obtained as yellow solid. MS (ESI): 636.2 [M+Na]⁺.

### Step b) tert-butyl N-[5,5,7-trifluoro-2-oxo-8-(2-oxo-3H-1,3,4-oxadiazol-5-yl)-1-[[4-[3-(trifluoromethyl)-1,2,4-triazol-1-yl]phenyl]methyl]-3,4-dihydro-1-benzazepin-3-yl]carbamate

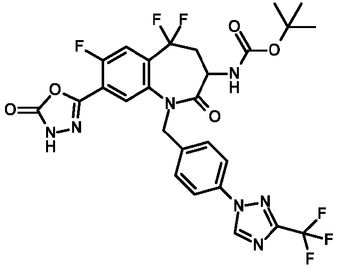

To a solution of tert-butyl N-[5,5,7-trifluoro-8-(hydrazinecarbonyl)-2-oxo-1-[[4-[3-(trifluoromethyl)-1,2,4-triazol-1-yl]phenyl]methyl]-3,4-dihydro-1-benzazepin-3-yl]carbamate (300.0 mg, 0.49 mmol, 1.0 eq) in THF (5 mL) was added CDI (158.58 mg, 0.98 mmol, 2.0 eq) at 25°C and the mixture was stirred at 25°C for 12 h. Then water (20 mL) was added and the mixture was extracted with EtOAc (10 mL x 3). The combined extracts were washed with brine (20 mL), dried (Na₂SO₄) and concetrated to give residue which was purified by column chromatography on silica gel (PE/EtOAc = 10:0 to 1:2) to give the title compound (210.0 mg, 0.33 mmol, 67% yield) as yellow solid. MS (ESI): 640.3 [M+H]⁺.

### Step c) tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate and tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate

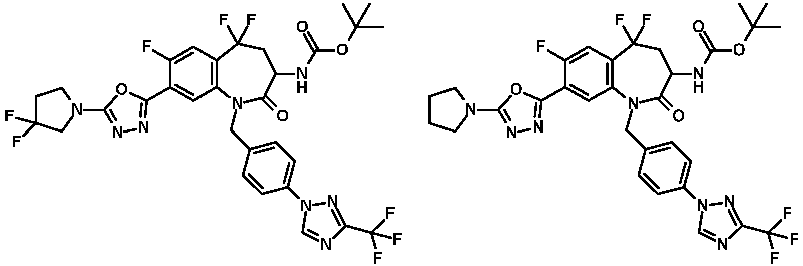

A solution of tert-butyl N-[5,5,7-trifluoro-2-oxo-8-(2-oxo-3H-1,3,4-oxadiazol-5-yl)-1-[[4-[3-(trifluoromethyl)-1,2,4-triazol-1-yl]phenyl]methyl]-3,4-dihydro-1-benzazepin-3-yl]carbamate (200.0 mg, 0.31 mmol, 1.0 eq), PyBroP (153.08 mg, 0.33 mmol, 1.05 eq), DIPEA (0.16 mL, 0.94 mmol, 3.0 eq) and 3,3-difluoropyrrolidine hydrochloride (67.35 mg, 0.47 mmol, 1.5 eq) in 1,4-dioxane (1 mL) was stirred at 20°C for 3 h. The reaction mixture was poured into water (20 mL), the layers were separated and the aqueous phase was extracted with EtOAc (10 mL x 3). The combined organic phases were washed with brine (20 mL), dried (Na₂SO₄) and concentrated. The remaining residue was purified by preparative TLC (PE/EtOAc = 1:2) to give tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate (140.0 mg, 0.19 mmol, 61% yield) as yellow solid (MS (ESI): 729.3 [M+H]⁺) and tert-butyl N-[5,5,7-trifluoro-2-oxo-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-3-yl]carbamate (30.0 mg, 0.04 mmol, 14% yield) as yellow solid. MS (ESI): 693.2 [M+H]⁺.

### Step d) tert-butyl N-[(35)-8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate and tert-butyl N-[(3R)-8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate

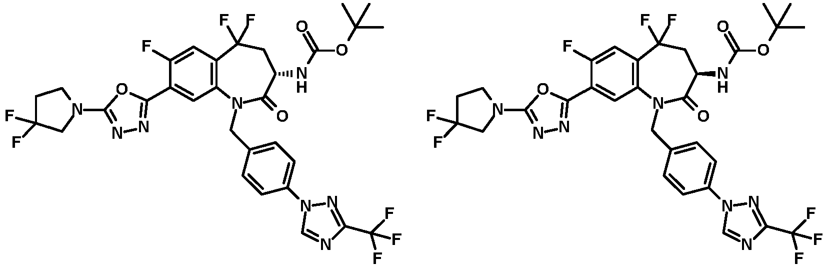

tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate (130 mg) was subjected to preparative SFC (Daicel Chiralpak OX (250 mm x 30 mm, 10 µm), 0.1% NH₃-H₂O in EtOH) to obtain tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate [isomer A] (55 mg, 41% yield) (MS (ESI): 729.2 [M+H]⁺, retention time 1.54 minutes (see conditions below) as light yellow solid and tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate [isomer B] (56 mg, 43% yield) as light yellow solid. MS (ESI): 729.2 [M+H]⁺, retention time 2.13 minutes (column: Chiralpak OX-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step e) (3S)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one and (3R)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one

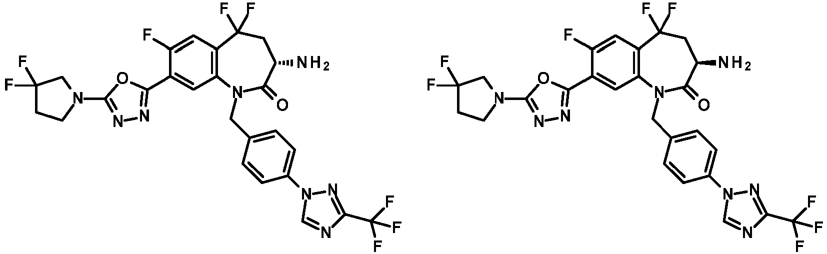

A solution of tert-butyl N-[8-[5-(3,3-difluoropyrrolidin-1-yl)-1,3,4-oxadiazol-2-yl]-5,5,7-trifluoro-2-oxo-1-[[4-[3-(trifluoromethyl)-1,2,4-triazol-1-yl]phenyl]methyl]-3,4-dihydro-1-benzazepin-3-yl]carbamate [isomer A] (50.0 mg, 0.07 mmol, 1.0 eq) in HCl/EtOAc (4 M, 10 mL, 40.0 mmol, 582.9 eq) was stirred at 20°C for 0.5 h. The mixture was concentrated and the remaining residue was dissolved in MeCN/water to obtain 3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one [isomer A] (40.1 mg, 0.06 mmol, 84% yield) by lyophilization as a white solid as hydrochloride salt. MS (ESI): 629.1 [M+H]⁺, retention time 0.94 minutes (see conditions below). 3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one [isomer B] was obtained in analogy. MS (ESI): 629.1 [M+H]⁺, retention time 1.91 minutes (column: Chiralpak IG-3 50×4.6 mm I.D., 3 µm; mobile phase: phase A for CO₂, and phase B for EtOH (0.05% DEA); elution: 60% EtOH (0.05% DEA) in CO₂; flow rate: 3 mL/min; detector: PDA; column temperatur: 35°C; back pressure: 100 bar).

### Example 23

### (3S)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one

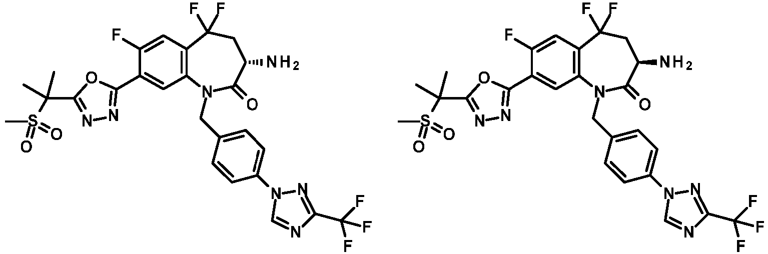

### Step a) tert-butyl N-[5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate

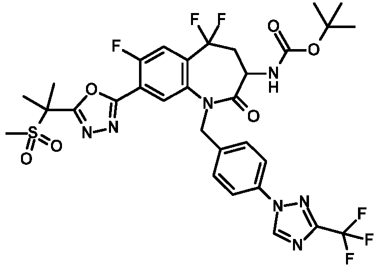

The title compound was prepared in analogy to Example 1, steps a to l (using 1-[4-(bromomethyl)phenyl]-3-(trifluoromethyl)-1,2,4-triazole (Intermediate 5) instead of 4-(trifluoromethoxy)benzyl bromide in step f and 2-methyl-2-methylsulfonyl-propanoic acid instead of pivalic acid in step k) and was obtained as light yellow solid. MS (ESI): 614.1 [M+H]⁺.

### Step b) tert-butyl N-[(3S)-5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate and tert-butylN-[(3R)-5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate

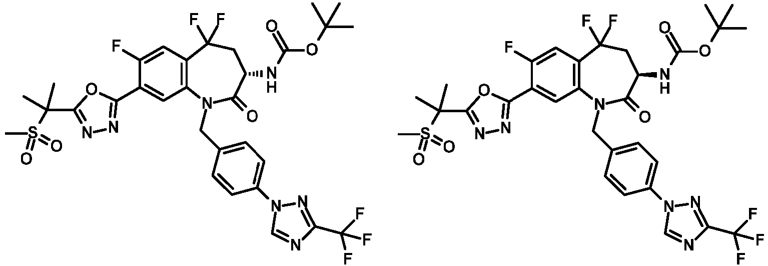

tert-butyl N-[5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate (340 mg) was subjected to preparative SFC (Daicel Chiralpak AD (250 mm x 30 mm, 10 µm), 0.1% NH₃-H₂O in EtOH) to obtain tert-butyl N-[5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate [isomer A] (30 mg) (MS (ESI): 688.1 [M-isobutene+H]⁺, retention time 1.20 minutes (see conditions below) as light yellow solid and tert-butyl N-[5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate [isomer B] (40 mg) as light yellow solid. MS (ESI): 688.3 [M-isobutene+H]⁺, retention time 2.08 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) (3S)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one and (3R)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one

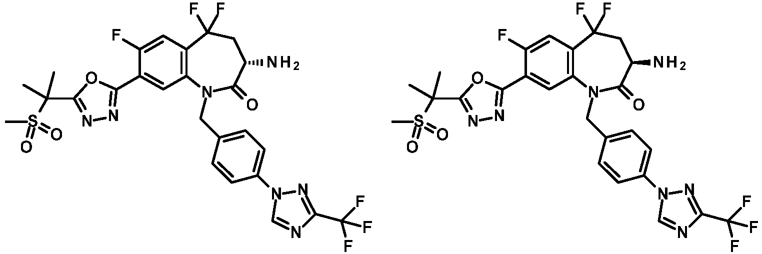

A solution of tert-butyl N-[5,5,7-trifluoro-8-[5-(1-methyl-1-methylsulfonyl-ethyl)-1,3,4-oxadiazol-2-yl]-2-oxo-1-[[4-[3-(trifluoromethyl)-1,2,4-triazol-1-yl]phenyl]methyl]-3,4-dihydro-1-benzazepin-3-yl]carbamate [isomer A] (30.0 mg, 0.04 mmol, 1.0 eq) in HCl/EtOAc (4 M, 7.5 mL, 30.0 mmol, 743.7 eq) was stirred at 20°C for 0.5 h. The mixture was concentrated to obtain 3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one [isomer A] (24.8 mg, 0.04 mmol, 87% yield) as a yellow solid as hydrochloride salt. MS (ESI): 644.1 [M+H]⁺, retention time 1.99 minutes (see conditions below). 3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one [isomer B] was obtained in analogy. MS (ESI): 644.1 [M+H]⁺, retention time 2.44 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 24

### 2-methyl-2-[5-[(3S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile

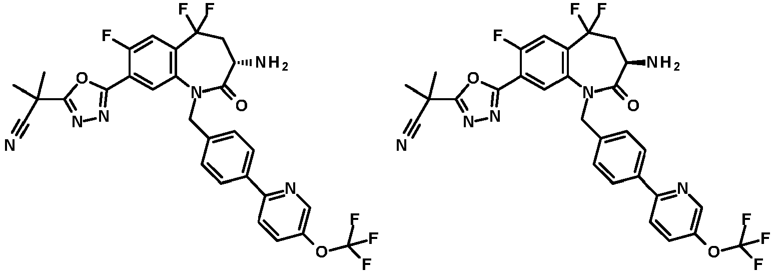

### Step a) tert-butyl N-[8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate

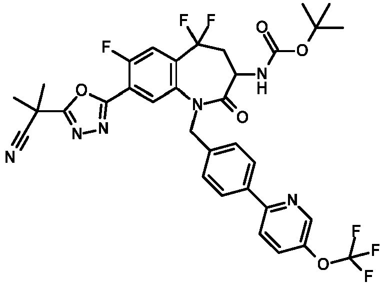

The title compound was prepared in analogy to Example 1, steps a to l (using 2-[4-(chloromethyl)phenyl]-5-(trifluoromethoxy)pyridine, hydrochloride [CAS#2338841-95-3] instead of 4-(trifluoromethoxy)benzyl bromide in step f and 2-cyano-2-methyl-propanoic acid instead of pivalic acid in step k) and was obtained as light yellow solid. MS (ESI): 717.5 [M+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate

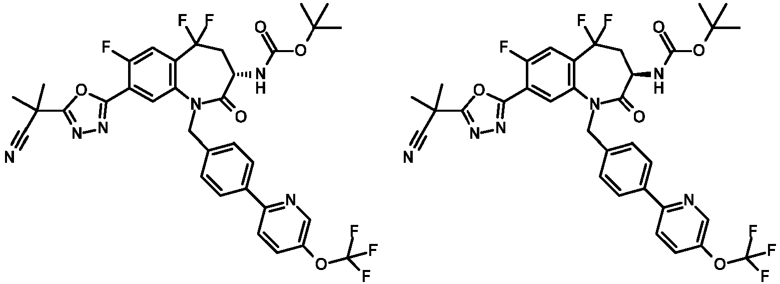

tert-butyl N-[8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate (260 mg) was subjected to preparative SFC (Daicel Chiralpak IC (250 mm x 30 mm, 10 µm), 0.1% NH₃-H₂O in iPrOH) to obtain tert-butyl N-[8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate [isomer A] (97 mg) (MS (ESI): 717.3 [M+H]⁺, retention time 1.74 minutes (see conditions below) as white solid and tert-butyl N-[8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate [isomer B] (98 mg) as white solid. MS (ESI): 717.4 [M+H]⁺, retention time 2.35 minutes (column: Chiralpak IC-3 50×4.6 mm I.D., 3 µm; mobile phase: phase A for CO₂, and phase B for iPrOH (0.05% DEA); gradient elution: 5% to 40% iPrOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile and 2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile

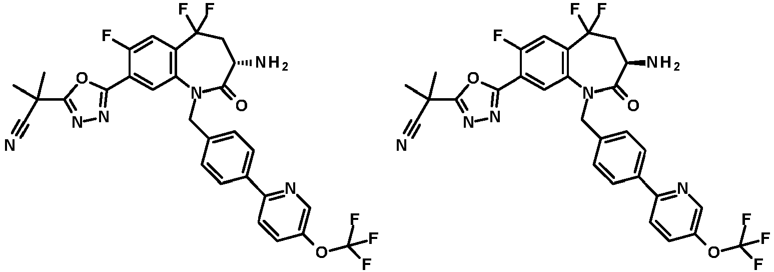

A solution of tert-butyl N-[8-[5-(1-cyano-1-methyl-ethyl)-1,3,4-oxadiazol-2-yl]-5,5,7-trifluoro-2-oxo-1-[[4-[5-(trifluoromethoxy)-2-pyridyl]phenyl]methyl]-3,4-dihydro-1-benzazepin-3-yl]carbamate [isomer A] (95.0 mg, 0.13 mmol, 1.0 eq) in a mixture of DCM/TFA (3:1, 4.0 mL) was stirred at 20°C for 1 h. Then DCM (20 mL) was added and the pH of the reaction mixture was adjusted to 8 by careful addition of saturated NaHCO₃ solution. The resulting mixture was extracted with DCM (10 mL x 3) and the combined organic layers were washed with brine (10 mL), dried (Na₂SO₄) and concentrated. The remaining residue was dissolved in EtOAc (30 mL) and HCl/EtOAc (4M, 0.1 mL) was added. The mixture was concentrated and water (20 mL) was added to the remaining solid. 2-methyl-2-[5-[3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile [isomer A] was obtained by lyophilization as white solid as a hydrochloride salt (67 mg, 0.1 mmol, 76%). MS (ESI): 617.4 [M+H]⁺, retention time 1.20 minutes (see conditions below). 2-methyl-2-[5-[3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile [isomer B] was obtained in analogy. MS (ESI): 617.5 [M+H]⁺, retention time 1.82 minutes (column: (S.S)Whelk-O1 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 26

### (3S)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one

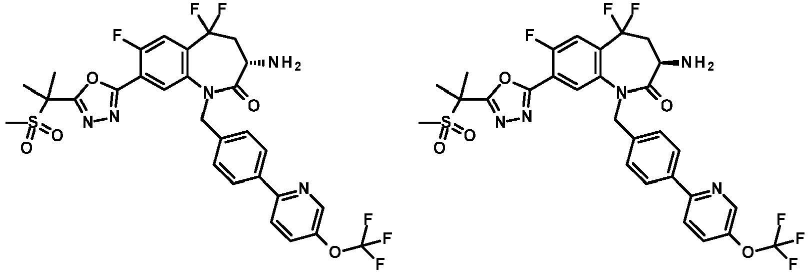

### Step a) tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl]methyl]-3,4-dihydro-1-benzazepin-3-yl]carbamate

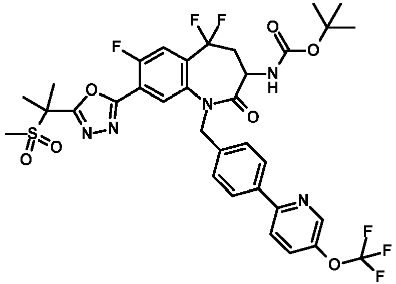

The title compound was prepared in analogy to Example 1, steps a to l (using 2-[4-(chloromethyl)phenyl]-5-(trifluoromethoxy)pyridine, hydrochloride [CAS# 2338841-95-3] instead of 4-(trifluoromethoxy)benzyl bromide in step f and 2-methyl-2-methylsulfonyl-propanoic acid instead of pivalic acid in step k) and was obtained as light yellow solid. MS (ESI): 770.3 [M+H]⁺.

### Step b) tert-butyl N-[(3S)-5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl[methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[[4-[5-(trifluoromethox)-2-pyridyl]phenyl]methyl]-3,4-dihydro-1-benzazepin-3-yl]carbamate

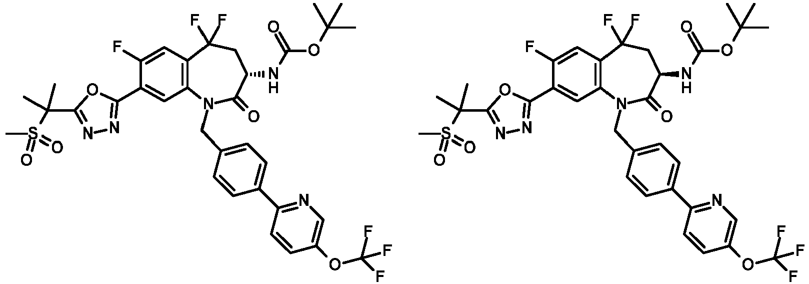

tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl]methyl]-3,4-dihydro-1-benzazepin-3-yl]carbamate (250 mg) was subjected to preparative SFC (Daicel Chiralpak IC (250 mm x 30 mm, 10 µm), 0.1% NH₃-H₂O in iPrOH) to obtain tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl]methyl]-3,4-dihydro-1-benzazepin-3-yl]carbamate [isomer A] (97 mg) (MS (ESI): 770.3 [M+H]⁺, retention time 2.01 minutes (see conditions below) as white solid and tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl]methyl]-3,4-dihydro-1-benzazepin-3-yl]carbamate [isomer B] (92 mg) as white solid. MS (ESI): 770.5 [M+H]⁺, retention time 2.57 minutes (column: Chiralpak IC-3 50×4.6 mm I.D., 3 µm; mobile phase: phase A for CO₂, and phase B for iPrOH (0.05% DEA); gradient elution: 5% to 40% iPrOH (0.05% DEA) in CO₂; flow rate: 3 mL/min; detector: PDA; column temperatur: 35°C; back pressure: 100 bar).

### Step c) (3S)-3-amino-5,5, 7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one and (3R)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one

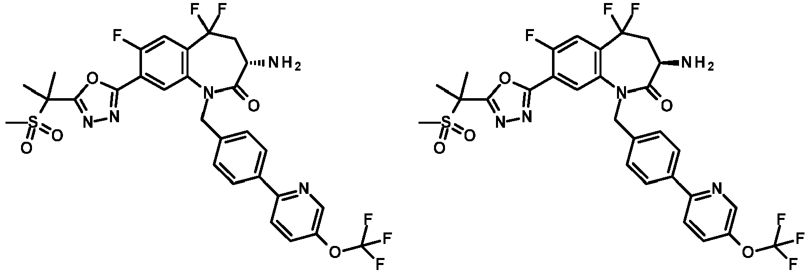

A solution of tert-butyl N-[5,5,7-trifluoro-8-[5-(1-methyl-1-methylsulfonyl-ethyl)-1,3,4-oxadiazol-2-yl]-2-oxo-1-[[4-[5-(trifluoromethoxy)-2-pyridyl]phenyl]methyl]-3,4-dihydro-1-benzazepin-3-yl]carbamate [isomer A] (95.0 mg, 0.12 mmol, 1.0 eq) in HCl/EtOAc (4 M, 10 mL, 40.0 mmol, 324.1 eq) was stirred at 20°C for 1 h. The mixture was concentrated and the remaining residue was dissolved in water (20 mL) to obtain 3-amino-5,5,7-trifluoro-8-[5-(1-methyl-1-methylsulfonylethyl)-1,3,4-oxadiazol-2-yl]-1-[[4-[5-(trifluoromethoxy)-2-pyridyl]phenyl]methyl]-3,4-dihydro-1-benzazepin-2-one [isomer A] (74.1 mg, 0.1 mmol, 83% yield) by lyophilization as a white solid as hydrochloride salt. MS (ESI): 670.4 [M+H]⁺, retention time 1.96 minutes (see conditions below). 3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one [isomer B] was obtained in analogy. MS (ESI): 670.4 [M+H]⁺, retention time 2.13 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 27

### 2-[5-[(3S)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile and

### 2-[5-[(3R)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile

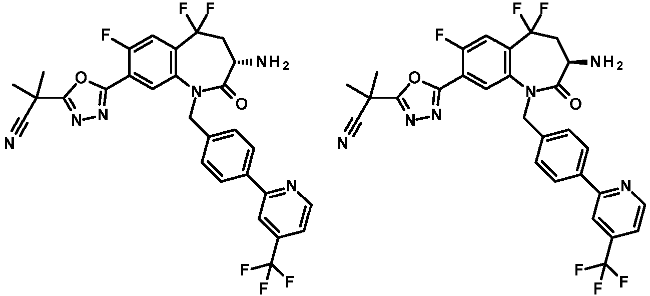

### Step a) tert-butyl N-[8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate

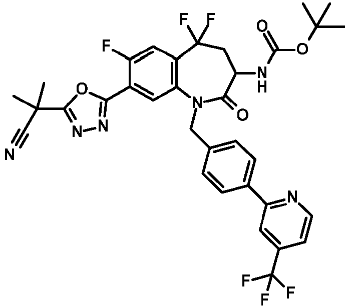

The title compound was prepared in analogy to Example 1, steps a to l (using 2-[4-(chloromethyl)phenyl]-4-(trifluoromethyl)pyridine; hydrochloride (Intermediate 6) instead of 4-(trifluoromethoxy)benzyl bromide in step f and 2-cyano-2-methyl-propanoic acid instead of pivalic acid in step k) and was obtained as light yellow solid. MS (ESI): 701.5 [M+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-[-benzazepin-3-yl]carbamate

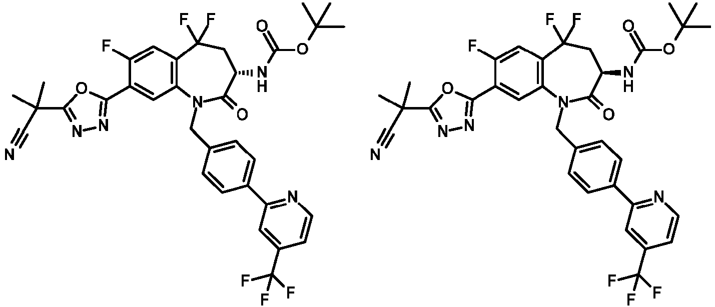

tert-butyl N-[8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate (270 mg) was subjected to preparative SFC (Daicel Chiralcel OD (250 mm x 30 mm, 10 µm), 0.1% NH₃-H₂O in iPrOH) to obtain tert-butyl N-[8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate [isomer A] (56 mg) (MS (ESI): 701.5 [M+H]⁺, retention time 1.08 minutes (see conditions below) as light yellow solid and tert-butyl N-[8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate [isomer B] (73 mg) as light yellow solid. MS (ESI): 701.4 [M+H]⁺, retention time 1.29 minutes (column: Chiralcel OD 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-[5-[(3S)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile and 2-[5-[(3R)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile

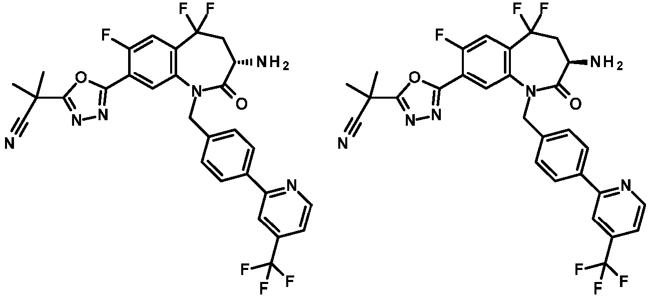

A solution of tert-butyl N-[8-[5-(1-cyano-1-methyl-ethyl)-1,3,4-oxadiazol-2-yl]-5,5,7-trifluoro-2-oxo-1-[[4-[4-(trifluoromethyl)-2-pyridyl]phenyl]methyl]-3,4-dihydro-1-benzazepin-3-yl]carbamate [isomer A] (55.2 mg, 0.08 mmol, 1.0 eq) in a mixture of DCM/TFA (3:1, 4.0 mL) was stirred at 20°C for 0.5 h. The pH of the reaction mixture was adjusted to 8 by careful addition of saturated NaHCO₃ solution. The resulting mixture was extracted with DCM (10 mL x 3) and the combined organic layers were washed with brine (10 mL), dried (Na₂SO₄) and concentrated. The remaining residue was dissolved in EtOAc (20 mL) and HCl/EtOAc (4M, 0.2 mL) was added. The mixture was concentrated and water (20 mL) was added to the remaining solid. 2-[5-[3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile [isomer A] was obtained by lyophilization as light yellow solid as a hydrochloride salt (49.6 mg, 0.08 mmol, 97%). MS (ESI): 601.4 [M+H]⁺, retention time 1.28 minutes (see conditions below). 2-[5-[3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile [isomer B] was obtained in analogy. MS (ESI): 601.2 [M+H]⁺, retention time 1.86 minutes (column: (S.S)Whelk-O1 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 28

### 2-[5-[(3S)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile and

### 2-[5-[(3R)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile

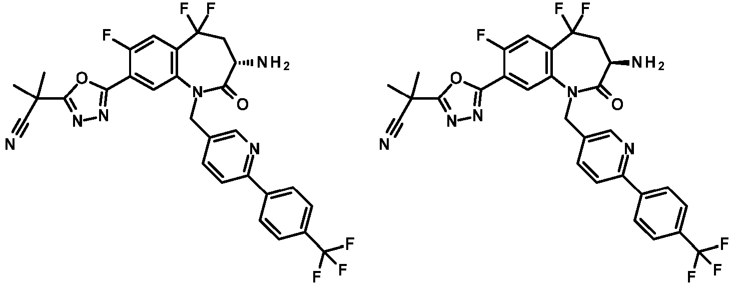

### Step a) tert-butyl N-[8-[5-(1-cyano-1-methyl-ethyl)-1, 3, 4-oxadiazol-2-yl]-5,5, 7-trifluoro-2-oxo-1-[[6-[4-(trifluoromethyl)phenyl]-3-pyridyl]methyl]-3,4-dihydro-1-benzazepin-3-yl]carbamate

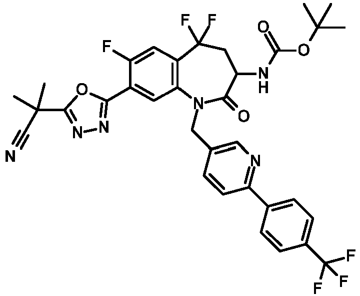

The title compound was prepared in analogy to Example 1, steps a to l (using 5-(chloromethyl)-2-[4-(trifluoromethyl)phenyl]pyridine [CAS# 851507-54-5] instead of 4-(trifluoromethoxy)benzyl bromide in step f and 2-cyano-2-methyl-propanoic acid instead of pivalic acid in step k) and was obtained as light yellow solid. MS (ESI): 701.4 [M+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl[methyl]-3,4-dihydro-1-benzazepin-3-yl]carbamate

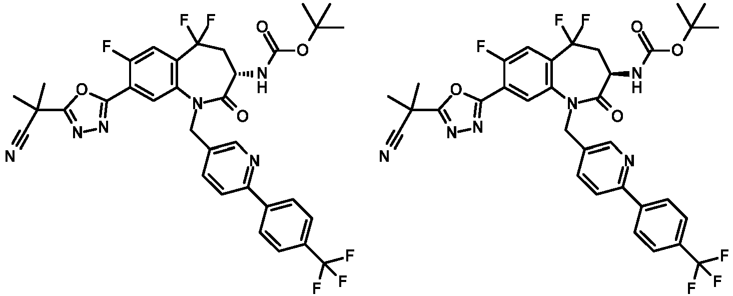

tert-butyl N-[8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl]methyl]-3,4-dihydro-1-benzazepin-3-yl]carbamate was subjected to preparative SFC (Daicel Chiralcel OD (250 mm x 30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl]methyl]-3,4-dihydro-1-benzazepin-3-yl]carbamate [isomer A] (42 mg) (MS (ESI): 701.5 [M+H]⁺, retention time 1.31 minutes (see conditions below) as light yellow solid and tert-butyl N-[8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl]methyl]-3,4-dihydro-1-benzazepin-3-yl]carbamate [isomer B] (68 mg) as light yellow solid. MS (ESI): 701.5 [M+H]⁺, retention time 1.59 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-[5-[(3S)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile and 2-[5-[(3R)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile

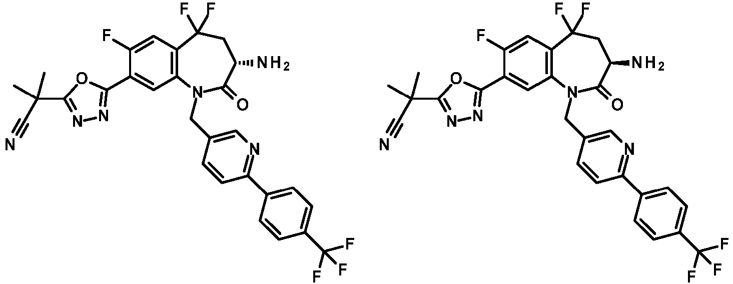

A solution of tert-butyl N-[8-[5-(1-cyano-1-methyl-ethyl)-1,3,4-oxadiazol-2-yl]-5,5,7-trifluoro-2-oxo-1-[[6-[4-(trifluoromethyl)phenyl]-3-pyridyl]methyl]-3,4-dihydro-1-benzazepin-3-yl]carbamate [isomer A] (41.2 mg, 0.06 mmol, 1.0 eq) in a mixture of DCM/TFA (3:1, 4.0 mL) was stirred at 20°C for 0.5 h. The pH of the reaction mixture was adjusted to 8 by careful addition of saturated NaHCO₃ solution. The resulting mixture was extracted with DCM (10 mL x 3) and the combined organic layers were washed with brine (10 mL), dried (Na₂SO₄) and concentrated. The remaining residue was dissolved in EtOAc (20 mL) and HCl/EtOAc (4M, 0.2 mL) was added. The mixture was concentrated and water (20 mL) was added to the remaining solid. 2-[5-[3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile [isomer A] was obtained by lyophilization as light yellow solid as a hydrochloride salt (36.4 mg, 0.06 mmol, 96%). MS (ESI): 601.2 [M+H]⁺, retention time 1.75 minutes (see conditions below). 2-[5-[3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile [isomer B] was obtained in analogy. MS (ESI): 601.3 [M+H]⁺, retention time 2.22 minutes (column: Chiralcel OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Example 29

### (3S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one

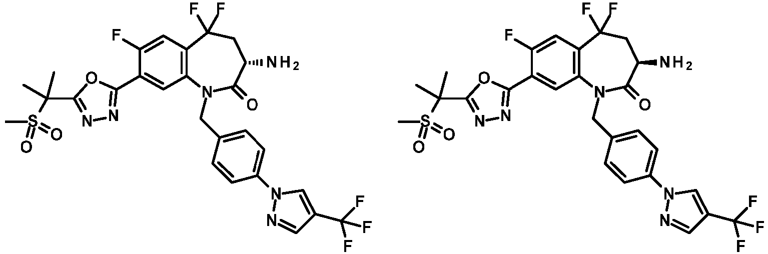

### Step a) tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate

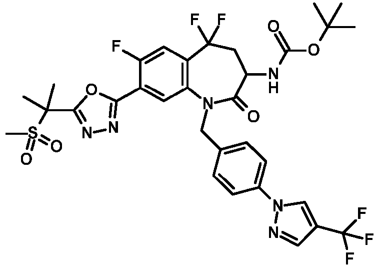

The title compound was prepared in analogy to Example 1, steps a to l (using 1-[4-(chloromethyl)phenyl]-4-(trifluoromethyl)pyrazole [CAS# 1486714-19-5] instead of 4-(trifluoromethoxy)benzyl bromide in step f and 2-methyl-2-methylsulfonyl-propanoic acid instead of pivalic acid in step k) and was obtained as yellow oil. MS (ESI): 687.0 [M-isobutene+H]⁺.

### Step b) tert-butyl N-[(3S)-5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate

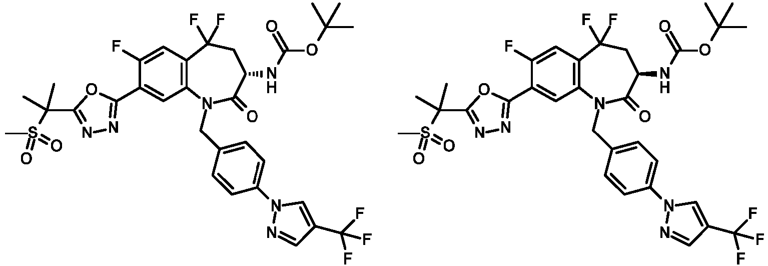

tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate was subjected to preparative SFC (Daicel Chiralpak AD (250 mm x 30 mm, 10 µm), 0.1% NH₃-H₂O in EtOH) to obtain tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate [isomer A] (70 mg) (MS (ESI): 743.4 [M+H]⁺, retention time 1.18 minutes (see conditions below) as yellow solid and tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate [isomer B] (70 mg) as yellow solid. MS (ESI): 743.4 [M+H]⁺, retention time 1.87 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) (3S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one and (3R)-3-amino-3,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one

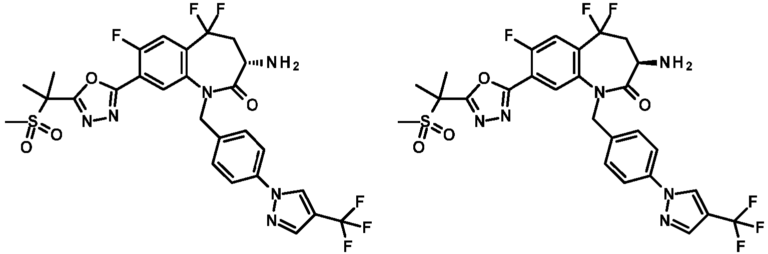

A solution of tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)pyrazol-1-yl]phenyl]methyl]-3,4-dihydro-1-benzazepin-3-yl]carbamate [isomer A] (65.0 mg, 0.09 mmol, 1.0 eq) in HCl/EtOAc (4 M, 3.45 mL, 13.8 mmol, 157.6 eq) was stirred at 25°C for 1 h. The mixture was concentrated and the remaining residue was purified by preparative HPLC (column: Phenomenex Luna C18 75x30mm, 3 µm; water (containing HCl) / MeCN) to obtain 3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one [isomer A] (22.3 mg, 0.03 mmol, 36% yield) as a white solid as hydrochloride salt. MS (ESI): 643.2 [M+H]⁺, retention time 1.72 minutes (see conditions below). 3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one [isomer B] was obtained in analogy. MS (ESI): 643.2 [M+H]⁺, retention time 2.04 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 30

### 2-[5-[(3S)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile and

### 2-[5-[(3R)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile

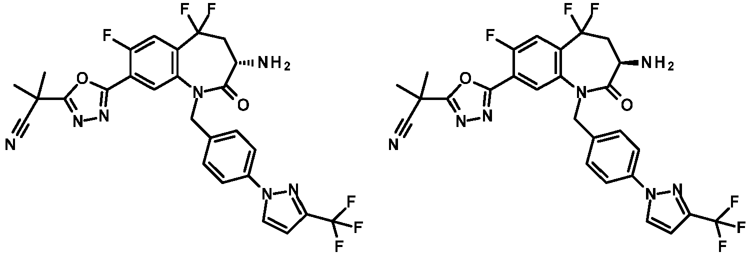

### Step a) tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro- 1-benzazepin-3-yl] carbamate

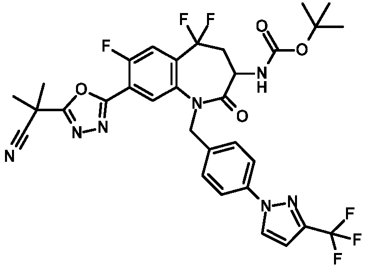

The title compound was prepared in analogy to Example 1, steps a to l (using 1-[4-(bromomethyl)phenyl]-3-(trifluoromethyl)pyrazole [CAS# 1284983-51-2] instead of 4-(trifluoromethoxy)benzyl bromide in step f and 2-cyano-2-methyl-propanoic acid instead of pivalic acid in step k) and was obtained as yellow oil. MS (ESI): 634.1 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate

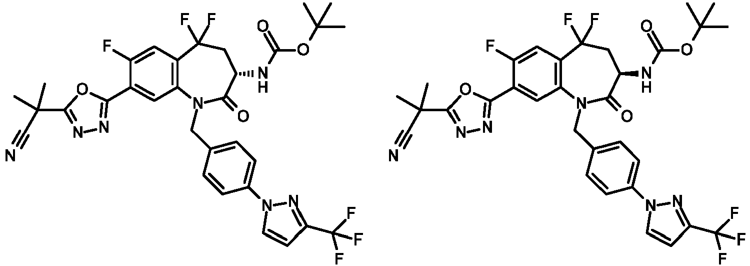

tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate (25 mg) was subjected to preparative SFC (Daicel Chiralcel OX-3 (50×4.6 mm, 3 µm), 0.05% DEA in EtOH) to obtain tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate [isomer A] (11 mg) (MS (ESI): 634.1 [M-isobutene+H]⁺, retention time 1.12 minutes (see conditions below) as off-white solid and tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate [isomer B] (10 mg) as off-white solid. MS (ESI): 634.1 [M-isobutene+H]⁺, retention time 1.50 minutes (column: Chiralcel OX-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-[5-[(3S)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile and 2-[5-[(3R)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile

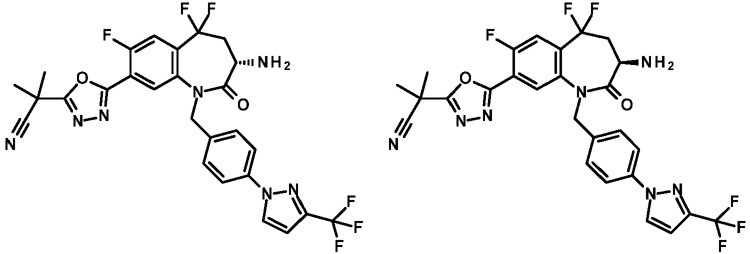

A solution of tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate [isomer A] (11 mg, 0.02 mmol, 1.0 eq) in a mixture of DCM/TFA (3:1, 4.0 mL) was stirred at 25°C for 1 h. DCM (3 mL) was added and the pH of the mixture was adjusted to 8 by careful addition of saturated NaHCO₃ solution. The resulting mixture was extracted with DCM (5 mL x 3) and the combined organic layers were washed with brine (10 mL), dried (Na₂SO₄) and concentrated to obtain 2-[5-[3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile [isomer A] as yellow solid (5.3 mg, 0.01 mmol, 54%). MS (ESI): 590.2 [M+H]⁺, retention time 1.55 minutes (see conditions below). 2-[5-[3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile [isomer B] was obtained in analogy. MS (ESI): 590.1 [M+H]⁺, retention time 2.27 minutes (column: Chiralcel OX-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 32

### (3S)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one and

### (3R)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl] phenyl] methyl]-3,4-dihydro-1-benzazepin-2-one

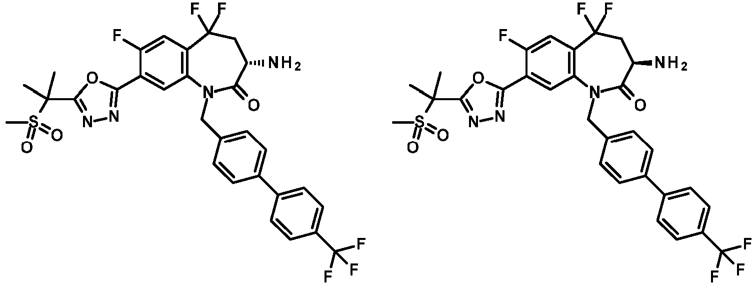

### Step a) tert-butyl N-[5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate

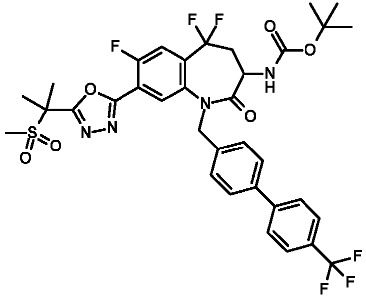

The title compound was prepared in analogy to Example 1, steps a to l (using 1-(chloromethyl)-4-[4-(trifluoromethyl)phenyl]benzene [CAS# 454464-38-1] instead of 4-(trifluoromethoxy)benzyl bromide in step f and 2-methyl-2-methylsulfonyl-propanoic acid instead of pivalic acid in step k) and was obtained as light yellow solid. MS (ESI): 697.2 [M-isobutene+H]⁺.

### Step b) tert-butyl N-[(3S)-5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate and tert-butyl N-[(3R)-5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate

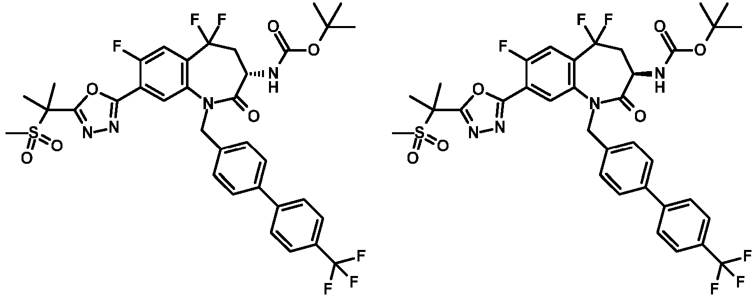

*tert*-butyl *N*-[5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate (190 mg) was subjected to preparative SFC (Daicel Chiralpak AD (250×30 mm, 10 µm), 0.1% NH₃-H₂O in EtOH) to obtain *tert*-butyl *N-*[5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate [isomer A] (60 mg) (MS (ESI): 697.3 [M-isobutene+H]⁺, retention time 1.16 minutes (see conditions below) as white solid and *tert*-butyl *N*-[5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate [isomer B] (65 mg) as white solid. MS (ESI): 697.3 [M-isobutene+H]⁺, retention time 1.81 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) (3S)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one and (3R)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one

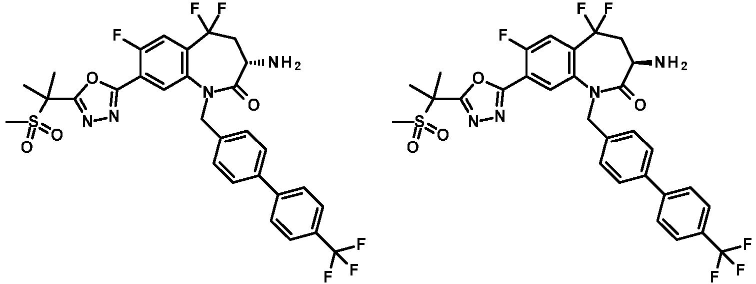

A solution of *tert*-butyl *N*-[5,5,7-trifluoro-8-[5-(1-methyl-1-methylsulfonyl-ethyl)-1,3,4-oxadiazol-2-yl]-2-oxo-1-[[4-[4-(trifluoromethyl)phenyl]phenyl]methyl]-3,4-dihydro-1-benzazepin-3-yl]carbamate [isomer A] (55.0 mg, 0.07 mmol, 1.0 eq) in HCl/EtOAc (4 M, 5.0 mL, 20.0 mmol, 273.7 eq) was stirred at 20°C for 1 h. The mixture was concentrated and water (20 mL) was added to the remaining residue. 3-amino-5,5,7-trifluoro-8-[5-(1-methyl-1-methylsulfonyl-ethyl)-1,3,4-oxadiazol-2-yl]-1-[[4-[3-(trifluoromethyl)phenoxy]phenyl]methyl]-3,4-dihydro-1-benzazepin-2-one [isomer A] (44 mg, 0.06 mmol, 86% yield) was obtained by lyophilization as an off-white solid as hydrochloride salt by . MS (ESI): 653.3 [M+H]⁺, retention time 1.82 minutes (see conditions below). 3-amino-5,5,7-trifluoro-8-[5-(1-methyl-1-methylsulfonyl-ethyl)-1,3,4-oxadiazol-2-yl]-1-[[4-[3-(trifluoromethyl)phenoxy]phenyl]methyl]-3,4-dihydro-1-benzazepin-2-one [isomer B] was obtained in analogy. MS (ESI): 653.3 [M+H]⁺, retention time 2.13 minutes (column: Chiralcel OD 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 33

### 2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile and

### 2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile

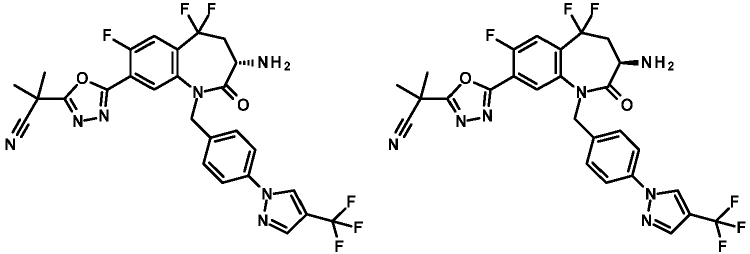

### Step a) tert-butyl N-[8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate

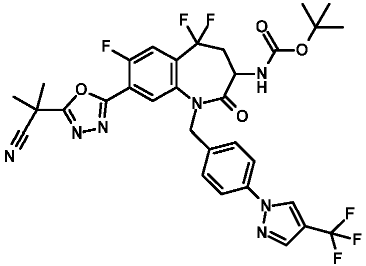

The title compound was prepared in analogy to Example 1, steps a to l (using 1-[4-(chloromethyl)phenyl]-4-(trifluoromethyl)pyrazole [CAS# 1486714-19-5] instead of 4-(trifluoromethoxy)benzyl bromide in step f and 2-cyano-2-methyl-propanoic acid instead of pivalic acid in step k) and was obtained as yellow oil. MS (ESI): 634.0 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate

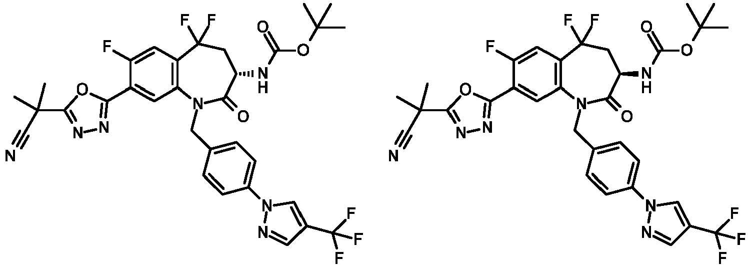

*tert-butyl N*-[8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate (28 mg) was subjected to preparative SFC (Daicel Chiralpak IG-3 (50×4.6 mm, 3 µm), 0.05% DEA in EtOH) to obtain *tert*-butyl *N*-[8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate [isomer A] (12 mg) (MS (ESI): 634.2 [M-isobutene+H]⁺, retention time 0.93 minutes (see conditions below) as off-white solid and *tert*-butyl *N*-[8-[5-(1-cyano-1-methyl-ethyl)-1,3,4-oxadiazol-2-yl]-5,5,7-trifluoro-2-oxo-1-[[4-[4-(trifluoromethyl)pyrazol-1-yl]phenyl]methyl]-3,4-dihydro-1-benzazepin-3-yl]carbamate [isomer B] (13 mg) as off-white solid. MS (ESI): 634.2 [M-isobutene+H]⁺, retention time 1.33 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-[5-[(3S)-3-amino-5,5, 7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile and 2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile

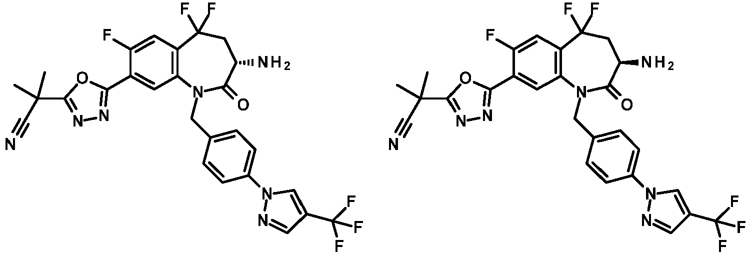

A solution of tert-butyl N-[8-[5-(1-cyano-1-methyl-ethyl)-1,3,4-oxadiazol-2-yl]-5,5,7-trifluoro-2-oxo-1-[[4-[3-(trifluoromethyl)pyrazol-1-yl]phenyl]methyl]-3,4-dihydro-1-benzazepin-3-yl]carbamate [isomer A] (11 mg, 0.02 mmol, 1.0 eq) in a mixture of DCM/TFA (3:1, 4.0 mL) was stirred at 25°C for 1 h. DCM (3 mL) was added and the pH of the mixture was adjusted to 8 by careful addition of saturated NaHCO₃ solution. The resulting mixture was extracted with DCM (5 mL x 3) and the combined organic layers were washed with brine (10 mL), dried (Na₂SO₄) and concentrated. The remaining residue was dissolved in EtOAc (30 mL) and HCl/EtOAc (4M, 0.1 mL, 0.4 mmol, 25.1 eq) was added. The mixture was concentrated to obtain 2-[5-[(3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile [isomer A] as yellow solid as hydrochloride salt (10 mg, 0.02 mmol, 100%). MS (ESI): 590.2 [M+H]⁺, retention time 1.41 minutes (see conditions below). 2-[5-[(3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile [isomer B] was obtained in analogy. MS (ESI): 590.1 [M+H]⁺, retention time 1.76 minutes (column: Chiralcel OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Example 34

### (3S)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one and

### (3R)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one

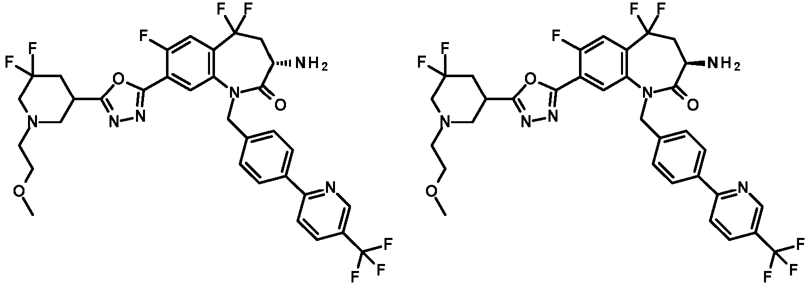

### Step a) ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate

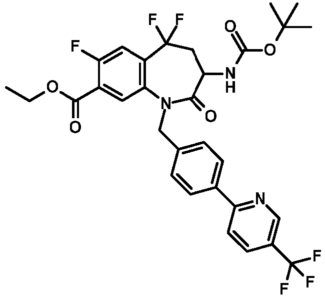

The title compound was prepared in analogy to Example 1, steps a to h (using 2-[4-(bromomethyl)phenyl]-5-(trifluoromethyl)pyridine [CAS#1056641-21-4]; hydrochloride instead of 4-(trifluoromethoxy)benzyl bromide in step f) and was obtained as light yellow solid. MS (ESI): 638.3 [M-isobutene+H]⁺.

### Step b) ethyl (3S)-3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate and ethyl (3R)-3-(tert-butoxycarbonylamino)-5,5,7-trifluoro-2-oxo-1-[[4-[5-(trifluoromethyl)-2pyridyl]phenyl]methyl]-3,4-dihydro-1-benzazepine-8-carboxylate

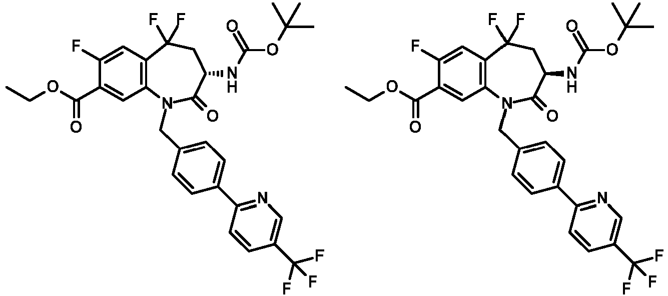

ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate (850 mg) was subjected to preparative SFC (Instrument: Waters 80Q; mobile phase: 50% iPrOH (0.1% NH₃-H₂O) in supercritical CO₂, flow rate: 50 g/min, back pressure: 100 bar) to obtain ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate [isomer A] (300 mg) (MS (ESI): 638.3 [M+H]⁺, retention time 1.00 minutes (see conditions below) as light yellow solid and ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate [isomer B] (350 mg) as light yellow solid. MS (ESI): 634.2 [M+H]⁺, retention time 1.20 minutes (column: Chiralpak AD-3 50×4.6 mm I.D., 3 µm; mobile phase: phase A for CO₂, and phase B for iPrOH (0.05% DEA); gradient elution: 5% to 40% iPrOH (0.05% DEA) in CO₂; flow rate: 3 mL/min; detector: PDA; column temperatur: 35°C; back pressure: 100 bar).

### Step c) tert-butyl N-[(3S)-8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate and tert-butyl N-[(3R)-8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate

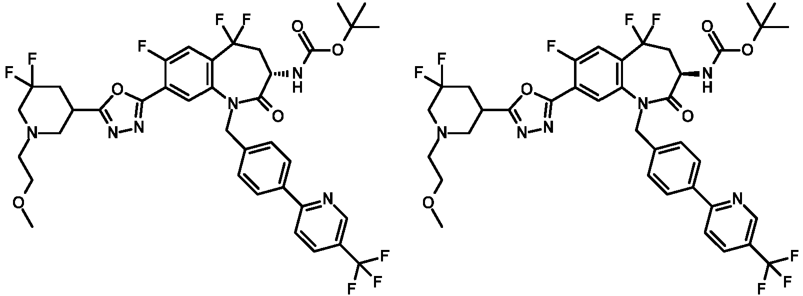

tert-butyl N-[8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer A] was prepared from ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate [isomer A] in analogy to Example 1, steps i to l (using 5,5-difluoro-1-(2-methoxyethyl)piperidine-3-carboxylic acid (Intermediate 16) instead of pivalic acid in step k) and was obtained as light yellow solid. MS (ESI): 811.3 [M+H]⁺. tert-butyl N-[8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer B] was prepared in analogy from ethyl 3-(tert-butoxycarbonylamino)-5,5,7-trifluoro-2-oxo-1-[[4-[5-(trifluoromethyl)-2-pyridyl]phenyl]methyl]-3,4-dihydro-1-benzazepine-8-carboxylate [isomer B] and was obtained as light yellow solid. MS (ESI): 811.3 [M+H]⁺.

### Step d) (3S)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one and (3R)-3-amino-8-[5-[5,5-difluoro-1-(2-methoxyethyl)-3-piperidyl]-1,3,4-oxadiazol-2-yl]-5,5, 7-trifluoro-1-[[4-[5-(trifluoromethyl)-2-pyridyl]phenyl]methyl]-3,4-dihydro-1-benzazepin-2-one

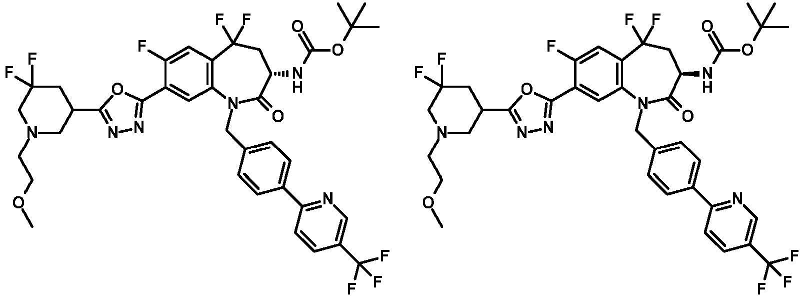

A solution of tert-butyl N-[8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer A] (33 mg, 0.04 mmol, 1.0 eq) in a mixture of DCM/TFA (3:1, 2.0 mL) was stirred at 20°C for 0.5 h. The reaction mixture was poured into water (10 mL) and the resulting mixture was extracted with DCM (10 mL x 3). The combined organic layers were washed with brine (5 mL), dried (Na₂SO₄) and concentrated. The remaining residue was dissolved in EtOAc (20 mL) and HCl/EtOAc (4M, 0.2 mL) was added. The mixture was concentrated and the remaining residue was dissolved in water (20 mL) to obtain 3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one [isomer A] by lyophilization as light yellow solid as hydrochloride salt (28 mg, 0.04 mmol, 89%). MS (ESI): 733.1 [M+Na]⁺, retention time 1.51 and 1.74 minutes (column: Chiralcel OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar). 3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one [isomer B] was obtained in analogy from tert-butyl N-[8-[5-[5,5-difluoro-1-(2-methoxyethyl)-3-piperidyl]-1,3,4-oxadiazol-2-yl]-5,5,7-trifluoro-2-oxo-1-[[4-[5-(trifluoromethyl)-2-pyridyl]phenyl]methyl]-3,4-dihydro-1-benzazepin-3-yl]carbamate [isomer B]. MS (ESI): 733.1 [M+Na]⁺, retention time 2.17 and 2.37 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 35

### methyl 1-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate and

### methyl 1-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate

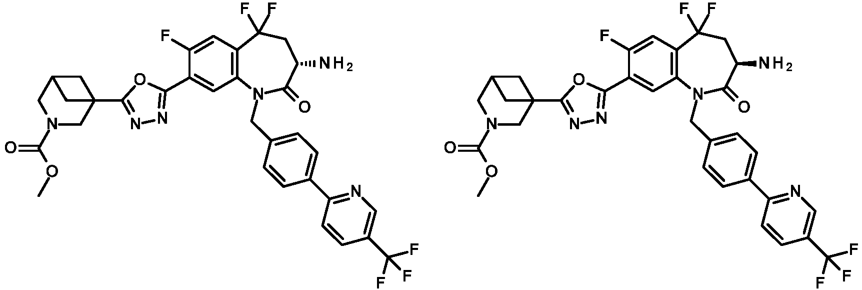

### Step a) tert-butyl 1-[5-[3-(tert-butoxycarbonylamino)-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate

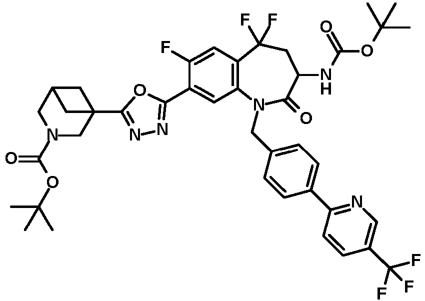

The title compound was prepared in analogy to Example 1, steps a to l (using 2-[4-(bromomethyl)phenyl]-5-(trifluoromethyl)pyridine [CAS#1056641-21-4]; hydrochloride instead of 4-(trifluoromethoxy)benzyl bromide in step f and 3-tert-butoxycarbonyl-3-azabicyclo[3.1.1]heptane-1-carboxylic acid instead of pivalic acid in step k) and was obtained as light yellow oil. MS (ESI): 829.4 [M+H]⁺.

### Step b) 3-amino-8-[5-(3-azabicyclo[3.1.1]heptan-1-yl)-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one

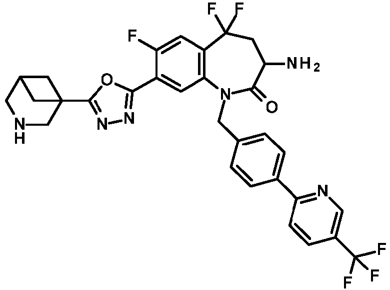

A solution of tert-butyl 1-[5-[3-(tert-butoxycarbonylamino)-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate (245.0 mg, 0.3 mmol, 1.0 eq) in HCl/EtOAc (4M, 4.0 mL, 16.0 mmol, 54.13 eq) was stirred at 20°C for 0.5 h. The reaction mixture was concentrated and the remaining residue was dissolved in water (30 mL) to obtain 3-amino-8-[5-(3-azabicyclo[3.1.1]heptan-1-yl)-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one (200.0 mg, 0.27 mmol, 92% yield) as white solid as trihydrochloride. MS (ESI): 629.4 [M+H]⁺.

### Step c) methyl 1-[5-[(3S)-3-amino-3,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate and methyl 1-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate

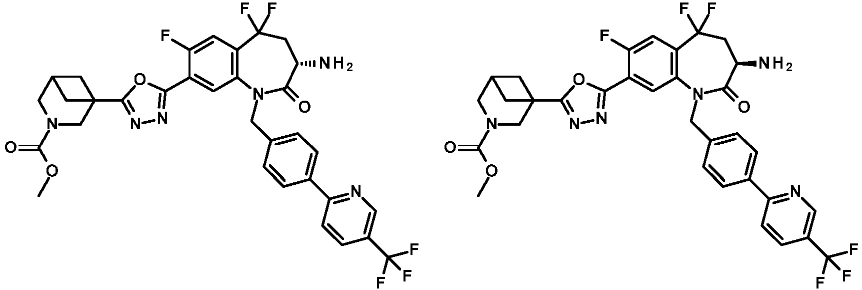

To a solution of 3-amino-8-[5-(3-azabicyclo[3.1.1]heptan-1-yl)-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one; trihydrochloride (100.0 mg, 0.14 mmol, 1.0 eq) and DIPEA (61.3 mg, 0.47 mmol, 3.5 eq) in DCM (1 mL) was added a solution of dimethyl dicarbonate (18.17 mg, 0.14 mmol, 1.0 eq) in DCM (0.5 mL) at 20°C and the mixture was stirred at 20°C for 1 h and then concentrated. The remaining residue was purified by preparative TLC and by preparative HPLC to obtain a solid (20 mg) that was subjected to preparative SFC (Instrument: Waters 80Q; mobile phase: 50% EtOH (0.1% NH₃-H₂O) in supercritical CO₂, flow rate: 70 g/min, back pressure:100 bar) to give methyl 1-[5-[3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate [isomer A] (10.0 mg, 0.01 mmol, 11% yield) as light yellow solid (MS (ESI): 687.3 [M+H]⁺, retention time 0.63 minutes (see conditions below)) and methyl 1-[5-[3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate [isomer B] (6.0 mg, 0.01 mmol, 6.5% yield) as light yellow solid. MS (ESI): 687.3 [M+H]⁺, retention time 1.34 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 36

### methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate and

### methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate

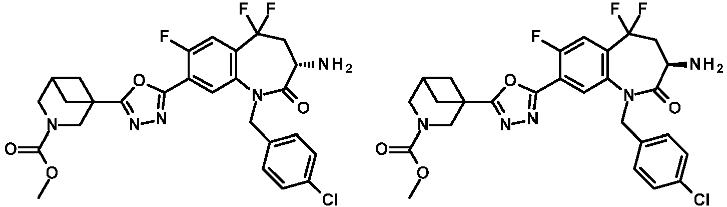

### Step a) tert-butyl 1-[5-[3-(tert-butoxycarbonylamino)-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane- 3-carboxylate

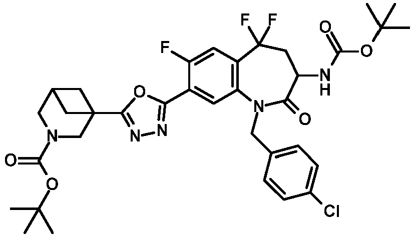

The title compound was prepared in analogy to Example 1, steps a to l (using 4-chlorobenzyl bromide instead of 4-(trifluoromethoxy)benzyl bromide in step f and 3-tert-butoxycarbonyl-3-azabicyclo[3.1.1]heptane-1-carboxylic acid instead of pivalic acid in step k) and was obtained as white solid. MS (ESI): 740.4 [M+Na]⁺.

### Step b) 3-amino-8-[5-(3-azabicyclo[3.1.1]heptan-1-yl)-1,3,4-oxadiazol-2-yl]-1-[(4-chlorophenyl)methyl]-5,5,7-trifluoro-3,4-dihydro-1-benzazepin-2-one

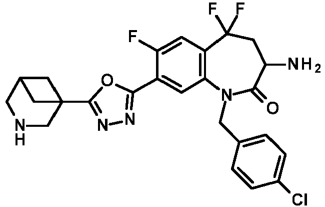

A solution of tert-butyl 1-[5-[3-(tert-butoxycarbonylamino)-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate (130.0 mg, 0.18 mmol, 1.0 eq) in HCl/EtOAc (8.67 mL, 34.67 mmol, 191.51 eq) was stirred at 20°C for 0.5 h. All volatiles were removed to obtain 3-amino-8-[5-(3-azabicyclo[3.1.1]heptan-1-yl)-1,3,4-oxadiazol-2-yl]-1-[(4-chlorophenyl)methyl]-5,5,7-trifluoro-3,4-dihydro-1-benzazepin-2-one (106.0 mg, 0.18 mmol, 88% yield) as a white solid as dihydrochloride salt. MS (ESI): 518.3 [M+H]⁺.

### Step c) methyl 1-[5-[3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate

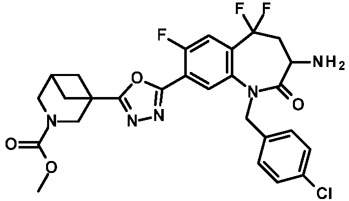

To a solution of 3-amino-8-[5-(3-azabicyclo[3.1.1]heptan-1-yl)-1,3,4-oxadiazol-2-yl]-1-[(4-chlorophenyl)methyl]-5,5,7-trifluoro-3,4-dihydro-1-benzazepin-2-one; dihydrochloride (106.0 mg, 0.18 mmol, 1.0 eq) and DIPEA (0.16 mL, 0.9 mmol, 5.0 eq) in DCM (10 mL) was added dimethyl dicarbonate (0.01 mL, 0.09 mmol, 0.5 eq) at 20°C and the solution was stirred at 20°C for 0.5 h. All volatiles were removed and the remaining residue was purified by prep-TLC (100% EtOAc) to afford methyl 1-[5-[3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate (80.0 mg, 0.14 mmol, 75% yield) as a white solid. MS (ESI): 576.2 [M+H]⁺.

### Step d) methyl 1-[5-[(3S)-3-amino-1-[(4-chlorophenyl)methyl]-5,5, 7-trifluoro-2-oxo-3,4-dihydro-1-benzazepin-8-yl]-1,3, 4-oxadiazol-2-yl]-3-azabicyclo[3.1.1]heptane-3-carboxylate and methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate

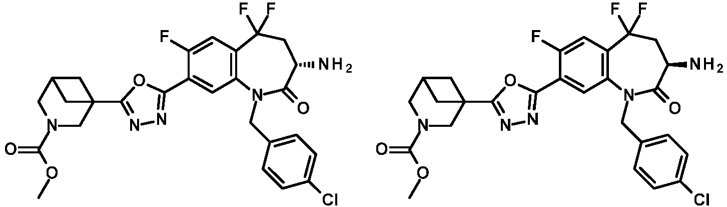

methyl 1-[5-[3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate (80 mg) was subjected to preparative SFC (Daicel Chiralpak OD (250×30 mm, 10 µm), 0.1% NH₃-H₂O in EtOH) to obtain methyl 1-[5-[3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate [isomer A] (30 mg) (MS (ESI): 576.2 [M+H]⁺, retention time 0.58 minutes (see conditions below) as light yellow solid and methyl 1-[5-[3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate [isomer B] (30 mg) as light yellow solid. MS (ESI): 576.2 [M+H]⁺, retention time 1.46 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 37

### 2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile and

### 2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile

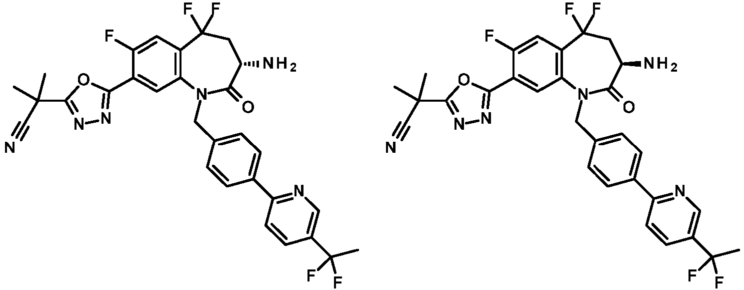

### Step a) tert-butyl N-[8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate

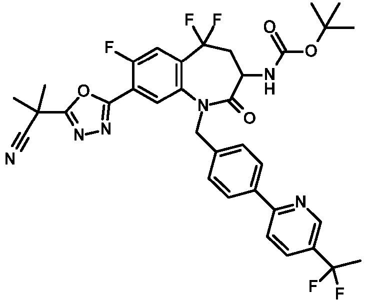

The title compound was prepared in analogy to Example 1, steps a to l (using 2-[4-(chloromethyl)phenyl]-5-(1,1-difluoroethyl)pyridine (Intermediate 7) instead of 4-(trifluoromethoxy)benzyl bromide in step f and 2-cyano-2-methyl-propanoic acid instead of pivalic acid in step k) and was obtained as solid. MS (ESI): 697.4 [M+H]⁺.

### Step b) tert-butyl N-[(3S)-8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate and tert-butyl N-[(3R)-8-[5-(1-cyano-1-methyl-ethyl)-1,3,4-oxadiazol-2-yl]-1-[[4-[5-(1,1-difluoroethyl)-2-pyridyl]phenyl]methyl]-5,5, 7-trifluoro-2-oxo-3,4-dihydro-1-benzazepin-3-yl]carbamate

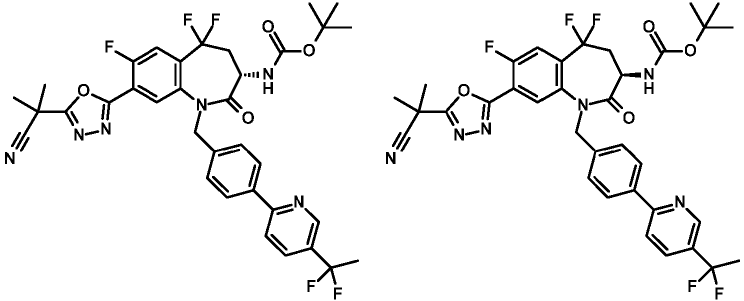

tert-butyl N-[8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate was subjected to preparative SFC (Daicel Chiralpak IC (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate [isomer A] (30 mg) (MS (ESI): 697.4 [M+H]⁺, retention time 1.67 minutes (see conditions below) as light yellow solid and tert-butyl N-[8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate [isomer B] (34 mg) as light yellow solid. MS (ESI): 697.3 [M+H]⁺, retention time 2.21 minutes (column: Chiralpak IC-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile and 2-[5-[(3R)-3-amino-1-[[4-[5-(1,1-difluoroethyl)-2-pyridyl]phenyl]methyl]-5,5, 7-trifluoro-2-oxo-3,4-dihydro-1-benzazepin-8-yl]-1,3,4-oxadiazol-2-yl]-2-methyl-propanenitrile

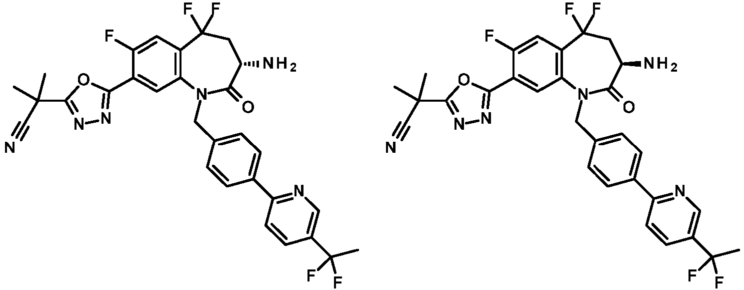

To a solution of tert-butyl N-[8-[5-(1-cyano-1-methyl-ethyl)-1,3,4-oxadiazol-2-yl]-1-[[4-[5-(1,1-difluoroethyl)-2-pyridyl]phenyl]methyl]-5,5,7-trifluoro-2-oxo-3,4-dihydro-1-benzazepin-3-yl]carbamate [isomer A] (20 mg, 0.03 mmol, 1.0 eq) in DCM (10 mL) was added TFA (0.33 mL, 4.46 mmol, 155.26 eq) at 25°C and the solution was stirred at 25°C for 1 h. The pH of the mixture was adjusted to 8 by careful addition of saturated NaHCO₃ solution and the resulting mixture was extracted with DCM (50 mL). The organic layer was washed with brine (50 mL), dried (Na₂SO₄) and concentrated. The remaining residue was dissolved in EtOAc (10 mL) and HCl/EtOAc (4M, 0.1 mL) was added. The mixture was concentrated and water (20 mL) was added to obtain 2-[5-[3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile [isomer A] after lyophilization as light yellow solid as hydrochloride salt (15.2 mg, 0.02 mmol, 82% yield). MS (ESI): 597.3 [M+H]⁺, retention time 0.64 minutes (see conditions below). 2-[5-[3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile [isomer B] was obtained in analogy. MS (ESI): 597.3 [M+H]⁺, retention time 1.37 minutes (column: Chiralcel OX-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 38

### 2-methyl-2-[5-[(3S)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile and

### 2-methyl-2-[5-[(3R)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile

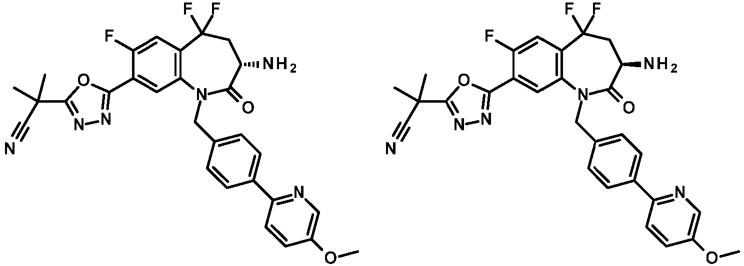

### Step a) tert-butyl N-[8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate

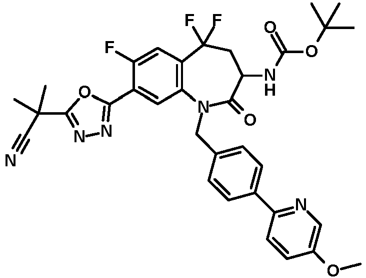

The title compound was prepared in analogy to Example 1, steps a to l (using 2-[4-(bromomethyl)phenyl]-5-methoxy-pyridine [CAS# 1160430-89-6] instead of 4-(trifluoromethoxy)benzyl bromide in step f and 2-cyano-2-methyl-propanoic acid instead of pivalic acid in step k) and was obtained as solid. MS (ESI): 663.3 [M+H]⁺.

### Step b) tert-butyl N-[(3S)-8-[5-(1-cyano-1-methyl-ethyl)-1,3,4-oxadiazol-2-yl]-5,5,7-trifluoro-1-[[4-(5-methoxy-2pyridyl)phenyl]methyl]-2-oxo-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate

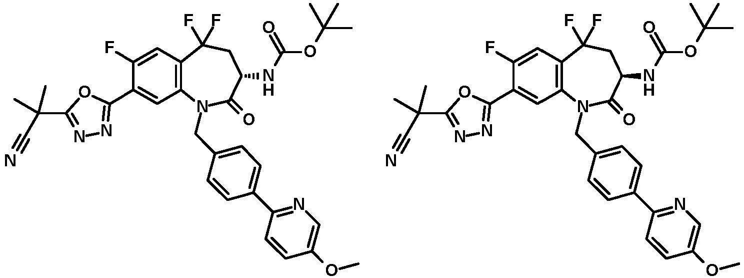

tert-butyl N-[8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate was subjected to preparative SFC (Daicel Chiralpak IC (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate [isomer A] (40 mg) (MS (ESI): 663.2 [M+H]⁺, retention time 0.94 minutes (see conditions below) as light yellow solid and tert-butyl N-[8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate [isomer B] (35 mg) as light yellow solid. MS (ESI): 663.2 [M+H]⁺, retention time 1.63 minutes (column: Chiralpak IC-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-1-[[4-(5-methoxy-2-pyridyl)phenyl]methyl]-2-oxo-3, 4-dihydro-1-benzazepin-8-yl]-1,3,4-oxadiazol-2-yl]propanenitrile and 2-methyl-2-[5-[(3R)-3-amino-5,5, 7-trifluoro-1-[[4-(5-methoxy-2-pyridyl)phenyl]methyl]-2-oxo-3, 4-dihydro-1-benzazepin-8-yl]-1,3,4-oxadiazol-2-yl]propanenitrile

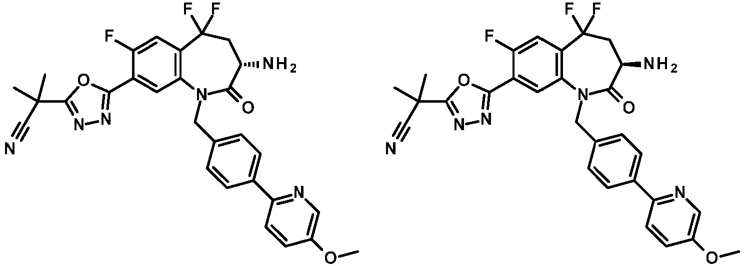

To a solution of tert-butyl N-[8-[5-(1-cyano-1-methyl-ethyl)-1,3,4-oxadiazol-2-yl]-5,5,7-trifluoro-1-[[4-(5-methoxy-2-pyridyl)phenyl]methyl]-2-oxo-3,4-dihydro-1-benzazepin-3-yl]carbamate [isomer A] (35.0 mg, 0.05 mmol, 1.0 eq) in DCM (3 mL) was added TFA (1.0 mL, 12.98 mmol, 245.76 eq) at 25°C and the solution was stirred at 25°C for 1 h. The pH of the mixture was adjusted to 8 by careful addition of saturated NaHCO₃ solution and the resulting mixture was extracted with DCM (50 mL). The organic layer was washed with brine (50 mL), dried (Na₂SO₄) and concentrated. The remaining residue was dissolved in EtOAc (10 mL) and HCl/EtOAc (4M, 0.1 mL) was added. The mixture was concentrated and water (20 mL) was added to obtain 2-methyl-2-[5-[3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile [isomer A] after lyophilization as light yellow solid as hydrochloride salt (22 mg, 0.04 mmol, 67% yield). MS (ESI): 563.2 [M+H]⁺, retention time 0.94 minutes (see conditions below). 2-methyl-2-[5-[3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile [isomer B] was obtained in analogy. MS (ESI): 563.2 [M+H]⁺, retention time 2.09 minutes (column: Chiralcel OX-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Example 39

### (3S)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one and

### (3R)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one

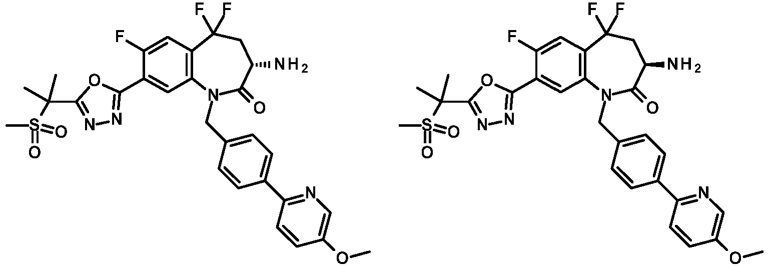

### Step a) tert-butyl N-[5,5,7-trifluoro-1-[[4-(5-methoxy-2-pyridyl)phenyl[methyl]-8-[5-(1-methyl-1-methylsulfonyl-ethyl)-1,3,4-oxadiazol-2-yl]-2-oxo-3,4-dihydro-1-benzazepin-3-yl]carbamate

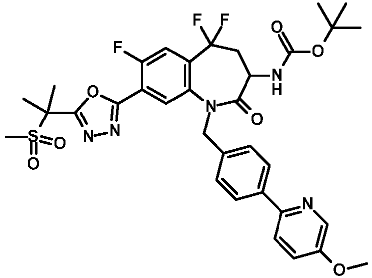

The title compound was prepared in analogy to Example 1, steps a to l (using 2-[4-(bromomethyl)phenyl]-5-methoxy-pyridine [CAS# 1160430-89-6] instead of 4-(trifluoromethoxy)benzyl bromide in step f and 2-methyl-2-methylsulfonyl-propanoic acid instead of pivalic acid in step k) and was obtained as solid. MS (ESI): 716.3 [M+H]⁺.

### Step b) tert-butyl N-[(3S)-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate and tert-butyl N-[(3R)-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate

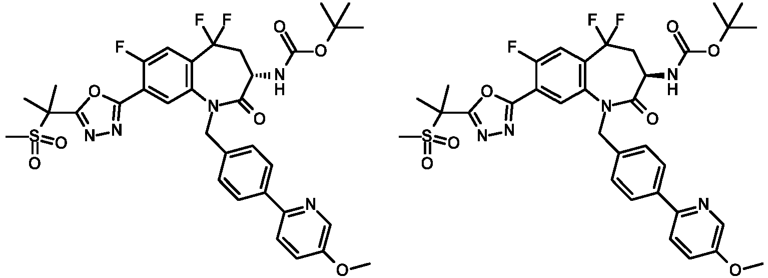

tert-butyl N-[5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate was subjected to preparative SFC (Daicel Chiralpak AD (250×30 mm, 10 µm), 0.1% NH₃-H₂O in EtOH) to obtain *tert*-butyl *N*-[5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer A] (65 mg) (MS (ESI): 716.3 [M+H]⁺, retention time 2.10 minutes (see conditions below) as light yellow solid and *tert*-butyl *N-*[5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer B] (60 mg) as light yellow solid. MS (ESI): 716.3 [M+H]⁺, retention time 2.59 minutes (column: Chiralpak IF-3 50×4.6 mm I.D., 3 µm; mobile phase: phase A for CO₂, and phase B for iPrOH (0.05% DEA); gradient elution: 5% to 40% iPrOH (0.05% DEA) in CO₂; flow rate: 3 mL/min; detector: PDA; column temperatur: 35°C; back pressure: 100 bar).

### Step c) (3S)-3-amino-5,5, 7-trifluoro-1-[[4-(5-methoxy-2-pyridyl)phenyl]methyl]-8-[5-(1-methyl-1-methylsulfonyl-ethyl)-1,3, 4-oxadiazol-2-yl]-3,4-dihydro-1-benzazepin-2-one and (3R)-3-amino-3,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one

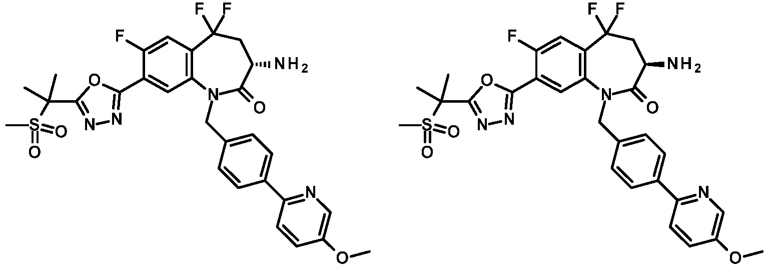

To a solution of *tert*-butyl *N-*[5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-2-oxo-3,4-dihydro-1-benzazepin-3-yl]carbamate [isomer A] (60.0 mg, 0.08 mmol, 1.0 eq) in DCM (10 mL) was added TFA (3.0 mL, 38.94 mmol, 464.52 eq) at 25°C and the solution was stirred at 25°C for 1 h. The pH of the mixture was adjusted to 8 by careful addition of saturated NaHCO₃ solution and the resulting mixture was extracted with DCM (50 mL). The organic layer was washed with brine (50 mL), dried (Na₂SO₄) and concentrated. The remaining residue was dissolved in EtOAc (10 mL) and HCl/EtOAc (4M, 0.1 mL) was added. The mixture was concentrated and water (20 mL) was added to obtain 3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one [isomer A] after lyophilization as light yellow solid as hydrochloride salt (11.5 mg, 0.02 mmol, 22% yield). MS (ESI): 616.2 [M+H]⁺, retention time 1.45 minutes (see conditions below). 3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one [isomer B] was obtained in analogy. MS (ESI): 616.2 [M+H]⁺, retention time 2.09 minutes (column: (S.S) Whelk-O1 50×4.6 mm I.D., 3.5 µm; mobile phase: phase A for CO₂, and phase B for MeOH/MeCN 4:1 (0.05% DEA); gradient elution: 5% to 40% MeOH/MeCN 4:1 (0.05% DEA) in CO₂; flow rate: 3 mL/min; detector: PDA; column temperatur: 35°C; back pressure: 100 bar).

### Example 40

### 2-methyl-2-[5-[(3S)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile and

### 2-methyl-2-[5-[(3R)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile

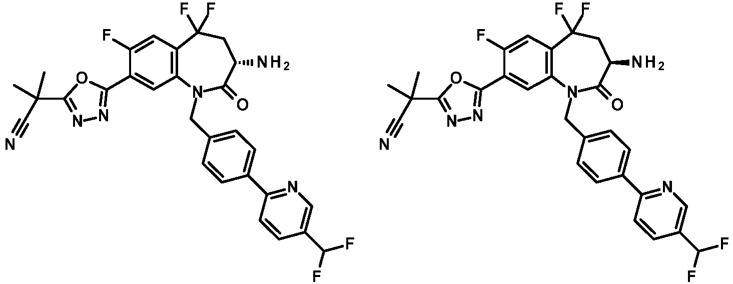

### Step a) tert-butyl N-[8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate

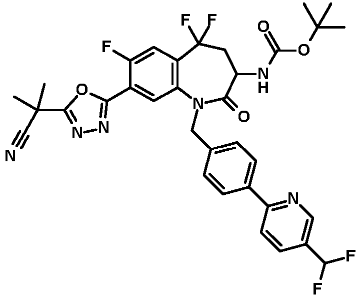

The title compound was prepared in analogy to Example 1, steps a to l (using 2-[4-(chloromethyl)phenyl]-5-(difluoromethyl)pyridine (Intermediate 8) instead of 4-(trifluoromethoxy)benzyl bromide in step f and 2-cyano-2-methyl-propanoic acid instead of pivalic acid in step k) and was obtained as solid. MS (ESI): 683.1 [M+H]⁺.

### Step b) tert-butyl N-[(3S)-8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate and tert-butyl N-[(3R)-8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate

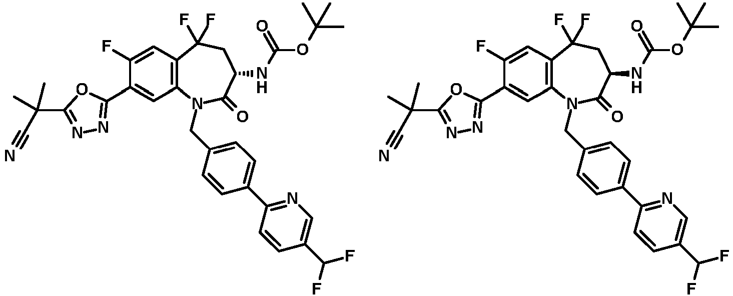

tert-butyl N-[8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate was subjected to preparative SFC (Daicel Chiralpak IC (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate [isomer A] (32 mg) (MS (ESI): 683.3 [M+H]⁺, retention time 0.82 minutes (see conditions below) as light yellow solid and tert-butyl N-[8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate [isomer B] (34 mg) as light yellow solid. MS (ESI): 683.1 [M+H]⁺, retention time 1.34 minutes (column: Chiralpak IC-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Step c) 2-methyl-2-[5-[(3S)-3-amino-1-[[4-[5-(difluoromethyl)-2-pyridyl]phenyl]methyl]-5,5,7-trifluoro-2-oxo-3, 4-dihydro-1-benzazepin-8-yl]-1,3,4-oxadiazol-2-yl]propanenitrile and 2-methyl-2-[5-[(3R)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile

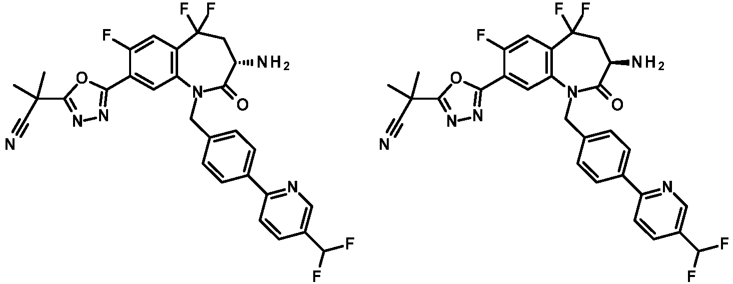

To a solution of tert-butyl N-[8-[5-(1-cyano-1-methyl-ethyl)-1,3,4-oxadiazol-2-yl]-1-[[4-[5-(difluoromethyl)-2-pyridyl]phenyl]methyl]-5,5,7-trifluoro-2-oxo-3,4-dihydro-1-benzazepin-3-yl]carbamate [isomer A] (30.0 mg, 0.04 mmol, 1.0 eq) in DCM (9 mL) was added TFA (3.0 mL, 38.94 mmol, 886.06 eq) at 25°C and the solution was stirred at 25°C for 1 h. The pH of the mixture was adjusted to 8 by careful addition of saturated NaHCO₃ solution and the resulting mixture was extracted with DCM (50 mL). The organic layer was washed with brine (50 mL), dried (Na₂SO₄) and concentrated. The remaining residue was dissolved in EtOAc (10 mL) and HCl/EtOAc (4M, 0.1 mL) was added. The mixture was concentrated and water (20 mL) was added to obtain 2-methyl-2-[5-[3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile [isomer A] after lyophilization as light yellow solid as hydrochloride salt (20.1 mg, 0.03 mmol, 76% yield). MS (ESI): 583.2 [M+H]⁺, retention time 1.53 minutes (see conditions below). 3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one [isomer B] was obtained in analogy. MS (ESI): 583.2 [M+H]⁺, retention time 2.21 minutes (column: (S.S) Whelk-O1 50×4.6 mm I.D., 3.5 µm; mobile phase: phase A for CO₂, and phase B for EtOH (0.05% DEA); 40% EtOH (0.05% DEA) in CO₂; flow rate: 3 mL/min; detector: PDA; column temperatur: 35°C; back pressure: 100 bar).

### Example 41

### 2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile and

### 2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile

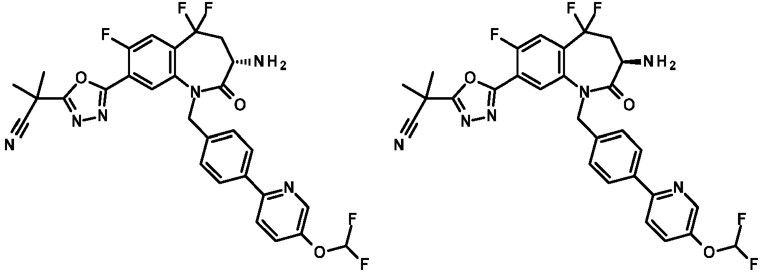

### Step a) tert-butyl N-[8-[5-(1-cyano-1-methyl-ethyl)-1,3,4-oxadiazol-2-yl]-1-[[4-[5-(difluoromethoxy)-2-pyridyl[phenyl]methyl]-5,5,7-trifluoro-2-oxo-3,4-dihydro-1-benzazepin-3-yl]carbamate

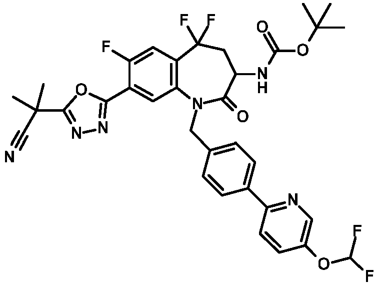

The title compound was prepared in analogy to Example 1, steps a to l (using 2-[4-(chloromethyl)phenyl]-5-(difluoromethoxy)pyridine (Intermediate 9) instead of 4-(trifluoromethoxy)benzyl bromide in step f and 2-cyano-2-methyl-propanoic acid instead of pivalic acid in step k) and was obtained as solid. MS (ESI): 699.4 [M+H]⁺.

### Step b) tert-butyl N-[(3S)-8-[5-(1-cyano-1-methyl-ethyl)-1,3,4-oxadiazol-2-yl]-1-[[4-[5-(difluoromethoxy)-2-pyridyl[phenyl]methyl]-5,5, 7-trifluoro-2-oxo-3,4-dihydro- 1-benzazepin-3-yl]carbamate and tert-butylN-[(3R)-8-[5-(1-cyano-1-methyl-ethyl)-1,3,4-oxadiazol-2-yl]-1-[[4-[5-(difluoromethoxy)-2-pyridyl]phenyl]methyl]-5,5,7-trifluoro-2-oxo-3,4-dihydro-1-benzazepin-3-yl]carbamate

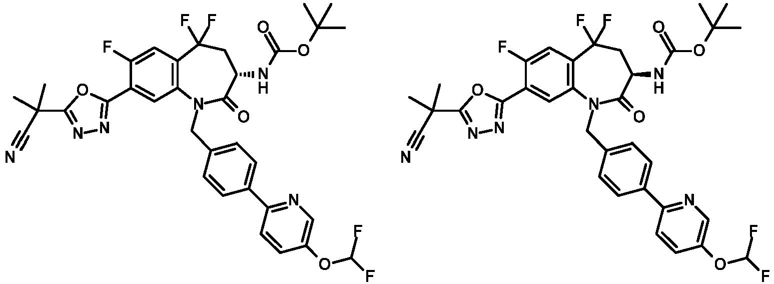

tert-butyl N-[8-[5-(1-cyano-1-methyl-ethyl)-1,3,4-oxadiazol-2-yl]-1-[[4-[5-(difluoromethoxy)-2-pyridyl]phenyl]methyl]-5,5,7-trifluoro-2-oxo-3,4-dihydro-1-benzazepin-3-yl]carbamate was subjected to preparative SFC (Daicel Chiralpak IC (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-1-[[4-[5-(difluoromethoxy)-2-pyridyl]phenyl]methyl]-5,5,7-trifluoro-2-oxo-3,4-dihydro-1-benzazepin-3-yl]carbamate [isomer A] (40 mg) (MS (ESI): 699.4 [M+H]⁺, retention time 1.69 minutes (see conditions below) as light yellow solid and tert-butyl N-[8-[5-(1-cyano-1-methyl-ethyl)-1,3,4-oxadiazol-2-yl]-1-[[4-[5-(difluoromethoxy)-2-pyridyl]phenyl]methyl]-5,5,7-trifluoro-2-oxo-3,4-dihydro-1-benzazepin-3-yl]carbamate [isomer B] (41 mg) as light yellow solid. MS (ESI): 699.4 [M+H]⁺, retention time 2.16 minutes (column: Chiralpak IC-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-1-[[4-[5-(difluoromethoxy)-2-pyridyl]phenyl]methyl]-5,5,7-trifluoro-2-oxo-3, 4-dihydro-1-benzazepin-8-yl]-1,3,4-oxadiazol-2-yl]propanenitrile and 2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile

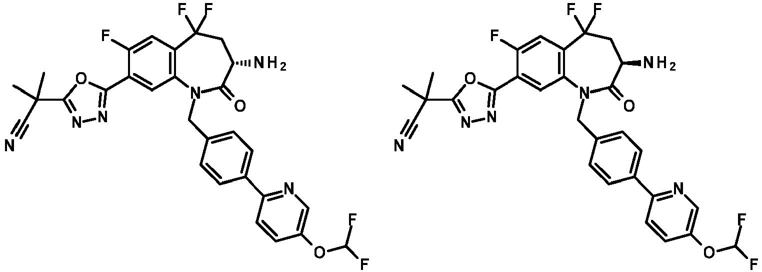

A solution of tert-butyl N-[8-[5-(1-cyano-1-methyl-ethyl)-1,3,4-oxadiazol-2-yl]-1-[[4-[5-(difluoromethoxy)-2-pyridyl]phenyl]methyl]-5,5,7-trifluoro-2-oxo-3,4-dihydro-1-benzazepin-3-yl]carbamate [isomer A] (35.0 mg, 0.05 mmol, 1.0 eq) in HCl/EtOAc (4 M, 10.0 mL, 40.0 mmol, 798.5 eq) was stirred at 20°C for 0.5 h. The mixture was concentrated and the remaining residue was purified by preperative HPLC (column Waters Xbridge 150x25mm, 5 µm, water (containing NH₄HCO₃)/MeCN = 3:2 to 3:7). The product was dissolved in EtOAc (5 mL) and HCl/EtOAc (4 M, 0.3 mL) was added. The mixture was concentrated and water was added to obtain 2-methyl-2-[5-[3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile [isomer A] after lyophilization as yellow solid as dihydrochloride salt (7.9 mg, 0.01 mmol, 23% yield). MS (ESI): 599.3 [M+H]⁺, retention time 0.86 minutes (see conditions below). 3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one [isomer B] was obtained in analogy. MS (ESI): 599.3 [M+H]⁺, retention time 1.86 minutes (column: Cellulose-2 50×4.6 mm I.D., 3 µm; mobile phase: phase A for CO₂, and phase B for EtOH (0.05% DEA); 40% EtOH (0.05% DEA) in CO₂; flow rate: 3 mL/min; detector: PDA; column temperatur: 35°C; back pressure: 100 bar).

### Example 42

### methyl 1-[5-[(3S)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate and

### methyl 1-[5-[(3R)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate

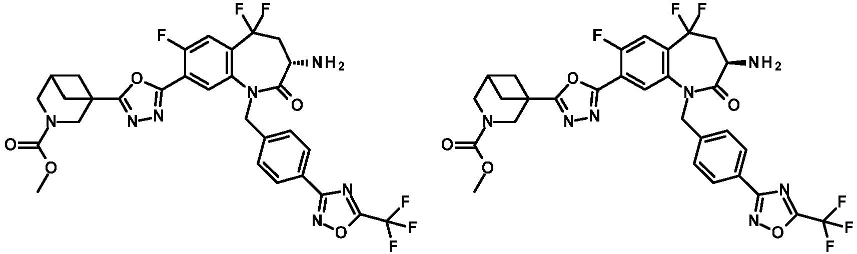

### Step a) tert-butyl 1-[5-[3-(tert-butoxycarbonylamino)-1-[(4-cyanophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate

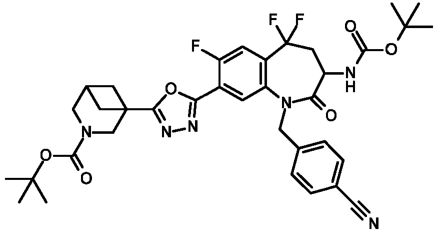

The title compound was prepared in analogy to Example 1, steps a to l (using 4-(bromomethyl)benzonitrile instead of 4-(trifluoromethoxy)benzyl bromide in step f and 3*-tert-*butoxycarbonyl-3-azabicyclo[3.1.1]heptane-1-carboxylic acid instead of pivalic acid in step k) and was obtained as light yellow solid. MS (ESI): 597.1 [M-(2 x isobutene)+H]⁺.

### Step b) tert-butyl 1-[5-[3-(tert-butoxycarbonylamino)-3,5,7-trifluoro-1-[[4-[(Z)-N'-hydroxycarbamimidoyl]phenyl]methyl]-2-oxo-3,4-dihydro-1-benzazepin-8-yl]-1,3,4-oxadiazol-2-yl]-3-azabicyclo[3.1.1]heptane-3-carboxylate

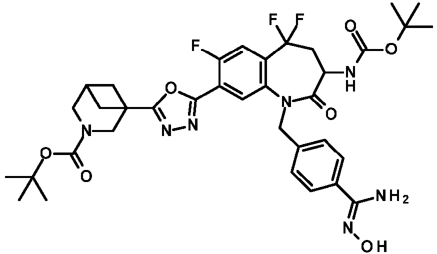

To a mixture of tert-butyl 1-[5-[3-(tert-butoxycarbonylamino)-1-[(4-cyanophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate (100.0 mg, 0.14 mmol, 1.0 eq) and sodium acetate (34.72 mg, 0.42 mmol, 3.0 eq) in ethanol (2 mL) was added hydroxylammonium chloride (14.71 mg, 0.21 mmol, 1.5 eq) at 20°C and the mixture was stirred at 50°C for 12 h. The reaction mixture was poured into water (15 mL) and the layers were separated. The aqueous phase was extracted with EtOAc (10 mL x 3) and the combined extracts were washed with brine (10 mL), dried (Na₂SO₄) and concentrated to give the title compound (100.0 mg, 0.13 mmol, 95% yield) as light yellow solid. MS (ESI): 742.1 [M+H]⁺.

### Step c) tert-butyl 1-[5-[3-(tert-butoxycarbonylamino)-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate

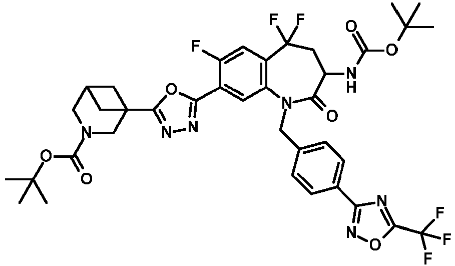

To a solution of tert-butyl 1-[5-[3-(tert-butoxycarbonylamino)-5,5,7-trifluoro-1-[[4-[(Z)-N'-hydroxycarbamimidoyl]phenyl]methyl]-2-oxo-3,4-dihydro-1-benzazepin-8-yl]-1,3,4-oxadiazol-2-yl]-3-azabicyclo[3.1.1]heptane-3-carboxylate (800.0 mg, 1.08 mmol, 1.0 eq) in THF (10 mL) was added trifluoroacetic anhydride (0.22 mL, 1.62 mmol, 1.5 eq) at 20°C and the mixture was sitrred at 20°C for 1 h. EtOAc (30 mL) and water (30 mL) were added to the reaction mixture and layers were separated. The pH of the aqueous layer was carefully adjusted to 8 with saturated aqueous NaHCO₃ and the mixture was extracted with EtOAc (50 mL x 3). The combined organic layers were washed with brine (30 mL), dried (Na₂SO₄) and concentrated. The remaining residue was purified by preparative TLC (PE/EtOAc = 2: 1) to give the title compound (260.0 mg, 0.32 mmol, 30% yield) as light yellow solid. MS (ESI): 664.1 [M-(2 x isobutene)-CO₂+H]⁺.

### Step d) 3-amino-8-[5-(3-azabicyclo[3.1.1]heptan-1-yl)-1,3,4-oxadiazol-2-yl]-5,5, 7-trifluoro-1-[[4-[5-(trifluoromethyl)-1, 2, 4-oxadiazol-3-yl]phenyl]methyl]-3,4-dihydro-1-benzazepin-2-one

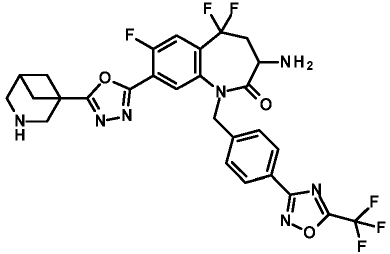

A solution of tert-butyl 1-[5-[3-(tert-butoxycarbonylamino)-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate (150.0 mg, 0.18 mmol, 1.0 eq) in HCl/EtOAc (5.0 mL, 10.0 mmol, 54.65 eq) was stirred at 20°C for 2 h. The reaction mixture was concentrated and the remaining residue was triturated with a mixture of PE/EtOAc = 10:1, 3 mL). The precipitate was collected and dried under vacuum to give the title compound (120.0 mg, 0.17 mmol, 83% yield) as dihydrochloride salt as off-white solid. MS (ESI): 620.3 [M+H]⁺.

### Step e) methyl 1-[5-[3-amino-5,5,7-trifluoro-2-oxo-1-[[4-[5-(trifluoromethyl)-1, 2, 4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate

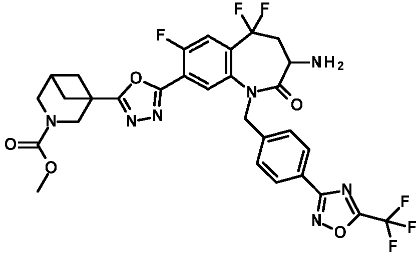

To a solution of 3-amino-8-[5-(3-azabicyclo[3.1.1]heptan-1-yl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one; dihydrochloride (30.0 mg, 0.04 mmol, 1.0 eq) and DIPEA (19.6 mg, 0.15 mmol, 3.5 eq) in DCM (1 mL) was added a solution of dimethyl dicarbonate (5.81 mg, 0.04 mmol, 1.0 eq) in DCM (0.5 mL) at 20°C and the mixture was stirred at 20°C for 1 h. The reaction mixture was concentrated and purified by preparative TLC (120 mL EA and 0.5 mL DIPEA) to give the title compound (30.0 mg, 0.04 mmol, 99% yield) as light yellow solid. MS (ESI): 678.4 [M+H]⁺.

### Step f) methyl 1-[5-[(3S)-3-amino-5,5, 7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate and methyl 1-[5-[(3R)-3-amino-5,5, 7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate

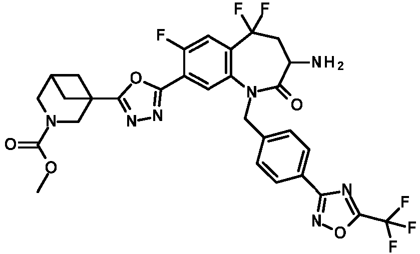

methyl 1-[5-[3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate (100 mg) was subjected to preparative SFC (Daicel Chiralcel OD (250×30 mm, 10 µm), 0.1% NH₃-H₂O in MeOH) to obtain methyl 1-[5-[3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate [isomer A] (35 mg) (MS (ESI): 678.2 [M+H]⁺, retention time 1.65 minutes (see conditions below) as off-white solid and methyl 1-[5-[3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate [isomer B] (35 mg) as light yellow solid. MS (ESI): 678.3 [M+H]⁺, retention time 2.42 minutes (column: Chiralcel OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Example 43

### 2-methyl-2-[5-[(3S)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile

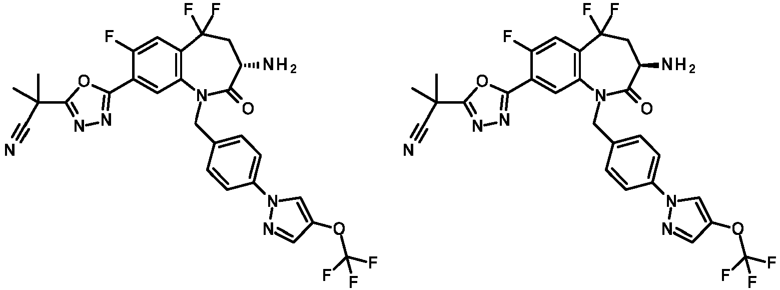

### Step a) tert-butyl N-[8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate

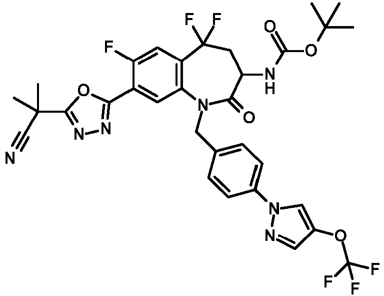

The title compound was prepared in analogy to Example 1, steps a to l (using 1-[4-(bromomethyl)phenyl]-4-(trifluoromethoxy)pyrazole (Intermediate 10) instead of 4-(trifluoromethoxy)benzyl bromide in step f and 2-cyano-2-methyl-propanoic acid instead of pivalic acid in step k) and was obtained as yellow solid. MS (ESI): 706.2 [M+H]⁺.

### Step b) tert-butyl N-[(3S)-8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate

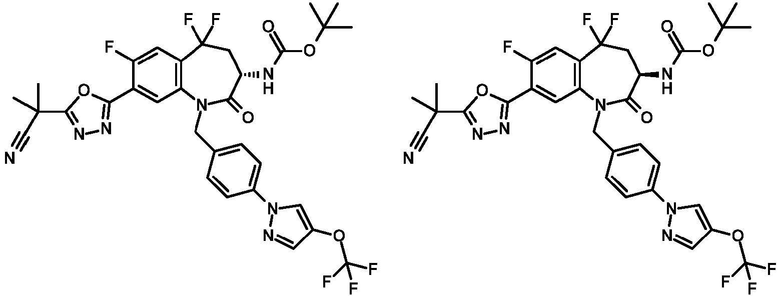

tert-butyl N-[8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate was subjected to preparative SFC (Daicel Chiralpak AD (50×4.6 mm, 3 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate [isomer A] (126 mg) (MS (ESI): 706.2 [M+H]⁺, retention time 0.84 minutes (see conditions below) as yellow solid and tert-butyl N-[8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate [isomer B] (128 mg) as yellow solid. MS (ESI): 706.2 [M+H]⁺, retention time 1.16 minutes (column: Chiralcel AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile and 2-methyl-2-[5-[(3R)-3-amino-3,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile

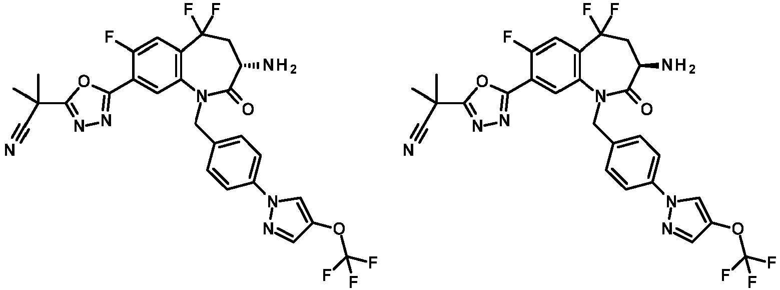

To a solution of tert-butyl N-[8-[5-(1-cyano-1-methyl-ethyl)-1,3,4-oxadiazol-2-yl]-5,5,7-trifluoro-2-oxo-1-[[4-[4-(trifluoromethoxy)pyrazol-1-yl]phenyl]methyl]-3,4-dihydro-1-benzazepin-3-yl]carbamate [isomer A] (126.0 mg, 0.18 mmol, 1.0 eq) in DCM (4.5 mL) was added TFA (1.5 mL) and the mixture was stirred at 25°C for 1 h. DCM (20mL) was added and the pH of the mixture was adjusted to 8 by careful addition of saturated NaHCO₃ solution. The resulting mixture was extracted with DCM (15 mL x 3) and the the organic extracts were washed with brine (30 mL), dried (Na₂SO₄) and concentrated. The remaining residue was dissolved in EtOAc (20 mL) and HCl/EtOAc (4 M, 0.2 mL) was added. The mixture was concentrated to obtain 2-methyl-2-[5-[3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile [isomer A] as yellow solid as hydrochloride salt (67.5 mg, 0.1 mmol, 61% yield). MS (ESI): 606.1 [M+H]⁺, retention time 1.32 minutes (see conditions below). 2-methyl-2-[5-[3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile [isomer B] was obtained in analogy. MS (ESI): 606.2 [M+H]⁺, retention time 1.63 minutes (column: Chiralpak OD 50×4.6 mm I.D., 3 µm; mobile phase: phase A for CO₂, and phase B for MeOH (0.05% DEA); 5% to 40% MeOH (0.05% DEA) in CO₂; flow rate: 3 mL/min; detector: PDA; column temperatur: 35°C; back pressure: 100 bar).

### Example 44

### 2-methyl-2-[5-[(3S)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile and

### 2-methyl-2-[5-[(3R)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile

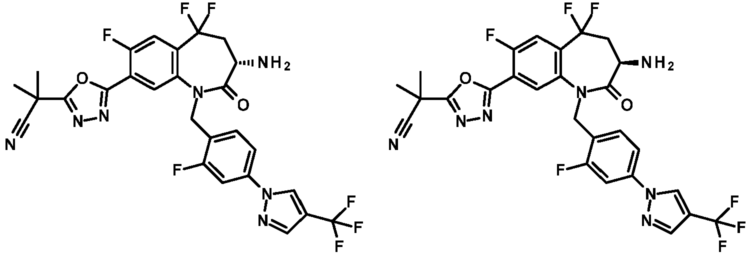

### Step a) tert-butyl N-[8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate

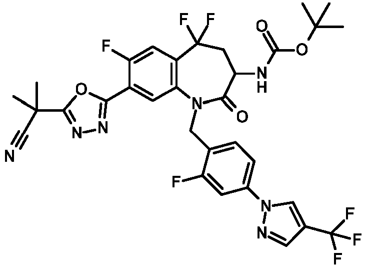

The title compound was prepared in analogy to Example 1, steps a to l (using 1-[4-(chloromethyl)-3-fluoro-phenyl]-4-(trifluoromethyl)pyrazole (Intermediate 11) instead of 4-(trifluoromethoxy)benzyl bromide in step f and 2-cyano-2-methyl-propanoic acid instead of pivalic acid in step k) and was obtained as yellow solid. MS (ESI): 652.1 [M-isobutene+H]⁺.

### Step b) tert-butyl N-[(3S)-8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate and tert-butyl N-[(3R)-8-[5-(1-cyano-1-methyl-ethyl)-1, 3, 4-oxadiazol-2-yl]-5,5, 7-trifluoro-1-[[2-fluoro-4-[4-(trifluoromethyl)pyrazol-1-yl]phenyl]methyl]-2-oxo-3,4-dihydro-1-benzazepin-3-yl]carbamate

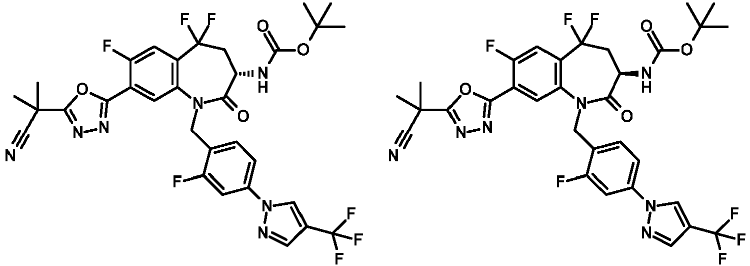

tert-butyl N-[8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate was subjected to preparative SFC (Daicel Chiralpak AD (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate [isomer A] (70 mg) (MS (ESI): 652.2 [M-isobutene+H]⁺, retention time 0.82 minutes (see conditions below) as white solid and tert-butyl N-[8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate [isomer B] (72 mg) as white solid. MS (ESI): 652.1 [M-isobutene+H]⁺, retention time 1.30 minutes (column: Chiralpak AD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-1-[[2-fluoro-4-[4-(trifluoromethyl)pyrazol-1-yl]phenyl]methyl]-2-oxo-3, 4-dihydro-1-benzazepin-8-yl]-1,3,4-oxadiazol-2-yl]propanenitrile and 2-methyl-2-[5-[(3R)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile

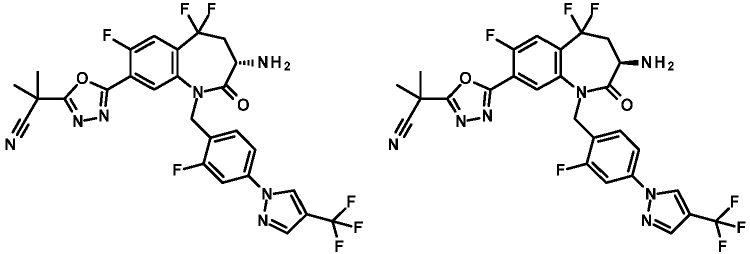

A solution of tert-butyl N-[8-[5-(1-cyano-1-methyl-ethyl)-1,3,4-oxadiazol-2-yl]-5,5,7-trifluoro-1-[[2-fluoro-4-[4-(trifluoromethyl)pyrazol-1-yl]phenyl]methyl]-2-oxo-3,4-dihydro-1-benzazepin-3-yl]carbamate [isomer A] (70.0 mg, 0.1 mmol, 1.0 eq) in a mixture of DCM/TFA (3:1, 4.0 mL) was stirred at 25°C for 1 h. DCM (5 mL) was added and the pH of the mixture was adjusted to 8 by careful addition of saturated NaHCO₃ solution. The resulting mixture was extracted with DCM (10 mL x 3) and the combined organic layers were washed with brine (20 mL), dried (Na₂SO₄) and concentrated. The remaining residue was purified by preperative HPLC (column Waters Xbridge 150x25mm, 5µm). The product was dissolved in EtOAc (30 mL) and HCl/EtOAc (4 M, 0.05 mL) was added. The mixture was concentrated to obtain 2-methyl-2-[5-[3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile [isomer A] as white solid as hydrochloride salt (32.2 mg, 0.05 mmol, 50% yield). MS (ESI): 608.2 [M+H]⁺, retention time 1.64 minutes (see conditions below). 2-methyl-2-[5-[3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile [isomer B] was obtained in analogy. MS (ESI): 608.1 [M+H]⁺, retention time 2.06 minutes (column: Chiralpak IK-3 50×4.6 mm I.D., 3 µm; mobile phase: phase A for CO₂, and phase B for MeOH (0.05% DEA); 5% to 40% MeOH (0.05% DEA) in CO₂; flow rate: 3 mL/min; detector: PDA; column temperatur: 35°C; back pressure: 100 bar).

### Example 45

### 2-methyl-2-[5-[(3S)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile and

### 2-methyl-2-[5-[(3R)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile

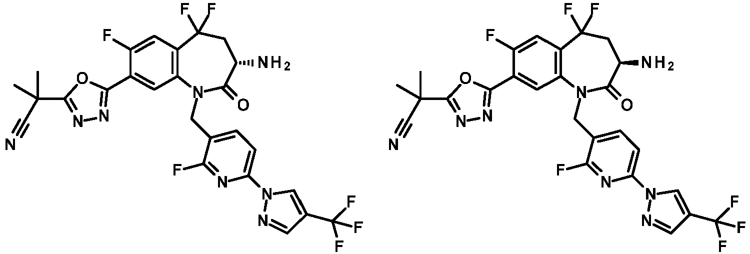

### Step a) tert-butyl N-[8-[5-(1-cyano-1-methyl-ethyl)-1, 3, 4-oxadiazol-2-yl]-5,5,7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate

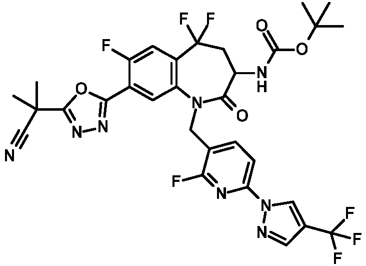

The title compound was prepared in analogy to Example 1, steps a to l (using 3-(chloromethyl)-2-fluoro-6-[4-(trifluoromethyl)pyrazol-1-yl]pyridine (Intermediate 12) instead of 4-(trifluoromethoxy)benzyl bromide in step f and 2-cyano-2-methyl-propanoic acid instead of pivalic acid in step k) and was obtained as light yellow solid. MS (ESI): 731.2 [M+Na]⁺.

### Step b) tert-butyl N-[(3S)-8-[5-(1-cyano-1-methyl-ethyl)-1, 3, 4-oxadiazol-2-yl]-5,5, 7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate and tert-butyl N-[(3R)-8-[5-(1-cyano-1-methyl-ethyl)-1,3, 4-oxadiazol-2-yl]-5,5, 7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate

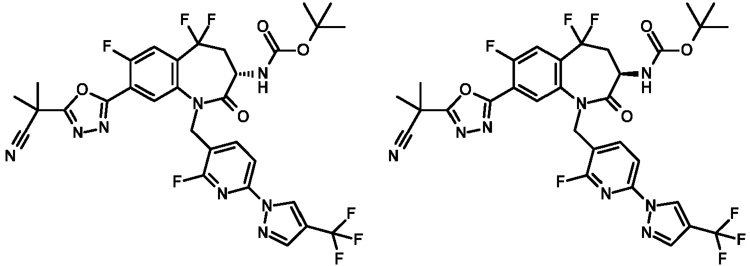

tert-butyl N-[8-[5-(1-cyano-1-methyl-ethyl)-1,3,4-oxadiazol-2-yl]-5,5,7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate was subjected to preparative SFC (Daicel Chiralpak OD (250×30 mm, 10 µm), 0.1% NH₃-H₂O in MeOH) to obtain tert-butyl N-[8-[5-(1-cyano-1-methyl-ethyl)-1,3,4-oxadiazol-2-yl]-5,5,7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate [isomer A] (110 mg) (MS (ESI): 731.2 [M+Na]⁺, retention time 0.96 minutes (see conditions below) as white solid and tert-butyl N-[8-[5-(1-cyano-1-methyl-ethyl)-1,3,4-oxadiazol-2-yl]-5,5,7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate [isomer B] (120 mg) as white solid. MS (ESI): 731.2 [M+Na]⁺, retention time 1.52 minutes (column: Chiralpak OD-3 50×4.6 mm I.D., 3 µm; mobile phase: phase A for CO₂, and phase B for MeOH (0.05% DEA); gradient elution: 5% to 40% MeOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile and 2-methyl-2-[5-[(3R)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile

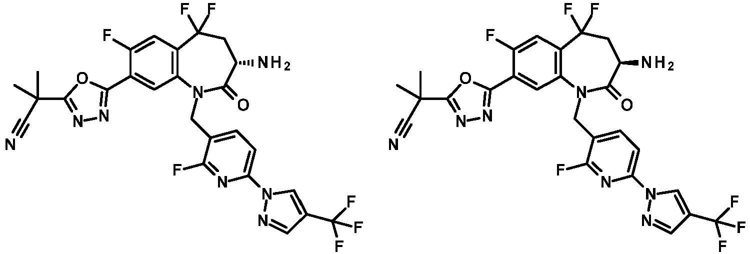

To a solution of tert-butyl N-[8-[5-(1-cyano-1-methyl-ethyl)-1,3,4-oxadiazol-2-yl]-5,5,7-trifluoro-1-[[2-fluoro-6-[4-(trifluoromethyl)pyrazol-1-yl]-3-pyridyl]methyl]-2-oxo-3,4-dihydro-1-benzazepin-3-yl]carbamate [isomer A] (100.0 mg, 0.14 mmol, 1.0 eq) in DCM (10.0 mL) was added TFA (3.0 mL, 38.94 mmol, 275.93 eq) and the mixture was stirred at 20°C for 0.5 h. Saturated NaHCO₃ solution (50 mL) was added carefully and the resulting mixture was extracted with DCM (50 mL). The organic phase was washed with brine (50 mL), dried (Na₂SO₄) and concentrated. The remaining residue was dissolved in EtOAc (10 mL) and HCl/EtOAc (4 M, 0.1 mL) was added. The mixture was concentrated and the remaining residue was dissolved in water (20 mL) to obtain 2-methyl-2-[5-[3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile [isomer A] as white solid as hydrochloride salt (70.2 mg, 0.11 mmol, 73% yield) after lyophilization. MS (ESI): 609.1 [M+H]⁺, retention time 1.35 minutes (see conditions below). 2-methyl-2-[5-[3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile [isomer B] was obtained in analogy. MS (ESI): 609.1 [M+H]⁺, retention time 2.08 minutes (column: Chiralpak OD 50×4.6 mm I.D., 3 µm; mobile phase: phase A for CO₂, and phase B for MeOH (0.05% DEA); 5% to 40% MeOH (0.05% DEA) in CO₂; flow rate: 3 mL/min; detector: PDA; column temperatur: 35°C; back pressure: 100 bar).

### Example 46

### 2-methyl-2-[5-[(3S)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile

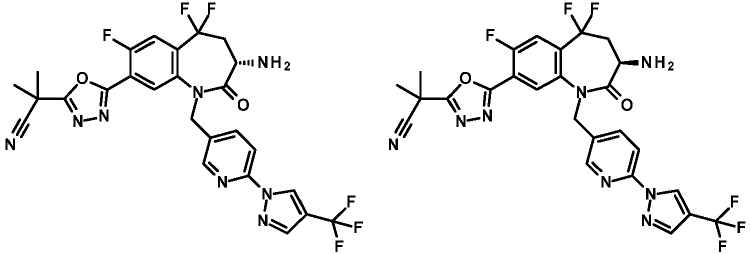

### Step a) tert-butyl N-[8-[5-(1-cyano-1-methyl-ethyl)-1, 3, 4-oxadiazol-2-yl]-5,5,7-trifluoro-2-oxo-1-[[6-[4-(trifluoromethyl)pyrazol-1-yl]-3-pyridyl]methyl]-3,4-dihydro-1-benzazepin-3-yl]carbamate

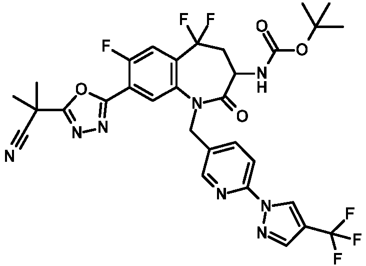

The title compound was prepared in analogy to Example 1, steps a to l (using 5-(chloromethyl)-2-[4-(trifluoromethyl)pyrazol-1-yl]pyridine (Intermediate 13) instead of 4-(trifluoromethoxy)benzyl bromide in step f and 2-cyano-2-methyl-propanoic acid instead of pivalic acid in step k) and was obtained as yellow solid. MS (ESI): 691.2 [M+H]⁺.

### Step b) tert-butyl N-[(3S)-8-[5-(1-cyano-1-methyl-ethyl)-1, 3, 4-oxadiazol-2-yl]-5,5, 7-trifluoro-2-oxo-1-[[6-[4-(trifluoromethyl)pyrazol-1-yl]-3-pyridyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5, 7-trifluoro-2-oxo-1-[[6-[4-(trifluoromethyl)pyrazol-1-yl]-3-pyridyl[methyl]-3,4-dihydro-1-benzazepin-3-yl]carbamate

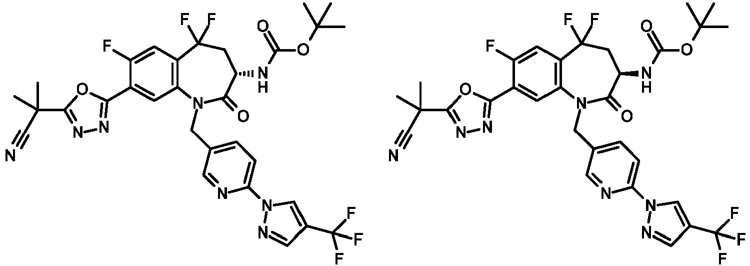

tert-butyl N-[8-[5-(1-cyano-1-methyl-ethyl)-1,3,4-oxadiazol-2-yl]-5,5,7-trifluoro-2-oxo-1-[[6-[4-(trifluoromethyl)pyrazol-1-yl]-3-pyridyl]methyl]-3,4-dihydro-1-benzazepin-3-yl]carbamate was subjected to preparative SFC (Daicel Chiralpak OD (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[6-[4-(trifluoromethyl)pyrazol-1-yl]-3-pyridyl]methyl]-3,4-dihydro-1-benzazepin-3-yl]carbamate [isomer A] (125 mg) (MS (ESI): 691.2 [M+H]⁺, retention time 1.03 minutes (see conditions below) as white solid and tert-butyl N-[8-[5-(1-cyano-1-methyl-ethyl)-1,3,4-oxadiazol-2-yl]-5,5,7-trifluoro-2-oxo-1-[[6-[4-(trifluoromethyl)pyrazol-1-yl]-3-pyridyl]methyl]-3,4-dihydro-1-benzazepin-3-yl]carbamate [isomer B] (125 mg) as white solid. MS (ESI): 691.2 [M+H]⁺, retention time 1.49 minutes (column: Chiralpak OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3, 4-oxadiazol-2-yl]propanenitrile and 2-methyl-2-[5-[(3R)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile

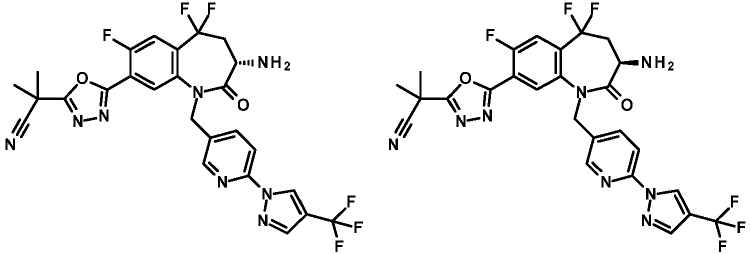

A solution of tert-butyl N-[8-[5-(1-cyano-1-methyl-ethyl)-1,3,4-oxadiazol-2-yl]-5,5,7-trifluoro-2-oxo-1-[[6-[4-(trifluoromethyl)pyrazol-1-yl]-3-pyridyl]methyl]-3,4-dihydro-1-benzazepin-3-yl]carbamate [isomer A] (120.0 mg, 0.17 mmol, 1.0 eq) in a mixture of DCM/TFA (3:1, 12.0 mL) was stirred at 25°C for 1 h. DCM (10 mL) was added and the pH of the mixture was adjusted to 8 by careful addition of saturated NaHCO₃ solution. The resulting mixture was extracted with DCM (20 mL x 3) and the the organic extracts were washed with brine (50 mL), dried (Na₂SO₄) and concentrated. The remaining residue was purified by preperative HPLC (column Waters Xbridge 150x25mm, 5µm). The product was dissolved in EtOAc (30 mL) and HCl/EtOAc (4 M, 0.1 mL) was added. The mixture was concentrated to obtain 2-methyl-2-[5-[3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile [isomer A] as white solid as hydrochloride salt (61.8 mg, 0.1 mmol, 55% yield). MS (ESI): 591.2 [M+H]⁺, retention time 1.38 minutes (see conditions below). 2-methyl-2-[5-[3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile [isomer B] was obtained in analogy. MS (ESI): 591.1 [M+H]⁺, retention time 2.00 minutes (column: Chiralpak OD 50×4.6 mm I.D., 3 µm; mobile phase: phase A for CO₂, and phase B for MeOH (0.05% DEA); 5% to 40% MeOH (0.05% DEA) in CO₂; flow rate: 3 mL/min; detector: PDA; column temperatur: 35°C; back pressure: 100 bar).

### Example 47

### 2-methyl-2-[5-[(3S)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile and

### 2-methyl-2-[5-[(3R)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile

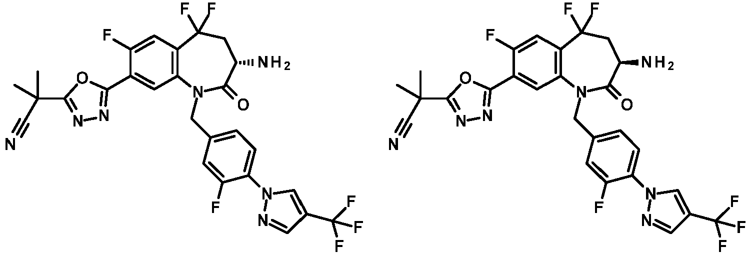

### Step a) tert-butyl N-[8-[5-(1-cyano-1-methyl-ethyl)-1, 3, 4-oxadiazol-2-yl]-5,5,7-trifluoro-1-[[3-fluoro-4-[4-(trifluoromethyl)pyrazol-1-yl]phenyl]methyl]-2-oxo-3,4-dihydro-1-benzazepin-3-yl]carbamate

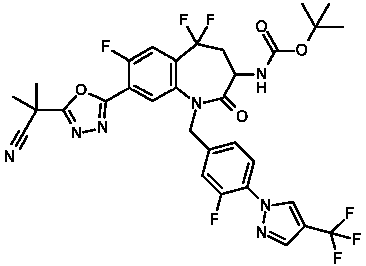

The title compound was prepared in analogy to Example 1, steps a to l (using 1-[4-(chloromethyl)-2-fluoro-phenyl]-4-(trifluoromethyl)pyrazole (Intermediate 14) instead of 4-(trifluoromethoxy)benzyl bromide in step f and 2-cyano-2-methyl-propanoic acid instead of pivalic acid in step k) and was obtained as light yellow solid. MS (ESI): 708.2 [M+H]⁺.

### Step b) tert-butyl N-[(3S)-8-[5-(1-cyano-1-methyl-ethyl)-1, 3, 4-oxadiazol-2-yl]-5,5, 7-trifluoro-1-[[3-fluoro-4-[4-(trifluoromethyl)pyrazol-1-yl]phenyl]methyl]-2-oxo-3,4-dihydro-1-benzazepin-3-yl]carbamate and tert-butyl N-[(3R)-8-[5-(1-cyano-1-methyl-ethyl)-1,3, 4-oxadiazol-2-yl]-5,5, 7-trifluoro-1-[[3-fluoro-4-[4-(trifluoromethyl)pyrazol-1-yl]phenyl]methyl]-2-oxo-3,4-dihydro-1-benzazepin-3-yl]carbamate

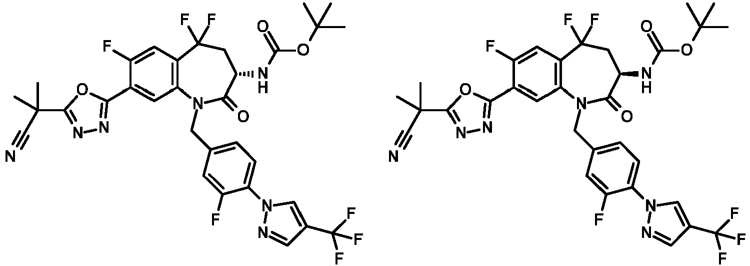

tert-butyl N-[8-[5-(1-cyano-1-methyl-ethyl)-1,3,4-oxadiazol-2-yl]-5,5,7-trifluoro-1-[[3-fluoro-4-[4-(trifluoromethyl)pyrazol-1-yl]phenyl]methyl]-2-oxo-3,4-dihydro-1-benzazepin-3-yl]carbamate was subjected to preparative SFC (Daicel Chiralpak OD-H (250×30 mm, 5 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-1-[[3-fluoro-4-[4-(trifluoromethyl)pyrazol-1-yl]phenyl]methyl]-2-oxo-3,4-dihydro-1-benzazepin-3-yl]carbamate [isomer A] (130 mg) (MS (ESI): 708.2 [M+H]⁺, retention time 0.88 minutes (see conditions below) as white solid and tert-butyl N-[8-[5-(1-cyano-1-methyl-ethyl)-1,3,4-oxadiazol-2-yl]-5,5,7-trifluoro-1-[[3-fluoro-4-[4-(trifluoromethyl)pyrazol-1-yl]phenyl]methyl]-2-oxo-3,4-dihydro-1-benzazepin-3-yl]carbamate [isomer B] (130 mg) as white solid. MS (ESI): 708.2 [M+H]⁺, retention time 1.16 minutes (column: Chiralcel OD-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: PDA; column temperatur: 35°C; back pressure: 100 bar).

### Step c) 2-methyl-2-[5-[(3S)-3-amino-5,5,7-trifluoro-1-[[3-fluoro-4-[4-(trifluoromethyl)pyrazol-1-yl]phenyl]methyl]-2-oxo-3, 4-dihydro-1-benzazepin-8-yl]-1,3,4-oxadiazol-2-yl]propanenitrile and 2-methyl-2-[5-[(3R)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile

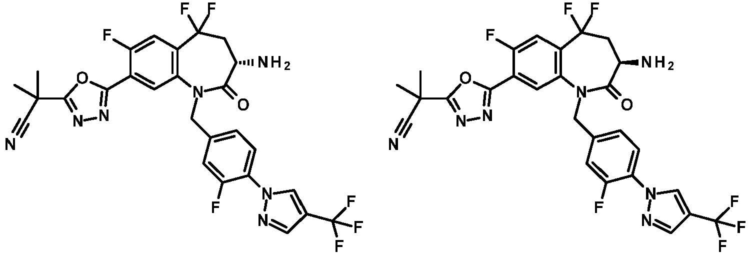

To a solution of tert-butyl N-[8-[5-(1-cyano-1-methyl-ethyl)-1,3,4-oxadiazol-2-yl]-5,5,7-trifluoro-1-[[3-fluoro-4-[4-(trifluoromethyl)pyrazol-1-yl]phenyl]methyl]-2-oxo-3,4-dihydro-1-benzazepin-3-yl]carbamate [isomer A] (120.0 mg, 0.17 mmol, 1.0 eq) in DCM (10.0 mL) was added TFA (3.0 mL) and the mixture was stirred at 20°C for 0.5 h. The pH of the mixture was adjusted to 8 by careful addition of saturated NaHCO₃ solution. The resulting mixture was extracted with DCM (50 mL) and the the organic layer was washed with brine (50 mL), dried (Na₂SO₄) and concentrated. The remaining residue was dissolved in EtOAc (10 mL) and HCl/EtOAc (4 M, 0.1 mL) was added. The mixture was concentrated and water (20 mL) was added to obtain 2-methyl-2-[5-[3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile [isomer A] as white solid as hydrochloride salt (67.5 mg, 0.1 mmol, 61% yield) after lyophilization. MS (ESI): 608.2 [M+H]⁺, retention time 1.33 minutes (see conditions below). 2-methyl-2-[5-[3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile [isomer B] was obtained in analogy. MS (ESI): 608.1 [M+H]⁺, retention time 1.73 minutes (column: Chiralpak OD 50×4.6 mm I.D., 3 µm; mobile phase: phase A for CO₂, and phase B for EtOH (0.05% DEA); 5% to 40% EtOH (0.05% DEA) in CO₂; flow rate: 3 mL/min; detector: PDA; column temperatur: 35°C; back pressure: 100 bar).

### Example 48

### 2-methyl-2-[5-[(3S)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile and

### 2-methyl-2-[5-[(3R)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile

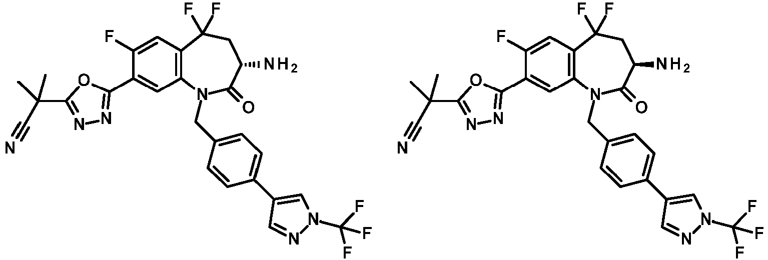

### Step a) tert-butyl N-[8-[5-(1-cyano-1-methyl-ethyl)-1, 3, 4-oxadiazol-2yl]-5,5,7-trifluoro-2-oxo-1-[[4-[1-(trifluoromethyl)pyrazol-4-yl]phenyl]methyl]-3,4-dihydro-1-benzazepin-3-yl]carbamate

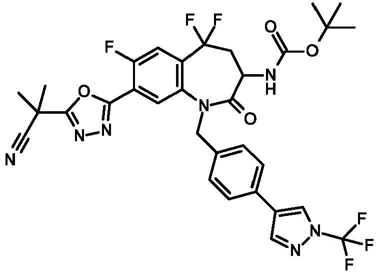

The title compound was prepared in analogy to Example 1, steps a to l (using 4-[4-(chloromethyl)phenyl]-1-(trifluoromethyl)pyrazole (Intermediate 15) instead of 4-(trifluoromethoxy)benzyl bromide in step f and 2-cyano-2-methyl-propanoic acid instead of pivalic acid in step k) and was obtained as light yellow solid. MS (ESI): 634.2 [M-isobutene+H]⁺.

### Step b) tert-butyl N-[(3S)-8-[5-(1-cyano-1-methyl-ethyl)-1, 3, 4-oxadiazol-2-yl]-5,5, 7-trifluoro-2-oxo-1-[[4-[1-(trifluoromethyl)pyrazol-4-yl]phenyl]methyl]-3,4-dihydro-1-benzazepin-3-yl]carbamate and tert-butyl N-[(3R)-8-[5-(1-cyano-1-methyl-ethyl)-1,3,4-oxadiazol-2-yl]-5,5, 7-trifluoro-2-oxo-1-[[4-[1-(trifluoromethyl)pyrazol-4-yl]phenyl]methyl]-3,4-dihydro-1-benzazepin-3-yl]carbamate

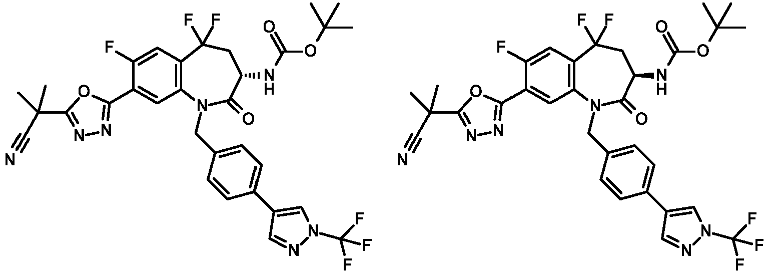

tert-butyl N-[8-[5-(1-cyano-1-methyl-ethyl)-1,3,4-oxadiazol-2-yl]-5,5,7-trifluoro-2-oxo-1-[[4-[1-(trifluoromethyl)pyrazol-4-yl]phenyl]methyl]-3,4-dihydro-1-benzazepin-3-yl]carbamate was subjected to preparative SFC (Daicel Chiralpak AD (250×30 mm, 10 µm), 0.1% NH₃-H₂O in EtOH) to obtain tert-butyl N-[8-[5-(1-cyano-1-methyl-ethyl)-1,3,4-oxadiazol-2-yl]-5,5,7-trifluoro-2-oxo-1-[[4-[1-(trifluoromethyl)pyrazol-4-yl]phenyl]methyl]-3,4-dihydro-1-benzazepin-3-yl]carbamate [isomer A] (MS (ESI): 634.2 [M-isobutene+H]⁺, retention time 1.27 minutes (see conditions below) as white solid and tert-butyl N-[8-[5-(1-cyano-1-methyl-ethyl)-1,3,4-oxadiazol-2-yl]-5,5,7-trifluoro-2-oxo-1-[[4-[1-(trifluoromethyl)pyrazol-4-yl]phenyl]methyl]-3,4-dihydro-1-benzazepin-3-yl]carbamate [isomer B] as white solid. MS (ESI): 634.2 [M-isobutene+H]⁺, retention time 1.89 minutes (column: Chiralpak IC-3 50×4.6 mm I.D., 3 µm; mobile phase: phase A for CO₂, and phase B for EtOH (0.05% DEA); gradient elution: 5% to 40% EtOH (0.05% DEA) in CO₂; flow rate: 3 mL/min; detector: DAD; column temperatur: 35°C; back pressure: 100 bar).

### Step c) 2-methyl-2-[5-[(3S)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3, 4-oxadiazol-2-yl]propanenitrile and 2-methyl-2-[5-[(3R)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile

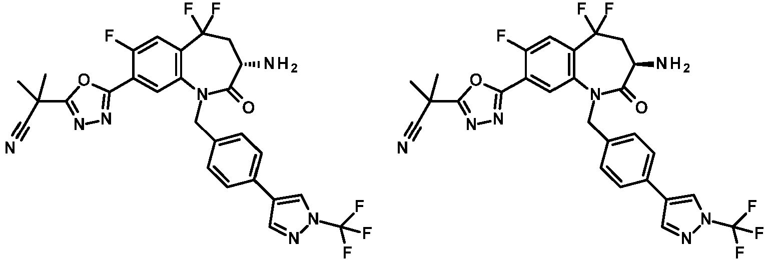

To a solution of tert-butyl N-[8-[5-(1-cyano-1-methyl-ethyl)-1,3,4-oxadiazol-2-yl]-5,5,7-trifluoro-2-oxo-1-[[4-[1-(trifluoromethyl)pyrazol-4-yl]phenyl]methyl]-3,4-dihydro-1-benzazepin-3-yl]carbamate [isomer A] (270.0 mg, 0.39 mmol, 1.0 eq) in DCM (9.0 mL) was added TFA (3.0 mL) and the mixture was stirred at 20°C for 1 h. The mixture was diluted with DCM (1.5 mL) and the pH of the mixture was adjusted to 8 by careful addition of saturated NaHCO₃ solution. The resulting mixture was extracted with DCM (3 mL x 3) and the the organic extracts were washed with brine (7 mL), dried (Na₂SO₄) and concentrated. The remaining residue was dissolved in EtOAc (10 mL) and HCl/EtOAc (4 M, 0.3 mL) was added. The mixture was concentrated and water (20 mL) was added to obtain 2-methyl-2-[5-[3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile [isomer A] as white solid as hydrochloride salt (237.1 mg, 0.38 mmol, 94% yield) after lyophilization. MS (ESI): 590.2 [M+H]⁺, retention time 1.41 minutes (see conditions below). 2-methyl-2-[5-[3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile [isomer B] was obtained in analogy. MS (ESI): 608.1 [M+H]⁺, retention time 1.85 minutes (column: Chiralpak OD-3 50×4.6 mm I.D., 3 µm; mobile phase: phase A for CO₂, and phase B for MeOH (0.05% DEA); 5% to 40% MeOH (0.05% DEA) in CO₂; flow rate: 3 mL/min; detector: PDA; column temperatur: 35°C; back pressure: 100 bar).

### 2) Biological examples

### 2.1) In vitro DGK Inhibition Assays

DGK α and ζ kinase use ATP to phosphorylate the substrate 1,2-dilauroyl-sn-glycerol (DLG, incorporated in the liposomes). ATP is converted to ADP as a result of this enzymatic reaction.

After the kinase reaction, an ATP-depletion reagent is added to terminate the kinase reaction and deplete any remaining ATP, leaving only ADP. Second, a detection reagent is added to simultaneously convert ADP to ATP and allow the newly synthesized ATP to be converted to light using a coupled luciferase/luciferin reaction.

### Reagents and Material

### Buffer Ingredients (solutions & salts)

| **Chemicals** | Vendor | Cat. Nr.: |
|---|---|---|
| DTT | AppliChem | Cat# A3668 |
| BSA | Sigma - Aldrich | Cat# A2153-10G |
| MOPS | Sigma - Aldrich | Cat# M1254 |
| Sodium chloride (NaCl) | Sigma - Aldrich | Cat# S7663-1KG |
| Magnesium chloride (MgCl₂) | MERCK | Cat# 1.05833.0250 |
| Calcium chloride (CaCl₂) | Sigma - Aldrich | Cat# C4901-500G |

### Protein / Substrates / Tracer

| **Proteins / Substrate / Tracer** | **Vendor** | **Lot#; Probe#** |
|---|---|---|
| DGKA | In house production | hDGKalpha(1-735)-N-His |
| DGKZ | In house production | hDGKzeta(1-928)-N-His |
| 18:1 PS (DOPS) | Sigma - Aldrich | Cat# 840035C |
| 16:0-18:1 PC | Sigma - Aldrich | Cat# 850457C |
| 1,2-dilauroyl-sn-glycerol (DLG) | Sigma - Aldrich | Cat# 800812C |
| Ultra Pure ATP | Promega | Cat# V9101 Part No. V915A |
| ADP-Glo Reagent | Promega | Cat# V9101 Part No. V912A |
| Kinase Detection Substr. | Promega | Cat# V9101 Part No. V914A |
| Kinase Detection Buffer | Promega | Cat# V9101 Part No. V913A |

Full length DGKA and Z were expressed in Sf21 insect cells by infecting the cells with the baculovirus stock at MOI of 2. Purification of both enzymes was performed as previously described by Takahashi et al., PeerJ, 2018 *(*Takahashi, D.; Sakane, F. Expression and purification of human diacylglycerol kinase alpha from baculovirus-infected insect cells for structural studies. PeerJ 2018, 6, No. e5449*).*

### Hardware

| **Material** | **Vendor** | **Cat. Nr. / Volume range** |
|---|---|---|
| 384 multiwell plate, white | Greiner | Cat# 781904 |
| 384 multiwell plate, white | Corning | Cat# CLS3574 |
| Matrix Multichannel Pipette | Thermo Fisher SCIENTIFIC | 2 -125 µl |
| Multidrop Combi | Thermo Fisher SCIENTIFIC | 0.5 - 50 µl/well |
| Envision plate reader | Perkin Elmer | |

### Assay Buffer (30ml)

| **Chemical** | **initial concentration (Stock)** | | **Working Solution (1-time)** | | | **final Assay concentration** | |
|---|---|---|---|---|---|---|---|
| | conc. | [mM, %....] | conc. | [mM, %....] | Volume [µl] | conc. | [mM, %....] |
| MOPS (pH 7.5) | 1000 | mM | 50 | mM | 1500 | 50 | mM |
| NaCl | 5000 | mM | 100 | mM | 600 | 100 | mM |
| MgCl₂ | 1000 | mM | 10 | mM | 300 | 10 | mM |
| CaCl₂ | 1 | mM | 0.001 | mM | 30 | 0.001 | mM |
| DTT | 1000 | mM | 1 | mM | 30 | 1 | mM |
| BSA | 10 | % | 0.01 | % | 30 | 0.01 | % |
| filled up with: | | | ddWater: | | 27510 | | |

### Assay procedure

A concentrated liposome solution was prepared in assay buffer without DTT and BSA: 2 mM of DLG in 21 mM of total liposome (2 mM DLG / 8 mM PS / 11 mM PC). The reaction mixtures contain the assay buffer with a final DLG concentration of 125 µM ATP concentrations of 25 µM (for DGKA assay) or 50 µM (for DGKZ assay). The reactions were started by addition of DGK α and ζ kinases at 4 nM and 2 nM final concentrations, respectively. After 1 hour reaction, the amount of ADP formed was detected with the ADP-Glo kinase assay (Promega) according to the manufacturer instructions. Compounds were added in 11-points dose response, starting at 10 mM, 1:3 dilutions, with a final DMSO concentration of 2%. The multidrop combi was used as a liquid handler and luminescence was read with 0.5 s by the envision reader (PE).

### Results

| Example | Isomer | DGKalpha ADP Glo assay IC₅₀ (µM) | DGKzeta ADP Glo assay IC₅₀ (µM) |
|---|---|---|---|
| 1 | A | 33.886 | 19.934 |
| 2 | A | 1.401 | 1.806 |
| 3 | A | 1.568 | 2.892 |
| 4 | A | 4.372 | 8.928 |
| 5 | A | 16.923 | 2.398 |
| 6 | A | 56.127 | 13.831 |
| 7 | A | 9.643 | 1.731 |
| 8 | A | 5.438 | 16.137 |
| 9 | A | 9.920 | 2.356 |
| 10 | A | 16.199 | 11.454 |
| 11 | A | 3.314 | 1.252 |
| 12 | A | 24.627 | 2.896 |
| 13 | A | 9.250 | 2.256 |
| 14 | A | >62.5 | 17.928 |
| 15 | A | 39.595 | 1.812 |
| 16 | A | 13.247 | 14.851 |
| 17 | A | >62.5 | 3.249 |
| 18 | A | >62.5 | >62.5 |
| 19 | A | 41.596 | 7.244 |
| 20 | A | 7.182 | 2.137 |
| 21 | A | 10.877 | 11.828 |
| 22 | A | 10.592 | 17.770 |
| 23 | A | 14.123 | 15.443 |
| 24 | A | 1.855 | 2.404 |
| 26 | A | 0.425 | 8.822 |
| 27 | A | 24.620 | 1.825 |
| 28 | A | 31.681 | 11.222 |
| 29 | A | 0.551 | 13.861 |
| 30 | A | 26.368 | 10.208 |
| 32 | A | 51.273 | 33.849 |
| 33 | A | 2.370 | 1.683 |
| 34 | A | >60.000 | 11.059 |
| 35 | A | >18.980 | >18.980 |
| 36 | A | 55.159 | 5.923 |
| 37 | A | 11.157 | 2.849 |
| 38 | A | 60.000 | 5.796 |
| 39 | A | 44.396 | 38.751 |
| 40 | A | 15.348 | 2.499 |
| 41 | A | 24.035 | 8.222 |
| 42 | A | 4.315 | 2.225 |
| 46 | A | 17.840 | 6.751 |
| 47 | A | 23.688 | 2.992 |
| 48 | A | 1.508 | 4.915 |

### In vitro DGK Inhibition Assays (ADP Glo)

DGK α and ζ kinase use ATP to phosphorylate the substrate 1,2-dilauroyl-sn-glycerol (DLG). ATP is converted to ADP as a result of this enzymatic reaction.

After the kinase reaction, an ATP-depletion reagent is added to terminate the kinase reaction and deplete any remaining ATP, leaving only ADP. Second, a detection reagent is added to simultaneously convert ADP to ATP and allow the newly synthesized ATP to be converted to light using a coupled luciferase/luciferin reaction.

### Experimental Procedure, Reagents and Material

DGK α and ζ kinase ADP Glo assays were ran by Reaction Biology Corp., 1 Great Valley Parkway, Suite 2, Malvern, 19355, PA, USA. Information provided by the service provider are the following: DGK α and ζ kinases were used at 2 nM final concentration. Reactions were carried out at 50 µM ATP. 500 uM of the substrate DLG (Dilauroyl-sn-glycerol) was used. Compounds were received at 10 mM DMSO stock solution and were tested in 10-dose IC50 duplicate with 3-fold serial dilution starting at 1 µM. A control compound, Calphostin C, was tested in 10-dose IC50 with 3-fold serial dilution starting at 100 µM.

### Results

| Example | Isomer | DGKalpha IC₅₀ (nM) | DGKzeta IC₅₀ (nM) |
|---|---|---|---|
| 2 | A | 10.5 | 22.1 |
| 5 | A | 124.4 | 57.9 |
| 7 | A | 70.3 | 49.5 |
| 9 | A | 43.6 | 32.6 |
| 10 | A | 93.1 | 1055.5 |
| 11 | A | 6.9 | 20.6 |
| 12 | A | 107.7 | 73.0 |
| 33 | A | 8.2 | 80.3 |
| 37 | A | 46.2 | 135.6 |

### 2.2) IL2 secretion measurements

As readouts for T-cell activation, IL2 secretion after 24 hours and proliferation after 5 days was measured. Increases in IL2 secretion and proliferation upon compound treatment were assessed as the % of the maximum of reference compound **A1**. WO 2016/139181 discloses reference compound **A1** as example 70. As a counter screen and to make sure that no unwanted TCR-independent activation was triggered, PBS conditions were run for all compounds.

### Reagents and Material

| **Reagents** | **Information** | **Stock conc.** | **End conc.** |
|---|---|---|---|
| Compound dilution Buffer (CDB) DMSO | Sigma, #D2650 | >99% | 0.2% DMSO |
| RPMI + GlutaMAX | Gibco, #61870-010 | | |
| Human serum (HS) | Sigma, #H3667-100ML | 100% | 5% |
| Sodium Pyruvate (100 mM) | Gibco, #11360-039 | 100x | 1x |
| 2-Mercaptoethanol | Gibco, #31350-010 | 50 mM | 50 µM |
| Penicillin-Streptomycin (10,000 U/mL) | Life Technologies, #15140122 | 100X | 1X |
| Primary T Lymphocyte cells enriched from isolated Peripheral blood mononuclear cells (PBMCs) | Buffy coats for PBMCs ordered from Blutspendezentrum Basel-Stadt, RNCB ID: CL008438 | | 312' 500 cells/cm2 in 96-well (100k/well) |
| Activator Control Ref cpd | reference compound A1, stock in DMSO, 0.2% DMSO final | 10 mM | 20 µM |
| Neutral Control DMSO | Sigma, #D2650 | 100% | 0.2% DMSO |
| Compounds | Stock in DMSO | 10 mM | DR starting at 20 µM ; Dil. Factor 3.333; 5 steps |
| No stimulation Control PBS++ | PBS++, Gibco #14040-091 | 100% | 100% |
| Stimulation Control CD3 | CD3 Monoclonal Antibody (OKT3), Thermo Fisher #16-0037-81 | 1mg/ml | Depending on donor (0.1 - 1.0ug/ml) |
| 96-well cell culture plates, Poly-D lysine coated | Corning, #354640 | | |
| Coating buffer for stim control CD3 | PBS++, Gibco #14040-091 | | |
| Washing buffer for coated plates and ELISA | PBS--, Gibco # 14190-094 | | |
| IL-2 Human ProQuantum Immunoassay Kit | Invitrogen, #A35603 | | |
| MicroAmp^{™} EnduraPlate^{™} Optical 384-Well for immunoassay | Applied Biosystems, #4483273 | | |
| LightCycler^{®} 480 Sealing Foil for immunoassay | Roche, #04729757001 | | |
| IL-2 Human Uncoated ELISA Kit | Thermo Fisher Scientific, #88-7025-88 | | |
| Tween 10% for ELISA | Bio-Rad, #161-0781 | 10% | 0.05% |
| Nunc MaxiSorp plates for ELISA | Thermo Fisher Scientific, #439454 | | |
| V-bottom plates for ELISA dilutions | Greiner Bio-One, #651201 | | |
| MACS filter (cell strainer) | Miltenyi Biotech, #130-041-407 | | |
| TopSeal-A Plus (sealing for sn collection) | Perkin Elmer, #6050185 | | |
| U-bottom plates for IL-2 / supernatant harvest | Costar, #3799 | | |
| LightCycler^{®} 480 Multiwell Plate 96 white, ProQuantum working plate | Roche, #04729692001 | | |

### Cell Culture

Expanded primary human T-cells were thawed and cultured in RPMI 1640 (Gibco, #61870-010) + 5% human serum (HS, Sigma, #H3667) + 1 mM Sodium Pyruvate (Gibco, #11360-039) + 50 µM 2-mercaptoethanol (Gibco, #31350-010) and 1x Pen-Strep (Life Technologies, #15140122) medium at density of 2 Mio/ml for 3 hours in 5% CO2, 37°C and 95% humidity. For coating of plates, PBS++ with PBS-- or PBS++ with CD3 antibody (concentration depending on donor and determined by CD3 titrations) was added 100 µl/well to Poly-D Lysine coated 96-well plates. Plates were sealed and incubated at room temperature for 3 hours on a table-top rocking platform. After incubation, plates were washed once with PBS-- and filled with 40 µl/well culture medium only. Compounds were then added (see next section) to medium only plates. After 3 hours of culturing the T-cells, cells were filtered through a cell strainer (Miltenyi Biotech, #130-041-407), counted again and concentration was adjusted to 1.25 Mio/ml.

Cells were then seeded 80µl/well to the 40µl/well including dispensed compounds according to plate layout. By adding cells, compounds were further diluted 1:3, and resulting in 100k cells/120µl/well. After 24 hours 40 µl of supernatant was collected carefully from the top while not disturbing the cells and transferred into a round bottom 96well plate. Collected and frozen supernatant was used for detection of IL2 using the IL-2 Human ProQuantum Immunoassay Kit (Invitrogen) or using the Human IL-2 ELISA Kit (Thermo Fisher).

### Compound treatment

Compounds were added in a 5 or 6pt dose response with the Tecan D300e Digital Dispenser, all conditions 3 times more concentrated than the end-concentration, since cells are added afterwards (80 µl cells to 40 µl prepared medium with treatment). The DR was starting at 20 µM or 10 µM final top concentration and a dilution factor of 3.333. The positive control was the reference compound A1 that was added in a dose response as well, additionally to 3 wells of only 20 µM representing the positive stimulator control. All wells were normalized with DMSO to a final concentration of 0.6% (0.2% end-concentration).

### IL2 ProQuantum Immunoassay

The immunoassay is done following the manufacturer's manual (Invitrogen, #A35603).

Additional information: For the immunoassay, MicroAmp^{™} EnduraPlate^{™} Optical 384-Well plates are used. Frozen supernatant is thawed and centrifuged for 5 minutes at 1000xg, both steps at 4°C. After centrifugation, required sample amount is taken from the top, and in a separate LightCycler V-bottom plate (working plate) diluted with assay dilution buffer, dilution factor depending on the PBS or CD3 condition but at least 1:3. IL-2 standard and blanks are prepared in the same V-bottom plate, standard with a range of 0.0128-5000 pg/ml (extended version). After preparation, 5µl of sample dilutions or standard/blanks are transferred to the optical 384-well plate (assay plate) and the 10 µl reaction protocol is being followed. For measurement, the QuantStudio 12K Flex system is used. Raw data is extracted and IL-2 concentrations are calculated with the Thermo Fisher online app (apps.thermofisher.com/apps/proquantum).

### IL2 Elisa

ELISA is done following the manufacturer's manual (Thermo Fisher Scientific, #88-7025-88).

Additional information: For the ELISA Nunc MaxiSorp 96well plates are used. Frozen supernatant is thawed and centrifuged for 5 minutes at 1000xg, both steps at 4°C. After that, required sample amount is taken from the top, and in a separate V-bottom plate diluted with ELISA diluent, dilution factor depending on the PBS or CD3 condition. IL-2 standard and blanks are prepared in the same V-bottom plate. After preparation, 50 µl of sample dilutions and 100 µl of standard or blanks are transferred to the Nunc plates.

### Calculations and data reporting

CD3 and PBS plates were analysed separately in Genedata Screener using Roche Normalization PCT_POS_CTRL with DMSO set as Neutral Control and 20 µM of the reference compound **A1** set as Stimulator Control/100%.

For CD3 conditions EC50 and Emax of the fitted sigmoidal curve were reported. If no curve could be fitted, the EC50 was reported as blank field and the Emax was based on individual data points. The Emax did not always correspond to the highest concentration tested. Compounds which activate unstimulated cells or compounds which negatively affected viability (see proliferation assay) were flagged.

### Results

| Example | Isomer | Tcell IL2 CD3 EC₅₀ (nM) | Emax IL2 (% activation) |
|---|---|---|---|
| 1 | A | 1,049.1 | 96 |
| 2 | A | 826.7 | 256 |
| 3 | A | >10,000.0 | 37 |
| 4 | A | 820.6 | 91 |
| 5 | A | 2,333.3 | 165 |
| 6 | A | >10,000.0 | 16 |
| 7 | A | 280.2 | 94 |
| 8 | A | 372.6 | 69 |
| 9 | A | 370.4 | 133 |
| 10 | A | 378.5 | 57 |
| 12 | A | 367.2 | 67 |
| 13 | A | >10,000.0 | 58 |
| 14 | A | 982.1 | 239 |
| 15 | A | 1285.2 | 152 |
| 16 | A | 1,457.7 | 183 |
| 17 | A | 3,789.5 | 107 |
| 19 | A | >2727.3 | 63 |
| 20 | A | 1011.9 | 161 |
| 21 | A | 7737.7 | 63 |
| 22 | A | >2727.3 | 31 |
| 23 | A | >2727.3 | 42 |
| 24 | A | 76.8 | 140 |
| 26 | A | 301.5 | 97 |
| 27 | A | >2727.3 | 181 |
| 28 | A | 678.5 | 73 |
| 29 | A | >10000.0 | 48 |
| 30 | A | 2611.4 | 77 |
| 32 | A | 600.0 | 27 |
| 33 | A | 237.3 | 62 |
| 34 | A | >10000.0 | 31 |
| 35 | A | 216.5 | 94 |
| 36 | A | 856.1 | 53 |
| 37 | A | 457.2 | 136 |
| 38 | A | 2078.5 | 105 |
| 39 | A | >10000.0 | 35 |
| 40 | A | >10000.0 | 27 |
| 41 | A | 883.7 | 131 |
| 42 | A | 2889.1 | 129 |

### 2.3) Proliferation assay

### Reagents and Material

| **Reagents** | **Information** | **Stock conc.** | **End conc.** |
|---|---|---|---|
| Compound dilution Buffer (CDB) DMSO | Sigma, #D2650 | >99% | 0.2% DMSO |
| RPMI + GlutaMAX | Gibco, #61870-010 | | |
| Human serum (HS) heat inactivated | Sigma, #H3667-100ML | | 5% |
| Sodium Pyruvate (100 mM) | Gibco, #11360-039 | 100x | 1x |
| 2-Mercaptoethanol | Gibco, #31350-010 | 50mM | 50µM |
| Penicillin-Streptomycin (10,000 U/mL) | Life Technologies, #15140122 | 100X | 1X |
| Primary T Lymphocyte cells enriched from isolated Peripheral blood mononuclear cells (PBMCs) | Buffy coats for PBMCs ordered from Blutspendezentrum Basel-Stadt, RNCB ID: CL008438 | | 312' 500 cells/cm2 in 96-well (100k/well) |
| Activator Control Ref cpd | reference compound A1, stock in DMSO, 0.2% DMSO final | 10 mM | 20 µM |
| Neutral Control DMSO | Sigma, #D2650 | | 0.2% DMSO |
| Compounds | Stock in DMSO | 10 mM | DR starting at 20 µM ; Dil. Factor 3.333; 5 steps |
| No stimulation Control PBS++ | PBS++, Gibco #14040-091 | 100% | 100% |
| Stimulation Control CD3 | CD3 Monoclonal Antibody (OKT3), Thermo Fisher #16-0037-81 | 1mg/ml | Depending on donor (0.1 - 1.0 µg/ml) |
| 96-well cell culture plates, Poly-D lysine coated | Corning, #354640 | | |
| Coating buffer for stim control CD3 | PBS++, Gibco #14040-091 | | |
| Washing buffer for coated plates | PBS--, Gibco # 14190-094 | | |
| CellTiter-Glo^{®} Luminescent Cell Viability Assay | Promega, #G7572 | 2x | 1x |
| MACS filter (cell strainer) | Miltenyi Biotech, #130-041-407 | | |
| TopSeal-A Plus (sealing for coating) | Perkin Elmer, #6050185 | | |
| Backing tape (for measurement) | Perkin Elmer, #6005199 | | |

Expanded primary human T-cells are thawed and cultured in RPMI 1640 (Gibco, #61870-010) + 5% human serum (HS, Sigma, #H3667) + 1mM Sodium Pyruvate (Gibco, #11360-039) + 50 µM 2-mercaptoethanol (Gibco, #31350-010) and 1x Pen-Strep (Life Technologies, #15140122) medium at density of 2 Mio/ml for 3 hours in 5% CO2, 37°C and 95% humidity. For coating of plates, PBS++ only or PBS++ with CD3 antibody (concentration depending on donor and determined by CD3 titrations) is added 100 µl/well to Poly-D Lysine coated 96-well plates. Plates are sealed and incubated at room temperature for 3 hours on a table-top rocking platform. After incubation, plates are washed once with PBS-- and filled with 40 µl/well culture medium only. Compounds are then added (see next section) to medium only plates. After 3 hours of culturing the T-cells, cells are filtered through a cell strainer (Miltenyi Biotech, #130-041-407), counted again and concentration is adjusted to 1.25 Mio/ml.

Cells are then seeded 80 µl/well to the 40 µl/well including dispensed compounds according to plate layout. By adding cells, compounds are further diluted 1:3, and resulting in 100k cells/120 µl/well. After 48 hours 40µl of supernatant is collected carefully from the top while not disturbing the cells. Cells are assessed for proliferation 5 days later by measuring ATP consumption using CellTiterGlo (Promega).

### Compound treatment

Compounds were added in a 5 or 6pt dose response with the Tecan D300e Digital Dispenser, all conditions 3 times more concentrated than the end-concentration, since cells are added afterwards (80µl cells to 40µl prepared medium with treatment). The DR was starting at 20µM or 10µM final top concentration and a dilution factor of 3.333. The positive control was the reference compound **A1** that was added in a dose response as well, additionally to 3 wells of only 20µM representing the positive stimulator control. All wells were normalized with DMSO to a final concentration of 0.6% (0.2% end-concentration).

### Cell Titer Glo Measurements

After 5 days, for detection of ATP which is directly proportional to the number of cells present per well, the CellTiter-Glo^{®} 2.0 Reagent is used. After visual control for toxicity or precipitations of the tested compounds, the plates are equilibrated to room temperature for 45 minutes. CellTiter-Glo^{®} 2.0 Reagent is equilibrated to room temperature as well. After equilibration, an equal amount of CellTiter-Glo reagent is added to the cells (80 µl/well) with an electronic multichannel pipette. Plates are placed on a rocking platform for 15 minutes at room temperature. After incubation, the bottom of the plates is sealed with backing tape. Luminescence is measured with PHERAstar FSX (interval time 0.5sec, gain 3000, focal height 15 mm) and exported as CSV file for analysis in Genedata screener.

### Calculations and data reporting

CD3 and PBS plates were analysed separately in Genedata Screener using Roche Normalization PCT_POS_CTRL with DMSO set as Neutral Control and 20 µM of the reference compound **A1** set as Stimulator Control/100%.

For CD3 conditions EC50 and Emax of the fitted sigmoidal curve were reported. If no curve could be fitted, the EC50 was reported as blank field and the Emax was based on individual data points. The Emax did not always correspond to the highest concentration tested. Compounds which activate unstimulated cells (see IL2 measurements) or compounds which negatively affected viability were flagged.

### Results

| Example | Isomer | Tcell Proliferation CD3 EC₅₀ (nM) | Eₘₐₓ proliferation (% activation) |
|---|---|---|---|
| 1 | A | 2,587.9 | 74 |
| 2 | A | 314.8 | 161 |
| 3 | A | 549.5 | 57 |
| 4 | A | 139.6 | 112 |
| 5 | A | 684.4 | 249 |
| 6 | A | 479.1 | 50 |
| 7 | A | 126.8 | 110 |
| 8 | A | 185.3 | 86 |
| 9 | A | 266.6 | 114 |
| 10 | A | 376.6 | 76 |
| 11 | A | 192.5 | 88 |
| 12 | A | 409.0 | 152 |
| 13 | A | 77.7 | 79 |
| 14 | A | 592.8 | 149 |
| 15 | A | 127.3 | 108 |
| 16 | A | 296.4 | 241 |
| 17 | A | 557.6 | 115 |
| 18 | A | 666.4 | 52 |
| 19 | A | 983.8 | 85 |
| 20 | A | 205.0 | 118 |
| 21 | A | >10000.0 | 43 |
| 22 | A | 2107.0 | 62 |
| 23 | A | >10000.0 | 98 |
| 24 | A | 161.2 | 129 |
| 26 | A | 949.5 | 155 |
| 27 | A | 598.5 | 79 |
| 28 | A | 978.4 | 96 |
| 29 | A | 91.7 | 93 |
| 30 | A | 589.0 | 94 |
| 32 | A | 2339.0 | 81 |
| 33 | A | 98.8 | 128 |
| 34 | A | 1219.7 | 75 |
| 35 | A | 123.8 | 128 |
| 36 | A | 167.8 | 82 |
| 37 | A | 214.8 | 138 |
| 38 | A | 1015.2 | 105 |
| 39 | A | 1416.3 | 98 |
| 40 | A | 655.3 | 99 |
| 41 | A | 195.7 | 113 |
| 42 | A | 1263.2 | 117 |
| 43 | A | 23.4 | 105 |
| 44 | A | 1458.4 | 126 |
| 45 | A | 2935.8 | 67 |
| 46 | A | 542.3 | 87 |
| 47 | A | 495.7 | 98 |
| 48 | A | 163.2 | 171 |

### 2.4) T-cell - TCB - MV3 killing assays

### Reagents and Material

| **Reagents** | **Information** | **Stock conc.** | **End conc.** |
|---|---|---|---|
| Compound dilution Buffer (CDB) DMSO | Sigma, #D2650 | >99% | 0.2% DMSO |

| MV-3 RFP medium | | | |
|---|---|---|---|
| DMEM + GlutaMAX | Gibco, #31966-021 | | |
| Fetal Bovine Serum (FBS) | VWR, #97068-085 | | |
| Penicillin-Streptomycin (PenStrep) (10,000 U/mL) | Life Technologies, #15140122 | 100X | 1X |
| Puromycin dihydrochloride | Sigma, #P9620-10M | | |

| T-cell medium | | | |
|---|---|---|---|
| RPMI + GlutaMAX | Gibco, #61870-010 | | |
| Human serum (HS) heat inactivated | Sigma, #H3667-100ML | | 5% |
| Sodium Pyruvate (100 mM) | Gibco, #11360-039 | 100x | 1x |
| 2-Mercaptoethanol | Gibco, #31350-010 | 50 mM | 50 µM |
| Penicillin-Streptomycin (PenStrep) (10,000 U/mL) | Life Technologies, #15140122 | 100X | 1X |
| Primary T Lymphocyte cells enriched from isolated Peripheral blood mononuclear cells (PBMCs) | PBMCs ordered from stemcell technologies, #70025 | | 312'500 cells/cm2 in 96-well (100k/well) |
| MV-3 RFP (nuclear expression), clone 22 | RNCB Specimen ID: | | 31'250 cells/cm2 in 96-well (10k/well) |
| Activator Control Ref cpd | reference compound A1, stock in DMSO, 0.2% DMSO final | 10 mM | 20 µM |
| Trypsin 0.05% | Gibco, #25300-054 | | |
| T-flask | Falcon, #353133 | | |
| Neutral Control DMSO | Sigma, #D2650 | | 0.2% DMSO |
| Compounds | Stock in DMSO | 10 mM | DR starting at 20 µM ; Dil. Factor 3.333; 8 steps |
| No stimulation Control PBS-- | PBS--, Gibco # 14190-094 | 100% | Depending on TCB concentration |
| Stimulation Control MCSP-TCB | In-house generated TCB, Concept ID P1AD8773-001, WO 2014/131711 A1 | Lot dependent | 1.5 pM - 5 pM (depending on donor sensitivity) |
| 96-well cell culture plates, low evaporation | TTP, #92696 | | |
| Round bottom plate | Costar, #3799 | | |
| 6-well cell culture plate | Corning, #3506 | | |
| MACS filter (cell strainer) | Miltenyi Biotech, #130-041-407 | | |

### Cell Culture

All culturing steps are executed at 5% CO2, 37°C and 95% humidity.

MV-3 RFP cells are cultured in MV-3 medium (DMEM + 10% FBS, 1x PenStrep and 0.5 µg/mL Puromycin) for at least 3 weeks. Cultured MV-3 cells at 80% confluency are washed once with PBS-- and trypsinized until detached. Cells are then counted and resuspended to 1*105 cells/mL in T-cell medium (RPMI 1640 + 5% human serum + 1mM Sodium Pyruvate + 50 µM 2-mercaptoethanol and 1x Pen-Strep). Cells are seeded with 100 µL/well into a 96-well plate (TTP, #92696), and placed for 40 minutes without moving at room temperature in order to achieve evenly distributed attachment of cells. Plates are then incubated until further use.

On the next day, expanded primary human T-cells are thawed and resuspended in T-cell medium to 4*106 cells/mL. For 3 hours, they are cultured in a 6-well plate with 6 mL per well at maximum. After culturing the T-cells, they are filtered through a cell strainer (Miltenyi Biotech, #130-041-407), counted again, and cell concentration is adjusted to 2*106 cells/mL.

### Compound treatment

MCSP-TCB or PBS are pre-diluted in T-cell medium (concentration depending on T-cell donor), 4 times more concentrated than the end-concentration. 60 µL/well of pre-dilutions are then distributed into a round bottom plate (Costar, #3799) according to plate layout. Compounds are added in a 9pt dose response with the Tecan D300e Digital Dispenser, as well 4 times more concentrated than the end-concentration. DMSO concentration of all wells is adjusted to 0.8 %, resulting in 0.2 % as final concentration.

60 µL per well of T-cell suspension are added to the prepared round bottom plate and resuspended with a manual multichannel. 100 µL/well of the resuspended T-cell suspension including treatments are then transferred cautiously to the over-night cultured MV-3 cells according to plate layout. 100 µL T-cell medium only is added to the outer MV-3 wells only. Final compound DR is starting at 20 µM with a dilution factor of 3.333. Final TCB concentration is between 1.5 pM to 5 pM and was determined for each T-cell donor individually by running TCB titrations. For each donor, a TCB concentration was chosen which corresponds to 10-20% of MV3 baseline cell killing in the absence of compound treatment. Positive control is the reference compound **A1** which is added in a DR, as well as additional wells with only 20 µM. 20 µM of reference compound **A1** represent the positive stimulator control, TCB only (DMSO wells) the neutral control.

### Calculations

After transfer of T-cells with treatment pre-dilutions, MV-3 cells are imaged by time-lapse microscopy using IncucyteZOOM^{™} (Essen BioScience, MI, USA). Imaging is performed every 3 hours for a total of 120 hours (10X objective, phase and red image channels, acquisition time 400 ms, Green/Red 4614 optical module). RFP object count per well is analysed in the IncucyteZOOM^{™} Software (Version 2019B Rev2) with a mask that was previously created and optimized for MV-3 cells. Raw data is exported as object count/well and values are normalized as % TCL compared to wells with MV-3 only, representing 100% growth and therefore 0% TCL.

### RFP measurements

Calculated % TCL values are analysed in Genedata Screener using Roche Normalization PCT_POS_CTRL with MCSP-TCB only set as Neutral Control and 20 µM of the reference compound **A1** set as Stimulator Control/100%.

EC50 and Emax values were provided in the table below.

Induced TCL by compounds without TCB treatment or toxicity (observed in the PBS condition) were be flagged.

### Results

| Example | Isomer | MSCP TCB killing MV-3 cells 5d EC₅₀ (µM) | MSCP TCB killing MV-3 cells 5d Eₘₐₓ (%) |
|---|---|---|---|
| 2 | A | 0.043 | 93 |
| 4 | A | 0.069 | 94 |
| 5 | A | 0.372 | 130 |
| 8 | A | 0.140 | 100 |
| 9 | A | 0.086 | 92 |
| 12 | A | 0.179 | 103 |
| 14 | A | 0.146 | 113 |
| 16 | A | 0.048 | 84 |
| 17 | A | 0.361 | 105 |
| 41 | A | 0.194 | 98 |

## Claims

1. A compound of formula (I) 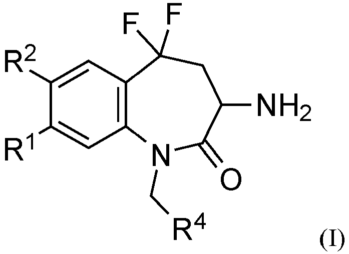 or a pharmaceutically acceptable salt thereof, wherein:
R¹ is 5 or 6-membered heteroaryl, wherein R¹ is optionally substituted with one or more R¹⁰ which can be the same or different;
R² is selected from hydrogen and halogen;
R⁴ is selected from phenyl and pyridinyl, wherein R⁴ is optionally substituted with one or more R¹¹ which can be the same or different;
R¹⁰ is selected from:
i) C₁₋₆-alkyl, optionally substituted with one or more halogen, amino, C₁₋₆-alkoxy, - S(O)₂(C₁₋₆-alkyl), cyano;
ii) C₃₋₁₀-cycloalkyl, optionally substituted with one or more halogen, cyano, amino;
iii) 3-10 membered heterocyclyl, optionally substituted with one or more halogen, C₁₋₁₀-alkyl, amino, halo-C₁₋₆-alkyl, hydroxy, cyano, -C(O)O-(R^{10q}), C₃₋₁₀-cycloalkyl, wherein C₁₋₁₀-alkyl is optionally substituted with one or more hydroxy, C₁₋₆-alkoxy;
iv) -N(R^{10e}R^{10f});
v) heteroaryl, optionally substituted with one or more C₁₋₁₀-alkyl, halogen;
R^{10e} and R^{10f} are each independently selected from:
i) hydrogen;
ii) C₁₋₆-alkyl, optionally substituted with one or more, cyano, halogen, hydroxy;
iii) C₃₋₁₀-cycloalkyl, optionally substituted with one or more halogen, C₁₋₁₀-alkyl;
R^{10q} is C₁₋₅-alkyl, wherein C₁₋₅-alkyl is optionally substituted with one or more hydroxy;
R¹¹ is selected from:
i) halogen;
ii) C₁₋₆-alkoxy, optionally substituted with one or more C₁₋₆-alkyl, C₅₋₆-aryl, C₃₋₁₀-cycloalkyl, halo-C₁₋₆-alkyl, C₃₋₁₀-heterocyclyl, wherein 3-10 membered heterocyclyl is optionally substituted with C₁₋₆-alkyl;
iii) 5-6 membered heteroaryl, optionally substituted with one or more halogen, halo-C₁₋₆-alkyl, C₁₋₆-alkoxy, C₃₋₁₀-cycloalkyl, C₁₋₆-alkyl, halo-C₁₋₆-alkoxy;
iv) phenyl, optionally substituted with one or more C₁₋₆-alkoxy, halo-C₁₋₆-alkyl;
v) -O(R^{11a});
R^{11a} is selected from C₁₋₆-alkyl, C₃₋₆-cycloalkyl, phenyl and halo-C₁₋₆-alkyl.

2. The compound of claim 1, or a pharmaceutically acceptable salt thereof, wherein R¹ is 5-membered heteroaryl, wherein R¹ is optionally substituted with one or more R¹⁰ which can be the same or different.

3. The compound of claim 1or 2, or a pharmaceutically acceptable salt thereof, wherein R¹ is 1,3,4-oxadiazole, substituted with one R¹⁰.

4. The compound of any one of claims 1 to 3, or a pharmaceutically acceptable salt thereof, wherein R² is fluorine.

5. The compound of any one of claims 1 to 4, or a pharmaceutically acceptable salt thereof, wherein R⁴ is phenyl.

6. The compound of any one of claims 1 to 5, or a pharmaceutically acceptable salt thereof, wherein R¹⁰ is selected from methyl-methylsulfonyl-ethyl, pyrrolidin, 4-oxa-7-azaspiro[2.5]octan-7-yl, methyl-propanenitrile, 1,2,2,2-tetrafluoro-methoxy-ethyl, tertbutyl, 1-ethyl-5,5-difluoro-3-piperidyl, and 4,4-difluoro-1-piperidyl.

7. The compound of any one of claims 1 to 6, or a pharmaceutically acceptable salt thereof, wherein R¹¹ is selected from 5-(trifluoromethyl)-2-pyridyl, phenoxy, trifluoromethoxy, methoxy-pyridyl, cyclopentoxy, 4-methoxyphenyl, (trifluoromethyl)-1,2,4-oxadiazol-3-yl, 5-(trifluoromethoxy)-2-pyridyl, 4-(trifluoromethyl)pyrazol-1-yl, chloro, 5-(difluoromethoxy)-2-pyridyl, 4-[4-(trifluoromethoxy)pyrazol-1-yl, 5-(trifluoromethyl)tetrazol-2-yl.

8. The compound of any one of claims 1 to 7, selected from:
3-amino-8-(5-tert-butyl-1,3,4-oxadiazol-2-yl)-5,5,7-trifluoro-1-[[4-(trifluoromethoxy)phenyl]methyl]-3,4-dihydro-1-benzazepin-2-one;
2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
(3S)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one;
(3R)-3-amino-5,5,7-trifluoro-8-(5-morpholino-1,3,4-oxadiazol-2-yl)-1-[[4-(trifluoromethoxy)phenyl]methyl]-3,4-dihydro-1-benzazepin-2-one;
(3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3S)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3R)-3-amino-5,5-difluoro-8-[5-(1-methyl-1-methylsulfonyl-ethyl)-1,3,4-oxadiazol-2-yl]-1-[(4-phenoxyphenyl)methyl]-3,4-dihydro-1-benzazepin-2-one;
(3S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3 S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
(3S)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one;
(3R)-3-amino-5,5,7-trifluoro-1-[[4-(4-methoxyphenyl)phenyl]methyl]-8-[5-(1-methyl-1-methylsulfonyl-ethyl)-1,3,4-oxadiazol-2-yl]-3,4-dihydro-1-benzazepin-2-one;
2-methyl-2-[5-[(3 S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
(3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3 S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-8-(5-tert-butyl-3-pyridyl)-1-[(4-chlorophenyl)methyl]-5,5,7-trifluoro-3,4-dihydro-1-benzazepin-2-one;
(3R)-3-amino-8-(5-tert-butyl-3-pyridyl)-1-[(4-chlorophenyl)methyl]-5,5,7-trifluoro-3,4-dihydro-1-benzazepin-2-one;
2-[5-[(3S)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methyl-propanenitrile;
2-[5-[(3R)-3-amino-5,5,7-trifluoro-1-[[4-[3-fluoro-5-(trifluoromethyl)-2-pyridyl]phenyl]methyl]-2-oxo-3,4-dihydro-1-benzazepin-8-yl]-1,3,4-oxadiazol-2-yl]-2-methyl-propanenitrile;
2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
(3*S*)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[3-(trifluoromethyl)-1,2,4-triazol-1-yl]phenyl]methyl]-3,4-dihydro-1-benzazepin-2-one;
(3*S*)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one;
(3R)-3-amino-8-[5-(3,3-difluoropyrrolidin-1-yl)-1,3,4-oxadiazol-2-yl]-5,5,7-trifluoro-1-[[4-[3-(trifluoromethyl)-1,2,4-triazol-1-yl]phenyl]methyl]-3,4-dihydro-1-benzazepin-2-one;
(3 S)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[3-(trifluoromethyl)-1,2,4-triazol-1-yl]phenyl]methyl]-3,4-dihydro-1-benzazepin-2-one;
2-methyl-2-[5-[(3 S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
(3S)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethoxy)-2-pyridyl]phenyl]methyl]-3,4-dihydro-1-benzazepin-2-one;
2-[5-[(3S)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)-2-pyridyl]phenyl]methyl]-3,4-dihydro-1-benzazepin-8-yl]-1,3,4-oxadiazol-2-yl]-2-methyl-propanenitrile;
2-[5-[(3S)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[6-[4-(trifluoromethyl)phenyl]-3-pyridyl]methyl]-3,4-dihydro-1-benzazepin-8-yl]-1,3,4-oxadiazol-2-yl]-2-methyl-propanenitrile;
(3 S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one;
2-[5-[(3S)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[3-(trifluoromethyl)pyrazol-1-yl]phenyl]methyl]-3,4-dihydro-1-benzazepin-8-yl]-1,3,4-oxadiazol-2-yl]-2-methyl-propanenitrile;
(3S)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)phenyl]phenyl]methyl]-3,4-dihydro-1-benzazepin-2-one;
2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
(3S)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one;
(3R)-3-amino-8-[5-[5,5-difluoro-1-(2-methoxyethyl)-3-piperidyl]-1,3,4-oxadiazol-2-yl]-5,5,7-trifluoro-1-[[4-[5-(trifluoromethyl)-2-pyridyl]phenyl]methyl]-3,4-dihydro-1-benzazepin-2-one;
methyl 1-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-methyl-2-[5-[(3S)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(5-methoxy-2-pyridyl)phenyl]methyl]-2-oxo-3,4-dihydro-1-benzazepin-8-yl]-1,3,4-oxadiazol-2-yl]propanenitrile;
(3S)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one;
(3R)-3-amino-5,5,7-trifluoro-1-[[4-(5-methoxy-2-pyridyl)phenyl]methyl]-8-[5-(1-methyl-1-methylsulfonyl-ethyl)-1,3,4-oxadiazol-2-yl]-3,4-dihydro-1-benzazepin-2-one;
2-methyl-2-[5-[(3S)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3R)-3-amino-1-[[4-[5-(difluoromethyl)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
methyl 1-[5-[(3S)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5,5,7-trifluoro-2-oxo-1-[[4-[5-(trifluoromethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptane-3-carboxylate;
2-methyl-2-[5-[(3*S*)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile;
2-methyl-2-[5-[(3*R*)-3-amino-5,5,7-trifluoro-2-oxo-1-[[4-[4-(trifluoromethoxy)pyrazol-1-yl]phenyl]methyl]-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[2-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[2-fluoro-6-[4-(trifluoromethyl)pyrazol-1-yl]-3-pyridyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[6-[4-(trifluoromethyl)pyrazol-1-yl]-3-pyridyl]methyl]-3,4-dihydro-1-benzazepin-8-yl]-1,3,4-oxadiazol-2-yl]propanenitrile;
2-methyl-2-[5-[(3*S*)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[3-fluoro-4-[4-(trifluoromethyl)pyrazol-1-yl]phenyl]methyl]-2-oxo-3,4-dihydro-1-benzazepin-8-yl]-1,3,4-oxadiazol-2-yl]propanenitrile;
2-methyl-2-[5-[(3*S*)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile; and
2-methyl-2-[5-[(3*R*)-3-amino-5,5,7-trifluoro-2-oxo-1-[[4-[1-(trifluoromethyl)pyrazol-4-yl]phenyl]methyl]-3,4-dihydro-1-benzazepin-8-yl]-1,3,4-oxadiazol-2-yl]propanenitrile;
or a pharmaceutically acceptable salt thereof.

9. The compound of any one of claims 1 to 8, selected from:
2-methyl-2-[5-[(3S)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-(trifluoromethoxy)phenyl]methyl]-3,4-dihydro-1-benzazepin-8-yl]-1,3,4-oxadiazol-2-yl]propanenitrile;
(3S)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3R)-3-amino-5,5,7-trifluoro-8-[5-(1,2,2,2-tetrafluoro-1-methoxy-ethyl)-1,3,4-oxadiazol-2-yl]-1-[[4-(trifluoromethoxy)phenyl]methyl]-3,4-dihydro-1-benzazepin-2-one;
(3S)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3R)-3-amino-8-(5-tert-butyl-1,3,4-oxadiazol-2-yl)-1-[[4-(cyclopentoxy)phenyl]methyl]-5,5,7-trifluoro-3,4-dihydro-1-benzazepin-2-one;
(3 S)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-2-pyridyl]phenyl]methyl]-3,4-dihydro-1-benzazepin-2-one;
2-methyl-2-[5-[(3S)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3R)-3-amino-5,5,7-trifluoro-1-[[4-(4-methoxyphenyl)phenyl]methyl]-2-oxo-3,4-dihydro-1-benzazepin-8-yl]-1,3,4-oxadiazol-2-yl]propanenitrile;
2-methyl-2-[5-[(3 S)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[(4-phenoxyphenyl)methyl]-3,4-dihydro-1-benzazepin-8-yl]-1,3,4-oxadiazol-2-yl]propanenitrile;
2-methyl-2-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]propanenitrile;
(3S)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3R)-3-amino-8-[5-(1-ethyl-5,5-difluoro-3-piperidyl)-1,3,4-oxadiazol-2-yl]-5,5,7-trifluoro-1-[[4-(trifluoromethoxy)phenyl]methyl]-3,4-dihydro-1-benzazepin-2-one;
(3 S)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-(5-pyrrolidin-1-yl-1,3,4-oxadiazol-2-yl)-1-[[4-[5-(trifluoromethyl)-1,2,4-oxadiazol-3-yl]phenyl]methyl]-3,4-dihydro-1-benzazepin-2-one;
(3S)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3R)-3-amino-8-[5-(4,4-difluoro-1-piperidyl)-1,3,4-oxadiazol-2-yl]-5,5,7-trifluoro-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3S)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
(3R)-3-amino-5,5,7-trifluoro-8-[5-(4-oxa-7-azaspiro[2.5]octan-7-yl)-1,3,4-oxadiazol-2-yl]-1-[[4-[5-(trifluoromethyl)-1,2,4-oxadiazol-3-yl]phenyl]methyl]-3,4-dihydro-1-benzazepin-2-one;
2-methyl-2-[5-[(3S)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethyl)tetrazol-2-yl]phenyl]methyl]-3,4-dihydro-1-benzazepin-8-yl]-1,3,4-oxadiazol-2-yl]propanenitrile;
2-methyl-2-[5-[(3S)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
2-methyl-2-[5-[(3R)-3-amino-5,5,7-trifluoro-2-oxo-1-[[4-[5-(trifluoromethoxy)-2-pyridyl]phenyl]methyl]-3,4-dihydro-1-benzazepin-8-yl]-1,3,4-oxadiazol-2-yl]propanenitrile;
(3 S)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one;
(3R)-3-amino-5,5,7-trifluoro-8-[5-(1-methyl-1-methylsulfonyl-ethyl)-1,3,4-oxadiazol-2-yl]-1-[[4-[4-(trifluoromethyl)pyrazol-1-yl]phenyl]methyl]-3,4-dihydro-1-benzazepin-2-one;
2-[5-[(3S)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
2-[5-[(3R)-3-amino-5,5,7-trifluoro-2-oxo-1-[[4-[4-(trifluoromethyl)pyrazol-1-yl]phenyl]methyl]-3,4-dihydro-1-benzazepin-8-yl]-1,3,4-oxadiazol-2-yl]-2-methyl-propanenitrile;
methyl 1-[5-[(3S)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-5,5,7-trifluoro-2-oxo-1-[[4-[5-(trifluoromethyl)-2-pyridyl]phenyl]methyl]-3,4-dihydro-1-benzazepin-8-yl]-1,3,4-oxadiazol-2-yl]-3-azabicyclo[3.1.1]heptane-3-carboxylate;
methyl 1-[5-[(3S)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
methyl 1-[5-[(3R)-3-amino-1-[(4-chlorophenyl)methyl]-5,5,7-trifluoro-2-oxo-3,4-dihydro-1-benzazepin-8-yl]-1,3,4-oxadiazol-2-yl]-3-azabicyclo[3.1.1]heptane-3-carboxylate;
2-[5-[(3S)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-[5-[(3R)-3-amino-1-[[4-[5-(1,1-difluoroethyl)-2-pyridyl]phenyl]methyl]-5,5,7-trifluoro-2-oxo-3,4-dihydro-1-benzazepin-8-yl]-1,3,4-oxadiazol-2-yl]-2-methyl-propanenitrile;
2-methyl-2-[5-[(3S)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile; and
2-methyl-2-[5-[(3R)-3-amino-1-[[4-[5-(difluoromethoxy)-2-pyridyl]phenyl]methyl]-5,5,7-trifluoro-2-oxo-3,4-dihydro-1-benzazepin-8-yl]-1,3,4-oxadiazol-2-yl]propanenitrile;
or a pharmaceutically acceptable salt thereof.

10. The compound according to any one of claims 1 to 9, or a pharmaceutically acceptable salt thereof, for use as therapeutically active substance.

11. A pharmaceutical composition comprising a compound according to any one of claims 1 to 9, or a pharmaceutically acceptable salt thereof, and a pharmaceutically acceptable excipient.

12. The pharmaceutical composition according to claim 11, further comprising an additional therapeutic agent.

13. The compound according to any one of claims 1 to 9, or a pharmaceutically acceptable salt thereof, for use in the treatment, prevention and/or delay of progression of cancer.

14. The compound for use according to claim 13, wherein the cancer is associated with aberrant diacylglycerol kinase signaling, wherein the diacylglycerol kinase is selected from DGKα and/or DGKζ.

15. The compound for use according to claim 13 or 14, wherein the cancer is selected from the group consisting of B-cell acute lymphoid leukemia, T-cell acute lymphoid leukemia, acute lymphoid leukemia, chronic myelogenous leukemia, chronic lymphocytic leukemia B-cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom's macroglobulinemia, preleukemia, sarcoma, carcinoma, melanoma, neuroblastoma, renal cell carcinoma, colon cancer, colorectal cancer, breast cancer, epithelial squamous cell cancer, melanoma, stomach cancer, brain cancer, lung cancer (e.g., NSCLC), pancreatic cancer, cervical cancer, ovarian cancer, liver cancer, bladder cancer, prostate cancer, testicular cancer, thyroid cancer, uterine cancer, adrenal cancer and head and neck cancer.

## Patentansprüche

1. Verbindung der Formel (I) 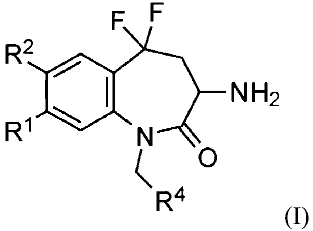 oder ein pharmazeutisch verträgliches Salz davon, wobei:
R¹ 5- oder 6-gliedriges Heteroaryl ist, wobei R¹ gegebenenfalls mit einem oder mehreren R¹⁰ substituiert ist, die gleich oder verschieden sein können;
R² ausgewählt ist aus Wasserstoff und Halogen;
R⁴ ausgewählt ist aus Phenyl und Pyridinyl, wobei R⁴ gegebenenfalls mit einem oder mehreren R¹¹ substituiert ist, die gleich oder verschieden sein können;
R¹⁰ ausgewählt ist aus:
i) C₁₋₆-Alkyl, gegebenenfalls substituiert mit einem oder mehreren Halogen, Amino, C₁₋₆-Alkoxy, -S(O)₂(C₁₋₆-Alkyl), Cyano;
ii) C₃₋₁₀-Cycloalkyl, gegebenenfalls substituiert mit einem oder mehreren Halogen, Cyano, Amino;
iii) 3-10-gliedrigem Heterocyclyl, gegebenenfalls substituiert mit einem oder mehreren Halogen, C₁₋₁₀-Alkyl, Amino, Halogen-C₁₋₆-alkyl, Hydroxy, Cyano, -C(O)O-(R^{10q}), C₃₋₁₀-Cycloalkyl, wobei C₁₋₁₀-Alkyl gegebenenfalls mit einem oder mehreren Hydroxy, C₁₋₆-Alkoxy substituiert ist;
iv) -N(R^{10e}R^{10f});
v) Heteroaryl, gegebenenfalls substituiert mit einem oder mehreren C₁₋₁₀-Alkyl, Halogen;
R^{10e} und R^{10f} jeweils unabhängig voneinander ausgewählt sind aus:
i) Wasserstoff;
ii) C₁₋₆-Alkyl, gegebenenfalls substituiert mit einem oder mehreren Cyano, Halogen, Hydroxy;
iii) C₃₋₁₀-Cycloalkyl, gegebenenfalls substituiert mit einem oder mehreren Halogen, C₁₋₁₀-Alkyl;
R^{10q} C₁₋₅-Alkyl ist, wobei C₁₋₅-Alkyl gegebenenfalls mit einem oder mehreren Hydroxy substituiert ist;
R¹¹ ausgewählt ist aus:
i) Halogen;
ii) C₁₋₆-Alkoxy, gegebenenfalls substituiert mit einem oder mehreren C₁₋₆-Alkyl, C₅₋₆-Aryl, C₃₋₁₀-Cycloalkyl, Halogen-C₁₋₆-alkyl, C₃₋₁₀-Heterocyclyl, wobei 3-10-gliedriges Heterocyclyl gegebenenfalls mit C₁₋₆-Alkyl substituiert ist;
iii) 5-6-gliedrigem Heteroaryl, gegebenenfalls substituiert mit einem oder mehreren Halogen, Halogen-C₁₋₆-alkyl, C₁₋₆-Alkoxy, C₃₋₁₀-Cycloalkyl, C₁₋₆-Alkyl, Halogen-C₁₋₆-alkoxy;
iv) Phenyl, gegebenenfalls substituiert mit einem oder mehreren C₁₋₆-Alkoxy, Halogen-C₁₋₆-alkyl;
v) -O(R^{11a});
R^{11a} ausgewählt ist aus C₁₋₆-Alkyl, C₃₋₆-Cycloalkyl, Phenyl und Halogen-C₁₋₆-alkyl.

2. Verbindung nach Anspruch 1 oder ein pharmazeutisch verträgliches Salz davon, wobei R¹ 5-gliedriges Heteroaryl ist, wobei R¹ gegebenenfalls mit einem oder mehreren R¹⁰ substituiert ist, die gleich oder verschieden sein können.

3. Verbindung nach Anspruch 1 oder 2 oder ein pharmazeutisch verträgliches Salz davon, wobei R¹ 1,3,4-Oxadiazol, substituiert mit einem R¹⁰, ist.

4. Verbindung nach einem der Ansprüche 1 bis 3 oder ein pharmazeutisch verträgliches Salz davon, wobei R² Fluor ist.

5. Verbindung nach einem der Ansprüche 1 bis 4 oder ein pharmazeutisch verträgliches Salz davon, wobei R⁴ Phenyl ist.

6. Verbindung nach einem der Ansprüche 1 bis 5 oder ein pharmazeutisch verträgliches Salz davon, wobei R¹⁰ ausgewählt ist aus Methyl-methylsulfonyl-ethyl, Pyrrolidin, 4-Oxa-7-azaspiro[2.5]octan-7-yl, Methyl-propannitril, 1,2,2,2-Tetrafluor-methoxy-ethyl, tert-Butyl, 1-Ethyl-5,5-difluor-3-piperidyl und 4,4-Difluor-1-piperidyl.

7. Verbindung nach einem der Ansprüche 1 bis 6 oder ein pharmazeutisch verträgliches Salz davon, wobei R¹¹ ausgewählt ist aus 5-(Trifluormethyl)-2-pyridyl, Phenoxy, Trifluormethoxy, Methoxy-pyridyl, Cyclopentoxy, 4-Methoxyphenyl, (Trifluormethyl)-1,2,4-oxadiazol-3-yl, 5-(Trifluormethoxy)-2-pyridyl, 4-(Trifluormethyl)pyrazol-1-yl, Chlor, 5-(Difluormethoxy)-2-pyridyl, 4-[4-(Trifluormethoxy)pyrazol-1-yl, 5-(Trifluormethyl)tetrazol-2-yl.

8. Verbindung nach einem der Ansprüche 1 bis 7, ausgewählt aus:
3-Amino-8-(5-tert-butyl-1,3,4-oxadiazol-2-yl)-5,5,7-trifluor-1-[[4-(trifluormethoxy)phenyl]methyl]-3,4-dihydro-1-benzazepin-2-on;
2-Methyl-2-[5-[(3S)-3-amino-5,5,7-trifluor-2-oxo-1-[[4-(trifluormethoxy)phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-(trifluormethoxy)phenyl]methyl]-3,4-dihydro-1-benzazepin-8-yl]-1,3,4-oxadiazol-2-yl]propannitril;
(3S)-3-Amino-5,5,7-trifluor-8-(5-morpholino-1,3,4-oxadiazol-2-yl)-1-[[4-(trifluormethoxy)phenyl]methyl]-3,4-dihydro-1-benzazepin-2-on;
(3R)-3-Amino-5,5,7-trifluor-8-(5-morpholino-1,3,4-oxadiazol-2-yl)-1-[[4-(trifluormethoxy)phenyl]methyl]-3,4-dihydro-1-benzazepin-2-on;
(3S)-3-Amino-5,5,7-trifluor-8-[5-(1,2,2,2-tetrafluor-1-methoxyethyl)-1,3,4-oxadiazol-2-yl]-1-[[4-(trifluormethoxy)phenyl]methyl]-3,4-dihydro-1-benzazepin-2-on;
(3R)-3-Amino-5,5,7-trifluor-8-[5-(1,2,2,2-tetrafluor-1-methoxyethyl)-1,3,4-oxadiazol-2-yl]-1-[[4-(trifluormethoxy)phenyl]methyl]-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[(4-phenoxyphenyl)methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[(4-phenoxyphenyl)methyl]-3,4-dihydro-1-benzazepin-2-on;
(3S)-3-Amino-5,5-difluor-8-[5-(1-methyl-1-methylsulfonylethyl)-1,3,4-oxadiazol-2-yl]-1-[(4-phenoxyphenyl)methyl]-3,4-dihydro-1-benzazepin-2-on;
(3R)-3-Amino-5,5-difluor-8-[5-(1-methyl-1-methylsulfonylethyl)-1,3,4-oxadiazol-2-yl]-1-[(4-phenoxyphenyl)methyl]-3,4-dihydro-1-benzazepin-2-on;
(3S)-3-Amino-8-(5-tert-butyl-1,3,4-oxadiazol-2-yl)-1-[[4-(cyclopentoxy)phenyl]methyl]-5,5,7-trifluor-3,4-dihydro-1-benzazepin-2-on;
(3R)-3-Amino-8-(5-tert-butyl-1,3,4-oxadiazol-2-yl)-1-[[4-(cyclopentoxy)phenyl]methyl]-5,5,7-trifluor-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[[4-[5-(trifluormethyl)-2-pyridyl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[5-(trifluormethyl)-2-pyridyl]phenyl]methyl]-3,4-dihydro-1-benzazepin-2-on;
2-Methyl-2-[5-[(3S)-3-amino-5,5,7-trifluor-1-[[4-(4-methoxyphenyl)phenyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[4-(4-methoxyphenyl)phenyl]methyl]-2-oxo-3,4-dihydro-1-benzazepin-8-yl]-1,3,4-oxadiazol-2-yl]propannitril;
(3S)-3-Amino-5,5,7-trifluor-1-[[4-(4-methoxyphenyl)phenyl]methyl]-8-[5-(1-methyl-1-methylsulfonylethyl)-1,3,4-oxadiazol-2-yl]-3,4-dihydro-1-benzazepin-2-on;
(3R)-3-Amino-5,5,7-trifluor-1-[[4-(4-methoxyphenyl)phenyl]methyl]-8-[5-(1-methyl-1-methylsulfonylethyl)-1,3,4-oxadiazol-2-yl]-3,4-dihydro-1-benzazepin-2-on;
2-Methyl-2-[5-[(3S)-3-amino-5,5,7-trifluor-2-oxo-1-[(4-phenoxyphenyl)methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[(4-phenoxyphenyl)methyl]-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]propannitril;
(3S)-3-Amino-8-[5-(1-ethyl-5,5-difluor-3-piperidyl)-1,3,4-oxadiazol-2-yl]-5,5,7-trifluor-1-[[4-(trifluormethoxy)phenyl]methyl]-3,4-dihydro-1-benzazepin-2-on;
(3R)-3-Amino-8-[5-(1-ethyl-5,5-difluor-3-piperidyl)-1,3,4-oxadiazol-2-yl]-5,5,7-trifluor-1-[[4-(trifluormethoxy)phenyl]methyl]-3,4-dihydro-1-benzazepin-2-on;
(3S)-3-Amino-5,5,7-trifluor-8-(5-pyrrolidin-1-yl-1,3,4-oxadiazol-2-yl)-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-(5-pyrrolidin-1-yl-1,3,4-oxadiazol-2-yl)-1-[[4-[5-(trifluormethyl)-1,2,4-oxadiazol-3-yl]phenyl]methyl]-3,4-dihydro-1-benzazepin-2-on;
(3S)-3-Amino-8-[5-(4,4-difluor-1-piperidyl)-1,3,4-oxadiazol-2-yl]-5,5,7-trifluor-1-[[4-[5-(trifluormethyl)-1,2,4-oxadiazol-3-yl]phenyl]methyl]-3,4-dihydro-1-benzazepin-2-on;
(3R)-3-Amino-8-[5-(4,4-difluor-1-piperidyl)-1,3,4-oxadiazol-2-yl]-5,5,7-trifluor-1-[[4-[5-(trifluormethyl)-1,2,4-oxadiazol-3-yl]phenyl]methyl]-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3S)-3-Amino-5,5,7-trifluor-8-[5-(4-oxa-7-azaspiro[2.5]octan-7-yl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-[5-(4-oxa-7-azaspiro[2.5]octan-7-yl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3S)-3-Amino-8-(5-tert-butyl-3-pyridyl)-1-[(4-chlorphenyl)methyl]-5,5,7-trifluor-3,4-dihydro-1-benzazepin-2-on;
(3R)-3-Amino-8-(5-tert-butyl-3-pyridyl)-1-[(4-chlorphenyl)methyl]-5,5,7-trifluor-3,4-dihydro-1-benzazepin-2-on;
2-[5-[(3S)-3-Amino-5,5,7-trifluor-1-[[4-[3-fluor-5-(trifluormethyl)-2-pyridyl]phenyl]methyl]-2-oxo-3,4-dihydro-1-benzazepin-8-yl]-1,3,4-oxadiazol-2-yl]-2-methylpropannitril;
2-[5-[(3R)-3-Amino-5,5,7-trifluor-1-[[4-[3-fluor-5-(trifluormethyl)-2-pyridyl]phenyl]methyl]-2-oxo-3,4-dihydro-1-benzazepin-8-yl]-1,3,4-oxadiazol-2-yl]-2-methylpropannitril;
2-Methyl-2-[5-[(3S)-3-amino-5,5,7-trifluor-2-oxo-1-[[4-[5-(trifluormethyl)tetrazol-2-yl]phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethyl)tetrazol-2-yl]phenyl]methyl]-3,4-dihydro-1-benzazepin-8-yl]-1,3,4-oxadiazol-2-yl]propannitril;
(3*S*)-3-Amino-5,5,7-trifluor-8-(5-pyrrolidin-1-yl-1,3,4-oxadiazol-2-yl)-1-[[4-[3-(trifluormethyl)-1,2,4-triazol-1-yl]phenyl]methyl]-3,4-dihydro-1-benzazepin-2-on;
(3R)-3-Amino-5,5,7-trifluor-8-(5-pyrrolidin-1-yl-1,3,4-oxadiazol-2-yl)-1-[[4-[3-(trifluormethyl)-1,2,4-triazol-1-yl]phenyl]methyl]-3,4-dihydro-1-benzazepin-2-on;
(3*S*)-3-Amino-8-[5-(3,3-difluorpyrrolidin-1-yl)-1,3,4-oxadiazol-2-yl]-5,5,7-trifluor-1-[[4-[3-(trifluormethyl)-1,2,4-triazol-1-yl]phenyl]methyl]-3,4-dihydro-1-benzazepin-2-on;
(3*R*)-3-Amino-8-[5-(3,3-difluorpyrrolidin-1-yl)-1,3,4-oxadiazol-2-yl]-5,5,7-trifluor-1-[[4-[3-(trifluormethyl)-1,2,4-triazol-1-yl]phenyl]methyl]-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[[4-[3-(trifluormethyl)-1,2,4-triazol-1-yl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[3-(trifluormethyl)-1,2,4-triazol-1-yl]phenyl]methyl]-3,4-dihydro-1-benzazepin-2-on;
2-Methyl-2-[5-[(3S)-3-amino-5,5,7-trifluor-2-oxo-1-[[4-[5-(trifluormethoxy)-2-pyridyl]-phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethoxy)-2-pyridyl]phenyl]methyl]-3,4-dihydro-1-benzazepin-8-yl]-1,3,4-oxadiazol-2-yl]propannitril;
(3S)-3-Amino-5,5,7-trifluor-8-[5-(1-methyl-1-methylsulfonylethyl)-1,3,4-oxadiazol-2-yl]-1-[[4-[5-(trifluormethoxy)-2-pyridyl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[5-(trifluormethoxy)-2-pyridyl]phenyl]methyl]-3,4-dihydro-1-benzazepin-2-on;
2-[5-[(3S)-3-Amino-5,5,7-trifluor-2-oxo-1-[[4-[4-(trifluormethyl)-2-pyridyl]phenyl]methyl]-3,4-dihydro-1-benzazepin-8-yl]-1,3,4-oxadiazol-2-yl]-2-methylpropannitril;
2-[5-[(3R)-3-Amino-5,5,7-trifluor-2-oxo-1-[[4-[4-(trifluormethyl)-2-pyridyl]phenyl]methyl]-3,4-dihydro-1-benzazepin-8-yl]-1,3,4-oxadiazol-2-yl]-2-methylpropannitril;
2-[5-[(3S)-3-Amino-5,5,7-trifluor-2-oxo-1-[[6-[4-(trifluormethyl)phenyl]-3-pyridyl]methyl]-3,4-dihydro-1-benzazepin-8-yl]-1,3,4-oxadiazol-2-yl]-2-methylpropannitril;
2-[5-[(3R)-3-Amino-5,5,7-trifluor-2-oxo-1-[[6-[4-(trifluormethyl)phenyl]-3-pyridyl]methyl]-3,4-dihydro-1-benzazepin-8-yl]-1,3,4-oxadiazol-2-yl]-2-methylpropannitril;
(3S)-3-Amino-5,5,7-trifluor-8-[5-(1-methyl-1-methylsulfonylethyl)-1,3,4-oxadiazol-2-yl]-1-[[4-[4-(trifluormethyl)pyrazol-1-yl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[4-(trifluormethyl)pyrazol-1-yl]phenyl]methyl]-3,4-dihydro-1-benzazepin-2-on;
2-[5-[(3S)-3-Amino-5,5,7-trifluor-2-oxo-1-[[4-[3-(trifluormethyl)pyrazol-1-yl]phenyl]methyl]-3,4-dihydro-1-benzazepin-8-yl]-1,3,4-oxadiazol-2-yl]-2-methylpropannitril;
2-[5-[(3R)-3-Amino-5,5,7-trifluor-2-oxo-1-[[4-[3-(trifluormethyl)pyrazol-1-yl]phenyl]methyl]-3,4-dihydro-1-benzazepin-8-yl]-1,3,4-oxadiazol-2-yl]-2-methylpropannitril;
(3S)-3-Amino-5,5,7-trifluor-8-[5-(1-methyl-1-methylsulfonylethyl)-1,3,4-oxadiazol-2-yl]-1-[[4-[4-(trifluormethyl)phenyl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[4-(trifluormethyl)phenyl]phenyl]methyl]-3,4-dihydro-1-benzazepin-2-on;
2-[5-[(3S)-3-Amino-5,5,7-trifluor-2-oxo-1-[[4-[4-(trifluormethyl)pyrazol-1-yl]phenyl]methyl]-3,4-dihydro-1-benzazepin-8-yl]-1,3,4-oxadiazol-2-yl]-2-methylpropannitril;
2-[5-[(3R)-3-Amino-5,5,7-trifluor-2-oxo-1-[[4-[4-(trifluormethyl)pyrazol-1-yl]phenyl]methyl]-3,4-dihydro-1-benzazepin-8-yl]-1,3,4-oxadiazol-2-yl]-2-methylpropannitril;
(3S)-3-Amino-8-[5-[5,5-difluor-1-(2-methoxyethyl)-3-piperidyl]-1,3,4-oxadiazol-2-yl]-5,5,7-trifluor-1-[[4-[5-(trifluormethyl)-2-pyridyl]phenyl]methyl]-3,4-dihydro-1-benzazepin-2-on;
(3R)-3-Amino-8-[5-[5,5-difluor-1-(2-methoxyethyl)-3-piperidyl]-1,3,4-oxadiazol-2-yl]-5,5,7-trifluor-1-[[4-[5-(trifluormethyl)-2-pyridyl]phenyl]methyl]-3,4-dihydro-1-benzazepin-2-on;
Methyl-1-[5-[(3S)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]-3-azabicyclo[3.1.1]heptan-3-carboxylat;
Methyl-1-[5-[(3R)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]-3-azabicyclo[3.1.1]heptan-3-carboxylat;
Methyl-1-[5-[(3S)-3-amino-1-[(4-chlorphenyl)methyl]-5,5,7-trifluor-2-oxo-3,4-dihydro-1-benzazepin-8-yl]-1,3,4-oxadiazol-2-yl]-3-azabicyclo[3 .1.1]heptan-3-carboxylat;
Methyl-1-[5-[(3R)-3-amino-1-[(4-chlorphenyl)methyl]-5,5,7-trifluor-2-oxo-3,4-dihydro-1-benzazepin-8-yl]-1,3,4-oxadiazol-2-yl]-3-azabicyclo[3.1.1]heptan-3-carboxylat;
2-[5-[(3S)-3-Amino-1-[[4-[5-(1,1-difluorethyl)-2-pyridyl]phenyl]methyl]-5,5,7-trifluor-2-oxo-3,4-dihydro-1-benzazepin-8-yl]-1,3,4-oxadiazol-2-yl]-2-methylpropannitril;
2-[5-[(3R)-3-Amino-1-[[4-[5-(1,1-difluorethyl)-2-pyridyl]phenyl]methyl]-5,5,7-trifluor-2-oxo-3,4-dihydro-1-benzazepin-8-yl]-1,3,4-oxadiazol-2-yl]-2-methylpropannitril;
2-Methyl-2-[5-[(3S)-3-amino-5,5,7-trifluor-1-[[4-(5-methoxy-2-pyridyl)phenyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[4-(5-methoxy-2-pyridyl)phenyl]methyl]-2-oxo-3,4-dihydro-1-benzazepin-8-yl]-1,3,4-oxadiazol-2-yl]propannitril;
(3S)-3-Amino-5,5,7-trifluor-1-[[4-(5-methoxy-2-pyridyl)phenyl]methyl]-8-[5-(1-methyl-1-methylsulfonylethyl)-1,3,4-oxadiazol-2-yl]-3,4-dihydro-1-benzazepin-2-on;
(3R)-3-Amino-5,5,7-trifluor-1-[[4-(5-methoxy-2-pyridyl)phenyl]methyl]-8-[5-(1-methyl-1-methylsulfonylethyl)-1,3,4-oxadiazol-2-yl]-3,4-dihydro-1-benzazepin-2-on;
2-Methyl-2-[5-[(3S)-3-amino-1-[[4-[5-(difluormethyl)-2-pyridyl]phenyl]methyl]-5,5,7-trifluor-2-oxo-3,4-dihydro-1-benzazepin-8-yl]-1,3,4-oxadiazol-2-yl]propannitril;
2-Methyl-2-[5-[(3R)-3-amino-1-[[4-[5-(difluormethyl)-2-pyridyl]phenyl]methyl]-5,5,7-trifluor-2-oxo-3,4-dihydro-1-benzazepin-8-yl]-1,3,4-oxadiazol-2-yl]propannitril;
2-Methyl-2-[5-[(3S)-3-amino-1-[[4-[5-(difluormethoxy)-2-pyridyl]phenyl]methyl]-5,5,7-trifluor-2-oxo-3,4-dihydro-1-benzazepin-8-yl]-1,3,4-oxadiazol-2-yl]propannitril;
2-Methyl-2-[5-[(3R)-3-amino-1-[[4-[5-(difluormethoxy)-2-pyridyl]phenyl]methyl]-5,5,7-trifluor-2-oxo-3,4-dihydro-1-benzazepin-8-yl]-1,3,4-oxadiazol-2-yl]propannitril;
Methyl-1-[5-[(3S)-3-amino-5,5,7-trifluor-2-oxo-1-[[4-[5-(trifluormethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]heptan-3-carboxylat;
Methyl-1-[5-[(3R)-3-amino-5,5,7-trifluor-2-oxo-1-[[4-[5-(trifluormethyl)-1,2,4-oxadiazol-3-yl]phenyl]methyl]-3,4-dihydro-1-benzazepin-8-yl]-1,3,4-oxadiazol-2-yl]-3-azabicyclo[3.1.1]-heptan-3-carboxylat;
2-Methyl-2-[5-[(3S)-3-amino-5,5,7-trifluor-2-oxo-1-[[4-[4-(trifluormethoxy)pyrazol-1-yl]phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[4-(trifluormethoxy)pyrazol-1-yl]phenyl]methyl]-3,4-dihydro-1-benzazepin-8-yl]-1,3,4-oxadiazol-2-yl]propannitril;
2-Methyl-2-[5-[(3S)-3-amino-5,5,7-trifluor-1-[[2-fluor-4-[4-(trifluormethyl)pyrazol-1-yl]phenyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[2-fluor-4-[4-(trifluormethyl)pyrazol-1-yl]phenyl]methyl]-2-oxo-3,4-dihydro-1-benzazepin-8-yl]-1,3,4-oxadiazol-2-yl]propannitril;
2-Methyl-2-[5-[(3S)-3-amino-5,5,7-trifluor-1-[[2-fluor-6-[4-(trifluormethyl)pyrazol-1-yl]-3-pyridyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[2-fluor-6-[4-(trifluormethyl)pyrazol-1-yl]-3-pyridyl]methyl]-2-oxo-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[[6-[4-(trifluormethyl)pyrazol-1-yl]-3-pyridyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[6-[4-(trifluormethyl)pyrazol-1-yl]-3-pyridyl]methyl]-3,4-dihydro-1-benzazepin-8-yl]-1,3,4-oxadiazol-2-yl]propannitril;
2-Methyl-2-[5-[(3S)-3-amino-5,5,7-trifluor-1-[[3-fluor-4-[4-(trifluormethyl)pyrazol-1-yl]phenyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[3-fluor-4-[4-(trifluormethyl)pyrazol-1-yl]phenyl]methyl]-2-oxo-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[[4-[1-(trifluormethyl)pyrazol-4-yl]phenyl]methyl]-3,4-dihydro-1-benzazepin-8-yl]-1,3,4-oxadiazol-2-yl]propannitril; und
2-Methyl-2-[5-[(3R)-3-amino-5,5,7-trifluor-2-oxo-1-[[4-[1-(trifluormethyl)pyrazol-4-yl]phenyl]methyl]-3,4-dihydro-1-benzazepin-8-yl]-1,3,4-oxadiazol-2-yl]propannitril;
oder ein pharmazeutisch verträgliches Salz davon.

9. Verbindung nach einem der Ansprüche 1 bis 8, ausgewählt aus:
2-Methyl-2-[5-[(3S)-3-amino-5,5,7-trifluor-2-oxo-1-[[4-(trifluormethoxy)phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-(trifluormethoxy)phenyl]methyl]-3,4-dihydro-1-benzazepin-8-yl]-1,3,4-oxadiazol-2-yl]propannitril;
(3S)-3-Amino-5,5,7-trifluor-8-[5-(1,2,2,2-tetrafluor-1-methoxyethyl)-1,3,4-oxadiazol-2-yl]-1-[[4-(trifluormethoxy)phenyl]methyl]-3,4-dihydro-1-benzazepin-2-on;
(3R)-3-Amino-5,5,7-trifluor-8-[5-(1,2,2,2-tetrafluor-1-methoxyethyl)-1,3,4-oxadiazol-2-yl]-1-[[4-(trifluormethoxy)phenyl]methyl]-3,4-dihydro-1-benzazepin-2-on;
(3S)-3-Amino-8-(5-tert-butyl-1,3,4-oxadiazol-2-yl)-1-[[4-(cyclopentoxy)phenyl]methyl]-5,5,7-trifluor-3,4-dihydro-1-benzazepin-2-on;
(3R)-3-Amino-8-(5-tert-butyl-1,3,4-oxadiazol-2-yl)-1-[[4-(cyclopentoxy)phenyl]methyl]-5,5,7-trifluor-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[[4-[5-(trifluormethyl)-2-pyridyl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[5-(trifluormethyl)-2-pyridyl]phenyl]methyl]-3,4-dihydro-1-benzazepin-2-on;
2-Methyl-2-[5-[(3S)-3-amino-5,5,7-trifluor-1-[[4-(4-methoxyphenyl)phenyl]methyl]-2-oxo-3,4-dihydro-1-benzazepin-8-yl]-1,3,4-oxadiazol-2-yl]propannitril;
2-Methyl-2-[5-[(3R)-3-amino-5,5,7-trifluor-1-[[4-(4-methoxyphenyl)phenyl]methyl]-2-oxo-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[(4-phenoxyphenyl)methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[(4-phenoxyphenyl)methyl]-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]propannitril;
(3S)-3-Amino-8-[5-(1-ethyl-5,5-difluor-3-piperidyl)-1,3,4-oxadiazol-2-yl]-5,5,7-trifluor-1-[[4-(trifluormethoxy)phenyl]methyl]-3,4-dihydro-1-benzazepin-2-on;
(3R)-3-Amino-8-[5-(1-ethyl-5,5-difluor-3-piperidyl)-1,3,4-oxadiazol-2-yl]-5,5,7-trifluor-1-[[4-(trifluormethoxy)phenyl]methyl]-3,4-dihydro-1-benzazepin-2-on;
(3S)-3-Amino-5,5,7-trifluor-8-(5-pyrrolidin-1-yl-1,3,4-oxadiazol-2-yl)-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-(5-pyrrolidin-1-yl-1,3,4-oxadiazol-2-yl)-1-[[4-[5-(trifluormethyl)-1,2,4-oxadiazol-3-yl]phenyl]methyl]-3,4-dihydro-1-benzazepin-2-on;
(3S)-3-Amino-8-[5-(4,4-difluor-1-piperidyl)-1,3,4-oxadiazol-2-yl]-5,5,7-trifluor-1-[[4-[5-(trifluormethyl)-1,2,4-oxadiazol-3-yl]phenyl]methyl]-3,4-dihydro-1-benzazepin-2-on;
(3R)-3-Amino-8-[5-(4,4-difluor-1-piperidyl)-1,3,4-oxadiazol-2-yl]-5,5,7-trifluor-1-[[4-[5-(trifluormethyl)-1,2,4-oxadiazol-3-yl]phenyl]methyl]-3,4-dihydro-1-benzazepin-2-on;
(3S)-3-Amino-5,5,7-trifluor-8-[5-(1-methyl-1-methylsulfonylethyl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3S)-3-Amino-5,5,7-trifluor-8-[5-(4-oxa-7-azaspiro[2.5]octan-7-yl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
(3R)-3-Amino-5,5,7-trifluor-8-[5-(4-oxa-7-azaspiro[2.5]octan-7-yl)-1,3,4-oxadiazol-2-yl]-1-[[4-[5-(trifluormethyl)-1,2,4-oxadiazol-3-yl]phenyl]methyl]-3,4-dihydro-1-benzazepin-2-on;
2-Methyl-2-[5-[(3S)-3-amino-5,5,7-trifluor-2-oxo-1-[[4-[5-(trifluormethyl)tetrazol-2-yl]phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethyl)tetrazol-2-yl]phenyl]methyl]-3,4-dihydro-1-benzazepin-8-yl]-1,3,4-oxadiazol-2-yl]propannitril;
2-Methyl-2-[5-[(3S)-3-amino-5,5,7-trifluor-2-oxo-1-[[4-[5-(trifluormethoxy)-2-pyridyl]-phenyl]methyl]-3,4-dihydro-1-benzazepin-8-yl]-1,3,4-oxadiazol-2-yl]propannitril;
2-Methyl-2-[5-[(3R)-3-amino-5,5,7-trifluor-2-oxo-1-[[4-[5-(trifluormethoxy)-2-pyridyl]-phenyl]methyl]-3,4-dihydro-1-benzazepin-8-yl]-1,3,4-oxadiazol-2-yl]propannitril;
(3S)-3-Amino-5,5,7-trifluor-8-[5-(1-methyl-1-methylsulfonylethyl)-1,3,4-oxadiazol-2-yl]-1-[[4-[4-(trifluormethyl)pyrazol-1-yl]phenyl]methyl]-3,4-dihydro-1-benzazepin-2-on;
(3R)-3-Amino-5,5,7-trifluor-8-[5-(1-methyl-1-methylsulfonylethyl)-1,3,4-oxadiazol-2-yl]-1-[[4-[4-(trifluormethyl)pyrazol-1-yl]phenyl]methyl]-3,4-dihydro-1-benzazepin-2-on;
2-[5-[(3S)-3-Amino-5,5,7-trifluor-2-oxo-1-[[4-[4-(trifluormethyl)pyrazol-1-yl]phenyl]methyl]-3,4-dihydro-1-benzazepin-8-yl]-1,3,4-oxadiazol-2-yl]-2-methylpropannitril;
2-[5-[(3R)-3-Amino-5,5,7-trifluor-2-oxo-1-[[4-[4-(trifluormethyl)pyrazol-1-yl]phenyl]-methyl]-3,4-dihydro-1-benzazepin-8-yl]-1,3,4-oxadiazol-2-yl]-2-methylpropannitril;
Methyl-1-[5-[(3S)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]-3-azabicyclo[3.1.1]heptan-3-carboxylat;
Methyl-1-[5-[(3R)-3-amino-5,5,7-trifluor-2-oxo-1-[[4-[5-(trifluormethyl)-2-pyridyl]phenyl]methyl]-3,4-dihydro-1-benzazepin-8-yl]-1,3,4-oxadiazol-2-yl]-3-azabicyclo[3.1.1]heptan-3-carboxylat;
Methyl-1-[5-[(3S)-3-amino-1-[(4-chlorphenyl)methyl]-5,5,7-trifluor-2-oxo-3,4-dihydro-1-benzazepin-8-yl]-1,3,4-oxadiazol-2-yl]-3-azabicyclo[3.1.1]heptan-3-carboxylat;
Methyl-1-[5-[(3R)-3-amino-1-[(4-chlorphenyl)methyl]-5,5,7-trifluor-2-oxo-3,4-dihydro-1-benzazepin-8-yl]-1,3,4-oxadiazol-2-yl]-3-azabicyclo[3.1.1]heptan-3-carboxylat;
2-[5-[(3S)-3-Amino-1-[[4-[5-(1,1-difluorethyl)-2-pyridyl]phenyl]methyl]-5,5,7-trifluor-2-oxo-3,4-dihydro-1-benzazepin-8-yl]-1,3,4-oxadiazol-2-yl]-2-methylpropannitril;
2-[5-[(3R)-3-Amino-1-[[4-[5-(1,1-difluorethyl)-2-pyridyl]phenyl]methyl]-5,5,7-trifluor-2-oxo-3,4-dihydro-1-benzazepin-8-yl]-1,3,4-oxadiazol-2-yl]-2-methylpropannitril;
2-Methyl-2-[5-[(3S)-3-amino-1-[[4-[5-(difluormethoxy)-2-pyridyl]phenyl]methyl]-5,5,7-trifluor-2-oxo-3,4-dihydro-1-benzazepin-8-yl]-1,3,4-oxadiazol-2-yl]propannitril; und
2-Methyl-2-[5-[(3R)-3-amino-1-[[4-[5-(difluormethoxy)-2-pyridyl]phenyl]methyl]-5,5,7-trifluor-2-oxo-3,4-dihydro-1-benzazepin-8-yl]-1,3,4-oxadiazol-2-yl]propannitril;
oder ein pharmazeutisch verträgliches Salz davon.

10. Verbindung nach einem der Ansprüche 1 bis 9 oder ein pharmazeutisch verträgliches Salz davon zur Verwendung als therapeutisch wirksame Substanz.

11. Pharmazeutische Zusammensetzung, umfassend eine Verbindung nach einem der Ansprüche 1 bis 9 oder ein pharmazeutisch verträgliches Salz davon und einen pharmazeutisch verträglichen Hilfsstoff.

12. Pharmazeutische Zusammensetzung nach Anspruch 11, ferner umfassend ein zusätzliches Therapeutikum.

13. Verbindung nach einem der Ansprüche 1 bis 9 oder ein pharmazeutisch verträgliches Salz davon zur Verwendung bei der Behandlung, Prävention und/oder Verzögerung des Fortschreitens von Krebs.

14. Verbindung zur Verwendung nach Anspruch 13, wobei der Krebs mit anormaler Diacylglycerinkinase-Signalübertragung assoziiert ist, wobei die Diacylglycerinkinase ausgewählt ist aus DGKα und/oder DGKζ.

15. Verbindung zur Verwendung nach Anspruch 13 oder 14, wobei der Krebs ausgewählt ist aus der Gruppe, bestehend aus akuter lymphatischer B-Zell-Leukämie, akuter lymphatischer T-Zell-Leukämie, akuter lymphatischer Leukämie, chronischer myeloischer Leukämie, chronischer lymphatischer Leukämie, prolymphatischer B-Zell-Leukämie, blastischem plasmazytoidem dendritischem Zell-Neoplasma, Burkitt-Lymphom, diffusem großzelligem B-Zell-Lymphom, follikulärem Lymphom, Haarzell-Leukämie, kleinzelligem oder großzelligem follikulärem Lymphom, malignen lymphoproliferativen Zuständen, MALT-Lymphom, Mantelzell-Lymphom, Marginalzonen-Lymphom, multiplem Myelom, Myelodysplasie und myelodysplastischem Syndrom, Non-Hodgkin-Lymphom, plasmablastischem Lymphom, plasmazytoidem dendritischem Zell-Neoplasma, Waldenström-Makroglobulinämie, Präleukämie, Sarkom, Karzinom, Melanom, Neuroblastom, Nierenzellkarzinom, Kolonkrebs, Kolorektalkrebs, Brustkrebs, epithelialem Plattenepithelkarzinom, Melanom, Magenkrebs, Gehirnkrebs, Lungenkrebs (z. B. NSCLC), Bauchspeicheldrüsenkrebs, Gebärmutterhalskrebs, Eierstockkrebs, Leberkrebs, Blasenkrebs, Prostatakrebs, Hodenkrebs, Schilddrüsenkrebs, Gebärmutterkrebs, Nebennierenkrebs und Kopf- und Halskrebs.

## Revendications

1. Composé de formule (I) 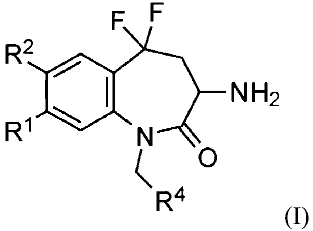 ou un sel pharmaceutiquement acceptable de celui-ci, dans lequel :
R¹ représente un hétéroaryle à 5 ou 6 chaînons, dans lequel R¹ est éventuellement substitué par un ou plusieurs R¹⁰ qui peuvent être identiques ou différents ;
R² est choisi parmi un hydrogène et un halogène ;
R⁴ est choisi parmi un phényle et un pyridinyle, dans lequel R⁴ est éventuellement substitué par un ou plusieurs R¹¹ qui peuvent être identiques ou différents ;
R¹⁰ est choisi parmi :
i) un alkyle en C₁₋₆, éventuellement substitué par un ou plusieurs halogène, amino, alcoxy en C₁₋₆, -S(O)₂(alkyle en C₁₋₆), cyano ;
ii) un cycloalkyle en C₃₋₁₀, éventuellement substitué par un ou plusieurs halogène, cyano, amino ;
iii) un hétérocyclyle à 3 à 10 chaînons, éventuellement substitué par un ou plusieurs halogène, alkyle en C₁₋₁₀, amino, halogénoalkyle en C₁₋₆, hydroxy, cyano, -C(O)O-(R^{10q}), cycloalkyle en C₃₋₁₀, dans lequel l'alkyle en C₁₋₁₀ est éventuellement substitué par un ou plusieurs hydroxy, alcoxy en C₁₋₆ ;
iv) -N(R^{10e}R^{10f}) ;
v) un hétéroaryle, éventuellement substitué par un ou plusieurs alkyle en C₁₋₁₀, halogène ;
R^{10e} et R^{10f} sont chacun indépendamment choisis parmi :
i) un hydrogène ;
ii) un alkyle en C₁₋₆, éventuellement substitué par un ou plusieurs cyano, halogène, hydroxy ;
iii) un cycloalkyle en C₃₋₁₀, éventuellement substitué par un ou plusieurs halogène, alkyle en C₁₋₁₀ ;
R^{10q} représente un alkyle en C₁₋₅, dans lequel l'alkyle en C₁₋₅ est éventuellement substitué par un ou plusieurs hydroxy ;
R¹¹ est choisi parmi :
i) un halogène ;
ii) un alcoxy en C₁₋₆, éventuellement substitué par un ou plusieurs alkyle en C₁₋₆, aryle en C₅₋₆, cycloalkyle en C₃₋₁₀, halogénoalkyle en C₁₋₆, hétérocyclyle en C₃₋₁₀, dans lequel l'hétérocyclyle à 3 à 10 chaînons est éventuellement substitué par un alkyle en C₁₋₆ ;
iii) un hétéroaryle à 5 à 6 chaînons, éventuellement substitué par un ou plusieurs halogène, halogénoalkyle en C₁₋₆, alcoxy en C₁₋₆, cycloalkyle en C₃₋₁₀, alkyle en C₁₋₆, halogénoalcoxy en C₁₋₆ ;
iv) un phényle, éventuellement substitué par un ou plusieurs alcoxy en C₁₋₆, halogénoalkyle en C₁₋₆ ;
v) -O(R^{11a}) ;
R^{11a} est choisi parmi un alkyle en C₁₋₆, un cycloalkyle en C₃₋₆, un phényle et un halogénoalkyle en C₁₋₆.

2. Composé selon la revendication 1, ou un sel pharmaceutiquement acceptable de celui-ci, dans lequel R¹ représente un hétéroaryle à 5 chaînons, dans lequel R¹ est éventuellement substitué par un ou plusieurs R¹⁰ qui peuvent être identiques ou différents.

3. Composé selon la revendication 1 ou 2, ou un sel pharmaceutiquement acceptable de celui-ci, dans lequel R¹ représente un 1,3,4-oxadiazole, substitué par un R¹⁰.

4. Composé selon l'une quelconque des revendications 1 à 3, ou un sel pharmaceutiquement acceptable de celui-ci, dans lequel R² représente un fluor.

5. Composé selon l'une quelconque des revendications 1 à 4, ou un sel pharmaceutiquement acceptable de celui-ci, dans lequel R⁴ représente un phényle.

6. Composé selon l'une quelconque des revendications 1 à 5, ou un sel pharmaceutiquement acceptable de celui-ci, dans lequel R¹⁰ est choisi parmi un méthyl-méthylsulfonyl-éthyle, une pyrrolidine, un 4-oxa-7-azaspiro[2.5]octan-7-yle, un méthyl-propanenitrile, un 1,2,2,2-tétrafluoro-méthoxy-éthyle, un tertbutyle, un 1-éthyl-5,5-difluoro-3-pipéridyle et un 4,4-difluoro-1-pipéridyle.

7. Composé selon l'une quelconque des revendications 1 à 6, ou un sel pharmaceutiquement acceptable de celui-ci, dans lequel R¹¹ est choisi parmi un 5-(trifluorométhyl)-2-pyridyle, un phénoxy, un trifluorométhoxy, un méthoxy-pyridyle, un cyclopentoxy, un 4-méthoxyphényle, un (trifluorométhyl)-1,2,4-oxadiazol-3-yle, un 5-(trifluorométhoxy)-2-pyridyle, un 4-(trifluorométhyl)pyrazol-1-yle, un chlore, un 5-(difluorométhoxy)-2-pyridyle, un 4-[4-(trifluorométhoxy)pyrazol-1-yle, un 5-(trifluorométhyl)tétrazol-2-yle.

8. Composé selon l'une quelconque des revendications 1 à 7, choisi parmi :
la 3-amino-8-(5-tert-butyl-1,3,4-oxadiazol-2-yl)-5,5,7-trifluoro-1-[[4-(trifluorométhoxy)phényl]méthyl]-3,4-dihydro-1-benzazépin-2-one ;
le 2-méthyl-2-[5-[(3S)-3-amino-5,5,7-trifluoro-2-oxo-1-[[4-(trifluorométhoxy)phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-(trifluorométhoxy)phényl]méthyl]-3,4-dihydro-1-benzazépin-8-yl]-1,3,4-oxadiazol-2-yl]propanenitrile ;
la (3S)-3-amino-5,5,7-trifluoro-8-(5-morpholino-1,3,4-oxadiazol-2-yl)-1-[[4-(trifluorométhoxy)phényl]méthyl]-3,4-dihydro-1-benzazépin-2-one ;
la (3R)-3-amino-5,5,7-trifluoro-8-(5-morpholino-1,3,4-oxadiazol-2-yl)-1-[[4-(trifluorométhoxy)phényl]méthyl]-3,4-dihydro-1-benzazépin-2-one ;
la (3S)-3-amino-5,5,7-trifluoro-8-[5-(1,2,2,2-tétrafluoro-1-méthoxy-éthyl)-1,3,4-oxadiazol-2-yl]-1-[[4-(trifluorométhoxy)phényl]méthyl]-3,4-dihydro-1-benzazépin-2-one ;
la (3R)-3-amino-5,5,7-trifluoro-8-[5-(1,2,2,2-tétrafluoro-1-méthoxy-éthyl)-1,3,4-oxadiazol-2-yl]-1-[[4-(trifluorométhoxy)phényl]méthyl]-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[(4-phénoxyphényl)méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[(4-phénoxyphényl)méthyl]-3,4-dihydro-1-benzazépin-2-one ;
la (3S)-3-amino-5,5-difluoro-8-[5-(1-méthyl-1-méthylsulfonyl-éthyl)-1,3,4-oxadiazol-2-yl]-1-[(4-phénoxyphényl)méthyl]-3,4-dihydro-1-benzazépin-2-one ;
la (3R)-3-amino-5,5-difluoro-8-[5-(1-méthyl-1-méthylsulfonyl-éthyl)-1,3,4-oxadiazol-2-yl]-1-[(4-phénoxyphényl)méthyl]-3,4-dihydro-1-benzazépin-2-one ;
la (3S)-3-amino-8-(5-tert-butyl-1,3,4-oxadiazol-2-yl)-1-[[4-(cyclopentoxy)phényl]méthyl]-5,5,7-trifluoro-3,4-dihydro-1-benzazépin-2-one ;
la (3R)-3-amino-8-(5-tert-butyl-1,3,4-oxadiazol-2-yl)-1-[[4-(cyclopentoxy)phényl]méthyl]-5,5,7-trifluoro-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[[4-[5-(trifluorométhyl)-2-pyridyl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[5-(trifluorométhyl)-2-pyridyl]phényl]méthyl]-3,4-dihydro-1-benzazépin-2-one ;
le 2-méthyl-2-[5-[(3S)-3-amino-5,5,7-trifluoro-1-[[4-(4-méthoxyphényl)phényl]méthyl]-2-oxo-3,4-dihydro-1-benzazépin-8-yl]-1,3,4-oxadiazol-2-yl]propanenitrile ;
le 2-méthyl-2-[5-[(3R)-3-amino-5,5,7-trifluoro-1-[[4-(4-méthoxyphényl)phényl]méthyl]-2-oxo-3,4-dihydro-1-benzazépin-8-yl]-1,3,4-oxadiazol-2-yl]propanenitrile ;
la (3S)-3-amino-5,5,7-trifluoro-1-[[4-(4-méthoxyphényl)phényl]méthyl]-8-[5-(1-méthyl-1-méthylsulfonyl-éthyl)-1,3,4-oxadiazol-2-yl]-3,4-dihydro-1-benzazépin-2-one ;
la (3R)-3-amino-5,5,7-trifluoro-1-[[4-(4-méthoxyphényl)phényl]méthyl]-8-[5-(1-méthyl-1-méthylsulfonyl-éthyl)-1,3,4-oxadiazol-2-yl]-3,4-dihydro-1-benzazépin-2-one ;
le 2-méthyl-2-[5-[(3S)-3-amino-5,5,7-trifluoro-2-oxo-1-[(4-phénoxyphényl)méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[(4-phénoxyphényl)méthyl]-3,4-dihydro-1-benzazépin-8-yl]-1,3,4-oxadiazol-2-yl]propanenitrile ;
le 2-méthyl-2-[5-[(3S)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]propanenitrile ;
la (3S)-3-amino-8-[5-(1-éthyl-5,5-difluoro-3-pipéridyl)-1,3,4-oxadiazol-2-yl]-5,5,7-trifluoro-1-[[4-(trifluorométhoxy)phényl]méthyl]-3,4-dihydro-1-benzazépin-2-one ;
la (3R)-3-amino-8-[5-(1-éthyl-5,5-difluoro-3-pipéridyl)-1,3,4-oxadiazol-2-yl]-5,5,7-trifluoro-1-[[4-(trifluorométhoxy)phényl]méthyl]-3,4-dihydro-1-benzazépin-2-one ;
la (3S)-3-amino-5,5,7-trifluoro-8-(5-pyrrolidin-1-yl-1,3,4-oxadiazol-2-yl)-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-(5-pyrrolidin-1-yl-1,3,4-oxadiazol-2-yl)-1-[[4-[5-(trifluorométhyl)-1,2,4-oxadiazol-3-yl]phényl]méthyl]-3,4-dihydro-1-benzazépin-2-one ;
la (3S)-3-amino-8-[5-(4,4-difluoro-1-pipéridyl)-1,3,4-oxadiazol-2-yl]-5,5,7-trifluoro-1-[[4-[5-(trifluorométhyl)-1,2,4-oxadiazol-3-yl]phényl]méthyl]-3,4-dihydro-1-benzazépin-2-one ;
la (3R)-3-amino-8-[5-(4,4-difluoro-1-pipéridyl)-1,3,4-oxadiazol-2-yl]-5,5,7-trifluoro-1-[[4-[5-(trifluorométhyl)-1,2,4-oxadiazol-3-yl]phényl]méthyl]-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3S)-3-amino-5,5,7-trifluoro-8-[5-(4-oxa-7-azaspiro[2.5]octan-7-yl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-[5-(4-oxa-7-azaspiro[2.5]octan-7-yl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3S)-3-amino-8-(5-tert-butyl-3-pyridyl)-1-[(4-chlorophényl)méthyl]-5,5,7-trifluoro-3,4-dihydro-1-benzazépin-2-one ;
la (3R)-3-amino-8-(5-tert-butyl-3-pyridyl)-1-[(4-chlorophényl)méthyl]-5,5,7-trifluoro-3,4-dihydro-1-benzazépin-2-one ;
le 2-[5-[(3S)-3-amino-5,5,7-trifluoro-1-[[4-[3-fluoro-5-(trifluorométhyl)-2-pyridyl]phényl]méthyl]-2-oxo-3,4-dihydro-1-benzazépin-8-yl]-1,3,4-oxadiazol-2-yl]-2-méthyl-propanenitrile ;
le 2-[5-[(3R)-3-amino-5,5,7-trifluoro-1-[[4-[3-fluoro-5-(trifluorométhyl)-2-pyridyl]phényl]méthyl]-2-oxo-3,4-dihydro-1-benzazépin-8-yl]-1,3,4-oxadiazol-2-yl]-2-méthyl-propanenitrile ;
le 2-méthyl-2-[5-[(3S)-3-amino-5,5,7-trifluoro-2-oxo-1-[[4-[5-(trifluorométhyl)tétrazol-2-yl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhyl)tétrazol-2-yl]phényl]méthyl]-3,4-dihydro-1-benzazépin-8-yl]-1,3,4-oxadiazol-2-yl]propanenitrile ;
la (3*S*)-3-amino-5,5,7-trifluoro-8-(5-pyrrolidin-1-yl-1,3,4-oxadiazol-2-yl)-1-[[4-[3-(trifluorométhyl)-1,2,4-triazol-1-yl]phényl]méthyl]-3,4-dihydro-1-benzazépin-2-one ;
la (3R)-3-amino-5,5,7-trifluoro-8-(5-pyrrolidin-1-yl-1,3,4-oxadiazol-2-yl)-1-[[4-[3-(trifluorométhyl)-1,2,4-triazol-1-yl]phényl]méthyl]-3,4-dihydro-1-benzazépin-2-one ;
la (3*S*)-3-amino-8-[5-(3,3-difluoropyrrolidin-1-yl)-1,3,4-oxadiazol-2-yl]-5,5,7-trifluoro-1-[[4-[3-(trifluorométhyl)-1,2,4-triazol-1-yl]phényl]méthyl]-3,4-dihydro-1-benzazépin-2-one ;
la (3*R*)-3-amino-8-[5-(3,3-difluoropyrrolidin-1-yl)-1,3,4-oxadiazol-2-yl]-5,5,7-trifluoro-1-[[4-[3-(trifluorométhyl)-1,2,4-triazol-1-yl]phényl]méthyl]-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[[4-[3-(trifluorométhyl)-1,2,4-triazol-1-yl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[3-(trifluorométhyl)-1,2,4-triazol-1-yl]phényl]méthyl]-3,4-dihydro-1-benzazépin-2-one ;
le 2-méthyl-2-[5-[(3S)-3-amino-5,5,7-trifluoro-2-oxo-1-[[4-[5-(trifluorométhoxy)-2-pyridyl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhoxy)-2-pyridyl]phényl]méthyl]-3,4-dihydro-1-benzazépin-8-yl]-1,3,4-oxadiazol-2-yl]propanenitrile ;
la (3S)-3-amino-5,5,7-trifluoro-8-[5-(1-méthyl-1-méthylsulfonyl-éthyl)-1,3,4-oxadiazol-2-yl]-1-[[4-[5-(trifluorométhoxy)-2-pyridyl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[5-(trifluorométhoxy)-2-pyridyl]phényl]méthyl]-3,4-dihydro-1-benzazépin-2-one ;
le 2-[5-[(3S)-3-amino-5,5,7-trifluoro-2-oxo-1-[[4-[4-(trifluorométhyl)-2-pyridyl]phényl]méthyl]-3,4-dihydro-1-benzazépin-8-yl]-1,3,4-oxadiazol-2-yl]-2-méthyl-propanenitrile ;
le 2-[5-[(3R)-3-amino-5,5,7-trifluoro-2-oxo-1-[[4-[4-(trifluorométhyl)-2-pyridyl]phényl]méthyl]-3,4-dihydro-1-benzazépin-8-yl]-1,3,4-oxadiazol-2-yl]-2-méthyl-propanenitrile ;
le 2-[5-[(3S)-3-amino-5,5,7-trifluoro-2-oxo-1-[[6-[4-(trifluorométhyl)phényl]-3-pyridyl]méthyl]-3,4-dihydro-1-benzazépin-8-yl]-1,3,4-oxadiazol-2-yl]-2-méthyl-propanenitrile ;
le 2-[5-[(3R)-3-amino-5,5,7-trifluoro-2-oxo-1-[[6-[4-(trifluorométhyl)phényl]-3-pyridyl]méthyl]-3,4-dihydro-1-benzazépin-8-yl]-1,3,4-oxadiazol-2-yl]-2-méthyl-propanenitrile ;
la (3S)-3-amino-5,5,7-trifluoro-8-[5-(1-méthyl-1-méthylsulfonyl-éthyl)-1,3,4-oxadiazol-2-yl]-1-[[4-[4-(trifluorométhyl)pyrazol-1-yl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[4-(trifluorométhyl)pyrazol-1-yl]phényl]méthyl]-3,4-dihydro-1-benzazépin-2-one ;
le 2-[5-[(3S)-3-amino-5,5,7-trifluoro-2-oxo-1-[[4-[3-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
le 2-[5-[(3R)-3-amino-5,5,7-trifluoro-2-oxo-1-[[4-[3-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
la (3S)-3-amino-5,5,7-trifluoro-8-[5-(1-méthyl-1-méthylsulfonyl-éthyl)-1,3,4-oxadiazol-2-yl]-1-[[4-[4-(trifluorométhyl)phényl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[4-(trifluorométhyl)phényl]phényl]méthyl]-3,4-dihydro-1-benzazépin-2-one ;
le 2-[5-[(3S)-3-amino-5,5,7-trifluoro-2-oxo-1-[[4-[4-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
le 2-[5-[(3R)-3-amino-5,5,7-trifluoro-2-oxo-1-[[4-[4-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
la (3S)-3-amino-8-[5-[5,5-difluoro-1-(2-méthoxyéthyl)-3-pipéridyl]-1,3,4-oxadiazol-2-yl]-5,5,7-trifluoro-1-[[4-[5-(trifluorométhyl)-2-pyridyl]phényl]méthyl]-3,4-dihydro-1-benzazépin-2-one ;
la (3R)-3-amino-8-[5-[5,5-difluoro-1-(2-méthoxyéthyl)-3-pipéridyl]-1,3,4-oxadiazol-2-yl]-5,5,7-trifluoro-1-[[4-[5-(trifluorométhyl)-2-pyridyl]phényl]méthyl]-3,4-dihydro-1-benzazépin-2-one ;
le 1-[5-[(3S)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 1-[5-[(3R)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 1-[5-[(3S)-3-amino-1-[(4-chlorophényl)méthyl]-5,5,7-trifluoro-2-oxo-3,4-dihydro-1-benzazépin-8-yl]-1,3,4-oxadiazol-2-yl]-3-azabicyclo[3.1.1]heptane-3-carboxylate de méthyle ;
le 1-[5-[(3R)-3-amino-1-[(4-chlorophényl)méthyl]-5,5,7-trifluoro-2-oxo-3,4-dihydro-1-benzazépin-8-yl]-1,3,4-oxadiazol-2-yl]-3-azabicyclo[3.1.1]heptane-3-carboxylate de méthyle ;
le 2-[5-[(3S)-3-amino-1-[[4-[5-(1,1-difluoroéthyl)-2-pyridyl]phényl]méthyl]-5,5,7-trifluoro-2-oxo-3,4-dihydro-1-benzazépin-8-yl]-1,3,4-oxadiazol-2-yl]-2-méthyl-propanenitrile ;
le 2-[5-[(3R)-3-amino-1-[[4-[5-(1,1-difluoroéthyl)-2-pyridyl]phényl]méthyl]-5,5,7-trifluoro-2-oxo-3,4-dihydro-1-benzazépin-8-yl]-1,3,4-oxadiazol-2-yl]-2-méthyl-propanenitrile ;
le 2-méthyl-2-[5-[(3S)-3-amino-5,5,7-trifluoro-1-[[4-(5-méthoxy-2-pyridyl)phényl]méthyl]-2-oxo-3,4-dihydro-1-benzazépin-8-yl]-1,3,4-oxadiazol-2-yl]propanenitrile ;
le 2-méthyl-2-[5-[(3R)-3-amino-5,5,7-trifluoro-1-[[4-(5-méthoxy-2-pyridyl)phényl]méthyl]-2-oxo-3,4-dihydro-1-benzazépin-8-yl]-1,3,4-oxadiazol-2-yl]propanenitrile ;
la (3S)-3-amino-5,5,7-trifluoro-1-[[4-(5-méthoxy-2-pyridyl)phényl]méthyl]-8-[5-(1-méthyl-1-méthylsulfonyl-éthyl)-1,3,4-oxadiazol-2-yl]-3,4-dihydro-1-benzazépin-2-one ;
la (3R)-3-amino-5,5,7-trifluoro-1-[[4-(5-méthoxy-2-pyridyl)phényl]méthyl]-8-[5-(1-méthyl-1-méthylsulfonyl-éthyl)-1,3,4-oxadiazol-2-yl]-3,4-dihydro-1-benzazépin-2-one ;
le 2-méthyl-2-[5-[(3S)-3-amino-1-[[4-[5-(difluorométhyl)-2-pyridyl]phényl]méthyl]-5,5,7-trifluoro-2-oxo-3,4-dihydro-1-benzazépin-8-yl]-1,3,4-oxadiazol-2-yl]propanenitrile ;
le 2-méthyl-2-[5-[(3R)-3-amino-1-[[4-[5-(difluorométhyl)-2-pyridyl]phényl]méthyl]-5,5,7-trifluoro-2-oxo-3,4-dihydro-1-benzazépin-8-yl]-1,3,4-oxadiazol-2-yl]propanenitrile ;
le 2-méthyl-2-[5-[(3S)-3-amino-1-[[4-[5-(difluorométhoxy)-2-pyridyl]phényl]méthyl]-5,5,7-trifluoro-2-oxo-3,4-dihydro-1-benzazépin-8-yl]-1,3,4-oxadiazol-2-yl]propanenitrile ;
le 2-méthyl-2-[5-[(3R)-3-amino-1-[[4-[5-(difluorométhoxy)-2-pyridyl]phényl]méthyl]-5,5,7-trifluoro-2-oxo-3,4-dihydro-1-benzazépin-8-yl]-1,3,4-oxadiazol-2-yl]propanenitrile ;
le 1-[5-[(3S)-3-amino-5,5,7-trifluoro-2-oxo-1-[[4-[5-(trifluorométhyl)-1,2,4-oxadiazol-3-yl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 1-[5-[(3R)-3-amino-5,5,7-trifluoro-2-oxo-1-[[4-[5-(trifluorométhyl)-1,2,4-oxadiazol-3-yl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 2-méthyl-2-[5-[(3*S*)-3-amino-5,5,7-trifluoro-2-oxo-1-[[4-[4-(trifluorométhoxy)pyrazol-1-yl]phényl]méthyl]-3,4-dihydro-1-benzazépin-8-yl]-1,3,4-oxadiazol-2-yl]propanenitrile ;
le 2-méthyl-2-[5-[(3*R*)-3-amino-5,5,7-trifluoro-2-oxo-1-[[4-[4-(trifluorométhoxy)pyrazol-1-yl]phényl]méthyl]-3,4-dihydro-1-benzazépin-8-yl]-1,3,4-oxadiazol-2-yl]propanenitrile ;
le 2-méthyl-2-[5-[(3*S*)-3-amino-5,5,7-trifluoro-1-[[2-fluoro-4-[4-(trifluorométhyl)pyrazol-1-yl]phényl]méthyl]-2-oxo-3,4-dihydro-1-benzazépin-8-yl]-1,3,4-oxadiazol-2-yl]propanenitrile ;
le 2-méthyl-2-[5-[(3*R*)-3-amino-5,5,7-trifluoro-1-[[2-fluoro-4-[4-(trifluorométhyl)pyrazol-1-yl]phényl]méthyl]-2-oxo-3,4-dihydro-1-benzazépin-8-yl]-1,3,4-oxadiazol-2-yl]propanenitrile ;
le 2-méthyl-2-[5-[(3*S*)-3-amino-5,5,7-trifluoro-1-[[2-fluoro-6-[4-(trifluorométhyl)pyrazol-1-yl]-3-pyridyl]méthyl]-2-oxo-3,4-dihydro-1-benzazépin-8-yl]-1,3,4-oxadiazol-2-yl]propanenitrile ;
le 2-méthyl-2-[5-[(3*R*)-3-amino-5,5,7-trifluoro-1-[[2-fluoro-6-[4-(trifluorométhyl)pyrazol-1-yl]-3-pyridyl]méthyl]-2-oxo-3,4-dihydro-1-benzazépin-8-yl]-1,3,4-oxadiazol-2-yl]propanenitrile ;
le 2-méthyl-2-[5-[(3*S*)-3-amino-5,5,7-trifluoro-2-oxo-1-[[6-[4-(trifluorométhyl)pyrazol-1-yl]-3-pyridyl]méthyl]-3,4-dihydro-1-benzazépin-8-yl]-1,3,4-oxadiazol-2-yl]propanenitrile ;
le 2-méthyl-2-[5-[(3*R*)-3-amino-5,5,7-trifluoro-2-oxo-1-[[6-[4-(trifluorométhyl)pyrazol-1-yl]-3-pyridyl]méthyl]-3,4-dihydro-1-benzazépin-8-yl]-1,3,4-oxadiazol-2-yl]propanenitrile ;
le 2-méthyl-2-[5-[(3*S*)-3-amino-5,5,7-trifluoro-1-[[3-fluoro-4-[4-(trifluorométhyl)pyrazol-1-yl]phényl]méthyl]-2-oxo-3,4-dihydro-1-benzazépin-8-yl]-1,3,4-oxadiazol-2-yl]propanenitrile ;
le 2-méthyl-2-[5-[(3*R*)-3-amino-5,5,7-trifluoro-1-[[3-fluoro-4-[4-(trifluorométhyl)pyrazol-1-yl]phényl]méthyl]-2-oxo-3,4-dihydro-1-benzazépin-8-yl]-1,3,4-oxadiazol-2-yl]propanenitrile ;
le 2-méthyl-2-[5-[(3*S*)-3-amino-5,5,7-trifluoro-2-oxo-1-[[4-[1-(trifluorométhyl)pyrazol-4-yl]phényl]méthyl]-3,4-dihydro-1-benzazépin-8-yl]-1,3,4-oxadiazol-2-yl]propanenitrile ; et
le 2-méthyl-2-[5-[(3*R*)-3-amino-5,5,7-trifluoro-2-oxo-1-[[4-[1-(trifluorométhyl)pyrazol-4-yl]phényl]méthyl]-3,4-dihydro-1-benzazépin-8-yl]-1,3,4-oxadiazol-2-yl]propanenitrile ;
ou un sel pharmaceutiquement acceptable de ceux-ci.

9. Composé selon l'une quelconque des revendications 1 à 8, choisi parmi :
le 2-méthyl-2-[5-[(3S)-3-amino-5,5,7-trifluoro-2-oxo-1-[[4-(trifluorométhoxy)phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-(trifluorométhoxy)phényl]méthyl]-3,4-dihydro-1-benzazépin-8-yl]-1,3,4-oxadiazol-2-yl]propanenitrile ;
la (3S)-3-amino-5,5,7-trifluoro-8-[5-(1,2,2,2-tétrafluoro-1-méthoxy-éthyl)-1,3,4-oxadiazol-2-yl]-1-[[4-(trifluorométhoxy)phényl]méthyl]-3,4-dihydro-1-benzazépin-2-one ;
la (3R)-3-amino-5,5,7-trifluoro-8-[5-(1,2,2,2-tétrafluoro-1-méthoxy-éthyl)-1,3,4-oxadiazol-2-yl]-1-[[4-(trifluorométhoxy)phényl]méthyl]-3,4-dihydro-1-benzazépin-2-one ;
la (3S)-3-amino-8-(5-tert-butyl-1,3,4-oxadiazol-2-yl)-1-[[4-(cyclopentoxy)phényl]méthyl]-5,5,7-trifluoro-3,4-dihydro-1-benzazépin-2-one ;
la (3R)-3-amino-8-(5-tert-butyl-1,3,4-oxadiazol-2-yl)-1-[[4-(cyclopentoxy)phényl]méthyl]-5,5,7-trifluoro-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[[4-[5-(trifluorométhyl)-2-pyridyl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[5-(trifluorométhyl)-2-pyridyl]phényl]méthyl]-3,4-dihydro-1-benzazépin-2-one ;
le 2-méthyl-2-[5-[(3S)-3-amino-5,5,7-trifluoro-1-[[4-(4-méthoxyphényl)phényl]méthyl]-2-oxo-3,4-dihydro-1-benzazépin-8-yl]-1,3,4-oxadiazol-2-yl]propanenitrile ;
le 2-méthyl-2-[5-[(3R)-3-amino-5,5,7-trifluoro-1-[[4-(4-méthoxyphényl)phényl]méthyl]-2-oxo-3,4-dihydro-1-benzazépin-8-yl]-1,3,4-oxadiazol-2-yl]propanenitrile ;
le 2-méthyl-2-[5-[(3S)-3-amino-5,5,7-trifluoro-2-oxo-1-[(4-phénoxyphényl)méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[(4-phénoxyphényl)méthyl]-3,4-dihydro-1-benzazépin-8-yl]-1,3,4-oxadiazol-2-yl]propanenitrile ;
le 2-méthyl-2-[5-[(3S)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]propanenitrile ;
la (3S)-3-amino-8-[5-(1-éthyl-5,5-difluoro-3-pipéridyl)-1,3,4-oxadiazol-2-yl]-5,5,7-trifluoro-1-[[4-(trifluorométhoxy)phényl]méthyl]-3,4-dihydro-1-benzazépin-2-one ;
la (3R)-3-amino-8-[5-(1-éthyl-5,5-difluoro-3-pipéridyl)-1,3,4-oxadiazol-2-yl]-5,5,7-trifluoro-1-[[4-(trifluorométhoxy)phényl]méthyl]-3,4-dihydro-1-benzazépin-2-one ;
la (3S)-3-amino-5,5,7-trifluoro-8-(5-pyrrolidin-1-yl-1,3,4-oxadiazol-2-yl)-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-(5-pyrrolidin-1-yl-1,3,4-oxadiazol-2-yl)-1-[[4-[5-(trifluorométhyl)-1,2,4-oxadiazol-3-yl]phényl]méthyl]-3,4-dihydro-1-benzazépin-2-one ;
la (3S)-3-amino-8-[5-(4,4-difluoro-1-pipéridyl)-1,3,4-oxadiazol-2-yl]-5,5,7-trifluoro-1-[[4-[5-(trifluorométhyl)-1,2,4-oxadiazol-3-yl]phényl]méthyl]-3,4-dihydro-1-benzazépin-2-one ;
la (3R)-3-amino-8-[5-(4,4-difluoro-1-pipéridyl)-1,3,4-oxadiazol-2-yl]-5,5,7-trifluoro-1-[[4-[5-(trifluorométhyl)-1,2,4-oxadiazol-3-yl]phényl]méthyl]-3,4-dihydro-1-benzazépin-2-one ;
la (3S)-3-amino-5,5,7-trifluoro-8-[5-(1-méthyl-1-méthylsulfonyl-éthyl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3S)-3-amino-5,5,7-trifluoro-8-[5-(4-oxa-7-azaspiro[2.5]octan-7-yl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
la (3R)-3-amino-5,5,7-trifluoro-8-[5-(4-oxa-7-azaspiro[2.5]octan-7-yl)-1,3,4-oxadiazol-2-yl]-1-[[4-[5-(trifluorométhyl)-1,2,4-oxadiazol-3-yl]phényl]méthyl]-3,4-dihydro-1-benzazépin-2-one ;
le 2-méthyl-2-[5-[(3S)-3-amino-5,5,7-trifluoro-2-oxo-1-[[4-[5-(trifluorométhyl)tétrazol-2-yl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhyl)tétrazol-2-yl]phényl]méthyl]-3,4-dihydro-1-benzazépin-8-yl]-1,3,4-oxadiazol-2-yl]propanenitrile ;
le 2-méthyl-2-[5-[(3S)-3-amino-5,5,7-trifluoro-2-oxo-1-[[4-[5-(trifluorométhoxy)-2-pyridyl]phényl]méthyl]-3,4-dihydro-1-benzazépin-8-yl]-1,3,4-oxadiazol-2-yl]propanenitrile ;
le 2-méthyl-2-[5-[(3R)-3-amino-5,5,7-trifluoro-2-oxo-1-[[4-[5-(trifluorométhoxy)-2-pyridyl]phényl]méthyl]-3,4-dihydro-1-benzazépin-8-yl]-1,3,4-oxadiazol-2-yl]propanenitrile ;
la (3S)-3-amino-5,5,7-trifluoro-8-[5-(1-méthyl-1-méthylsulfonyl-éthyl)-1,3,4-oxadiazol-2-yl]-1-[[4-[4-(trifluorométhyl)pyrazol-1-yl]phényl]méthyl]-3,4-dihydro-1-benzazépin-2-one ;
la (3R)-3-amino-5,5,7-trifluoro-8-[5-(1-méthyl-1-méthylsulfonyl-éthyl)-1,3,4-oxadiazol-2-yl]-1-[[4-[4-(trifluorométhyl)pyrazol-1-yl]phényl]méthyl]-3,4-dihydro-1-benzazépin-2-one ;
le 2-[5-[(3S)-3-amino-5,5,7-trifluoro-2-oxo-1-[[4-[4-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
le 2-[5-[(3R)-3-amino-5,5,7-trifluoro-2-oxo-1-[[4-[4-(trifluorométhyl)pyrazol-1-yl]phényl]méthyl]-3,4-dihydro-1-benzazépin-8-yl]-1,3,4-oxadiazol-2-yl]-2-méthyl-propanenitrile ;
le 1-[5-[(3S)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 1-[5-[(3R)-3-amino-5,5,7-trifluoro-2-oxo-1-[[4-[5-(trifluorométhyl)-2-pyridyl]phényl]méthyl]-3,4-dihydro-1-benzazépin-8-yl]-1,3,4-oxadiazol-2-yl]-3-azabicyclo[3.1.1]heptane-3-carboxylate de méthyle ;
le 1-[5-[(3S)-3-amino-1-[(4-chlorophényl)méthyl]-5,5,7-trifluoro-2-oxo-3,4-dihydro-1-benzazépin-8-yl]-1,3,4-oxadiazol-2-yl]-3-azabicyclo[3.1.1]heptane-3-carboxylate de méthyle ;
le 1-[5-[(3R)-3-amino-1-[(4-chlorophényl)méthyl]-5,5,7-trifluoro-2-oxo-3,4-dihydro-1-benzazépin-8-yl]-1,3,4-oxadiazol-2-yl]-3-azabicyclo[3.1.1]heptane-3-carboxylate de méthyle ;
le 2-[5-[(3S)-3-amino-1-[[4-[5-(1,1-difluoroéthyl)-2-pyridyl]phényl]méthyl]-5,5,7-trifluoro-2-oxo-3,4-dihydro-1-benzazépin-8-yl]-1,3,4-oxadiazol-2-yl]-2-méthyl-propanenitrile ;
le 2-[5-[(3R)-3-amino-1-[[4-[5-(1,1-difluoroéthyl)-2-pyridyl]phényl]méthyl]-5,5,7-trifluoro-2-oxo-3,4-dihydro-1-benzazépin-8-yl]-1,3,4-oxadiazol-2-yl]-2-méthyl-propanenitrile ;
le 2-méthyl-2-[5-[(3S)-3-amino-1-[[4-[5-(difluorométhoxy)-2-pyridyl]phényl]méthyl]-5,5,7-trifluoro-2-oxo-3,4-dihydro-1-benzazépin-8-yl]-1,3,4-oxadiazol-2-yl]propanenitrile ; et
le 2-méthyl-2-[5-[(3R)-3-amino-1-[[4-[5-(difluorométhoxy)-2-pyridyl]phényl]méthyl]-5,5,7-trifluoro-2-oxo-3,4-dihydro-1-benzazépin-8-yl]-1,3,4-oxadiazol-2-yl]propanenitrile ;
ou un sel pharmaceutiquement acceptable de ceux-ci.

10. Composé selon l'une quelconque des revendications 1 à 9, ou un sel pharmaceutiquement acceptable de celui-ci, pour une utilisation comme substance thérapeutiquement active.

11. Composition pharmaceutique comprenant un composé selon l'une quelconque des revendications 1 à 9, ou un sel pharmaceutiquement acceptable de celui-ci, et un excipient pharmaceutiquement acceptable.

12. Composition pharmaceutique selon la revendication 11, comprenant en outre un agent thérapeutique supplémentaire.

13. Composé selon l'une quelconque des revendications 1 à 9, ou un sel pharmaceutiquement acceptable de celui-ci, pour une utilisation dans le traitement, la prévention et/ou le ralentissement de la progression d'un cancer.

14. Composé pour une utilisation selon la revendication 13, dans lequel le cancer est associé à une signalisation aberrante de la diacylglycérol kinase, dans lequel la diacylglycérol kinase est choisie parmi DGKα et/ou DGKζ.

15. Composé pour une utilisation selon la revendication 13 ou 14, dans lequel le cancer est choisi dans le groupe constitué par une leucémie lymphoïde aiguë à cellules B, une leucémie lymphoïde aiguë à cellules T, une leucémie lymphoïde aiguë, une leucémie myéloïde chronique, une leucémie lymphocytaire chronique, une leucémie prolymphocytaire à cellules B, un néoplasme à cellules dendritiques plasmacytoïdes blastiques, un lymphome de Burkitt, un lymphome diffus à grandes cellules B, un lymphome folliculaire, une leucémie à tricholeucocytes, un lymphome folliculaire à petites cellules ou à grandes cellules, des affections lymphoprolifératives malignes, un lymphome de MALT, un lymphome à cellules du manteau, un lymphome de la zone marginale, un myélome multiple, une myélodysplasie et un syndrome myélodysplasique, un lymphome non hodgkinien, un lymphome plasmablastique, un néoplasme à cellules dendritiques plasmacytoïdes, une macroglobulinémie de Waldenström, une préleucémie, un sarcome, un carcinome, un mélanome, un neuroblastome, un carcinome à cellules rénales, un cancer du côlon, un cancer colorectal, un cancer du sein, un cancer épidermoïde à cellules squameuses, un mélanome, un cancer de l'estomac, un cancer du cerveau, un cancer du poumon (par exemple un CPNPC), un cancer du pancréas, un cancer du col de l'utérus, un cancer de l'ovaire, un cancer du foie, un cancer de la vessie, un cancer de la prostate, un cancer des testicules, un cancer de la thyroïde, un cancer de l'utérus, un cancer surrénalien et un cancer de la tête et du cou.
